(19) Europäisches Patentamt
European Patent Office
Office européen des brevets

(11) EP 3 373 902 B1

(12) EUROPEAN PATENT SPECIFICATION

(45) Date of publication and mention of the grant of the patent:
**29.04.2020 Bulletin 2020/18**

(21) Application number: **16794652.4**

(22) Date of filing: **14.11.2016**

(51) Int Cl.:
***A61K 8/49*** (2006.01) ***A61Q 5/06*** (2006.01)
***A61K 8/41*** (2006.01) ***A61K 8/46*** (2006.01)

(86) International application number:
**PCT/EP2016/077555**

(87) International publication number:
**WO 2017/081314 (18.05.2017 Gazette 2017/20)**

(54) **CATIONIC DIRECT DYE COMPRISING AN ALIPHATIC CHAIN AND BEARING A DISULFIDE/THIOL/PROTECTED-THIOL FUNCTION FOR DYEING KERATIN FIBRES**

KATIONISCHER DIREKTFARBSTOFF MIT EINER ALIPHATISCHEN KETTE UND MIT EINER DISULFID/THIOL/GESCHÜTZTES-THIOL-FUNKTION ZUR FÄRBUNG VON KERATINFASERN

COLORANT DIRECT CATIONIQUE COMPRENANT UNE CHAÎNE ALIPHATIQUE ET PORTANT UNE FONCTION DE DISULFURE/THIOL/THIOL PROTÉGÉ POUR COLORER DES FIBRES DE KÉRATINE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.11.2015 FR 1560806**

(43) Date of publication of application:
**19.09.2018 Bulletin 2018/38**

(73) Proprietor: **L'Oréal**
**92110 Clichy (FR)**

(72) Inventors:
- **BLAISE, Christian**
  **93601 Aulnay-sous-Bois (FR)**
- **BURCKBUCHLER, Virginie**
  **93601 Aulnay-sous-Bois (FR)**

(74) Representative: **L'Oreal**
**Service D.I.P.I.**
**9, rue Pierre Dreyfus**
**92110 Clichy (FR)**

(56) References cited:
**EP-A1- 1 647 580**
**WO-A2-2008/019977**
**FR-A1- 2 921 256**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 373 902 B1

## Description

A subject of the invention is a process for dyeing keratin fibres such as the hair, using at least one cationic direct dye bearing a disulfide, thiol or protected-thiol function and comprising at least one ammonium group bearing an aliphatic chain **(I).** Another subject of the invention is the use i) of at least one cationic direct dye bearing a disulfide, thiol or protected-thiol function **(I)** combined with ii) steam and/or a heating iron for straightening keratin fibres, for dyeing keratin fibres, and novel cationic direct dyes bearing a disulfide, thiol or protected-thiol function **(I),** and also a cosmetic composition comprising same.

It is known to dye keratin fibres by direct dyeing or semi-permanent dyeing. Direct dyeing or semi-permanent dyeing consists in introducing the colour via a coloured molecule which becomes adsorbed at the surface of the individual hair or which penetrates into the individual hair. Thus, the process conventionally used in direct dyeing consists in applying to keratin fibres direct dyes, which are coloured and colouring molecules that have affinity for the fibres, leaving the fibres in contact with the colouring molecules and then rinsing the fibres. Generally, this technique leads to chromatic colourings.

Scientific research has been conducted for several years to modify the colour of keratin materials, in particular keratin fibres, and in particular to mask white fibres, to modify the colour of the fibres permanently or temporarily, and to satisfy new desires and needs in terms of colours and durability.

Patent applications EP 1 647 580, WO 2005/097 051, EP 2 004 759, EP 2 075 289, WO 2007/110 541, WO 2007/110 540, WO 2007/110 539, WO 2007/110 538, WO 2007/110 537, WO 2007/110 536, WO 2007/110 535, WO 2007/110 534, WO 2007/110 533, WO 2007/110 532, WO 2007/110 531, EP 2 070 988, WO 2009/040 354 and WO 2009/034 059 disclose direct dyes bearing a disulfide, thiol or protected-thiol function, which may be grafted onto the hair using a reductive treatment. As it happens, the majority of reducing agents have a tendency to impair the integrity of keratin fibres, having the effect of making them brittle. In addition, when combined with dyes bearing a disulfide function, reducing agents generally generate unpleasant odours.
Moreover, the colourations obtained do not always give the keratin fibre a quality of softness and easy untangling, or the associated treatments are not always long-lasting.

The aim of the present invention is to provide novel hair dyeing systems for obtaining colourations which are fast with respect to external agents, uniform and very strong, and/or which do not impair the cosmetic properties of keratin fibres, or even which give said fibres a care valency, this being even without the use of a reducing agent and/or of a chemical oxidizing agent.

These aims are achieved with the present invention, the first subject of which is a process for dyeing keratin fibres, in particular human keratin fibres, such as the hair, consisting in applying to the fibres at least one cationic direct dye bearing a disulfide, thiol or protected-thiol function of formula **(I):**

$$\mathbf{A - (X)_p - C_{sat} - S - U} \qquad \mathbf{(I)}$$

salts thereof with an organic or mineral acid, optical or geometric isomers thereof, tautomers thereof, and solvates thereof such as the hydrates,
in which formula **(I):**

- **U** represents a radical chosen from: *a)* $\mathbf{- S - C'_{sat} - (X')_{p'} - A'}$; and *b)* - **Y**;
- **A** and **A',** which may be identical or different, represent, a cationic or non-cationic, chromophore, preferably a cationic chromophore, it being understood that at least one of the two chromophores bears at least one linear or branched, saturated or unsaturated $C_{10}$-$C_{30}$ aliphatic chain;
- **Y** represents i) a hydrogen atom; or ii) a thiol-function protecting group;
- **X** and **X',** which may be identical or different, represent a linear or branched, saturated or unsaturated divalent $C_1$-$C_{30}$ hydrocarbon-based chain, optionally interrupted and/or optionally terminated at one or both of its ends with one or more divalent groups or combinations thereof chosen from:

    - ➢ -N(R)-, $-N^+(R)(R)-$, -O-, -S-, -CO-, $-SO_2-$ with R, which may be identical or different, chosen from a hydrogen and a $C_1$-$C_4$ alkyl, hydroxyalkyl or aminoalkyl radical;
    - ➢ an aromatic or non-aromatic, saturated or unsaturated, fused or non-fused (hetero)cyclic radical optionally comprising one or more identical or different, optionally substituted heteroatoms;

- **p** and **p'**, which may be identical or different, are equal to 0 or 1;
- $\mathbf{C_{sat}}$ et $\mathbf{C'_{sat}}$, which may be identical or different, represent an optionally cyclic, optionally substituted linear or branched $C_1$-$C_{18}$ alkylene chain.

Another subject of the invention is the use, for dyeing keratin fibres, in particular the hair, of at least one cationic direct dye of formula (I) as defined above, preferably combined with a treatment with steam and/or an iron for straightening the keratin fibres. Another subject of the invention is a dye composition comprising at least one disulfide, thiol or protected-thiol dye of formula **(I)** as defined above, and a compound of formula (I) as defined above.

The dyeing process of the invention in particular makes it possible to dye human keratin fibres such as the hair in a uniform, persistent, odourless manner, while providing care to said fibres. Furthermore, the dyeing and care provided to the keratin fibres are long-lasting. In addition, the keratin fibres are protected against heat treatments at a temperature greater than or equal to 80°C provided, for example, using steam in particular originating from a steam iron.

In particular, the combination of the heat of the straightening iron combined with the application of a disulfide cationic derivative bearing a fatty chain in solution in a hydrophilic or hydrophobic medium gives the fibre a uniform colouration and persistent long-lasting care while at the same time respecting the integrity of the fibre (smooth feel, silky sheen appearance, facilitated untangling when dry and in a wet medium, improved manageability). The colourations obtained are thus aesthetic, very vivid and very fast with respect to common attacking factors such as sunlight, perspiration, sebum and other hair treatments such as successive shampooing, while at the same time respecting the keratin fibres. The vividness obtained is particularly noteworthy. The same is true for the uniformity of the colour and of the care given to the keratin fibres, this being even after heat treatment given to the keratin fibres.

For the purposes of the present invention and unless otherwise indicated:

- a *"cationic direct dye bearing a disulfide function"* is a direct dye comprising one or more cationic chromophores that absorb light in the visible spectrum, and comprising a disulfide bond: -S-S- between two carbon atoms and which is indirectly bonded to the chromophore(s) of the dye, i.e. between the chromophores and the -S-S- function there is at least one methylene group;
- a *"direct dye bearing a protected-thiol function"* is a direct dye comprising a chromophore, comprising a protected-thiol function -SY in which Y is a protective group known to those skilled in the art, for instance those described in the publications "Protective Groups in Organic Synthesis", T.W. Greene, John Wiley & Sons ed., NY, 1981, pp. 193-217; "Protecting Groups", P. Kocienski, Thieme, 3rd ed., 2005, chap. 5; and Ullmann's Encyclopedia, "Peptide Synthesis", pp. 4-5, 2005 Wiley-VCH Verlag GmbH & Co. KGaA, Weinheim 10.1002/14356007.a19 157; it being understood that said protected-thiol function is indirectly bonded to the chromophore of the dye, i.e. between the chromophore and the function -SY there is at least one methylene group;
- a *"direct dye bearing a thiol function"* is a direct dye comprising a chromophore, and comprising a thiol function -SY' in which Y' is i) a hydrogen atom; ii) an alkali metal; iii) an alkaline-earth metal; iv) an ammonium group: $N^+R^\alpha R^\beta R^\gamma R^\delta$ or a phosphonium group: $P^+R^\alpha R^\beta R^\gamma R^\delta$ with $R^\alpha$, $R^\beta$, $R^\gamma$ and $R^\delta$, which may be identical or different, representing a hydrogen atom or a $(C_1\text{-}C_4)$alkyl group, preferentially comprising a thiol function -SH, it being understood that said thiol function is indirectly bonded to the chromophore of the dye, i.e. between the chromophore and the function -SY' there is at least one methylene group;
- A *"chromophore"* is a radical derived from a dye, i.e. a radical derived from a molecule that absorbs light in the visible range of radiation that is visually and physiologically noticeable by those skilled in the art, *i.e.* at an absorption wavelength $\lambda_{abs}$ of between 400 and 800 nm inclusive;
- a *"chromophore"* is said to be *"cationic"* if it comprises at least one cationic aryl or heteroaryl group as defined below;
- the dyes according to the invention contain one or more chromophores, and these dyes are capable of absorbing light at a wavelength $\lambda_{abs}$ particularly of between 400 and 700 nm inclusive;
- chromophores are said to be *"different"* when they differ in their chemical structure and may be chromophores derived from different families or from the same family on condition that they have different chemical structures: for example, the chromophores may be chosen from the family of azo dyes but differ in the chemical structure of the radicals constituting them or in the respective position of these radicals;
- an *"alkylene chain"* represents a divalent $C_1$-$C_{20}$, particularly $C_1$-$C_6$ and more particularly $C_1$-$C_2$ chain when the chain is linear; optionally substituted with one or more identical or different groups chosen from hydroxyl, $(C_1\text{-}C_2)$alkoxy, (poly)hydroxy$(C_2\text{-}C_4)$alkoxy, (di)$(C_1\text{-}C_2)$ (alkyl)amino, $R^a\text{-}Z^a\text{-}C(Z^b)\text{-}$, and $R^a\text{-}Z^a\text{-}S(O)_t\text{-}$with $Z^a$, $Z^b$, which may be identical or different, representing an oxygen or sulfur atom, or a group $NR^{a'}$, $R^a$ representing an alkali metal, a hydrogen atom or an alkyl group, or alternatively is absent if another part of the molecule is cationic, and $R^{a'}$ representing a hydrogen atom or an alkyl group and t is equal to 1 or 2;
- a *"saturated or unsaturated, optionally substituted $C_1$-$C_{30}$ divalent hydrocarbon-based chain"* represents a hydrocarbon-based chain, which is in particular a $C_1$-$C_8$ hydrocarbon-based chain, optionally comprising one or more conjugated or unconjugated $\pi$ double bonds, the hydrocarbon-based chain being in particular saturated; said chain is optionally substituted with one or more identical or different groups chosen from hydroxyl, $(C_1\text{-}C_2)$alkoxy, (poly)hydroxy$(C_2\text{-}C_4)$alkoxy, (di)$(C_1\text{-}C_2)$ (alkyl)amino, $R^a\text{-}Z^a\text{-}C(Z^b)\text{-}$, and $R^a\text{-}Z^a\text{-}S(O)_t\text{-}$ with $Z^a$, $Z^b$, which may be identical or different, representing an oxygen or sulfur atom, or a group $NR^{a'}$, $R^a$ representing an alkali metal, a hydrogen atom or an alkyl group, or alternatively is absent if another part of the molecule is cationic, and $R^{a'}$ representing a

hydrogen atom or an alkyl group and t is equal to 1 or 2;

▪ the *"aryl"* or *"heteroaryl"* radicals or the aryl or heteroaryl part of a radical may be substituted with at least one substituent borne by a carbon atom, chosen from:

- a $C_1$-$C_{16}$ and preferably $C_1$-$C_8$ alkyl radical optionally substituted with one or more radicals chosen from hydroxyl, $C_1$-$C_2$ alkoxy, $C_2$-$C_4$ (poly)hydroxyalkoxy, acylamino, amino substituted with two $C_1$-$C_4$ alkyl radicals, which may be identical or different, optionally bearing at least one hydroxyl group, or the two radicals possibly forming, with the nitrogen atom to which they are attached, a saturated or unsaturated, optionally substituted 5- to 7-membered and preferably 5- or 6-membered heterocycle optionally comprising another nitrogen or non-nitrogen heteroatom;
- a halogen atom;
- a hydroxyl group;
- a $C_1$-$C_2$ alkoxy radical;
- a $C_2$-$C_4$ (poly)hydroxyalkoxy radical;
- an amino radical;
- a 5- or 6-membered heterocycloalkyl radical;
- an optionally cationic 5- or 6-membered heteroaryl radical, preferentially imidazolium, optionally substituted with a ($C_1$-$C_4$)alkyl radical, preferentially methyl;
- an amino radical substituted with one or two identical or different $C_1$-$C_6$ alkyl radicals, optionally bearing at least:

  i) a hydroxyl group,
  ii) an amino group optionally substituted with one or two optionally substituted $C_1$-$C_3$ alkyl radicals, said alkyl radicals possibly forming with the nitrogen atom to which they are attached a saturated or unsaturated, optionally substituted 5- to 7-membered heterocycle, optionally comprising at least one other nitrogen or non-nitrogen heteroatom,
  iii) a quaternary ammonium group -N+R'R"R''', $M^-$ for which R', R" and R''', which may be identical or different, represent a hydrogen atom or a $C_1$-$C_4$ alkyl group; and $M^-$ represents the counterion of the organic or mineral acid or of the corresponding halide;
  iv) or an optionally cationic 5- or 6-membered heteroaryl radical, preferentially imidazolium, optionally substituted with a ($C_1$-$C_4$)alkyl radical, preferentially methyl;

- an acylamino radical (-NR-C(O)-R') in which the radical R is a hydrogen atom, a $C_1$-$C_4$ alkyl radical optionally bearing at least one hydroxyl group and the radical R' is a $C_1$-$C_2$ alkyl radical; a carbamoyl radical ($(R)_2$N-C(O)-) in which the radicals R, which may be identical or different, represent a hydrogen atom, a $C_1$-$C_4$ alkyl radical optionally bearing at least one hydroxyl group; an alkylsulfonylamino radical (R'-$S(O)_2$-N(R)-) in which the radical R represents a hydrogen atom, a $C_1$-$C_4$ alkyl radical optionally bearing at least one hydroxyl group and the radical R' represents a $C_1$-$C_4$ alkyl radical or a phenyl radical; an aminosulfonyl radical ($(R)_2$N-$S(O)_2$-) in which the radicals R, which may be identical or different, represent a hydrogen atom or a $C_1$-$C_4$ alkyl radical optionally bearing at least one hydroxyl group,
- a carboxyl radical in the acid or salified form (preferably salified with an alkali metal or a substituted or unsubstituted ammonium);
- a cyano group;
- a nitro or nitroso group;
- a polyhaloalkyl group, preferentially the trifluoromethyl group;

the cyclic or heterocyclic part of a non-aromatic radical may be substituted by at least one substituent chosen from the following groups:

- hydroxyl;
- $C_1$-$C_4$ alkoxy or $C_2$-$C_4$ (poly)hydroxyalkoxy;
- $C_1$-$C_4$ alkyl;
- alkylcarbonylamino (R-C(O)-NR'-) in which the radical R' is a hydrogen atom or a $C_1$-$C_4$ alkyl radical optionally bearing at least one hydroxyl group, and the radical R is a $C_1$-$C_2$ alkyl radical or an amino radical optionally substituted with one or two $C_1$-$C_4$ alkyl groups, which may be identical or different, optionally bearing at least one hydroxyl group, said alkyl radicals possibly forming, with the nitrogen atom to which they are attached a saturated or unsaturated, optionally substituted 5- to 7-membered heterocycle optionally comprising at least one other nitrogen or non-nitrogen heteroatom;
- alkylcarbonyloxy (R-C(O)-O-) in which the radical R is a $C_1$-$C_4$ alkyl radical or an amino group optionally substituted with one or two identical or different $C_1$-$C_4$ alkyl groups optionally bearing at least one hydroxyl group, said alkyl radicals possibly forming with the nitrogen atom to which they are attached a saturated or unsaturated, optionally substituted 5- to 7-membered heterocycle, optionally comprising at least one other nitrogen or non-nitrogen heteroatom;

- alkoxycarbonyl (R-G-C(O)-) in which the radical R is a $C_1$-$C_4$ alkoxy radical, G is an oxygen atom or an amino group optionally substituted with a $C_1$-$C_4$ alkyl group optionally bearing at least one hydroxyl group, said alkyl radical possibly forming with the nitrogen atom to which they are attached a saturated or unsaturated, optionally substituted 5- to 7-membered heterocycle, optionally comprising at least one other nitrogen or non-nitrogen heteroatom;

▪ a cyclic or heterocyclic radical, or a non-aromatic part of an aryl or heteroaryl radical, which may also be substituted with one or more oxo groups;

▪ a hydrocarbon-based chain is unsaturated when it comprises one or more double bonds and/or one or more triple bonds;

▪ an "*aryl*" radical represents a monocyclic or fused or non-fused polycyclic carbon-based group containing from 6 to 22 carbon atoms, at least one ring of which is aromatic; preferentially, the aryl radical is a phenyl, biphenyl, naphthyl, indenyl, anthracenyl or tetrahydronaphthyl;

▪ a "*heteroaryl radical*" represents an optionally cationic, 5- to 22-membered, monocyclic or fused or non-fused polycyclic group, comprising from 1 to 6 heteroatoms chosen from nitrogen, oxygen, sulfur and selenium, at least one ring of which is aromatic; preferentially, a heteroaryl radical is chosen from acridinyl, benzimidazolyl, benzobistriazolyl, benzopyrazolyl, benzopyridazinyl, benzoquinolyl, benzothiazolyl, benzotriazolyl, benzoxazolyl, pyridinyl, tetrazolyl, dihydrothiazolyl, imidazopyridyl, imidazolyl, indolyl, isoquinolyl, naphthoimidazolyl, naphthoxazolyl, naphthopyrazolyl, oxadiazolyl, oxazolyl, oxazolopyridyl, phenazinyl, phenoxazolyl, pyrazinyl, pyrazolyl, pyrilyl, pyrazoyltriazyl, pyridyl, pyridinoimidazolyl, pyrrolyl, quinolyl, tetrazolyl, thiadiazolyl, thiazolyl, thiazolopyridinyl, thiazoylimidazolyl, thiopyrylyl, triazolyl, xanthyl and the ammonium salt thereof;

▪ a "*cyclic radical*" is a non-aromatic, monocyclic or polycyclic, fused or non-fused cycloalkyl radical, containing from 5 to 22 carbon atoms, which may comprise one or more unsaturations;

▪ a "*heterocyclic radical*" is a fused or non-fused, 5- to 22-membered monocyclic or polycyclic non-aromatic radical which may contain one or two unsaturations and which comprises from 1 to 6 heteroatoms chosen from nitrogen, oxygen, sulfur and selenium atoms;

▪ a "*heterocycloalkyl radical*" is a saturated heterocyclic radical;

▪ a "*cationic heteroaryl radical*" is a heteroaryl group as defined above, which comprises an endocyclic or exocyclic cationic group,

◦ when the charge is endocyclic, it is included in the electron delocalization via the mesomeric effect; for example, it is a pyridinium, imidazolium or indolinium group:

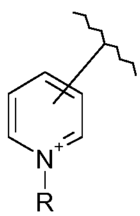 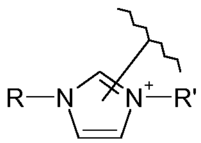 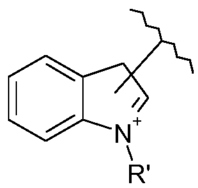

with R and R’ being a heteroaryl substituent as defined above and particularly a (hydroxy)($C_1$-$C_8$)alkyl group such as methyl;

◦ when the charge is exocyclic, it is not included in the electron delocalization via the mesomeric effect; for example, it is an ammonium or phosphonium $R^+$ substituent, such as trimethylammonium, which is outside the heteroaryl, such as pyridyl, indolyl, imidazolyl or naphthalimidyl, in question:

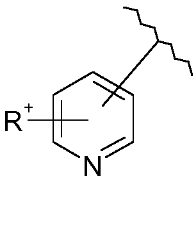 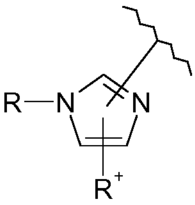 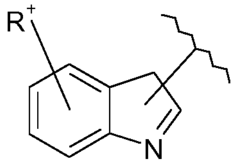 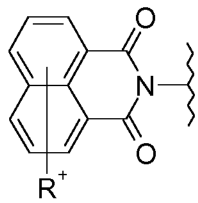 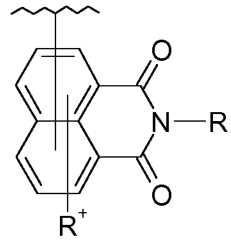

with R a heteroaryl substituent as defined above and $R^+$ an ammonium $R_aR_bR_cN^+$-, phosphonium $R_aR_bR_cP^+$- or ammonium $R_aR_bR_cN^+$-($C_1$-$C_6$)alkylamino group with $R_a$, $R_b$ and $R_c$, which may be identical or different, representing a hydrogen atom or a ($C_1$-$C_8$)alkyl group such as methyl;

- a *"cationic aryl bearing an exocyclic charge"* is intended to mean an aryl ring whose cationic group is outside said ring; it is in particular an ammonium or phosphonium R+ substituent such as trimethylammonium outside the aryl such as phenyl or naphthyl:

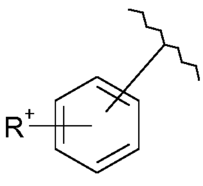 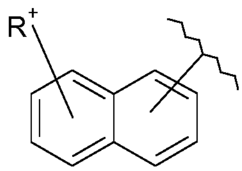

;

- an *"alkyl radical"* is a linear or branched $C_1$-$C_{20}$ and preferably $C_1$-$C_8$ hydrocarbon-based radical;
- an *"alkenylene radical"* is an alkyl radical as defined above, which may contain from 1 to 4 conjugated or unconjugated double bonds -C=C-; the alkenylene group particularly contains 1 or 2 unsaturations;
- the term "optionally substituted" applied to the alkyl radical implies that said alkyl radical may be substituted with one or more radicals chosen from the following radicals: i) hydroxyl, ii) $C_1$-$C_4$ alkoxy, iii) acylamino, iv) amino optionally substituted with one or two identical or different $C_1$-$C_4$ alkyl radicals, said alkyl radicals possibly forming with the nitrogen atom that bears them a 5- to 7-membered heterocycle optionally comprising another nitrogen or non-nitrogen heteroatom; v) or a quaternary ammonium group $-N^+R'R''R'''$, $M^-$ for which R', R" and R''', which may be identical or different, represent a hydrogen atom or a $C_1$-$C_4$ alkyl group, or alternatively $-N^+R'R''R'''$ forms a heteroaryl such as imidazolium optionally substituted with a $C_1$-$C_4$ alkyl group, and $M^-$ represents the counterion of the organic or mineral acid or of the corresponding halide;
- an *"alkoxy radical"* is an alkyl-oxy radical for which the alkyl radical is a linear or branched $C_1$-$C_{16}$ and preferentially $C_1$-$C_8$ hydrocarbon-based radical;
- when the alkoxy group is optionally substituted, this implies that the alkyl group is optionally substituted as defined hereinabove;;
- the term "at least one" is intended to mean "one or more".

*1.1. Direct dyes bearing a disulfide or thiol function of the invention:*

The direct dye(s) bearing a disulfide, thiol or protected-thiol function used in the invention are of formula **(I)** as defined above.

One particular mode of the invention relates to the dyes bearing a disulfide function of formula **(I)** as defined above, i.e. for which **U** represents the following radical *a)* $\mathbf{-S-C'_{sat}-(X')_{p'}-A'}$, preferentially the disulfide dye(s) of the invention are symmetrical, i.e. comprise an axis of symmetry $C_2$, in other words, A = A', $C_{sat}$ = $C'_{sat}$, X = X' and p = p'.

Another particular mode of the invention relates to the dyes of formula **(I)** bearing a thiol function as defined above, i.e. **U** representing the radical *b)* **Y.**

*1.1.1.* ***Y*:**

According to one particular embodiment of the invention, the cationic direct dye of formula **(I)** is a thiol dye, i.e. Y represents i) a hydrogen atom.

In accordance with another particular embodiment of the invention, in the abovementioned formula **(I), Y** is a protective group known to those skilled in the art, for instance those described in the publications "Protective Groups in

Organic Synthesis", T. W. Greene, published by John Wiley & Sons, NY, 1981, pp. 193-217; "Protecting Groups", P. Kocienski, Thieme, 3rd edition, 2005, chapter 5, and Ullmann's Encyclopedia, "Peptide Synthesis", pp. 4-5, 2005 Wiley-VCH Verlag GmbH & Co. KGaA, Weinheim 10.1002/14356007.a19 157.

In particular, **Y** represents a thiol-function protecting group chosen from the following radicals:

- $(C_1-C_4)$alkylcarbonyl;
- $(C_1-C_4)$alkylthiocarbonyl;
- $(C_1-C_4)$alkoxycarbonyl;
- $(C_1-C_4)$alkoxythiocarbonyle;
- $(C_1-C_4)$alkylthiothiocarbonyl;
- (di) $(C_1-C_4)$ (alkyl)aminocarbonyl;
- (di) $(C_1-C_4)$ (alkyl)aminothiocarbonyl;
- arylcarbonyl such as phenylcarbonyl;
- aryloxycarbonyl;
- aryl$(C_1-C_4)$alkcoxycarbonyl;
- (di) $(C_1-C_4)$ (alkyl)aminocarbonyl such as dimethylaminocarbonyl;
- $(C_1-C_4)$(alkyl)arylaminocarbonyl;
- carboxyl;
- $SO_3^-$; $M^+$ with $M^+$ representing an alkali metal such as sodium or potassium, or alternatively a counterion of the cationic chromophore **A** and $M^+$ are absent;
- optionally substituted aryl such as phenyl, dibenzosuberyl or 1,3,5-cycloheptatrienyl;
- optionally substituted heteroaryl; in particular including the following cationic or non-cationic heteroaryl radicals comprising from 1 to 4 heteroatoms, chosen from i), ii) and iii):

  i) 5-, 6- or 7-membered monocyclic radicals such as furanyl or furyl, pyrrolyl or pyrryl, thiophenyl or thienyl, pyrazolyl, oxazolyl, oxazolium, isoxazolyl, isoxazolium, thiazolyl, thiazolium, isothiazolyl, isothiazolium, 1,2,4-triazolyl, 1,2,4-triazolium, 1,2,3-triazolyl, 1,2,3-triazolium, 1,2,4-oxazolyl, 1,2,4-oxazolium, 1,2,4-thiadiazolyl, 1,2,4-thiadiazolium, pyrylium, thiopyridyl, pyridinium, pyrimidinyl, pyrimidinium, pyrazinyl, pyrazinium, pyridazinyl, pyridazinium, triazinyl, triazinium, tetrazinyl, tetrazinium, azepine, azepinium, oxazepinyl, oxazepinium, thiepinyl, thiepinium, imidazolyl, imidazolium;

  ii) 8- to 11-membered bicyclic radicals such as indolyl, indolinium, benzimidazolyl, benzimidazolium, benzoxazolyl, benzoxazolium, dihydrobenzoxazolinyl, benzothiazolyl, benzothiazolium, pyridoimidazolyl, pyridoimidazolium, thienocycloheptadienyl, these monocyclic or bicyclic groups being optionally substituted with one or more groups such as $(C_1-C_4)$alkyl, for instance methyl, or polyhalo$(C_1-C_4)$alkyl, for instance trifluoromethyl;

  iii) the following tricyclic <u>ABC</u> radical:

  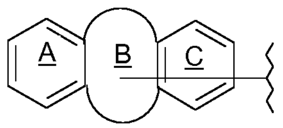

  in which the two rings <u>A</u> and <u>C</u> optionally comprise a heteroatom, and ring <u>B</u> is a 5-, 6- or 7-membered ring, particularly a 6-membered ring, and contains at least one heteroatom, for instance piperidyl or pyranyl;

- optionally cationic, optionally substituted heterocycloalkyl, the heterocycloalkyl group in particular represents a saturated or partially saturated 5-, 6- or 7-membered monocyclic group comprising from 1 to 4 heteroatoms chosen from oxygen, sulfur and nitrogen, such as di/tetrahydrofuranyl, di/tetrahydrothiophenyl, di/tetrahydropyrrolyl, di/tetrahydropyranyl, di/tetra/hexahydrothiopyranyl, dihydropyridyl, piperazinyl, piperidinyl, tetramethylpiperidyl, morpholinyl, di/tetra/hexahydroazepinyl, di/tetrahydropyrimidinyl, these groups being optionally substituted with one or more groups such as $(C_1-C_4)$ alkyl, oxo or thioxo; or the heterocycle represents the following group:

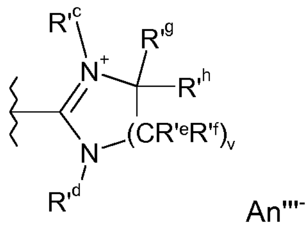

in which R'$^c$, R'$^d$, R'$^e$, R'$^f$, R'$^g$ and R'$^h$, which may be identical or different, represent a hydrogen atom or a ($C_1$-$C_4$) alkyl group, or alternatively two groups R'$^g$ with R'$^h$, and/or R'$^e$ with R'$^f$, form an oxo or thioxo group, or alternatively R'$^g$ with R'$^e$ together form a cycloalkyl; and v represents an integer between 1 and 3 inclusive; preferentially, R'$^c$ to R'$^h$ represent a hydrogen atom; and An'''$^-$ represents a counterion;

- -C(NR'$^c$R'$^d$)=N$^+$R'$^e$R'$^f$; An'''$^-$ with R'$^c$, R'$^d$, R'$^e$ and R'$^f$, which may be identical or different, representing a hydrogen atom or a ($C_1$-$C_4$)alkyl group; preferentially, R'$^c$ to R'$^f$ represent a hydrogen atom; and An'''$^-$ represents a counterion;
- -C(NR'$^c$R'$^d$)=NR'$^e$; with R'$^c$, R'$^d$ and R'$^e$ as defined above;
- optionally substituted (di)aryl($C_1$-$C_4$)alkyl such as 9-anthracenylmethyl, phenylmethyl or diphenylmethyl optionally substituted with one or more groups in particular chosen from ($C_1$-$C_4$) alkyl, ($C_1$-$C_4$) alkoxy such as methoxy, hydroxyl, alkylcarbonyl or (di)($C_1$-$C_4$)(alkyl)amino such as dimethylamino;
- optionally substituted (di)heteroaryl($C_1$-$C_4$)alkyl, the heteroaryl group in particular being a cationic or noncationic, 5- or 6-membered monocyclic radical comprising from 1 to 4 heteroatoms chosen from nitrogen, oxygen and sulfur, such as pyrrolyl, furanyl, thiophenyl, pyridyl, pyridyl N-oxide groups such as 4-pyridyl or 2-pyridyl-N-oxide, pyrylium, pyridinium or triazinyl, optionally substituted with one or more groups such as alkyl, particularly methyl; advantageously, the (di)heteroaryl($C_1$-$C_4$)alkyl is (di)heteroarylmethyl or (di)heteroarylethyl;
- $CR^1R^2R^3$ with $R^1$, $R^2$ and $R^3$, which may be identical or different, representing a halogen atom or a group chosen from:

  - ($C_1$-$C_4$)alkyl;
  - ($C_1$-$C_4$)alkoxy;
  - optionally substituted aryl such as phenyl optionally substituted with one or more groups, for instance ($C_1$-$C_4$)alkyl, ($C_1$-$C_4$)alkoxy or hydroxyl;
  - optionally substituted heteroaryl such as thiophenyl, furanyl, pyrrolyl, pyranyl or pyridyl, optionally substituted with a ($C_1$-$C_4$)alkyl group;
  - P($Z^1$)R'$^1$R'$^2$R'$^3$ with R'$^1$ and R'$^2$, which may be identical or different, representing a hydroxyl, ($C_1$-$C_4$)alkoxy or alkyl group, R'$^3$ representing a hydroxyl or ($C_1$-$C_4$)alkoxy group, and $Z^1$ representing an oxygen or sulfur atom;

- a sterically hindered ring; and
- optionally substituted alkoxyalkyl, such as methoxymethyl (MOM), ethoxyethyl (EOM) and isobutoxymethyl.

According to one particular embodiment, the thiol-protected dyes of formula **(I)** comprise a group **Y** chosen from i) aromatic cationic 5- or 6-membered monocyclic heteroaryl comprising from 1 to 4 heteroatoms chosen from oxygen, sulfur and nitrogen, such as oxazolium, isoxazolium, thiazolium, isothiazolium, 1,2,4-triazolium, 1,2,3-triazolium, 1,2,4-oxazolium, 1,2,4-thiadiazolium, pyrylium, pyridinium, pyrimidinium, pyrazinyl, pyrazinium, pyridazinium, triazinium, tetrazinium, oxazepinium, thiepinyl, thiepinium, imidazolium; ii) cationic 8- to 11-membered bicyclic heteroaryl such as indolinium, benzimidazolium, benzoxazolium, benzothiazolium, these monocyclic or bicyclic heteroaryl groups optionally being substituted with one or more groups such as alkyl, for instance methyl, or polyhalo($C_1$-$C_4$)alkyl such as trifluoromethyl; iii) or the following heterocyclic:

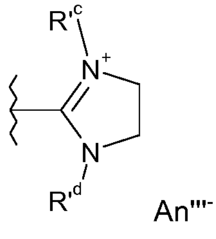

in which R'$^c$ and R'$^d$, which may be identical or different, represent a hydrogen atom or a group ($C_1$-$C_4$)alkyl; preferentially

R'$^c$ to R'$^d$ represent a group ($C_1$-$C_4$)alkyl such as methyl; and An'''- represents a counterion.

In particular, **Y** represents a group chosen from oxazolium, isoxazolium, thiazolium, isothiazolium, 1,2,4-triazolium, 1,2,3-triazolium, 1,2,4-oxazolium, 1,2,4-thiadiazolium, pyrylium, pyridinium, pyrimidinium, pyrazinium, pyridazinium, triazinium and imidazolium, benzimidazolium, benzoxazolium, benzothiazolium, these groups being optionally substituted with one or more ($C_1$-$C_4$)alkyl groups, in particular methyl.

In particular, **Y** represents a protective group such as:

- ($C_1$-$C_4$)alkylcarbonyl, for instance methylcarbonyl or ethylcarbonyl;
- arylcarbonyl such as phenylcarbonyl;
- ($C_1$-$C_4$)alkoxycarbonyl;
- aryloxycarbonyl;
- aryl($C_1$-$C_4$)alkoxycarbonyl;
- (di)($C_1$-$C_4$) (alkyl)aminocarbonyl such as dimethylaminocarbonyl;
- ($C_1$-$C_4$)(alkyl)arylaminocarbonyl;
- optionally substituted aryl such as phenyl;
- 5- or 6-membered monocyclic heteroaryl such as imidazolyl or pyridyl;
- cationic 5- or 6-membered monocyclic heteroaryl such as pyrylium, pyridinium, pyrimidinium, pyrazinium, pyridazinium, triazinium, imidazolium; these groups being optionally substituted with one or more identical or different ($C_1$-$C_4$)alkyl groups such as methyl;
- cationic 8- to 11-membered bicyclic heteroaryl such as benzimidazolium or benzoxazolium; these groups being optionally substituted with one or more identical or different ($C_1$-$C_4$)alkyl groups such as methyl;
- cationic heterocycle of formula below:

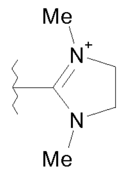

An'''-

- -$C(NH_2)=N^+H_2$; An'''-; with An'''- being an anionic counterion as defined above;
- -$C(NH_2)=NH$;
- $SO_3^-$, $M^+$ with $M^+$ representing an alkali metal such as sodium or potassium.

*1.1.2. **$C_{sat}$** and **$C'_{sat}$**:*

As indicated previously, in formula (I), $C_{sat}$ and $C'_{sat}$, independently of each other, represent a linear or branched, optionally substituted, optionally cyclic $C_1$-$C_{18}$ alkylene chain.

Substituents that may be mentioned include amino groups, ($C_1$-$C_4$)alkylamino groups, ($C_1$-$C_4$)dialkyl amino groups, or the group $R^a$-$Z^a$-$C(Z^b)$- (in which $Z^a$, $Z^b$, which may be identical or different, represent an oxygen or sulfur atom or a group $NR^{a'}$, and $R^a$ represents an alkali metal, a hydrogen atom or a $C_1$-$C_4$ alkyl group and $R^{a'}$ represents a hydrogen atom or a $C_1$-$C_4$ alkyl group) preferably present on the carbon in the beta or gamma position relative to the sulfur atoms.

Preferably, in the case of formulae **(I),** $C_{sat}$ and $C'_{sat}$ represent a chain $-(CH_2)_k-$ with k being an integer between 1 and 8 inclusive.

*1.1.3. **X** and **X'**:*

In accordance with one particular embodiment of the invention, in the abovementioned formulae **(I),** when **p** and **p'** are equal to 1, **X** and **X',** which may be identical or different, represent the following sequence: **$-(T)_t-(Z)_z-(T')_{t'}-$** , said sequence being linked in formulae **(I)** symmetrically as follows: - **$C_{sat}$ (or $C'_{sat}$t)-$(T)_t$-$(Z)_z$-(A** or **A');**

in which:
**T** and **T'**, which may be identical or different, represent one or more radicals or combinations thereof chosen from: -O-; -S-; -N(R)-; $-N^+(R)(R^\circ)-$; -S(O)-; $-S(O)_2-$; - C(O)-; with R, R°, which may be identical or different, representing a hydrogen atom, a $C_1$-$C_4$ alkyl radical, $C_1$-$C_4$ hydroxyalkyl radical or an aryl($C_1$-$C_4$)alkyl radical; and a cationic or non-cationic, preferentially monocyclic heterocycloalkyl or heteroaryl radical, preferentially containing two heteroatoms (more preferentially two nitrogen atoms) and preferentially being 5- to 7-membered, more preferentially imidazolium;
the indices **t** and **t'**, which may be identical or different, are equal to 0 or 1;

- **Z** represents:
  - $-(CH_2)_m-$ with m being an integer between 1 and 8;
  - $-(CH_2CH_2O)_q-$ or $-(OCH_2CH_2)_q-$ in which q is an integer between 1 and 5 inclusive;
  - an aryl, alkylaryl or arylalkyl radical in which the alkyl radical is $C_1$-$C_4$ and the aryl radical is preferably $C_6$, being optionally substituted with at least one group $SO_3M$ with M representing a hydrogen atom, an alkali metal or an ammonium group substituted with one or more identical or different, linear or branched $C_1$-$C_{18}$ alkyl radicals optionally bearing at least one hydroxyl;
- **z** is 0 or 1.

Moreover, according to one particular embodiment of the invention, **Z** represents:

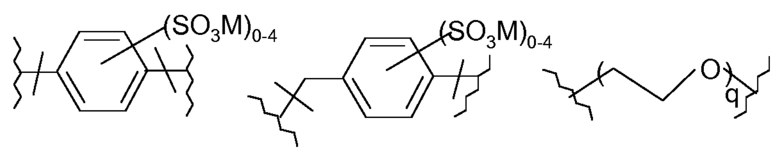 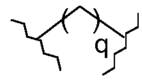

in which **M** represents a hydrogen atom, an alkali metal or an ammonium group or an ammonium group substituted with one or more identical or different, linear or branched $C_1$-$C_{10}$ alkyl radicals optionally bearing at least one hydroxyl; **0-4** represents an integer between 0 and 4 inclusive, and **q** represents an integer between 1 and 6.

*1.1.4. **A** and **A'**:*

The radicals **A** and **A'** of formulae **(I)** represent a cationic chromophore, it being understood that at least one of the two chromophores preferably bears at least one ammonium group bearing a linear or branched, preferably linear, saturated or unsaturated, preferably saturated, $C_{10}$-$C_{30}$ aliphatic chain; more preferentially, the two radicals A and A' each bear an ammonium group bearing a $C_{10}$-$C_{30}$ aliphatic chain.
In particular, **A** and/or **A'** bear a linear or branched ($C_{10}$-$C_{30}$)alkyl group, or a linear or branched ($C_{10}$-$C_{30}$)alkenyl group; and $R^2$ and $R^3$, which may be identical or different, represent a hydrogen atom, or a linear or branched ($C_1$-$C_{30}$)alkyl group or linear or branched ($C_2$-$C_{30}$)alkenyl group, preferably a ($C_1$-$C_6$)alkyl group.
According to one preferred embodiment of the invention, the dyes **(I)** according to the invention are disulfides and comprise identical cationic chromophores **A** and **A'.**
More particularly, the dyes of formula **(I)** according to the invention are disulfides and symmetrical, *i.e.* they contain a $C_2$ axis of symmetry, i.e. formula **(I)** is such that:
$\mathbf{A\text{-}(X)_p\text{-}C_{sat}\text{-}S\text{-}S\text{-}C'_{sat}\text{-}(X')_{p'}\text{-}A'}$ with A = A', X = X', p = p', $C_{sat}$ = $C'_{sat}$.
As cationic chromophores that are useful in the present invention, mention may be made of those derived from the following dyes: acridines; acridones; anthranthrones; anthrapyrimidines; anthraquinones; azines; (poly)azos, hydrazono or hydrazones, in particular arylhydrazones; azomethines; benzanthrones; benzimidazoles; benzimidazolones; benzindoles; benzoxazoles; benzopyrans; benzothiazoles; benzoquinones; bisazines; bis-isoindolines; carboxanilides; coumarins; cyanins such as azacarbocyanins, diazacarbocyanins, diazahemicyanins, tetraazacarbocyanins or (poly)methines; diazines; diketopyrrolopyrroles; dioxazines; diphenylamines; diphenylmethanes; dithiazines; flavonoids such as flavanthrones and flavones; fluorindines; formazans; indamines; indanthrones; indigoids and pseudo-indigoids; indophenols; indoanilines; isoindolines; isoindolinones; isoviolanthrones; lactones; naphthoquinones; nitro, in particular nitro(hetero)aromatics; oxadiazoles; oxazines; perilones; perinones; perylenes; phenazines; phenoxazine; phenothiazines; phthalocyanine; polyenes/carotenoids; porphyrins; pyranthrones; pyrazolanthrones; pyrazolones; pyrimidinoanthrones; pyronines; quinacridones; quinolines; quinophthalones; squaranes; tetrazoliums; thiazines, thioindigo; thiopyronines; triarylmethanes, or xanthenes. Preferentially, the chromophores are chosen from poly(azo), hydrazono and azomethine chromophores, more particularly from azo chromophores.

Among the cationic azo chromophores, mention may be made particularly of those derived from the cationic dyes described in the Kirk Othmer Encyclopedia of Chemical Technology, "Dyes, Azo", J. Wiley & Sons, updated on 19/04/2010.

Among the cationic azo chromophores **A** and/or **A'** that may be used according to the invention, mention may be made of radicals derived from the cationic azo dyes described in patent applications WO 95/15144, WO95/01772 and EP-714954.

According to one preferred embodiment of the invention, the coloured chromophore **A** and/or **A'** is chosen from cationic chromophores, preferentially those derived from dyes known as *"basic dyes"*. Among the azo chromophores, mention may be made of those described in the Colour Index International 3rd edition, and in particular the compounds of Arianor type: Basic Red 22; Basic Red 76; Basic Yellow 57; Basic Brown 16; Basic Brown 17.

Among the cationic quinone chromophores **A** and/or **A',** those mentioned in the abovementioned Colour Index International are suitable for use, and among those, mention may be made, inter alia, of the radicals derived from the following dyes: Basic Blue 22; Basic Blue 99. Among the cationic azine chromophores **A** and/or **A',** those listed in the Colour Index International are suitable for use, and for example the radicals derived from the following dyes: Basic Blue 17; Basic Red 2.

Among the cationic triarylmethane chromophores **A** and/or **A'** that may be used according to the invention, mention may be made, besides those listed in the Colour Index, of the radicals derived from the following dyes: Basic Green 1; Basic Violet 3; Basic Violet 14; Basic Blue 7; Basic Blue 26.

Mention may also be made of the cationic chromophores derived from the dyes described in documents US 5 888 252, EP 1 133 975, WO 03/029 359, EP 860 636, WO 95/01772, WO 95/15144 and EP 714 954. Mention may also be made of those listed in the encyclopaedia "The Chemistry of Synthetic Dyes" by K. Venkataraman, 1952, Academic Press, vol. 1 to 7, in the "Kirk-Othmer Encyclopedia of Chemical Technology", in the chapter "Dyes and Dye Intermediates", 1993, Wiley and Sons, and in various chapters of "Ullmann's Encyclopedia of Industrial Chemistry", 7th edition, Wiley and Sons.

Preferably, the cationic chromophores **A** and/or **A'** are chosen from those derived from dyes of azo and hydrazono type.

According to one particular embodiment, the cationic radicals **A** and/or **A'** in formula (I) comprise at least one cationic azo chromophore derived from a dye described in EP 850 636, FR 2 788 433, EP 920 856, WO 99/48465, FR 2 757 385, EP 850 637, EP 918 053, WO 97/44004, FR 2 570 946, FR 2 285 851, DE 2 538 363, FR 2 189 006, FR 1 560 664, FR 1 540 423, FR 1 567 219, FR 1 516 943, FR 1 221 122, DE 4 220 388, DE 4 137 005, WO 01/66646, US 5 708 151, WO 95/01772, WO 515 144, GB 1 195 386, US 3 524 842, US 5 879 413, EP 1 062 940, EP 1 133 976, GB 738 585, DE 2 527 638, FR 2 275 462, GB 1974-27645, Acta Histochem, 61(1), 48-52 (1978); Tsitologiya, 10(3), 403-5 (1968); Zh. Obshch. Khim., 40(1), 195-202 (1970); Ann. Chim. (Rome), 65(5-6), 305-14 (1975); Journal of the Chinese Chemical Society (Taipei), 45(1), 209-211 (1998); Rev. Roum. Chim., 33(4), 377-83 (1988); Text. Res. J. (1984), 54(2), 105-7; Chim. Ind. (Milan), 56(9), 600-3 (1974); Khim. Tekhnol., 22(5), 548-53 (1979); Ger. Monatsh. Chem., 106(3), 643-8 (1975); MRL Bull. Res. Dev., 6(2), 21-7 (1992); Lihua Jianyan, Huaxue Fence, 29(4), 233-4 (1993); Dyes Pigm., 19(1), 69-79 (1992); Dyes Pigm., 11(3), 163-72 (1989).

In addition to the presence on at least one of the two chromephores of an ammonium group bearing a linear or branched, saturated or unsaturated $C_{10}$-$C_{30}$ aliphatic chain, as defined above, according to one advantageous variant, **A** and/or **A'** of formulae **(I)** contain at least one cationic radical borne by or included in at least one of the chromophores. Preferably, said cationic radical is a quaternary ammonium; more preferentially, the cationic charge is endocyclic.

These cationic radicals are, for example, a cationic radical:

- bearing an exocyclic (di/tri)($C_1$-$C_8$)alkylammonium charge, or
- bearing an endocyclic charge, such as the following cationic heteroaryl groups: acridinium, benzimidazolium, benzobistriazolium, benzopyrazolium, benzopyridazinium, benzoquinolium, benzothiazolium, benzotriazolium, benzoxazolium, bipyridinium, bis-tetrazolium, dihydrothiazolium, imidazopyridinium, imidazolium, indolium, isoquinolium, naphthoimidazolium, naphthoxazolium, naphthopyrazolium, oxadiazolium, oxazolium, oxazolopyridinium, oxonium, phenazinium, phenoxazolium, pyrazinium, pyrazolium, pyrazoyltriazolium, pyridinium, pyridinoimidazolium, pyrrolium, pyrylium, quinolium, tetrazolium, thiadiazolium, thiazolium, thiazolopyridinium, thiazoylimidazolium, thiopyrylium, triazolium or xanthylium.

Mention may be made of the hydrazono cationic chromophores of formulae **(II)** and **(III'),** and the azo cationic chromophores **(IV), (IV'), (V)** and **(V')** below:

**(*)-$Het^+$-C($R^a$)=N-N($R^b$)-Ar, $Q^-$ (II)**

**$Q^-$, $Het^+$-C($R^a$)=N-N($R^b$)-Ar'-(*), (II')**

**(*)-$Het^+$-N($R^a$)-N=C($R^b$)-Ar, $Q^-$ (III)**

**$Q^-$, $Het^+$-N($R^a$)-N=C($R^b$)-Ar'-(*), (III')**

**(*)-$Het^+$-N=N-Ar, $Q^-$ (IV)**

**$Q^-$, $Het^+$-N=N-Ar'-(*), (IV')**

**(*)-$Ar^+$-N=N-Ar", $Q^-$ (V)**

**$Q^-$, $Ar^+$-N=N-Ar"-(*) (V')**

formulae **(II)** to **(V')** with:

- **$Het^+$** representing a cationic heteroaryl radical, preferentially bearing an endocyclic cationic charge, such as imidazolium, indolium or pyridinium, optionally substituted, preferentially with at least one ($C_1$-$C_8$) alkyl group such as methyl;
- **$Ar^+$** representing an aryl radical, such as phenyl or naphthyl, bearing an exocyclic cationic charge, preferentially ammonium, particularly tri($C_1$-$C_8$)alkylammonium such as trimethylammonium;
- **Ar** represents an aryl group, in particular phenyl, which is optionally substituted, preferentially with one or more electron-donating groups such as i) optionally substituted ($C_1$-$C_8$)alkyl, ii) optionally substituted ($C_1$-$C_8$)alkoxy, iii) (di)($C_1$-$C_8$)(alkyl)amino optionally substituted on the alkyl group(s) with a hydroxyl group, iv) aryl($C_1$-$C_8$)alkylamino, v) optionally substituted *N*-($C_1$-$C_8$)alkyl-*N*-aryl($C_1$-$C_8$)alkylamino or alternatively **Ar** represents a julolidine group;
- **Ar'** is an optionally substituted divalent (hetero)arylene group such as phenylene, particularly para-phenylene, or naphthalene, which are optionally substituted, preferentially with one or more groups ($C_1$-$C_8$)alkyl, hydroxyl or ($C_1$-$C_8$)alkoxy;
- **Ar"** is an optionally substituted (hetero)aryl group such as phenyl or pyrazolyl, which are optionally substituted, preferentially with one or more groups ($C_1$-$C_8$)alkyl, hydroxyl, (di)($C_1$-$C_8$)(alkyl)amino, ($C_1$-$C_8$)alkoxy or phenyl;
- **$R^a$** and **$R^b$**, which may be identical or different, represent a hydrogen atom or a ($C_1$-$C_8$)alkyl group, which is optionally substituted, preferentially with a hydroxyl group;
  or else the substituent **$R^a$** with a substituent of **$Het^+$** and/or **$R^b$** with a substituent of **Ar** form, together with the atoms that bear them, a (hetero)cycloalkyl;
  particularly, **$R^a$** and **$R^b$** represent a hydrogen atom or a ($C_1$-$C_4$)alkyl group, which is optionally substituted with a hydroxyl group;
- **$Q^-$** represents an organic or mineral anionic counterion such as a halide or an alkyl sulfate;
- **(*)** represents the part of the chromophore linked to the rest of the molecule of formula **(I);**
- it being understood that at least one of the $Het^+$, $Ar^+$, Ar and Ar' groups bears at least one linear or branched, saturated or unsaturated $C_{10}$-$C_{30}$ aliphatic chain; preferably a linear or branched ($C_{10}$-$C_{30}$)alkyl group or a linear or branched ($C_{10}$-$C_{30}$)alkenyl group.

In particular, mention may be made of the azo and hydrazono chromophores bearing an endocyclic cationic charge of formulae **(II)** to **(IV')** as defined above. More particularly those of formulae **(II)** to **(IV')** derived from the dyes described in patent applications WO 95/15144, WO 95/01772 and EP-714954. Preferentially the following chromophores:

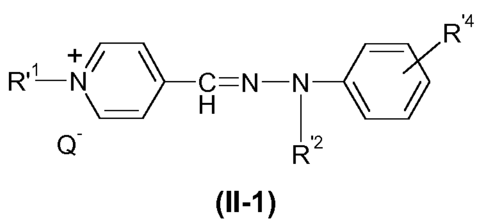

**(II-1)**

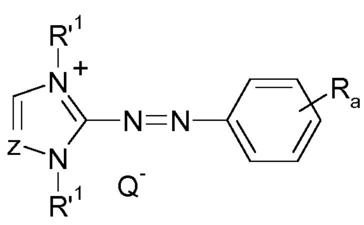

**(IV-1)**

in which formulae **(III-1)** and **(IV-1):**

- **R'1** and **R'2**, which may be identical or different, represent a linear or branched ($C_1$-$C_{30}$)alkyl or linear or branched ($C_2$-$C_{30}$)alkenyl group;
- **$R_a$** represents an amino group $NR'_2R'_3$ with **$R'_2$** and **$R'_3$**, which may be identical or different, representing a hydrogen atom or a linear or branched ($C_1$-$C_{30}$)alkyl or linear or branched ($C_2$-$C_{30}$)alkenyl group; and
- **R'4** represents a hydrogen atom or an electron-donating group such as optionally substituted ($C_1$-$C_{30}$)alkyl, optionally substituted ($C_1$-$C_{30}$)alkoxy, or (di)($C_1$-$C_{30}$)(alkyl)amino optionally substituted on the alkyl group(s) with a hydroxyl group;
- **Z** represents a CH group or a nitrogen atom, preferentially CH,
- **Q'** is as defined above, particularly a halide such as chloride or an alkyl sulfate such as methyl sulfate or mesityl;

it being understood that:

- the chromophore **(II-1)** or **(IV-1)** is linked to the rest of the molecule of formula **(I)** by **R'2**, **R'1**, **R'4** or **$R_a$**, in which case one of the hydrogen atoms of **R'2**, **R'1** or **R'4** is substituted with **X** or **X'** if p = 1 or p'=1 or else with **$C_{sat}$** or **$C_{sat'}$** if p = 0 or p'=0, and
- at least one of the R'1, R'2, R'3, R'4 and $R_a$ groups bears at least one linear or branched, saturated or unsaturated $C_{10}$-$C_{30}$ aliphatic chain; preferably R'1 represents a linear or branched ($C_{10}$-$C_{30}$)alkyl group or a linear or branched ($C_{10}$-$C_{30}$)alkenyl group.

Particularly, the chromophores **(II-1)** and **(IV-1)** are derived from Basic Red 51, Basic Yellow 87 and Basic Orange 31 or derivatives thereof:

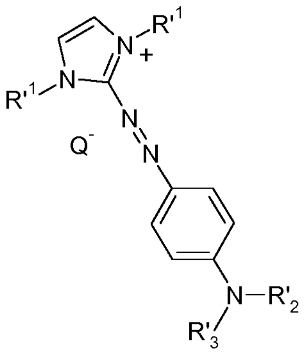

Basic Red 51

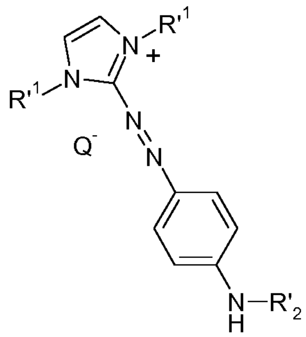

Basic Orange 31

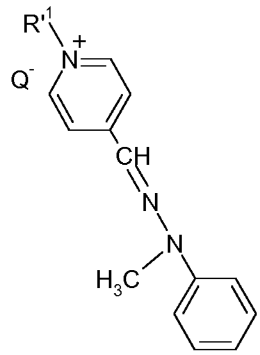

Basic Yellow 87

with:
with:

- **Q'** being an anionic counterion as defined above, particularly a halide such as chloride or an alkyl sulfate such as methyl sulfate or mesityl,
- R'1 representing a linear or branched ($C_{10}$-$C_{30}$)alkyl group or a linear or branched ($C_{10}$-$C_{30}$)alkenyl group, preferably a linear ($C_{10}$-$C_{30}$)alkyl group.

As examples of dyes of the invention, mention may be made of the disulfide dyes chosen from formulae **(V)** to **(XII)** and the thiol or protected-thiol dyes chosen from formulae **(V')** to **(XII')** below:

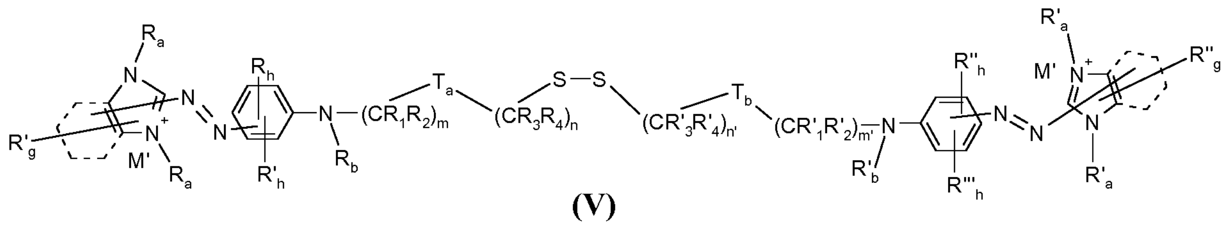

**(V)**

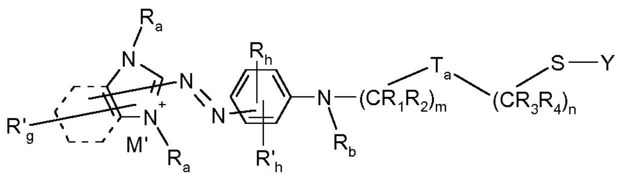

**(V')**

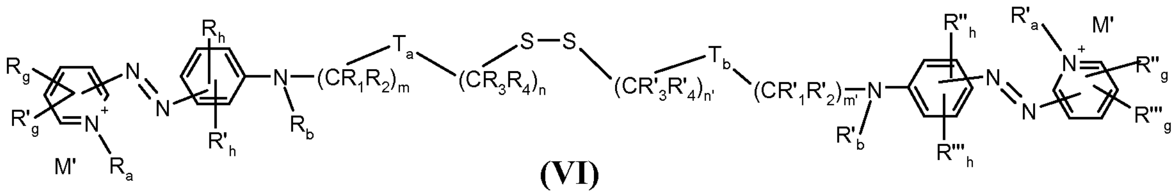

**(VI)**

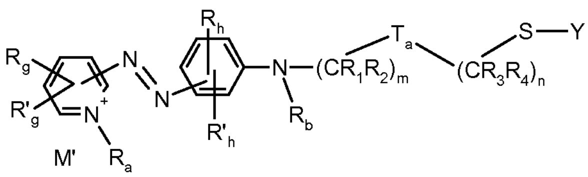

**(VI')**

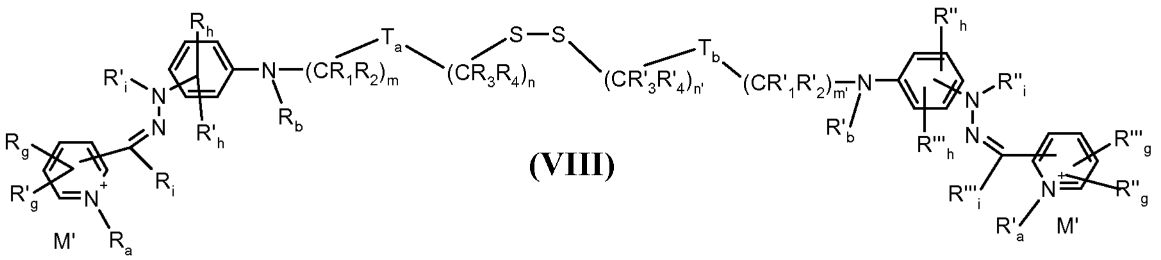

**(VIII)**

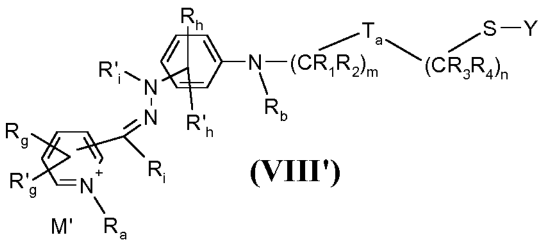

**(VIII')**

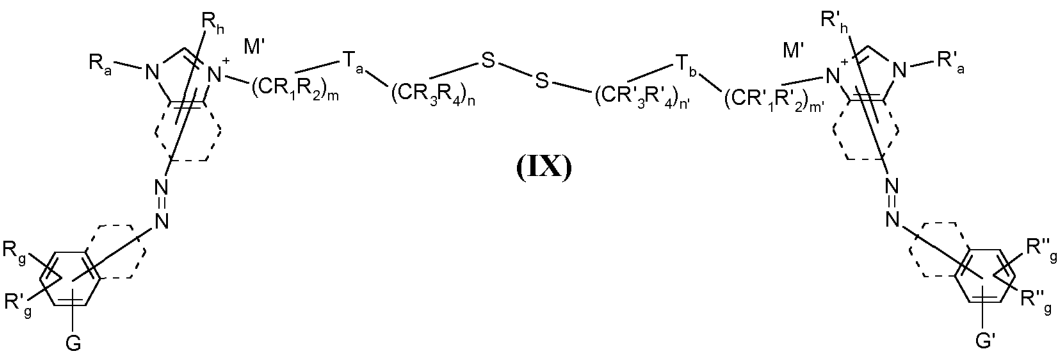

**(IX)**

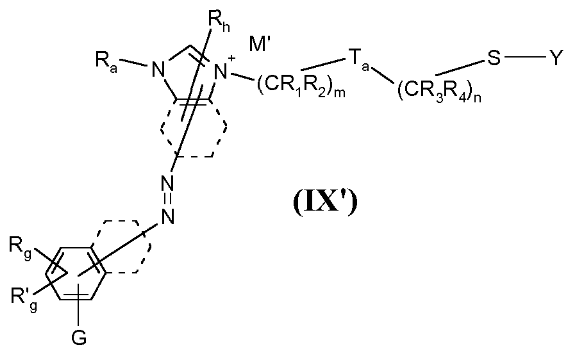

(IX')

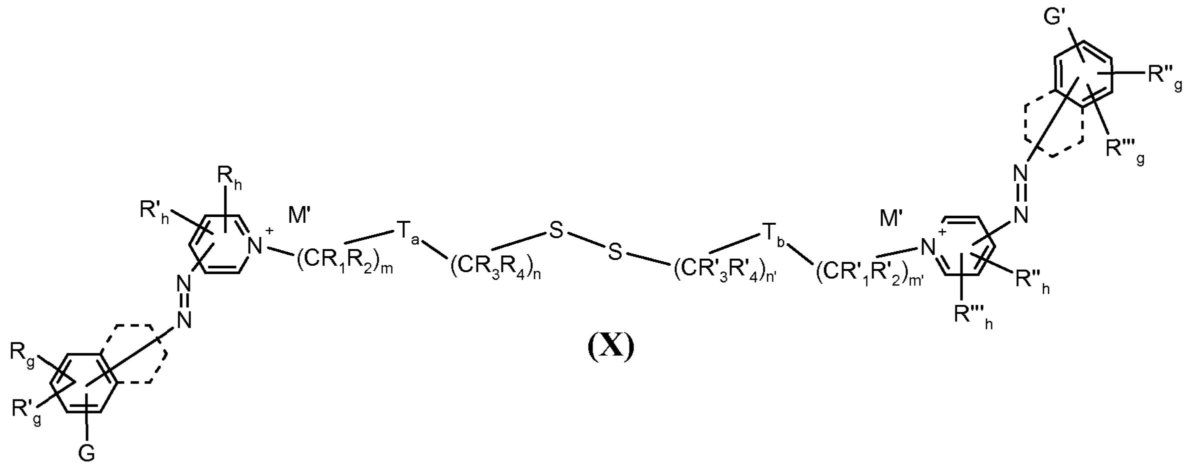

(X)

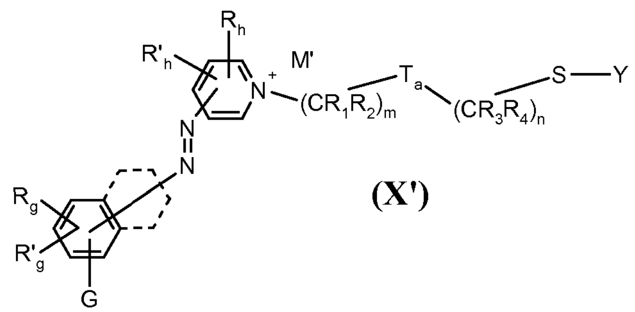

(X')

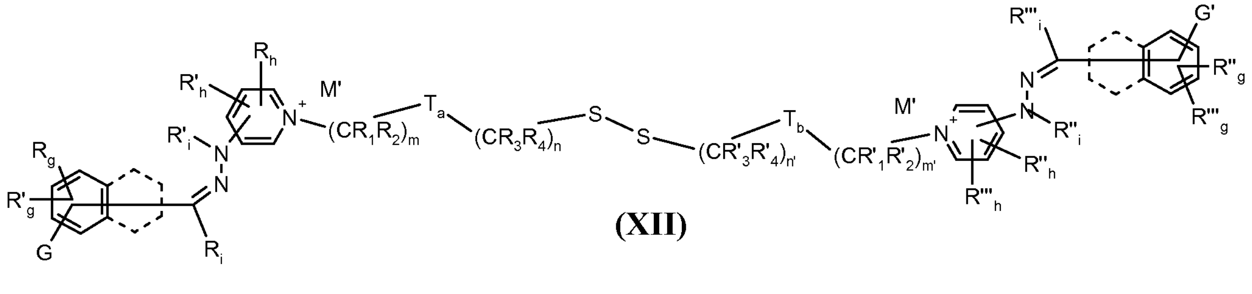

(XII)

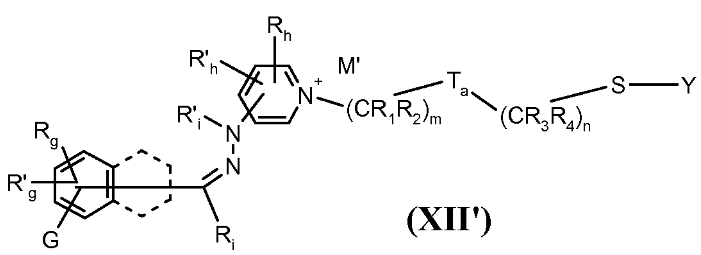

(XII')

in which formulae **(V)** to **(XII)** and **(V')** to **(XII'):**

- G and G', which may be identical or different, represent a hydrogen atom, or i) an - $NR_cR_d$ or $-NR'_cR'_d$ group; ii) optionally substituted, preferentially unsubstituted, $C_1$-$C_6$ alkoxy; or iii) a linear or branched ($C_{10}$-$C_{30}$)alkoxy group or a linear or branched ($C_{10}$-$C_{30}$)alkenyloxy group;
- $R_a$ et $R'_a$, which may be identical or different, represent an aryl($C_1$-$C_4$)alkyl group or a $C_1$-$C_6$ alkyl group optionally substituted with a hydroxyl or amino group, a linear or branched ($C_{10}$-$C_{30}$)alkyl group, or a linear or branched ($C_{10}$-$C_{30}$)alkenyl group; preferentially, $R_a$ and $R'_a$ represent a linear or branched ($C_{10}$-$C_{30}$)alkyl group;
- $R_b$, and $R'_b$, which may be identical or different, represent a hydrogen atom, an aryl($C_1$-$C_4$)alkyl group, or an optionally substituted $C_1$-$C_6$ alkyl group;
- $R_c$, $R'_c$, $R_d$ and $R'_d$, which may be identical or different, represent a hydrogen atom, an aryl($C_1$-$C_4$)alkyl group, an optionally substituted $C_1$-$C_6$ alkyl group or a linear or branched ($C_{10}$-$C_{30}$)alkyl group, or a linear or branched ($C_{10}$-$C_{30}$)alkenyl group; $R_c$, $R'_c$, $R_d$ and $R'_d$ preferentially represent a hydrogen atom, an optionally substituted $C_1$-$C_6$ alkyl group or a linear or branched ($C_{10}$-$C_{30}$)alkyl group;
  or alternatively two adjacent radicals $R_c$ and $R_d$, $R'_c$ and $R'_d$ borne by the same nitrogen atom together form a heterocyclic or heteroaryl group; preferentially, the heterocycle or heteroaryl is monocyclic and 5- to 7-membered; more preferentially, the groups are chosen from imidazolyl and pyrrolidinyl;
- $R_g$, $R'_g$, $R''_g$, $R'''_g$, $R_h$, $R'_h$, $R''_h$, and $R'''_h$, which may be identical or different, represent a hydrogen atom, a halogen atom, an amino, $C_1$-$C_4$ alkylamino, $C_1$-$C_4$ dialkylamino, cyano, carboxyl, hydroxyl or trifluoromethyl group, an acylamino, $C_1$-$C_4$ alkoxy, (poly)hydroxy($C_2$-$C_4$)alkoxy, alkylcarbonyloxy, alkoxycarbonyl or alkylcarbonylamino radical, an acylamino, carbamoyl or alkylsulfonylamino radical, an aminosulfonyl radical, or a $C_1$-$C_{16}$ alkyl radical optionally substituted with a group chosen from $C_1$-$C_{12}$ alkoxy, hydroxyl, cyano, carboxyl, amino, $C_1$-$C_4$ alkylamino and $C_1$-$C_4$ dialkylamino, or alternatively the two alkyl radicals borne by the nitrogen atom of the amino group form a 5- to 7-membered heterocycle optionally comprising another heteroatom identical to or different from that of the nitrogen atom; preferentially, $R_g$, $R'_g$, $R''_g$, $R'''_g$, $R_h$, $R'_h$, $R''_h$, and $R'''_h$ represent a hydrogen or halogen atom or a $C_1$-$C_3$ alkyl group;
- or alternatively two groups $R_g$ and $R'_g$; $R''_g$ and $R'''_g$; $R_h$ and $R'_h$; $R''_h$ and $R'''_h$ borne by two adjacent carbon atoms together form a benzo or indeno ring, a fused heterocycloalkyl or fused heteroaryl group; the benzo, indeno, heterocycloalkyl or heteroaryl ring being optionally substituted with a halogen atom, an amino, $C_1$-$C_4$ alkylamino, $C_1$-$C_4$ dialkylamino, nitro, cyano, carboxyl, hydroxyl or trifluoromethyl group, an acylamino, $C_1$-$C_4$ alkoxy, (poly)hydroxy($C_2$-$C_4$)alkoxy, alkylcarbonyloxy, alkoxycarbonyl or alkylcarbonylamino radical, an acylamino, carbamoyl or alkylsulfonylamino radical, an aminosulfonyl radical, or a $C_1$-$C_{16}$ alkyl radical optionally substituted with: a group chosen from $C_1$-$C_{12}$ alkoxy, hydroxyl, cyano, carboxyl, amino, $C_1$-$C_4$ alkylamino, $C_1$-$C_4$ dialkylamino, or alternatively two alkyl radicals borne by the nitrogen atom of the amino group form a 5- to 7-membered heterocycle optionally comprising another heteroatom identical to or different from that of the nitrogen atom; preferentially, $R_g$ and $R'_g$; $R''_g$ and $R'''_g$ together form a benzo group;
- or alternatively when G represents $-NR_cR_d$ and G' represents $-NR'_cR'_d$, two groups $R_c$ and $R'_g$; $R'_c$ and $R''_g$; $R_d$ and $R_g$; $R'_d$ and $R'''_g$ together form a saturated heteroaryl or heterocycle, optionally substituted with one or more groups ($C_1$-$C_6$)alkyl, preferentially a 5- to 7-membered heterocycle containing one or two heteroatoms chosen from nitrogen and oxygen; more preferentially the heterocycle is chosen from morpholinyl, piperazinyl, piperidinyl and pyrrolidinyl groups;
- $R_i$, $R'_i$, $R''_i$, and $R'''_i$, which may be identical or different, represent a hydrogen atom or a $C_1$-$C_4$ alkyl group;
- $R_1$, $R_2$, $R_3$, $R_4$, $R'_1$, $R'_2$, $R'_3$, and $R'_4$, which may be identical or different, represent a hydrogen atom or a $C_1$-$C_4$ alkyl, $C_1$-$C_4$ alkoxy, hydroxyl, cyano, carboxyl, amino, $C_1$-$C_4$ alkylamino or $C_1$-$C_4$ dialkylamino group, said alkyl radicals possibly forming with the nitrogen atom that bears them a 5- to 7-membered heterocycle optionally comprising another nitrogen or non-nitrogen heteroatom; preferentially, $R_1$, $R_2$, $R_3$, $R_4$, $R'_1$, $R'_2$, $R'_3$, and $R'_4$ are hydrogen atoms or an amino group; more preferentially, $R_1$, $R_2$, $R_3$, $R_4$, $R'_1$, $R'_2$, $R'_3$, and $R'_4$ represent a hydrogen atom;
- $T_a$, $T_b$, which may be identical or different, represent i) either a covalent bond $\sigma$, ii) or one or more radicals or combinations thereof chosen from $-SO_2-$, -O-, -S-, - N(R)-, $-N^+(R)(R^\circ)-$, -C(O)-, with R, R°, which may be identical or different, representing a hydrogen atom, a $C_1$-$C_4$ alkyl radical, a $C_1$-$C_4$ hydroxyalkyl radical; or an aryl($C_1$-$C_4$)alkyl; preferentially, $T_a$ is identical to $T_b$ and represents a covalent bond $\sigma$ or a group chosen from -N(R)-, -C(O)-N(R)-, -N(R)-C(O)-, -O-C(O)-, -C(O)-O- and $-N^+(R)(R^\circ)-$, $Q^-$, with R, R°, which may be identical or different, representing a hydrogen atom or a $C_1$-$C_4$ alkyl group; an optionally substituted $C_1$-$C_6$ alkyl group or a linear or branched ($C_{10}$-$C_{30}$)alkyl group, and $Q^-$ representing an anionic counterion; more preferentially, $T_a$ and $T_b$ represent a bond $\sigma$ or -C(O)-N(R)- or - N(R)-C(O)-; iii) or a cationic or non-cationic, preferentially monocyclic, preferentially identical, heterocycloalkyl or heteroaryl radical, preferentially containing two heteroatoms (more preferentially two nitrogen atoms) and preferentially comprising from 5 to 7 ring members, such as imidazolium;

- 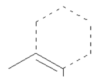

  represents an aryl or heteroaryl group fused to the imidazolium or phenyl ring; or alternatively is absent from the imidazolium or phenyl ring; in particular, when the ring is present, the ring is a benzo; preferentially is absent from the imidazolium or phenyl ring;
- m, m', n and n', which may be identical or different, represent an integer between 0 and 6 inclusive, with m+n and m'+n', which may be identical or different, representing an integer between 1 and 10 inclusive; preferentially, m+n = m'+n' = an integer between 2 and 4 inclusive; more preferentially, m+n = m'+n' = an integer equal to 2;
- **Y** is as defined above; in particular, Y represents a hydrogen atom or a protective group such as:

  ➢ $(C_1\text{-}C_4)$alkylcarbonyl, for instance methylcarbonyl or ethylcarbonyl;

  ➢ arylcarbonyl such as phenylcarbonyl;

  ➢ $(C_1\text{-}C_4)$alkoxycarbonyl;

  ➢ aryloxycarbonyl;

  ➢ aryl$(C_1\text{-}C_4)$alkoxycarbonyl;

  ➢ (di) $(C_1\text{-}C_4)$ (alkyl)aminocarbonyl such as dimethylaminocarbonyl;

  ➢ $(C_1\text{-}C_4)$(alkyl)arylaminocarbonyl;

  ➢ optionally substituted aryl such as phenyl;

  ➢ 5- or 6-membered monocyclic heteroaryl such as imidazolyl or pyridyl;

  ➢ cationic 5- or 6-membered monocyclic heteroaryl such as pyrylium, pyridinium, pyrimidinium, pyrazinium, pyridazinium, triazinium, imidazolium; these groups being optionally substituted with one or more identical or different $(C_1\text{-}C_4)$alkyl groups such as methyl;

  ➢ cationic 8- to 11-membered bicyclic heteroaryl such as benzimidazolium or benzoxazolium; these groups being optionally substituted with one or more identical or different $(C_1\text{-}C_4)$alkyl groups such as methyl;

  ➢ cationic heterocycle of formula below:

  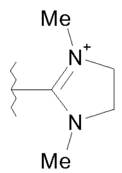

  An'''-

  ➢ $-C(NH_2)=N^+H_2$; An'''-; with An'''- being an anionic counterion as defined above;

  ➢ $-C(NH_2)=NH$;

  ➢ $SO_3^-$, $M^+$ with $M^+$ representing an alkali metal such as sodium or potassium; preferentially, Y represents a hydrogen atom; and
- **M'** representing an anionic counterion, derived from a salt of an organic or mineral acid, or from an organic or mineral base that ensures the electrical neutrality of the molecule;
- it being understood that at least one of the G, G', $R_a$, $R'_a$, $R_c$, $R'_c$, $R_d$ and $R'_d$ groups bears at least one linear or branched $(C_{10}\text{-}C_{30})$alkyl group, or a linear or branched $(C_{10}\text{-}C_{30})$alkenyl group, preferably at least one linear or branched, more preferentially linear, $(C_{10}\text{-}C_{30})$alkyl group.

In particular, the dyes of formula **(I)** are chosen from disulfide, thiol or thiol-protected azo chromophore dyes, preferably chosen from formulae **(V)** and **(V'),** as defined above, preferably with $\mathbf{R_a}$ and $\mathbf{R'_a}$, which are identical, repre-

senting a linear or branched, more preferentially linear, $(C_{10}-C_{30})$alkyl group.

According to one preferred mode of the invention, the dyes of formula **(I)** are chosen from disulfide, thiol or protected-thiol dyes chosen from formulae **(XIII)** to **(XIII')** below:

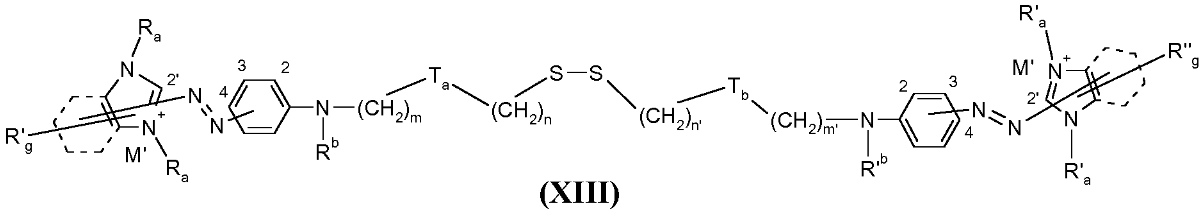

**(XIII)**

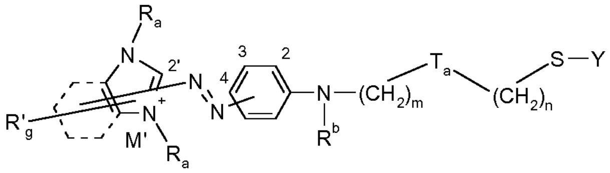

**(XIII')**

salts thereof with organic or mineral acids, optical isomers and geometrical isomers thereof, and solvates such as hydrates; in which formulae **(XIII)** to **(XIII'):**

- $\mathbf{R_a}$ and $\mathbf{R'_a}$, which may be identical or different, represent a linear or branched $(C_{10}-C_{30})$alkyl group, or a linear or branched $(C_{10}-C_{30})$alkenyl group; preferably, $R_a$ and $R'_a$ are identical and represent a linear or branched, in particular linear, $(C_{10}-C_{30})$alkyl group;
- $\mathbf{R_b}$ and $\mathbf{R'_b}$, which may be identical or different, represent a hydrogen atom or a $C_1-C_6$ alkyl group;
- $\mathbf{R'_g}$ and $\mathbf{R''_g}$, which may be identical or different, represent a hydrogen atom, a halogen group, an amino, $C_1-C_4$ alkylamino, $C_1-C_4$ dialkylamino, cyano, carboxy or hydroxyl group, an acylamino, $C_1-C_4$ alkoxy, alkylcarbonyloxy, alkoxycarbonyl or alkylcarbonylamino radical, an acylamino, carbamoyl or alkylsulfonylamino radical, an aminosulfonyl radical, or a $C_1-C_6$ alkyl radical; in particular, $R'_g$ and $R''_g$ represent a hydrogen atom or a $(C_1-C_4)$alkyl group, preferably hydrogen;
- $\mathbf{T_a}$ and $\mathbf{T_b}$, which may be identical or different, represent i) either a covalent bond $\sigma$, ii) or one or more radicals or combinations thereof chosen from -O-, -N(R)-, - $N^+(R)(R°)$-, $Q^-$ and -C(O)-, with R, R°, which may be identical or different, representing a hydrogen atom or a $C_1-C_4$ alkyl radical; preferentially, $T_a$ is identical to $T_b$ and they represent a covalent bond $\sigma$ or a group chosen from -N(R)-, -C(O)-N(R)- and -N(R)-C(O)-, with R representing a hydrogen atom or a $C_1-C_4$ alkyl group; more preferentially, $T_a$ and $T_b$ represent a bond $\sigma$;

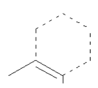

is as defined above; preferentially is absent from the imidazolium or phenyl ring;
- **m, m', n** and **n'**, which may be identical or different, represent an integer between 0 and 6 inclusive, with m+n and m'+n', which may be identical or different, representing an integer between 1 and 10 inclusive; preferentially, m+n = m'+n' = an integer between 2 and 4 inclusive; more preferentially, m+n = m'+n' = an integer equal to 2;
- **Y** represents a hydrogen atom, or a protective group chosen from:

  - ➢ $(C_1-C_4)$alkylcarbonyl, for instance methylcarbonyl or ethylcarbonyl;
    - ➢ arylcarbonyl such as phenylcarbonyl;
  - ➢ $(C_1-C_4)$alkoxycarbonyl;
    - ➢ aryloxycarbonyl;
  - ➢ aryl$(C_1-C_4)$alkoxycarbonyl;
  - ➢ (di) $(C_1-C_4)$ (alkyl)aminocarbonyl such as dimethylaminocarbonyl;

- ➢ $(C_1-C_4)$(alkyl)arylaminocarbonyl;
  - ➢ optionally substituted aryl such as phenyl;
  - ➢ 5- or 6-membered monocyclic heteroaryl such as imidazolyl or pyridyl;

➢ cationic 5- or 6-membered monocyclic heteroaryl such as pyrylium, pyridinium, pyrimidinium, pyrazinium, pyridazinium, triazinium, imidazolium; these groups being optionally substituted with one or more identical or different $(C_1-C_4)$alkyl groups such as methyl;

➢ cationic 8- to 11-membered bicyclic heteroaryl such as benzimidazolium or benzoxazolium; these groups being optionally substituted with one or more identical or different $(C_1-C_4)$alkyl groups such as methyl;

➢ cationic heterocycle of formula below:

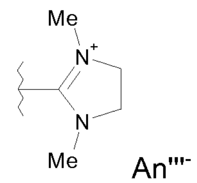

➢ $-C(NH_2)=N^+H_2$; $An'''^-$; with $An'''^-$ being an anionic counterion as defined above;

➢ $-C(NH_2)=NH$;

➢ $SO_3^-$, $M^+$ with $M^+$ representing an alkali metal such as sodium or potassium; preferably, **Y** represents a hydrogen atom; and

- **M'** representing an anionic counterion, derived from a salt of an organic or mineral acid, or from an organic or mineral base that ensures the electrical neutrality of the molecule.

According to one particular mode of the invention, the dyes of the invention belong to formula **(XIII)** or **(XIII')** which bear an azo group linking the (benzo)imidazolium part to the phenyl ortho or para to the pyridinium, i.e. in the 2'-4, 4-2', 2'-4 and 4-2' position, preferably in the 2'-4 and 4-2' para position.

As examples of disulfide, thiol and protected-thiol direct dyes of formula **(I)** of the invention, mention may be made of those having the following chemical structures:

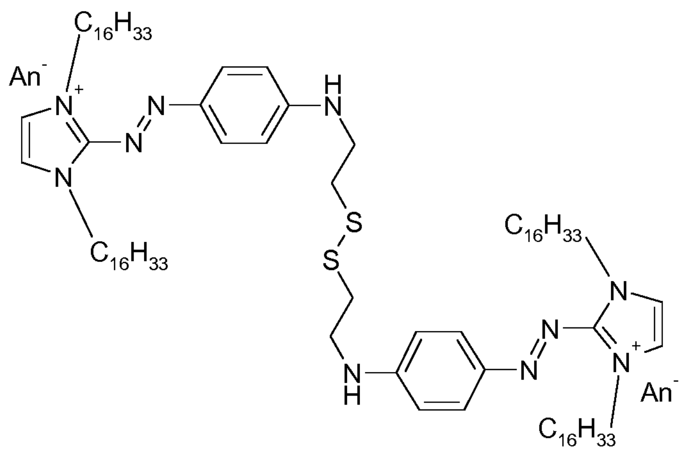

**1**

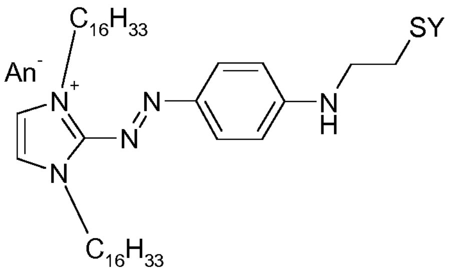

**2**

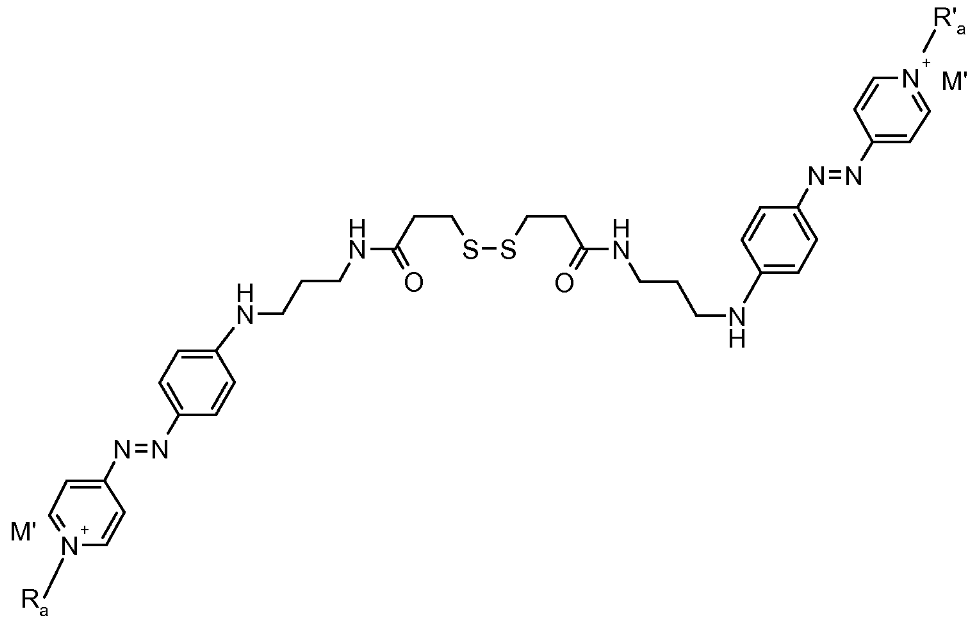

**3**

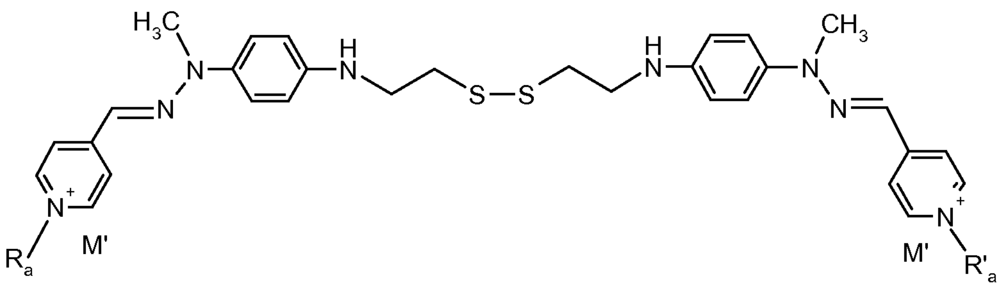

**4**

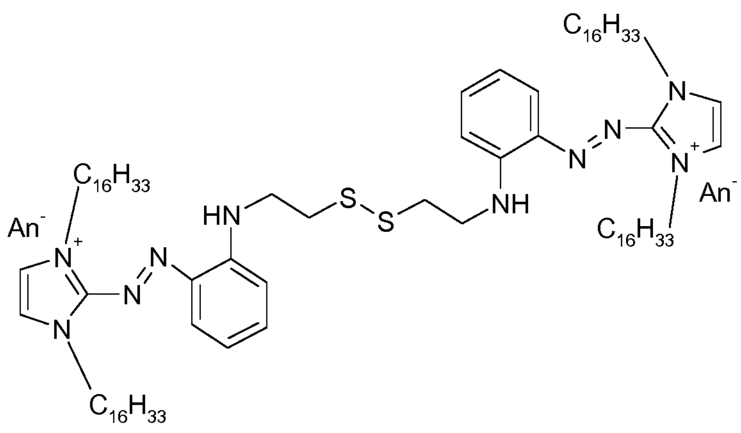

**5**

with **An**$^-$, **M'**, which may be identical or different, preferentially identical, representing anionic counterions; more particularly, the anionic counterion is chosen from halides such as chloride, alkyl sulfate such as methyl sulfate and

mesylate; **Y** is as defined above, and preferably represents

- ➢ $(C_1\text{-}C_4)$alkylcarbonyl, for instance methylcarbonyl or ethylcarbonyl;
- ➢ arylcarbonyl such as phenylcarbonyl;
- ➢ $(C_1\text{-}C_4)$alkoxycarbonyl;
- ➢ aryloxycarbonyl;
- ➢ aryl$(C_1\text{-}C_4)$alkoxycarbonyl;
- ➢ optionally substituted aryl such as phenyl;
- ➢ 5- or 6-membered monocyclic heteroaryl such as imidazolyl or pyridyl;
- ➢ $-C(NH_2)=N^+H_2$; An'''-; with An'''- an anionic counterion as defined above; more preferentially, Y is a hydrogen atom;

**R'$_a$** represents an n-$(C_{14}\text{-}C_{20})$alkyl group such as an n-($C_{16}$ alkyl) group.

*1.1.5 The cosmetically acceptable organic or mineral acid salt and counterion of the dyes of the invention:*

an *"organic or mineral acid salt"* is more particularly chosen from a salt derived from i) hydrochloric acid HCl, ii) hydrobromic acid HBr, iii) sulfuric acid $H_2SO_4$, iv) alkylsulfonic acids: Alk-$S(O)_2OH$ such as methanesulfonic acid and ethanesulfonic acid; v) arylsulfonic acids: Ar-$S(O)_2OH$ such as benzenesulfonic acid and toluenesulfonic acid; vi) citric acid; vii) succinic acid; viii) tartaric acid; ix) lactic acid; x) alkoxysulfinic acids: Alk-O-S(O)OH such as methoxysulfinic acid and ethoxysulfinic acid; xi) aryloxysulfinic acids such as tolueneoxysulfinic acid and phenoxysulfinic acid; xii) phosphoric acid $H_3PO_4$; xiii) acetic acid $CH_3C(O)OH$; xiv) triflic acid $CF_3SO_3H$; and xv) tetrafluoroboric acid $HBF_4$;
an *"anionic counterion"* is an anion or an anionic group associated with the cationic charge of the dye; more particularly, the anionic counterion is chosen from: i) halides such as chloride or bromide; ii) nitrates; iii) sulfonates, including $C_1\text{-}C_6$ alkylsulfonates: Alk-$S(O)_2O^-$such as methanesulfonate or mesylate and ethanesulfonate; iv) arylsulfonates: Ar-$S(O)_2O^-$ such as benzenesulfonate and toluenesulfonate or tosylate; v) citrate; vi) succinate; vii) tartrate; viii) lactate; ix) alkyl sulfates: Alk-O-$S(O)O^-$ such as methyl sulfate and ethyl sulfate; x) aryl sulfates: Ar-O-$S(O)O^-$ such as benzene sulfate and toluene sulfate; xi) alkoxy sulfates: Alk-O-$S(O)_2O^-$ such as methoxy sulfate and ethoxy sulfate; xii) aryloxy sulfates: Ar-O-$S(O)_2O^-$; xiii) phosphate; xiv) acetate; xv) triflate; and xvi) borates such as a tetrafluoroborate.

Moreover, the addition salts that may be used in the context of the invention are in particular chosen from addition salts with a cosmetically acceptable base such as basifying agents as defined below, for instance alkali metal hydroxides, such as sodium hydroxide or potassium hydroxide, aqueous ammonia, amines or alkanolamines.

*1.2. Preparation of the direct dyes bearing a disulfide or thiol function of the invention:*

The disulfide, thiol and protected-thiol dyes are prepared according to conventional methods known by those skilled in the art, in particular using the methods described in applications WO 2009/034059, FR 290780, EP 1 647 580, EP 2 004 759, WO 2007/110541, WO 2007/110540, WO 2007/110539, WO 2007/110538, WO 2007/110537, WO 2007/110536, WO 2007/110535, WO 2007/110534, WO 2007/110533, WO 2007/110532, WO 2007/110531, EP 2 070 988 and WO 2009/040354

These compounds can be obtained using the similar preparation processes described in the books Advanced Organic Chemistry, "Reactions, Mechanisms and Structures", J. March, 4th Ed, John Wiley & Sons, 1992, T. W. Greene "Protective Groups in Organic Synthesis" or "Color Chemistry", H Zollinger, 3rd Ed, Wiley VCH.

It is in particular possible to prepare the disulfide, thiol or protected-thiol dyes of the invention **(I)** as defined above using two equivalents of reagents comprising a chromophore and at least one nucleofugal group such as halogeno **(a)** and one equivalent of amino disulfide compound **(b)**

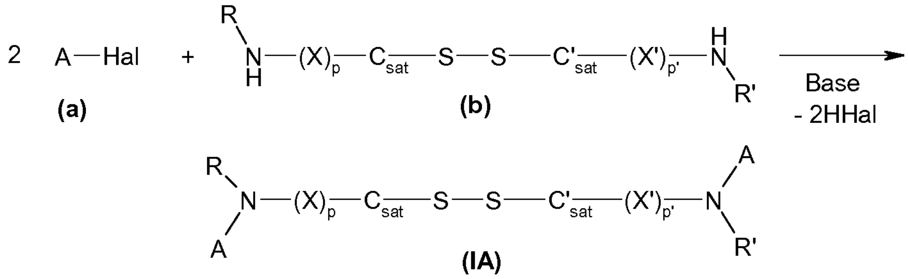


with **Hal** representing a nucleofugal group such as halogeno, (poly)halogeno($C_1$-$C_4$)alkoxy, (poly)halogeno($C_1$-$C_4$)sulfoxy, **X, X', p, p', $C_{sat}$** and **$C'_{sat}$** being as defined for the compound **(I), R** and **R'** representing a hydrogen atom or an optionally substituted ($C_1$-$C_4$)alkyl group; **A** representing a chromophore as defined above for **(I)** or else **A** represents a chromophore comprising a nucleofugal group as defined, which reacts with one or two equivalents of aminoalkylene followed by an alkylation reaction using for example ($C_{10}$-$C_{30}$)alkyl halide, preferably **A** represents a hydrazono cationic chromophore of formulae **(II)** and **(III')**, or azo cationic chromophore **(IV), (IV'), (V)** and **(V')** as defined above.

These reactions are preferably carried out in a polar protic solvent, in particular at the solvent reflux; preferably, the solvent is an alcohol such as ethanol.

According to another particular embodiment, the disulfide dyes of the invention are obtained by a divergent method: the first step consists in preparing the disulfide reagent **(c)** onto which will be grafted a heteroaryl substituted with at least one ($C_1$-$C_6$)alkyl group such as a pyridine substituted with a hydrazino group **(d)** which in turn reacts with an aryl(thio)aldehyde or aryl(thio)ketone reagent comprising at least one group bearing a linear or branched, saturated or unsaturated $C_{10}$-$C_{30}$ aliphatic chain; with elimination of $H_2O$ or $H_2S$ so as to give a disulfide dye bearing a hydrazono chromophore **(I')**, and also optical and geometric isomers thereof, belonging to formula (I) according to the invention:

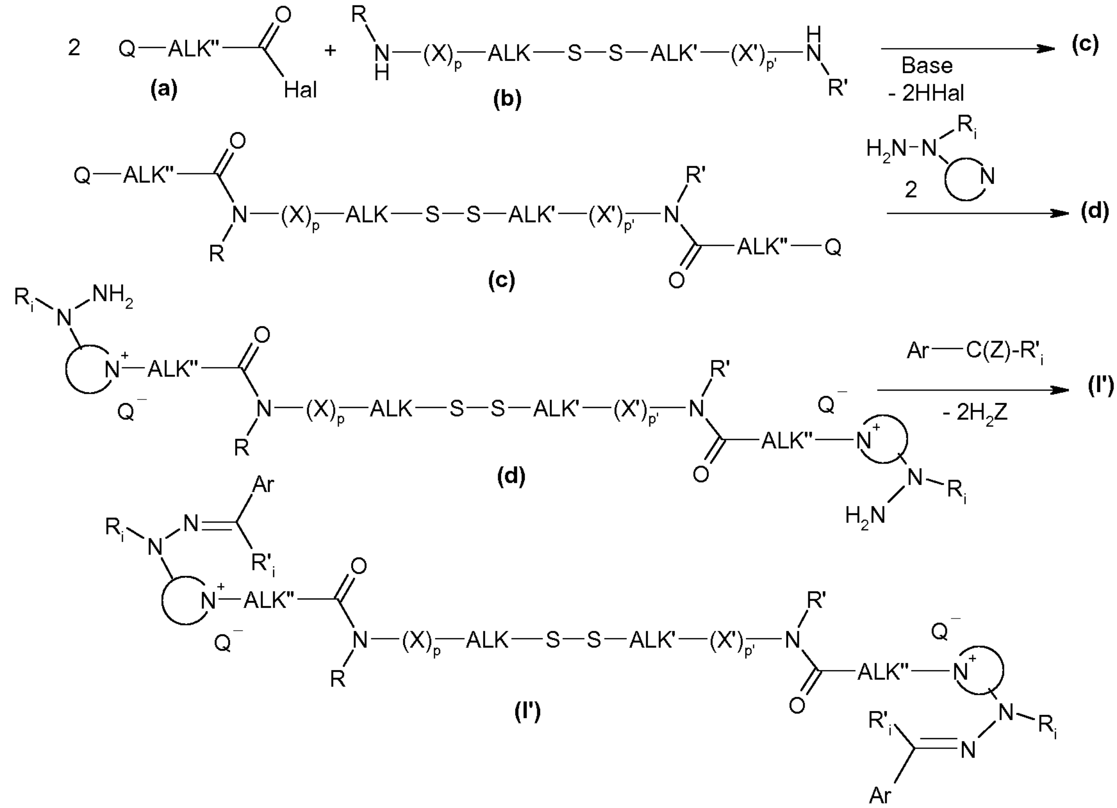


Compounds **(a), (b), (c), (d)** and **(I')**:

- **Q** represents an electrofugal group such as halogeno, (poly)halo($C_1$-$C_6$)-$S(O)_2$-O-, alkyl sulfonyl ($C_1$-$C_6$)Alk-$S(O)_2$O- such as methylsulfonyl and ethylsulfonyl; arylsulfonyls: Ar-$S(O)_2O^-$ such as benzenesulfonyl and toluenesulfonyl; (poly)(hydroxy)($C_1$-$C_6$)alkylcarbonyloxy, alkylsulfonyl: Alk-O-S(O)O-; arylsulfonyls: Ar-O-S(O)O- such as benzenesulfonyl and toluenesulfonyl; alkoxysulfonyls: Alk-O-$S(O)_2$O-; aryloxysulfates: Ar-O-$S(O)_2$O-, phosphonyl; and

borates such as tetrahaloborate;

- **Q$^-$** represents an anionic counterion which derives from Q as defined above;
- **ALK, ALK', ALK"**, which may be identical or different, represent an optionally substituted linear or branched ($C_1$-$C_6$)alkylene group, such as ethylene or propylene;
- **Hal, X, X', p** and **p'** are as defined above;
- **Z** represents an oxygen or sulfur, preferably oxygen, atom;
- **$R_i$** and **$R'_i$** are as defined above;
- 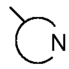

  represents a heteroaryl group comprising at least one nitrogen atom substituted with at least one ($C_1$-$C_6$)alkyl group, preferably methyl;
- 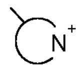

  represents a cationic heteroaryl group substituted with at least one ($C_1$-$C_6$)alkyl group, preferably methyl, and comprising at least one ammonium;
- **Ar** represents an optionally substituted (hetero)aryl group, preferably phenyl, comprising at least one group bearing a linear or branched, saturated or unsaturated $C_{10}$-$C_{30}$ aliphatic chain.

According to one variant, the disulfide compound has an alkylene linker which is uninterrupted between the chromophore and the disulfide group via a heteroatom or an - N(R)-, -N$^+$(R)(R)-, -O-, -S-, -CO-, -$SO_2$- group or a combination thereof with R as defined above and can be prepared according to the following synthesis scheme:

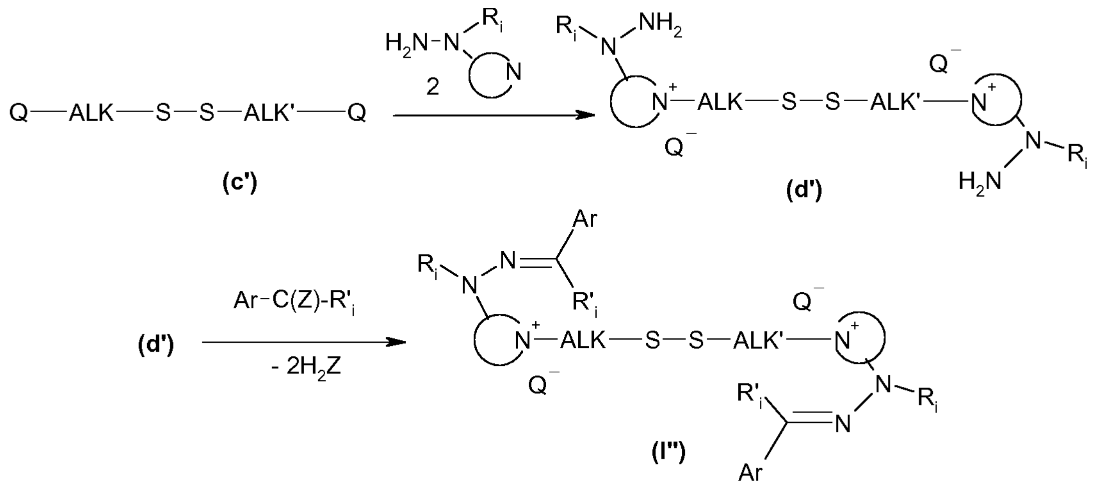


(c') (d') (d') (I")

with ALK, ALK', Q, $R_1$, Z, $R'_1$, Ar as defined above; and

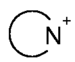

represents a cationic heteroaryl group comprising at least one ammonium.

According to another particular embodiment, the disulfide dyes of the invention are obtained by a convergent method: the first step consists in preparing the azo chromophore reagent **(f)** comprising a leaving group GP such as halide, mesylate or tosylate, a (hetero)aromatic part Ar and a heteroaryl group comprising at least one nitrogen atom such as a (benzo)imidazolyl from a (hetero)aromatic compound (e) comprising at least one GP and an amino group in the presence i) of acid, in particular a mixture of organic and inorganic acid, preferably in a mixture with a carboxylic acid and of hydrochloric acid, and of $MNO_2$ with M representing an alkali or alkaline-earth metal preferably in water and at a temperature between 0°C et 5°C, then ii) of a base such as sodium hydroxide or potassium hydroxide, then iii) of a heteroaryl comprising at least one nitrogen atom, such as (benzo)imidazole, preferably imidazole. The compound **(f)** can then be alkylated in particular with $R^a$ a linear or branched, saturated or unsaturated $C_{10}$-$C_{30}$ aliphatic chain using

a reagent $R_a$-Q with Q representing a leaving group such as halide so as to give the intermediate **(g),** the latter can then react with the diamino disulfide reagent (b) so as to give the azo compound **(I'''')** according to the invention:

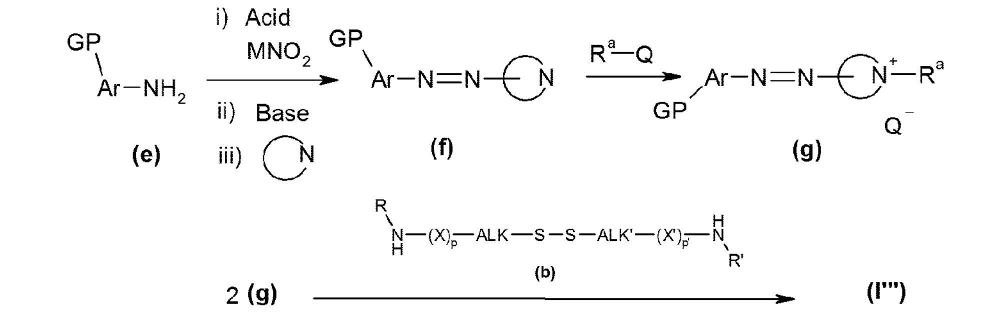


(e) (f) (g) (b) 2 (g) (I''')

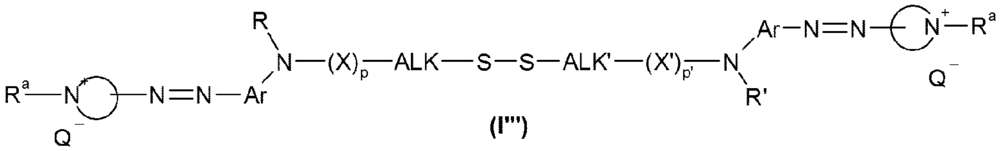

**(I''')**

with ALK, ALK', X, X', p, Q, $Q^-$, Ar, GP and

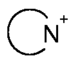

as defined above;

R and R', which may be identical different, represent a hydrogen atom or a $(C_1-C_6)$alkyl group;

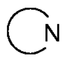

represents a heteroaryl group comprising at least one nitrogen atom.

According to one preferred embodiment of the invention, the process for preparing the compounds of formulae **(Va)** and **(V')** consists in reacting an aniline substituted with an alkoxy or benzyloxy group (h) with i) an acid, preferably a mineral acid such as hydrochloric acid or a carboxylic acid such as acetic acid, in the presence of a nitrogenous compound $MNO_2$ with M representing a cationic counterion such as Na or K, then ii) with an organic or mineral base, preferably a mineral base, such as sodium hydroxide, then iii) an imidazolyl derivative, optionally fused with a (hetero)aryl group, preferably a benzo group, so as to give the azo compound (i); the latter reacting with at least 2 molar equivalents of an alkylating agent such as $R^a$-Q with $R_a$ and Q as defined above, so as to give the compound (j);

- either two equivalents of (j) reacting with an amino disulfide compound (d), so as to give the disulfide dye (Va);
- or one molar equivalent of (j) reacting with a thiol or protected-thiol compound (d') so as to give the thiol or protected-thiol dye (V').

According to the following scheme:

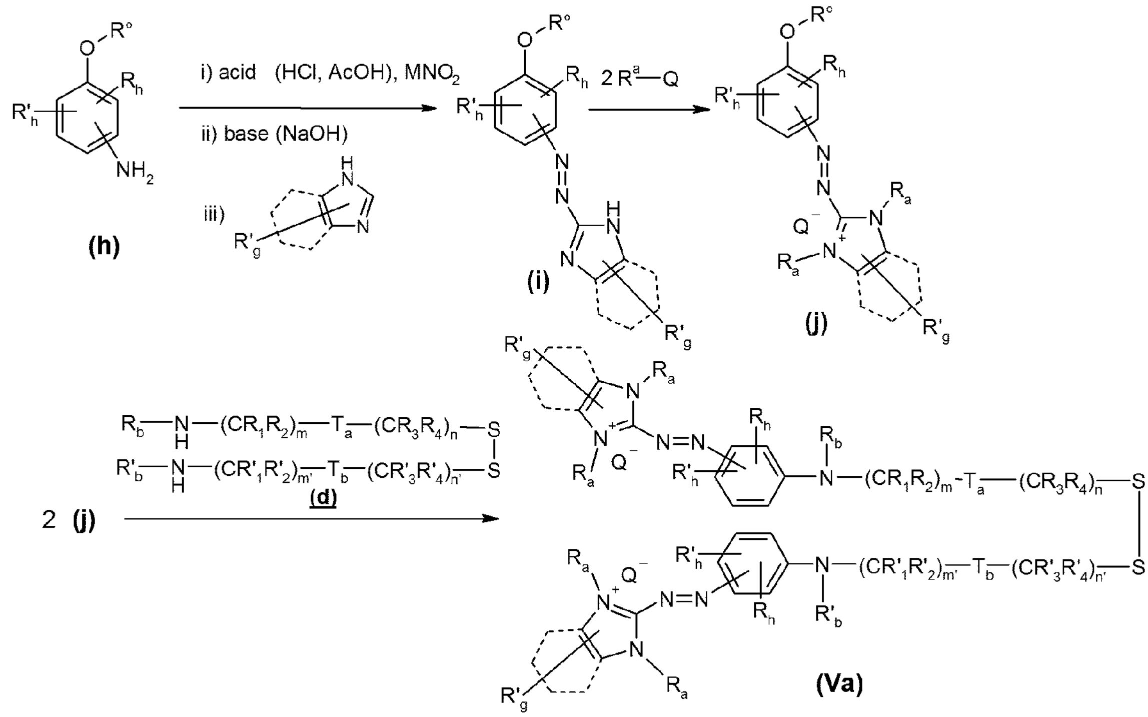


(h) (i) (j)

2 (j)

(Va)

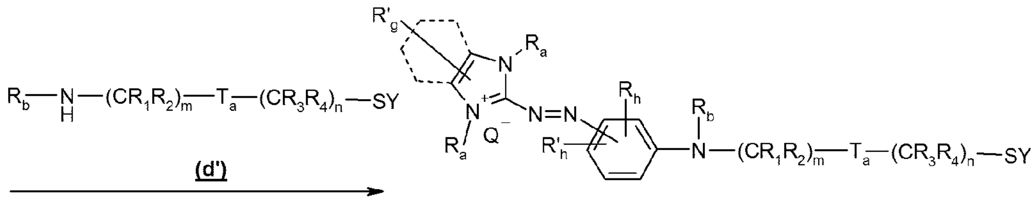


(j)

(V')

with $R_a$, $R_b$, $R'_b$, $R'_g$, $R_h$, $R'_h$, $R_1$, $R_2$, $R_3$, $R_4$, $R'_1$, $R'_2$, $R'_3$, $R'_4$, Y, m, m', n, n', $T_a$, $T_b$, Q and

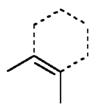

being as defined above, and R° representing a ($C_1$-$C_6$)alkyl, (hetero)aryl or benzyl group.

According to one embodiment, the process for synthesizing the compounds of formula **(I),** more particularly **(Xa),** used in the invention can consist in carrying out the following steps:

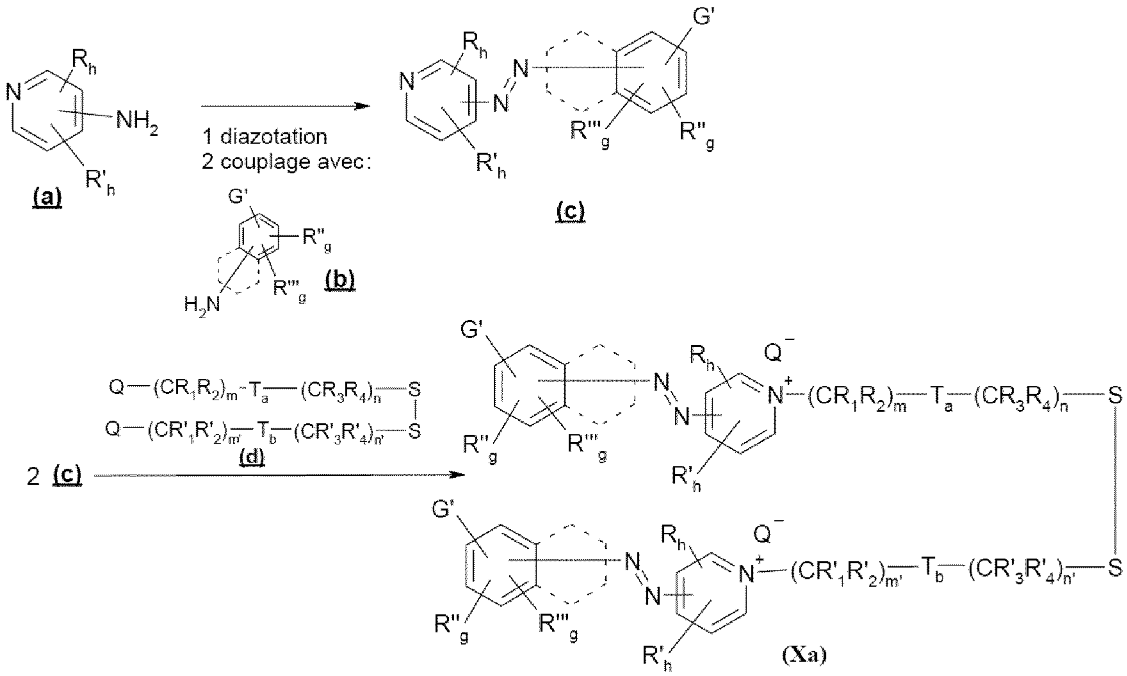


with $R_h$, $R'_h$, G', $R''_g$, $R'''_g$, m, n, m', n', $T_a$, $T_b$, $R_1$, $R_2$, $R_3$, $R_4$, $R'_1$, $R'_2$, $R'_3$ and $R'_4$ as defined above and Q represents a leaving group such as halide, tosylate or mesylate and $Q^-$represents an anionic counterion as defined above.

According to this process, a first step of diazotization of an amin opyridine or quinoline **(a)** is carried out in a manner known to those skilled in the art. They can be obtained from the references described in Color Chemistry, H Zollinger, 3rd Edn, Wiley VCH, pages 166-169.

Thus, said amine is for example brought into contact with phosphoric acid and tert-butylnitrite. Usually, this reaction is carried out at a temperature between -20°C and 30°C; preferably between -10°C and 20°C, at a pH of between 0 and 12.

Conventionally, the reaction is carried out in the presence of a suitable solvent, among which mention may be made of water, alcohols, in particular aliphatic alcohols comprising up to 4 carbon atoms, organic acids, for example a carboxylic acid or sulfonic acid comprising up to 10 carbon atoms, and/or mineral acids of the hydrochloric or sulfuric acid type.

Once the reaction has been carried out, coupling of the product obtained with a compound of the aniline type **(b)** is carried out.

Conventionally, this reaction is carried out in the presence of a solvent which may be that of the preceding step.

The temperature is conventionally between -15°C and 30°C; preferably between - 10°C and 20°C, at a pH preferably of between 0 and 8.

The product may be isolated via the techniques known to those skilled in the art (precipitation, evaporation, etc.).

In a final step, the resulting product **(c)** is then dimerized in a manner known to those skilled in the art. Thus, said resulting product **(c)** is brought into contact with a double alkylating agent having a disulfide unit **(d)** in the presence of a polar or non-polar aprotic solvent such as acetonitrile, dimethylformamide, toluene, 1,3-dimethyl-2-oxohexahydropyrimidine (DMPU) or N-methylpyrrolidone. Usually, the temperature is between 10 and 180°C, preferably between 20°C and 140°C.

The double alkylating agent having a disulfide unit **(d)** can be prepared according to the methods described in the literature and which are known to those skilled in the art:

- Synthesis of the disulfide diol: see for example J. Org. Chem. 1985, 50(26), 5716-5719; J. Am. Chem. Soc. 1970, 92(24), 7224-7225; Tetrahedron 2004, 60(51), 11911-11922; J. Org. (Rome) (1990), 55(59), 2580-2586; J. Chinese Chem. Chem. 1985, 50(26), 5716-5719; J. Chem. Soc., Chem. Comm. 1981, 15, 741-742; Tet. Lett. 2005, 46(36), 6097-6099; J. Org. (Rome) (1990), 55(59), 2580-2586; J. Chinese Chem. Chem. 1985, 50(26), 5716-5719).

- Synthesis of the double alkylating agent bearing a disulfide unit **(d):** By way of example, see Bioorganic § Medicinal Chemistry Letters, 2004, 14(21), 5347-5350; WO 04/039771; Chem. Berichte, 1983, 116(1), 323-347, Tetrahedron 1985, 41(15), 3063-3069; J. Am. Chem. Soc. 1987, 109(25), 7648-7653; J. Org. Chem. 1995, 60(8), 2638-2639; Sulfur Lett. 2000, 24(3), 137-145.

According to another embodiment, the process for synthesizing the compounds used in the invention can consist in carrying out the following steps:

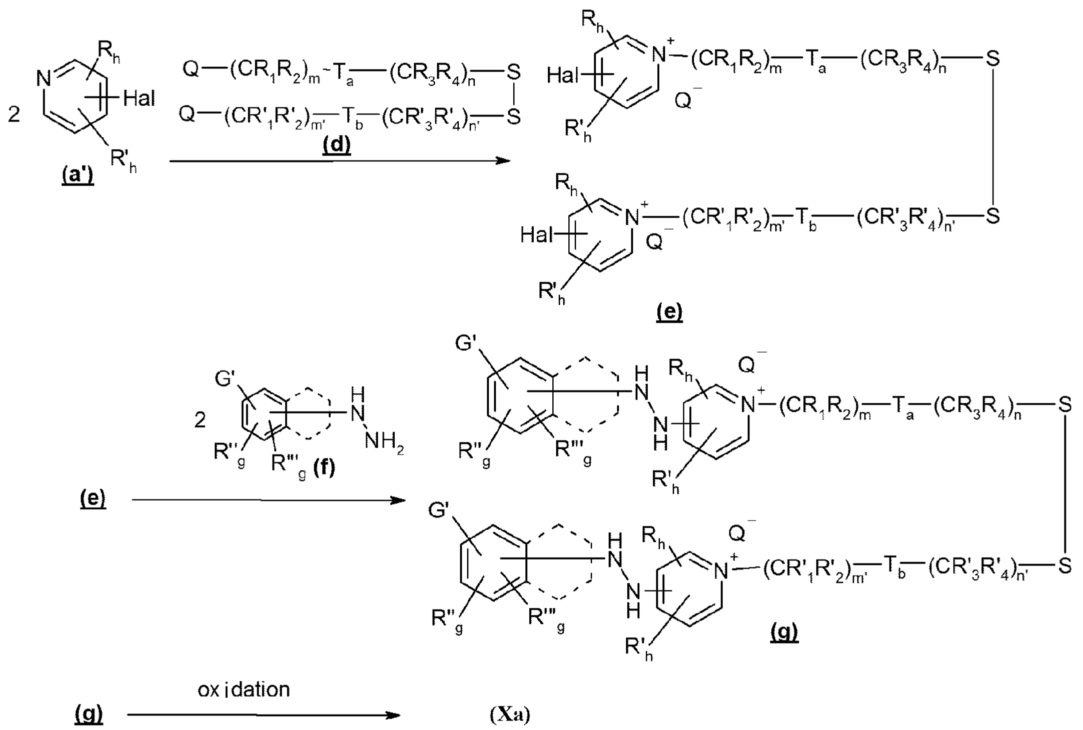


with $R_h$, $R'_h$, G', $R''_g$, $R'''_g$, m, n, m', n', $T_a$, $T_b$, $R_1$, $R_2$, $R_3$, $R_4$, $R'_1$, $R'_2$, $R'_3$, $R'_4$, Q, $Q^-$ and **(Xa)** as defined above and Hal represents a halogen atom, preferably Cl or Br.

According to this process, a first step of alkylation of a pyridine or quinoline compound **(a)** is carried out in a manner known to those skilled in the art. The conditions for carrying out such a step have been summarized previously.

Once the reaction has been carried out, a nucleophilic substitution of the product obtained **(e)** with a compound of arylhydrazine type **(f)** is carried out. Conventionally, this reaction is carried out in the presence of a solvent which may be that of the preceding step.

The temperature is conventionally between -15°C and 60°C; preferably between 0°C and 40°C, at a pH preferably of between 4 and 9.

The product may be isolated via the techniques known to those skilled in the art (precipitation, evaporation, etc.).

Once the reaction has been carried out, the hydrazine derivative **(g)** is oxidized by addition of an oxidizing agent or by simple addition of air. Conventionally, this reaction is carried out in the presence of a solvent which may be that of the preceding step. The temperature is conventionally between -15°C and 60°C; preferably between 0°C and 40°C, at a pH preferably of between 4 and 9. The product **(Xa)** may be isolated via the techniques known to those skilled in the art (precipitation, evaporation, preparative chromatography, etc.).

According to another embodiment, the process for synthesizing the compounds used in the invention can consist in carrying out the following steps:

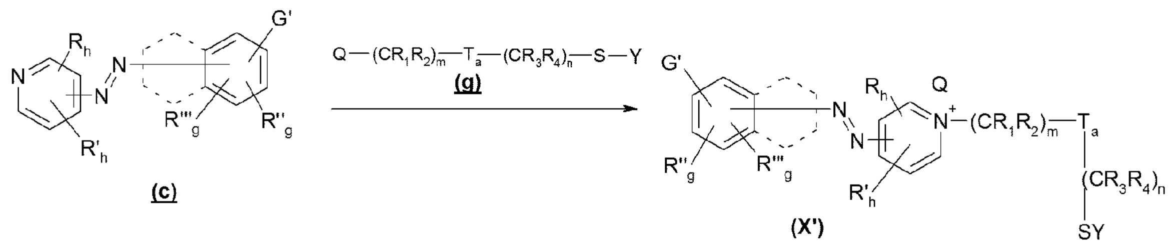

(c) (g) (X')

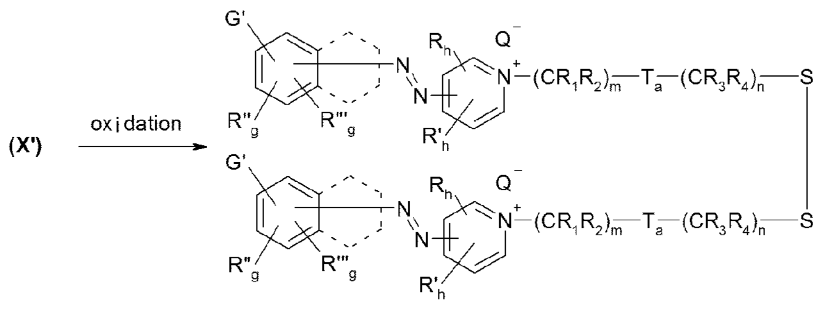


(X') (Xb)

with $R_h$, $R'_h$, G', $R''_g$, $R'''_g$, m, n, $T_a$, $R_1$, $R_2$, $R_3$, $R_4$, Q, $Q^-$ and Y as defined above.

According to this process, a first step of quaternization of a non-cationic azo pyridine or quinoline compound **(c)** is carried out in the usual manner with a thiol or protected-thiol compound **(g)** in the presence of a polar or non-polar, protic or aprotic solvent such as dichloromethane, toluene, ethyl acetate or water at spontaneous or alkaline pH, so as to give the compound according to invention **(X')**. This quaternization step is known to those skilled in the art. Usually, the temperature is between 10 and 180°C, preferably between 20°C and 100°C.

Once the reaction has been carried out, deprotection, if Y is other than hydrogen, of the thiol group and then oxidation of the thiol are carried out. The conditions for carrying out such a step have been summarized below. Usually, the temperature is between 10 and 180°C, preferably between 20°C and 100°C.

This process makes it possible to obtain, during the first step, a dye according to the invention bearing a protected-thiol unit (for which x=y=1), during the thiol deprotection step, a dye bearing a thiol unit (for which x=y=1 and Y represents a hydrogen atom) or a thiolate unit (for which Y represents a metal, an ammonium or a phosphonium), and finally a disulfide dye (for which x=2 and y=0)

According to another embodiment, the process for synthesizing the compounds used in the invention can consist in carrying out the following steps:

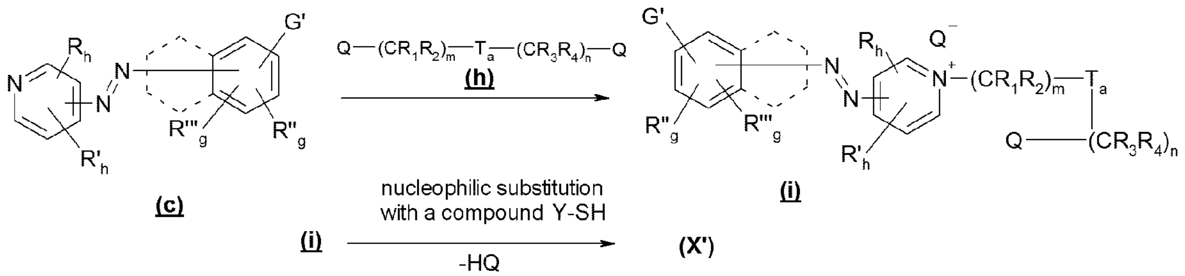


(c) (h) (i) (i) (X')

with $R_h$, $R'_h$, G', $R''_g$, $R'''_g$, m, n, $T_a$, $R_1$, $R_2$, $R_3$, $R_4$, Y, Q, $Q^-$ and **(X')** as defined above.

According to this process, a first step of quaternization of a non-cationic azo pyridine or quinoline compound **(c)** is carried out in the usual manner with a compound **(h)** in the presence of a polar or non-polar, protic or aprotic solvent such as dichloromethane, toluene, ethyl acetate or water at spontaneous or alkaline pH, so as to give the intermediate **(i)**. Q represents a leaving group such as tosylate, mesylate and halide, in particular chloride or bromide.

This quaternization step is known to those skilled in the art. Usually, the temperature is between 10°C and 180°C, preferably between 20°C and 100°C. The quaternization is followed by a nucleophilic substitution with a reagent YSH.

Once the reaction has been carried out, a nucleophilic substitution of the intermediate is obtained **(i)** with a compound of type Y-SH (Y is as previously defined) is carried out. Usually, this reaction is carried out in the presence of a polar or non-polar, protic or aprotic solvent such as dichloromethane, toluene, ethyl acetate, water or alcohols. The

temperature is conventionally between -15°C and 60°C; preferably between 0°C and 50°C, at a pH preferably of between 7 and 9.

The product may be isolated via the techniques known to those skilled in the art (precipitation, evaporation, etc.).

Once the reaction has been carried out, the compound **(X')** can be deprotected and then oxidized so as to give **(Xb)** as previously defined.

According to another embodiment, a disulfide/thiol dye according to the invention can be obtained by nucleophilic substitution of an amine $HNR_2R_3$ **(j)** on an aromatic nucleus such as phenyl of an azo dye bearing a pyridinium or quinolinium unit (X'), bearing, in the ortho- or para-position with respect to said phenyl, a nucleofugal group, for example a halide or an alkoxy:

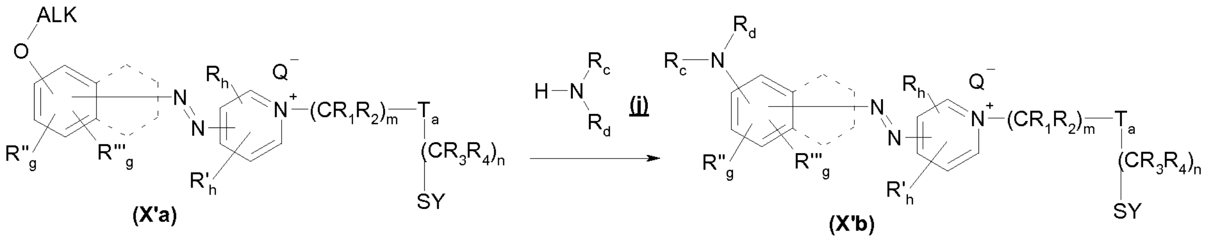


**(X'a)** **(X'b)**

with $R_h$, $R'_h$, $R''_g$, $R'''_g$, m, n, $T_a$, $R_1$, $R_2$, $R_3$, $R_4$, Y, Q, $Q^-$, $R_c$ and $R_d$ as defined above and ALK represents a linear or branched $(C_1\text{-}C_6)$alkyl group such as methyl.

Once the reaction has been carried out, the compound **(X'b)** can be deprotected and then oxidized as defined above so as to give **(Xc).**

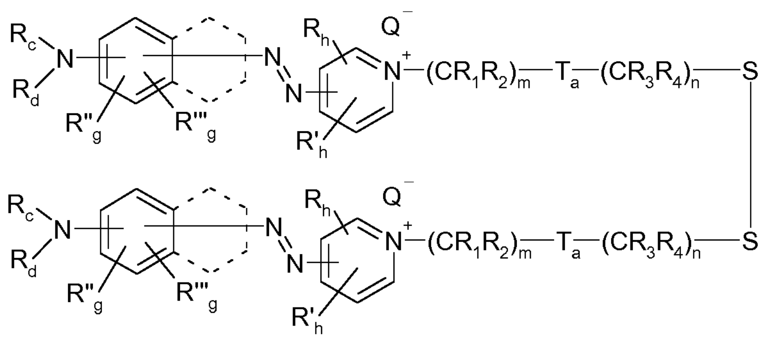

**(Xc)**

This reaction is carried out in a manner known to those skilled in the art, in a polar solvent, preferably a protic polar solvent, such as alcohols. The precursor bearing the nucleofugal group can be easily obtained according to the first steps of the processes previously described.

If the process has recourse to diazotization of an aminopyridinium or aminoquinolinium followed by coupling, the coupler chosen may be a phenol, that can be subsequently alkylated according to conditions known to those skilled in the art.

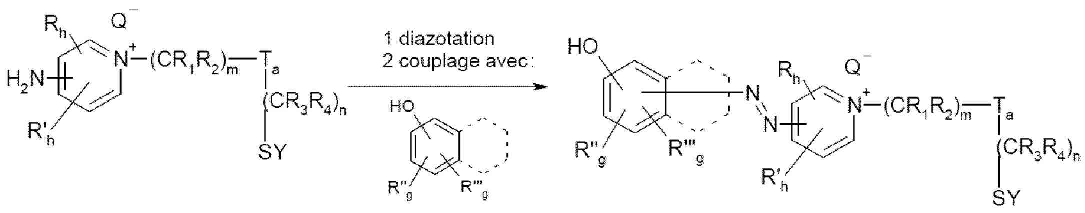


(k) (X'c)

(X'c)

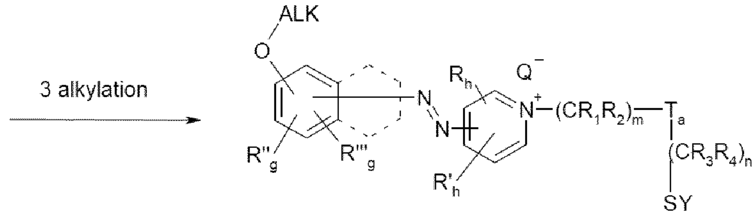


(X'a)

with $R_h$, $R'_h$, $R''_g$, $R'''_g$, m, n, $T_a$, $R_1$, $R_2$, $R_3$, $R_4$, Q, $Q^-$, Y, $R_c$, $R_d$ and ALK as defined above.

According to another embodiment, the process for synthesizing the compounds of formula (VI) used in the invention can consist in carrying out the following steps:

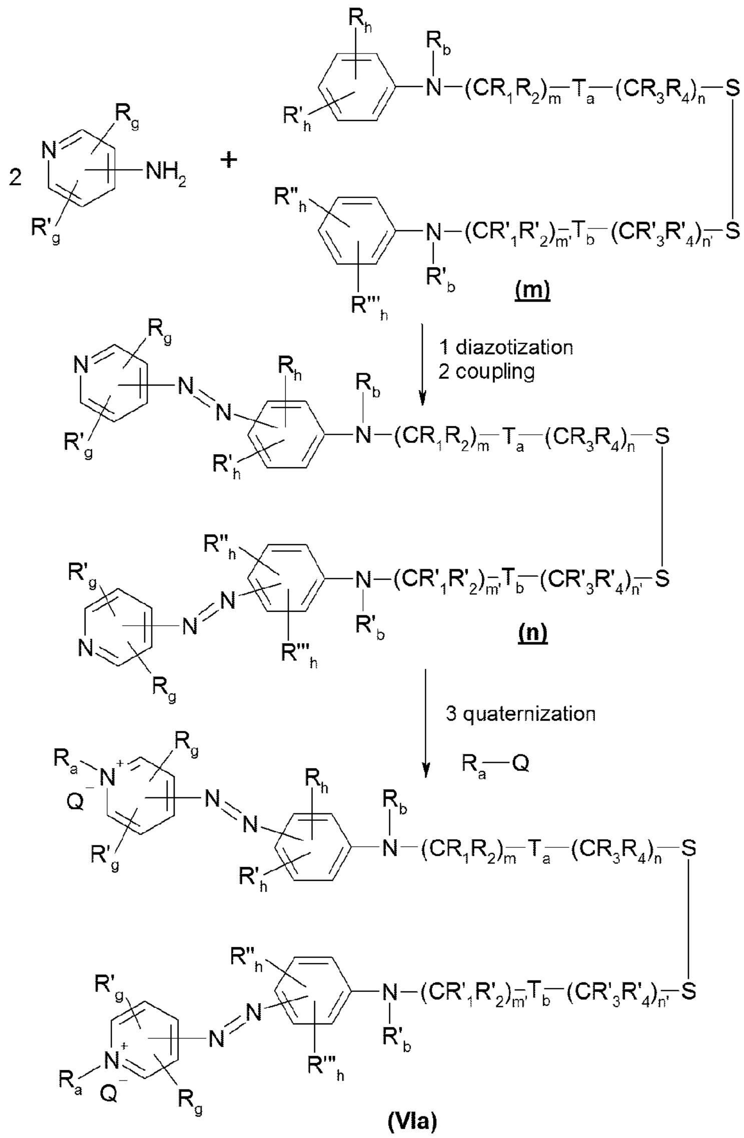


**(VIa)**

with $R_h$, $R'_h$, $R_g$, $R'_g$, m, n, m', n', $T_a$, $T_b$, $R_1$, $R_2$, $R_3$, $R_4$, $R'_1$, $R'_2$, $R'_3$, $R'_4$, Q, et $Q^-$ as defined above.

According to this process, a first step of diazotization of an amino pyridine or quinoline is carried out in a manner known to those skilled in the art. The conditions for carrying out such a step have been summarized previously.

Once the reaction has been carried out, coupling of the product obtained with a compound of the disulfide type **(m)** is carried out so as to give the compound **(n)**.

Conventionally, this reaction is carried out in the presence of a solvent which may be that of the preceding step.

The temperature is conventionally between -15°C and 30°C; preferably between - 10°C and 20°C, at a pH preferably of between 0 and 8.

The product **(n)** may be isolated via the techniques known to those skilled in the art (precipitation, evaporation, etc.).

The product **(n)** is then quaternized in the usual way (see for example Synth. Comm. 2005, 35(23), 3021-3026; EP1386916; Synthesis, 1986, 5, 382-383; Liebigs Annalen der Chemie, 1987, 1, 77-79); Bull. Chem. Soc. Jap. 1977, 50(6), 1510-1512; J. Org. Chem. 1979, 44(4), 638-639. For example, the product obtained can be brought into contact with an alkyl sulfate such as dimethyl sulfate, diethyl sulfate or dipropyl sulfate or an alkyl halide or alkylaryl halide such as iodomethane, iodoethane, 2-bromoethanol or benzyl bromide in the presence of a polar or non-polar, protic or aprotic solvent such as dichloromethane, toluene, ethyl acetate or water at spontaneous or alkaline pH. The product obtained can also be brought into contact with dialkyl carbonate such as dimethyl carbonate or diethyl carbonate in the presence of a base. Usually, the temperature is between 10 and 180°C, preferably between 20°C and 100°C.

According to another embodiment, the process for synthesizing the compounds used in the invention can consist in carrying out the following steps:

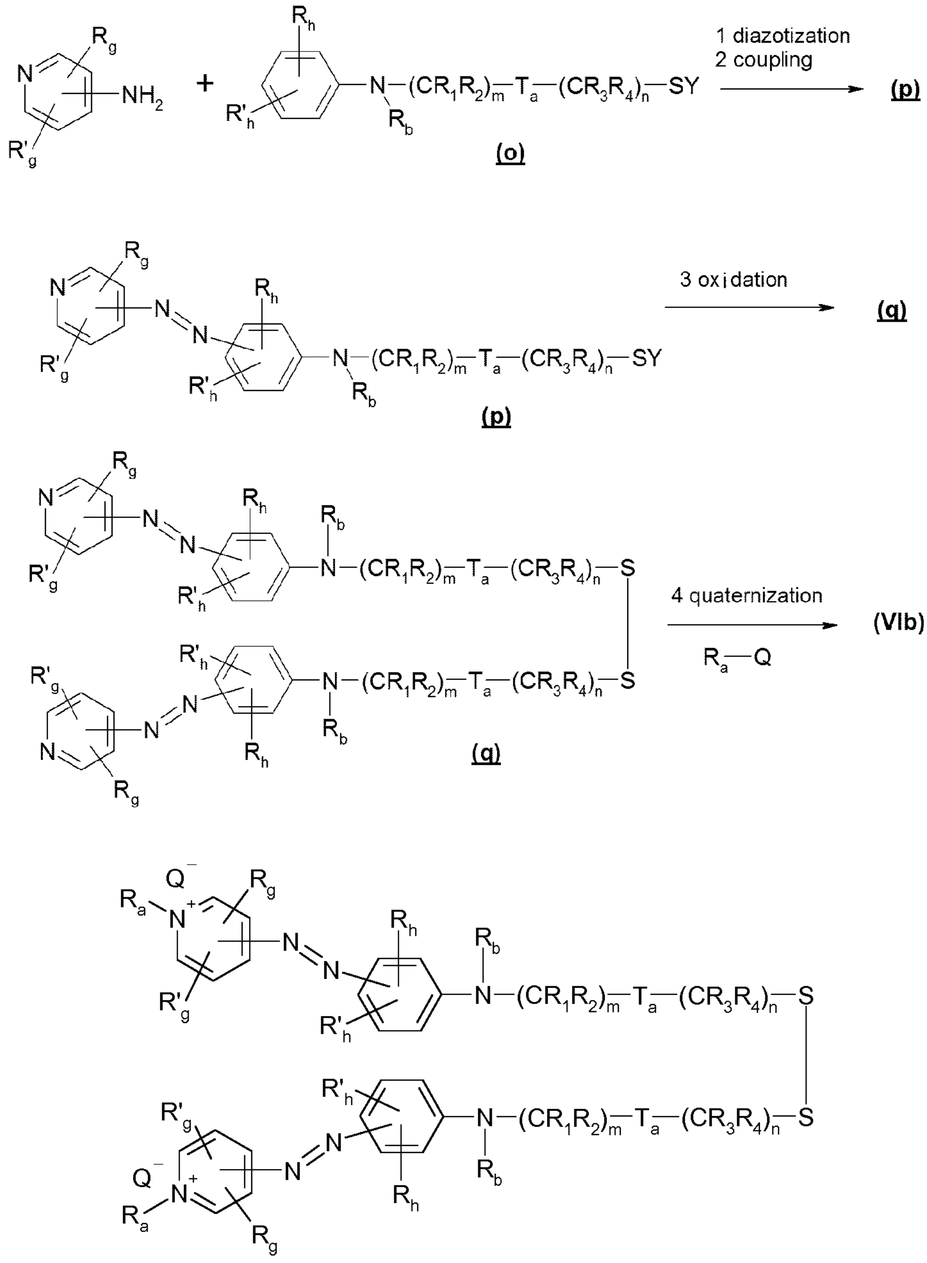


(VIb)

with $R_h$, $R'_h$, $R_g$, $R'_g$, m, n, $T_a$, $R_1$, $R_2$, $R_3$, $R_4$, Y, Q, and $Q^-$ as defined above.

According to this process, a first step of diazotization of an amino pyridine or quinoline is carried out in a manner known to those skilled in the art, so as to give the compound **(p).**

Once the reaction has been carried out, coupling of the product obtained **(p)** with a compound of the thiol or protected-thiol type **(o)** is carried out so as to give the compound **(q)**.

Once the reaction has been carried out, if Y represents a protective group, deprotection is carried out followed by oxidation of the thiol, and if Y represents a hydrogen atom the oxidation is carried out directly, so as to give the compound according to the invention **(VIb).** Usually, the temperature is between 10 and 180°C, preferably between 20°C and 100°C.

The resulting product is then quaternized in the usual way.

The conditions for carrying out all the steps of this process have been summarized previously. At each step of this synthesis, the intermediate, thus the final product, may be isolated via the techniques known to those skilled in the art (precipitation, evaporation, etc.).

According to another embodiment, the process for synthesizing the compounds used in the invention can consist in carrying out the following steps:

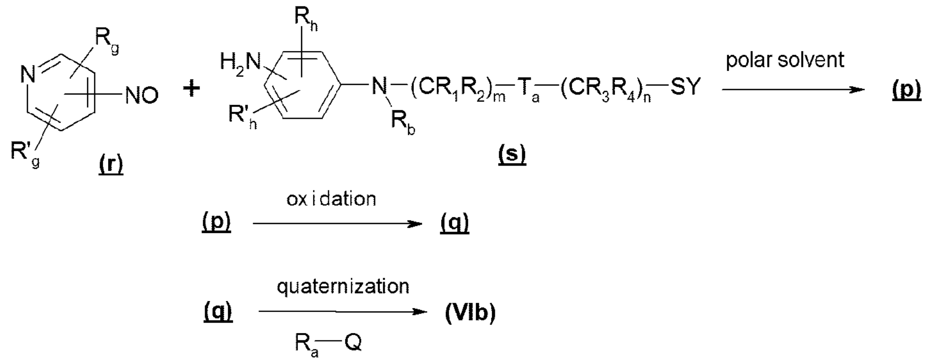


with $R_h$, $R'_h$, $R_g$, $R'_g$, m, n, $T_a$, $R_1$, $R_2$, $R_3$, $R_4$, Y, Q, and $Q^-$ as defined above.

According to this process, the azo compound is prepared by reaction of a nitroso compound **(r)** and of an aromatic amine **(s)** in a polar solvent such as $Ac_2O$. This process is known to those skilled in the art and described in J. Hetero. Chem 21(2), 501-3, 1984. The conditions for carrying the other steps of the synthesis have been summarized previously.

A variant of this synthesis is the use of a disulfide compound **(t)** with 2 equivalents of nitroso reagent **(r)**:

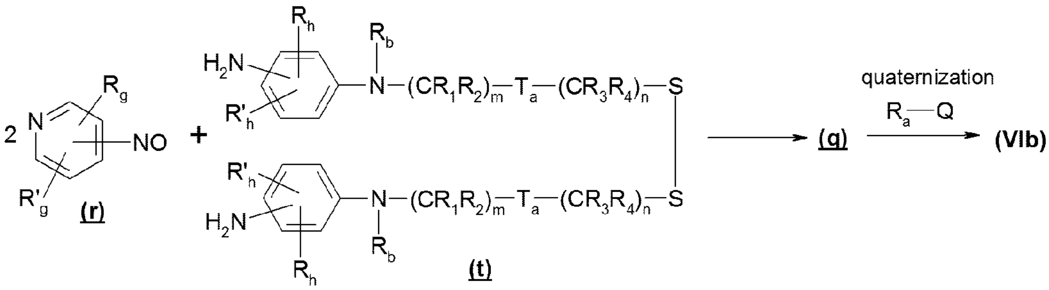


with $R_h$, $R'_h$, $R_g$, $R'_g$, m, n, $T_a$, $R_1$, $R_2$, $R_3$, $R_4$, Y, Q, and $Q^-$ as defined above.

A variant of this synthesis route is the use of a hydroxylamine compound **(r')**:

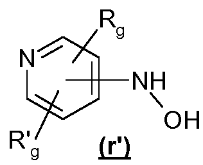

which can react with **(s)** or **(t)** as defined above, so as to also give the compounds **(q)** and **(VIb)** as defined above. This process is known to those skilled in the art and also described in J. Hetero. Chem 21(2), 501-3, 1984.

According to another embodiment, the process for synthesizing the compounds used in the invention can consist in carrying out the following steps:

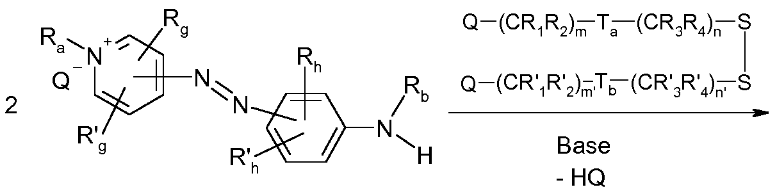


**(VIb)**

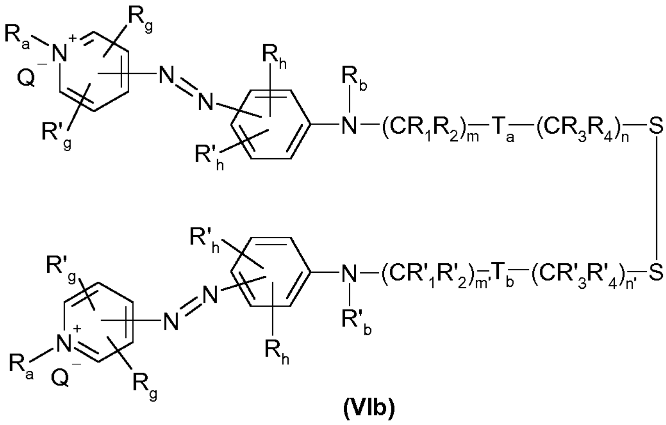

**(VIb)**

with $R_h$, $R'_h$, $R_g$, $R'_g$, m, n, m', n', $T_a$, $T_b$, $R_1$, $R_2$, $R_3$, $R_4$, $R'_1$, $R'_2$, $R'_3$, $R'_4$, Q, and $Q^-$ as defined above.

According to another embodiment, the process for synthesizing the compounds used in the invention can consist in carrying out the following steps:

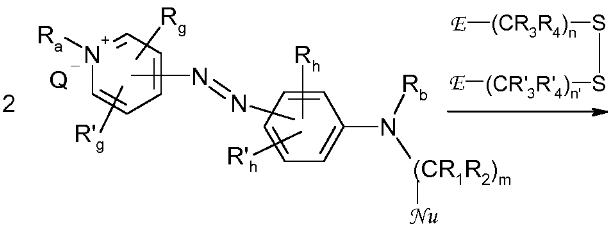

**(VIc)**

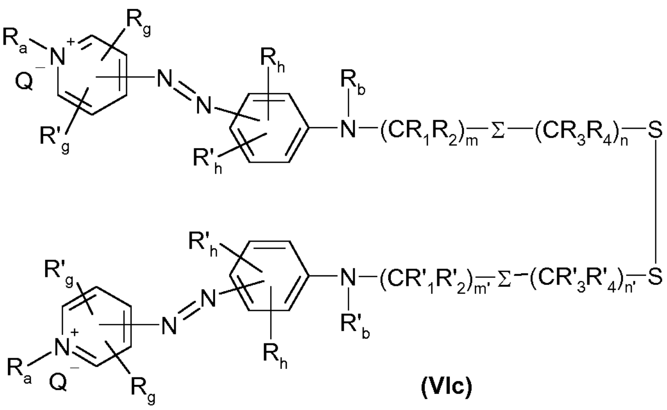

**(VIc)**

with $R_h$, $R'_h$, $R_g$, $R'_g$, m, n, m', n', $T_a$, $T_b$, $R_1$, $R_2$, $R_3$, $R_4$, $R'_1$, $R'_2$, $R'_3$, $R'_4$, Q, and $Q^-$ as defined above.

$\mathcal{Nu}$ represents a nucleophilic group; $\mathcal{E}$ represents an electrophilic group; $\Sigma$ the bond generated after an attack of the nucleophile on the electrophile; the combination of the group -$\Sigma$- is contained by the bond $T_a$ or $T_b$ as defined above. By way of example, the covalent bonds $\Sigma$ that may be generated are listed in the table below, starting with condensation of electrophiles with nucleophiles:

| Electrophiles $\mathcal{E}$ | Nucleophiles $\mathcal{Nu}$ | Covalent bonds $\Sigma$ |
|---|---|---|
| Activated esters* | Amines | Carboxamides |

(continued)

| | | |
|---|---|---|
| Acyl azides** | Amines | Carboxamides |
| Acyl halides | Amines | Carboxamides |
| Acyl halides | Alcohols | Esters |
| Acyl cyanides | Alcohols | Esters |
| Acyl cyanides | Amines | Carboxamides |
| Alkyl halides | Amines | Alkylamines |
| Alkyl halides | Carboxylic acids | Esters |
| Alkyl halides | Thiols | Thioesters |
| Alkyl halides | Alcohols | Ethers |
| Sulfonic acids and salts thereof | Thiols | Thioethers |
| Sulfonic acids and salts thereof | Carboxylic acids | Esters |
| Sulfonic acids and salts thereof | Alcohols | Ethers |
| Anhydrides | Alcohols | Esters |
| Anhydrides | Amines | Carboxamides |
| Aryl halides | Thiols | Thioethers |
| Aryl halides | Amines | Arylamines |
| Aziridines | Thiols | Thioethers |
| Carboxylic acids | Amines | Carboxamides |
| Carboxylic acids | Alcohols | Esters |
| Carbodiimides | Carboxylic acids | N-acylureas |
| Diazoalkanes | Carboxylic acids | Esters |
| Epoxides | Thiols | Thioethers |
| Haloacetamides | Thiols | Thioethers |
| Imide esters | Amines | Amidines |
| Isocyanates | Amines | Ureas |
| Isocyanates | Alcohols | Urethanes |
| Isothiocyanates | Amines | Thioureas |
| Maleimides | Thiols | Thioethers |
| Sulfonic esters | Amines | Alkylamines |
| Sulfonic esters | Thiols | Thioethers |
| Sulfonic esters | Carboxylic acids | Esters |
| Sulfonic esters | Alcohols | Ethers |
| Sulfonyl halides | Amines | Sulfonamides |
| *the activated esters or general formula -CO-GP with GP representing a leaving group such as oxysuccinimidyl, oxybenzotriazolyl or aryloxy, optionally substituted; **the acyl azides can rearrange to give isocyanates. | | |

By way of non-limiting indication, a dye with a link containing carboxamido groups is synthesized by reacting an azo compound containing a nucleophilic amino group with a compound containing two acyl halide groups.

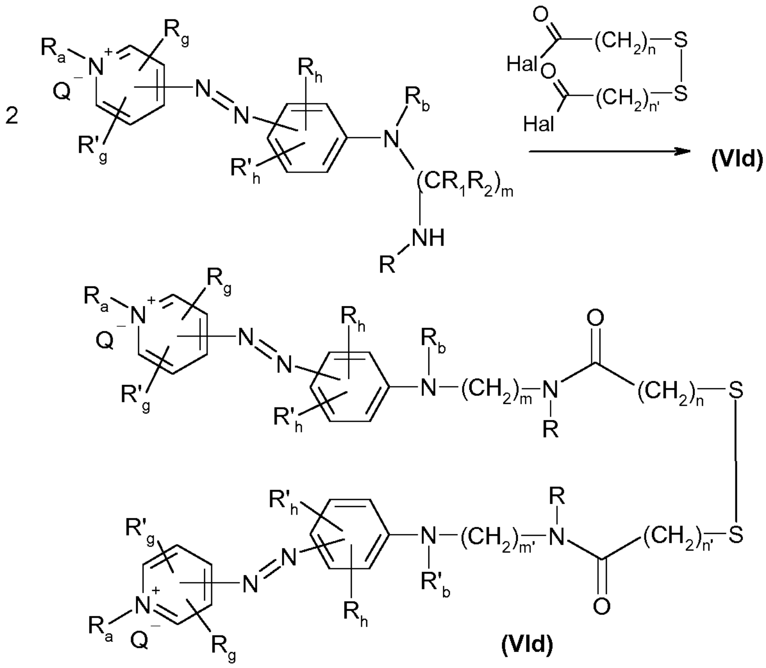

(VId)

(VId)

with $R_h$, $R'_h$, $R_g$, $R'_g$, m, n, m', n', Q, et $Q^-$ as defined above and Hal represents a halogen atom, preferably Cl or Br.

This reaction is carried out in a manner known to those skilled in the art, in a polar solvent, preferably alcohols in the presence of a base such as triethylamine. The final product can be isolated by techniques known to those skilled in the art (precipitation, evaporation, etc.) and the precursors can be easily obtained according to the first steps of the processes previously described.

According to another embodiment, a disulfide/thiol dye according to the invention can be obtained by aromatic nucleophilic substitution on an azo dye bearing a pyridinium or quinolinium unit bearing a nucleofugal group, for example a halogen or an alkyloxy, with an amino thiol or protected-thiol reagent **(v)** or an amino disulfide reagent **(u)** (for example cysteamine, cysteine):

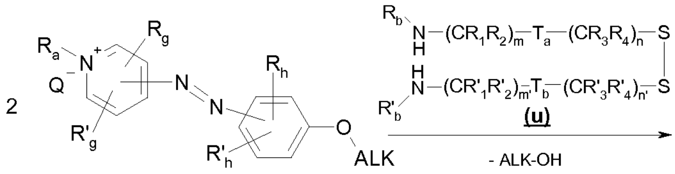


(VIb)

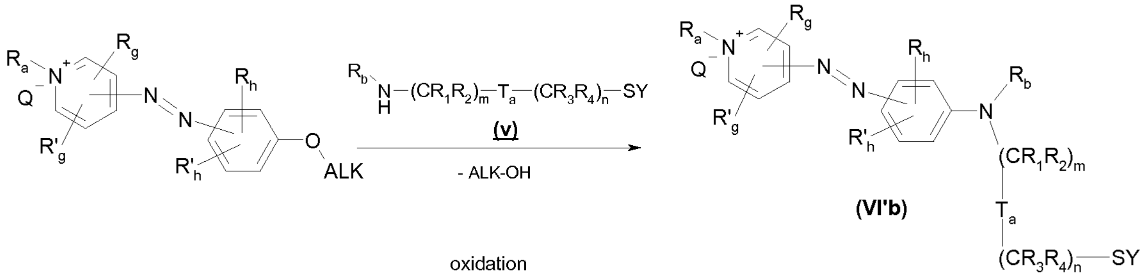


(VI'b)

(VI'b) oxidation (VId)

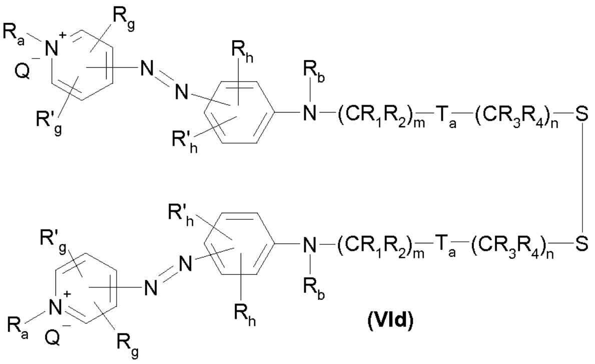

(VId)

with $R_h$, $R'_h$, $R_g$, $R'_g$, m, n, m', n', $T_a$, $T_b$, $R_1$, $R_2$, $R_3$, $R_4$, $R'_1$, $R'_2$, $R'_3$, $R'_4$, Y, Q, and $Q^-$ as defined above.

This reaction is carried out in a manner known to those skilled in the art, in a polar solvent, preferably a protic polar solvent, such as $(C_1\text{-}C_6)$alkanols, in particular ethanol. The precursor bearing the nucleofugal group ALK-O-, with ALK as defined above, can be easily obtained according to the first steps of the processes previously described. If the process has recourse to diazotization of an aminopyridine or aminoquinoline followed by coupling, the coupler chosen may be a phenol, that can be subsequently alkylated, in particular methylated, according to conditions known to those skilled in the art.

The protected-thiol dye compounds **(I)** in which **Y** is other than a hydrogen atom can be synthesized in two steps. The first step consists in preparing the non-protected-thiol dye **(I-H)** according to methods known to those skilled in the art, for instance "Thiols and organic Sulfides", "Thiocyanates and Isothiocyanates, organic", Ullmann's Encyclopedia, Wiley-VCH, Weinheim, 2005. Furthermore the second step consists in protecting the thiol function according to conventional methods known to those skilled in the art, so as to give the protected-thiol dyes of formula **(I-Y).** By way of example, to protect the thiol function -SH of the thiol dye, it is possible to use the methods in the books "Protective Groups in Organic Synthesis", T. W. Greene, John Wiley & Sons ed., NY, 1981, pp.193-217; and "Protecting Groups", P. Kocienski, Thieme, 3rd ed., 2005, chap. 5.

This method can be illustrated by the method consisting i) in generating thiol dyes of formulae **(I-H)** and **(I'-H)** by reduction of a heterocyclic two-chromophore dye bearing a disulfide function -S-S-, such as **(I-S)** , and ii) in protecting, according to conventional methods, said thiol function of **(I-H)** and **(I'-H)** with the reagent (u) Y'R in order to obtain the protected-thiol dyes of formulae **(I-Y)** and **(I'-Y).** The thiol compounds **(I-H)** and **(I'-H)** can also be metallized with an alkali metal or alkaline-earth metal Met* so as to give the thiolate dye of formulae **(I-Mét*)** and **(I'-Mét*).**

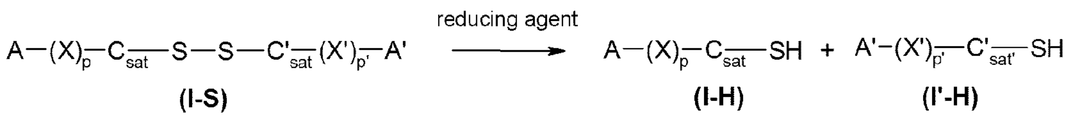


(I-S) (I-H) (I'-H)

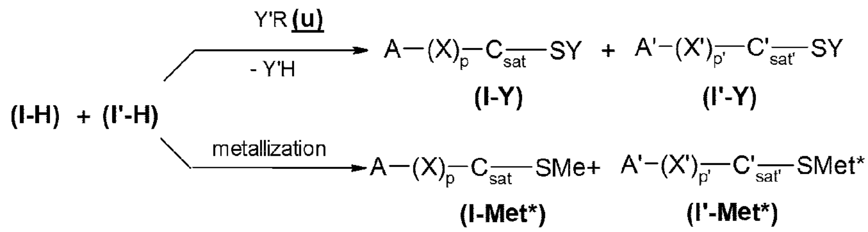


(I-H) + (I'-H)

(I-Y) (I'-Y)

(I-Met*) (I'-Met*)

with A, A', X, X', p, p', $C_{sat}$ and $C'_{sat}$ as defined above, Y' representing a thiol-function-protecting group; Met* representing an alkali metal or alkaline-earth metal, particularly sodium or potassium, it being understood that, when the metal is an alkaline-earth metal, 2 chromophores bearing a thiolate function $-S^-$ may be combined with 1 $Metal^{2+}$; R represents a nucleofugal leaving group, for instance mesylate, tosylate, triflate or halide.

Reference may be made to the book Advanced Organic Chemistry, "Reactions, Mechanisms and Structures", J. March, 4th Ed, John Wiley & Sons, 1992 or T. W. Greene "Protective Groups in Organic Synthesis", for further details on the operating conditions implemented for the processes mentioned above.

The thiol dyes formed can be converted into -S Y' protected-thiol dyes by protection of the thiol -SH using conventional protective groups. The thiol dyes are metallized by likewise using conventional methods known to those skilled in the art, such as those described in Advanced Organic Chemistry, Reactions, Mechanisms and Structures", J. March, 4th Ed, John Wiley & Sons, NY, 1992.

The protected-thiol dyes can be deprotected via conventional routes, such as those described in the books "Protective Groups in Organic Synthesis", T. W. Greene, John Wiley & Sons ed., NY, 1981; "Protecting Groups ", P. Kocienski, Thieme, 3rd ed., 2005.

The starting reagents are commercially available or obtainable via conventional methods known to those skilled in the art. By way of example, mention may be made of the document US 4 579 949.

*1.3. The composition of the dyeing process*

The dye(s) bearing a disulfide, thiol or protected-thiol function in particular of formula **(I)** as defined above may be applied directly to keratin fibres in powder form or may be in a liquid composition.

The dye composition that is useful then contains, in a cosmetically acceptable medium, an amount of the dyes bearing a disulfide, thiol or protected-thiol function as defined above in particular of formula **(I)** as defined above, generally of between 0.001% and 30% relative to the total weight of the composition.

Preferably, the amount of dyes bearing a disulfide, thiol or protected-thiol function as defined above, in particular of formula **(I),** is between 0.01% and 5% by weight relative to the total weight of the composition. By way of example, the dye(s) are in an amount of between 0.01% and 2%.

Preferably, the composition of the dyeing process of the invention is in liquid form and contains one or more cationic direct dyes of formula **(I)** bearing a disulfide function as defined above.

*The medium:*

The medium that is suitable for dyeing, also known as the dye support, is a cosmetic medium generally formed from water or a mixture of water and one or more organic solvents or a mixture of organic solvents.

The term "organic solvent" is intended to mean an organic substance capable of dissolving another substance without chemically modifying it.

*1.2.1 The organic solvents:*

Mention may be made, as organic solvent, for example, of lower $C_1$-$C_4$ alkanols, such as ethanol and isopropanol, polyols and polyol ethers, such as 2-butoxyethanol, propylene glycol, propylene glycol monomethyl ether, diethylene glycol monoethyl ether or diethylene glycol monomethyl ether, and also aromatic alcohols, such as benzyl alcohol or phenoxyethanol, and mixtures thereof.

The solvents are preferably present in proportions preferably of between 1% and 40% by weight approximately and even more preferably between 5% and 30% by weight approximately relative to the total weight of the dye composition.

*1.2.2 The adjuvants:*

The composition comprising the dye(s) bearing a disulfide, thiol or protected-thiol function in particular of formula **(I)** as defined above of the process of the invention may also contain various adjuvants conventionally used in hair dye compositions, such as anionic, cationic, nonionic, amphoteric or zwitterionic surfactants or mixtures thereof, anionic, cationic, nonionic, amphoteric or zwitterionic non-thiol and siliceous polymers or mixtures thereof, mineral or organic thickeners, and in particular anionic, cationic, nonionic and amphoteric polymeric associative thickeners, antioxidants, penetrants, sequestrants, fragrances, buffers, dispersants, conditioning agents, for instance volatile or non-volatile, modified or unmodified silicones, film-forming agents, ceramides, preserving agents and opacifiers.
The above adjuvants are generally present in an amount, for each of them, of between 0.01% and 20% by weight, with respect to the weight of the composition.
Needless to say, those skilled in the art will take care to select this or these optional additional compound(s) such that the advantageous properties intrinsically associated with the dye composition in accordance with the invention are not, or are not substantially, adversely affected by the envisaged addition(s).

*1.2.3 The additional dyes:*

The composition comprising the dye(s) bearing a disulfide, thiol or protected-thiol function in particular of formula **(I)** as defined above of the process of the invention may also contain one or more additional direct dyes other than the disulfide, thiol or protected-thiol direct dyes of formula **(I)** according to the invention. These direct dyes are chosen, for example, from those conventionally used in direct dyeing, among which may be mentioned all the commonly used aromatic and/or non-aromatic dyes, such as neutral, acidic or cationic nitrobenzene direct dyes, neutral, acidic or cationic azo direct dyes, natural direct dyes, neutral, acidic or cationic quinone and in particular anthraquinone direct dyes, azine, triarylmethane or indoamine direct dyes, methines, styryls, porphyrins, metalloporphyrins, phthalocyanines, methinecyanines and fluorescent dyes.
Mention may be made, among the natural direct dyes, of lawsone, juglone, alizarin, purpurin, carminic acid, kermesic acid, purpurogallin, protocatechualdehyde, indigo, isatin, curcumin, spinulosin, apigenidin or orceins. Use may also be made of extracts or decoctions comprising these natural dyes and in particular henna-based poultices or extracts.
According to the invention, the additional direct dye(s) used according to the invention preferably represent from 0.001% to 10% by weight approximately relative to the total weight of the dye composition comprising the dye(s) bearing a disulfide, thiol or protected-thiol function in particular of formula **(I)** as defined above and even more preferentially from 0.05% to 5% by weight approximately.
The composition comprising the dye(s) bearing a disulfide, thiol or protected-thiol function in particular of formula **(I)** as defined above of the process of the invention may also contain one or more oxidation bases and/or one or more couplers conventionally used for the dyeing of keratin fibres.
Among the oxidation bases, mention may be made of para-phenylenediamines, bis(phenyl)alkylenediamines, para-aminophenols, bis-para-aminophenols, ortho-aminophenols and heterocyclic bases, and the addition salts thereof.
Mention may in particular be made, among these couplers, of meta-phenylenediamines, meta-aminophenols, meta-diphenols, naphthalene couplers, heterocyclic couplers and their addition salts.
The coupler(s) are each generally present in an amount of between 0.001% and 10% by weight and preferably between 0.005% and 6% by weight relative to the total weight of the dye composition.
The oxidation base(s) present in the dye composition are each generally present in an amount of between 0.001% and 10% by weight and preferably between 0.005% and 6% by weight relative to the total weight of the dye composition.
In general, the addition salts of the oxidation bases and couplers that can be used in the context of the invention are in particular chosen from the salts of addition with an acid, such as the hydrochlorides, hydrobromides, sulfates, citrates, succinates, tartrates, lactates, tosylates, benzenesulfonates, phosphates and acetates, and the salts of addition with a base, such as alkali metal hydroxides, for instance sodium hydroxide, potassium hydroxide, ammonia, amines or alkanolamines.
According to one particular embodiment, the composition of the process of the invention contains at least one oxidation base and optionally at least one coupler as defined above.
The process of the invention may also use another composition that comprises one or more chemical oxidizing agents. The term "chemical oxidizing agent" is intended to mean chemical oxidizing agents other than atmospheric oxygen.
The chemical oxidizing agent may be chosen, for example, from hydrogen peroxide, urea peroxide, alkali metal bromates such as sodium bromate, persalts such as perborates and persulfates, and enzymes such as peroxidases and two-electron or four-electron oxidoreductases, for instance uricases, and four-electron oxidases such as laccases.
The use of hydrogen peroxide is particularly preferred.

The content of oxidizing agent is generally between 1% and 40% by weight relative to the weight of the composition and preferably between 1% and 20% by weight relative to the weight of the composition.

*1.2.4 The pH:*

The pH of the composition comprising the dye(s) bearing a disulfide, thiol or protected-thiol function in particular of formula **(I)** as defined above according to the invention is generally between 2 and 12 approximately and preferably between 3 and 11 approximately. It can be adjusted to the desired value by means of acidifying or basifying agents regularly used in the dyeing of keratin fibres or alternatively using conventional buffer systems.

The pH of the composition is preferentially between 6 and 9.

Among the acidifying agents that may be mentioned, for example, are mineral or organic acids, for instance hydrochloric acid, orthophosphoric acid or sulfuric acid, carboxylic acids, for instance acetic acid, tartaric acid, citric acid and lactic acid, and sulfonic acids.

Among the basifying agents, examples that may be mentioned include aqueous ammonia, alkali metal carbonates, alkanolamines such as monoethanolamine, diethanolamine and triethanolamine, and also derivatives thereof, sodium hydroxide, potassium hydroxide and the compounds of formula ($\alpha$) below:

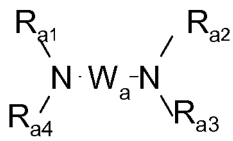
(α)

in which $W_a$ is a linear or branched, preferentially linear, divalent ($C_1$-$C_{10}$)alkylene group, optionally interrupted with one or more heteroatoms such as O, S and $NR_{a1}$ and/or optionally substituted with one or more hydroxyl groups; $R_{a1}$, $R_{a2}$, $R_{a3}$ and $R_{a4}$, which may be identical or different, represent a hydrogen atom or a $C_1$-$C_4$ alkyl or $C_1$-$C_4$ hydroxyalkyl radical; preferentially, $W_a$ represents a propylene group.

*1.2.5 Forms of the composition:*

The dye composition comprising the dye(s) bearing a disulfide, thiol or protected-thiol function in particular of formula **(I)** as defined above may be in various galenical forms, such as in the form of a liquid, a lotion, a cream or a gel, or in any other form that is suitable for dyeing keratin fibres. They may also be conditioned under pressure in an aerosol can in the presence of a propellant and form a mousse.

*1.4. Mode of application of the dyes (I) optionally in the presence of a heat source*

A subject of the invention is also a process for dyeing keratin materials, in particular keratin fibres such as the hair, using one or more dyes of formula (I) as defined above.

According to one particular embodiment of the invention, the process for dyeing keratin materials comprises i) the application to said materials, in particular keratin fibres such as the hair, of one or more dyes bearing a disulfide, thiol or protected-thiol function of formula **(I)** as defined above and ii) a heat source such as steam and/or an iron for straightening said fibres.

Process for treating keratin materials, in particular keratin fibres such as the hair, comprising the following steps:

i) applying to the hair fibres a dye composition comprising, in a cosmetic medium, one or more dyes bearing a disulfide, thiol or protected-thiol function of formula (I) as defined above,
ii) increasing the temperature of the hair fibres to a temperature of between 50 and 280°C, steps i) and ii) possibly being carried out simultaneously or sequentially.

After applying the dye composition, and before increasing the temperature of the hair fibres, said composition can be left on, generally for 30 seconds to 60 minutes, preferably 5 to 45 minutes. The process according to the invention comprises, preferably after the step of applying the dye composition, a step of increasing the temperature of the hair fibres to a temperature between 100 and 250°C. Preferably, the step of heating the keratin fibres is carried out at a temperature ranging from 150 to 220°C, preferably ranging from 160°C to 220°C, preferentially ranging from 160°C to 200°C, in particular ranging from 170°C to 190°C.

According to one particular embodiment of the invention, the temperature is increased by means of an iron.

For the purposes of the present invention, the term "iron" is intended to mean a device for heating keratin fibres

which places said fibres and the heating device in contact.

The irons that can be used may be curling irons, straightening irons, crimping irons or steam irons.

As examples of irons that may be used in the process according to the invention, mention may be made of any type of flat or round irons, and in particular, in a non-limiting manner, those described in patents US 4 103 145, US 4 308 878, US 5 983 903, US 5 957 140, US 5 494 058 and US 5 046 516.

The end of the iron which comes into contact with the keratin fibres generally has two flat surfaces. These two surfaces may be made of metal or ceramic. In particular, these two surfaces may be smooth or crimped or curved.

The iron may be applied by successive separate strokes lasting a few seconds or by gradual movement or sliding along the tresses of keratin fibres, in particular of hair.

In particular, the iron is applied in the process according to the invention by a continuous movement from the root to the tip of the hair, in one or more passes, in particular in two to twenty passes. The duration of each pass of the iron may last from 2 seconds to 1 minute.

Preferably, the iron is applied in the process according to the invention by a continuous movement from the root to the tip of the hair, in one or more passes.

The process according to the invention may also comprise an additional step of totally or partially pre-drying the hair fibres before the step of increasing the temperature, so as to prevent significant amounts of steam being given off, which might burn the stylist's hands and the subject's scalp. This pre-drying step can be carried out for example by means of a dryer, or of a hood at a temperature below 50°C or else by drying in the open air.

The temperature increase may be simultaneous with the application of the composition according to the invention.

Preferably, the increase in temperature follows the application of the composition according to the invention.

The above embodiments can be implemented using a device comprising a heat source combined with a reservoir of cosmetic composition containing an aqueous composition such as water. By way of example, such a conditioning and application device may comprise:

- a reservoir, which is heated or unheated,
- an aqueous cosmetic composition, preferably water,
- an application device,
- a heating means placed on either side of the application device which makes it possible to heat the aqueous cosmetic composition to the desired temperature, simultaneously with or after its application. The heating means can thus act as an applicator.

According to one embodiment of the invention, the treatment according to the invention is combined with an existing treatment of the fibre.

Thus, in a first step, permanent-waving or oxidation dyeing or bleaching or shampooing or a styling product or alkaline relaxing can be applied and, in a second step, the described process of the invention can be applied. The durability of the first treatment is thus reinforced.

According to one embodiment, the process according to the invention is carried out on natural keratin fibres, in particular natural hair.

According to one embodiment, the process according to the invention is carried out on damaged keratin fibres, in particular damaged hair. As indicated previously, the term "damaged hair" is intended to mean dry or coarse or brittle or split or limp hair.

In other words, the treatment process according to the invention is preferably performed on keratin fibres, in particular sensitized hair, such as bleached, relaxed or permanent-waved fibres.

The process according to the invention may be carried out on keratin fibres, in particular hair, which is dry or wet. Preferentially, the process is carried out on dry keratin fibres, in particular dry hair.

After application to the keratin fibres of the compound(s) **(I)** or of a cosmetic composition containing the same, and before performing the step of heating the keratin fibres, the compound(s) (I) or the composition containing the same may be left on for a time ranging from 1 to 60 minutes, preferably ranging from 2 to 50 minutes and preferentially ranging from 5 to 45 minutes. The leave-on period may take place at a temperature ranging from 15°C to 45°C, preferably at ambient temperature (25°C).

The cosmetic composition described previously is advantageously applied to the keratin fibres in an amount ranging from 0.1 to 10 grams and preferably from 0.2 to 5 grams of composition per gram of keratin fibres.

After application of the cosmetic composition to the keratin fibres, the latter may be wrung out to remove the excess composition or washed with water or with a shampoo.

The treatment process according to the invention may be performed before, during and/or after an additional process of cosmetic treatment of the keratin fibres, such as a process for temporarily shaping, such as shaping with curlers, a curling iron or a straightening iron, or a process for durably shaping, permanent-waving or relaxing the keratin

fibres. Preferably, the dyeing process of the invention implements a post-treatment step consisting in applying a heat source, preferably using an iron for straightening the keratin fibres and/or steam, in particular originating from a steam iron, i.e. from a device which combines steam and a straightening iron.

In particular, the treatment process according to the invention may be carried out on damaged keratin fibres. The treatment process according to the invention is preferably carried out on sensitized keratin fibres such as bleached, relaxed or permanent-waved fibres.

According to one particular embodiment of the invention, the step of applying or treating keratin fibres with steam is carried out extemporaneously with that of the application or treatment of the keratin fibres with one or more dyes bearing a disulfide, thiol or protected-thiol function of formula **(I)** as defined above.

According to another particular embodiment of the process of the invention, the treatment of fibres with one or more dyes bearing a disulfide, thiol or protected-thiol function of formula **(I)** as defined above and the step of treating the keratin fibres is performed in two stages. In a first step, the keratin fibres are treated with one or more dyes bearing a disulfide, thiol or protected-thiol function of formula **(I)** as defined above, and then, after a leave-on time, step ii) of straightening the keratin fibres with an iron is carried out without intermediate rinsing. In particular, the dye(s) bearing a disulfide, thiol or protected-thiol function in particular of formula **(I)** as defined above are in a dye composition as defined above in liquid form (point 1.2). The leave-on time after application of the composition containing the dyes of formula **(I)** is set at between 5 minutes and 2 hours. Preferentially it is between 15 minutes and 1 hour, such as 30 minutes.

The application of one or more dyes bearing a disulfide, thiol or protected-thiol function in particular of formula **(I)** as defined above is generally carried out at ambient temperature. It may, however, be carried out at temperatures ranging from 20 to 80°C and preferentially between 20 and 60°C, and the keratin fibres are then subjected to a treatment with steam.

The fibres may be treated with an iron for straightening keratin fibres assisted with steam. These irons are those that may be obtained commercially or those of professionals.

Another means is to arrange the keratin fibres treated beforehand with at least one dye bearing a disulfide, thiol or protected-thiol function in particular of formula **(I)** according to step i) as defined above, over a source of steam such as a kettle, a boiling water container or a steam iron, for example the commercially available irons such as Joico K-Pak ReconstRx Vapor Iron and Babyliss Pro230 steam.

The treatment time for the keratin fibres with the steam is between 5 minutes and 2 hours. Preferentially it is between 15 minutes and 1 hour, such as 30 minutes.

According to another process for dyeing keratin fibres, the composition that comprises at least one dye bearing a disulfide, thiol or protected-thiol function in particular of formula **(I)** as defined above is an aqueous composition, this composition being applied to the hair followed by application of a straightening iron that generates steam *in situ.*

According to one variant of the process for dyeing keratin fibres, the composition that comprises at least one dye bearing a disulfide, thiol or protected-thiol function in particular of formula **(I)** as defined above is applied to wet or moistened hair followed by application of a straightening iron that also generates steam *in situ.*

Preferentially, the process for dyeing keratin fibres does not use a reducing agent.

A treatment with a chemical oxidizing agent may optionally be combined as a post-treatment. Any type of oxidizing agent that is conventional in the field as described previously may be used. Thus, it may be chosen from hydrogen peroxide, urea peroxide, alkali metal bromates, persalts such as perborates and persulfates, and also enzymes, among which mention may be made of peroxidases, 2-electron oxidoreductases such as uricases, and 4-electron oxygenases such as laccases. The use of hydrogen peroxide is particularly preferred. The duration of the optional post-treatment with an oxidizing agent is between 1 second and 40 minutes. It is preferably between 1 and 10 minutes.

Preferentially, the chemical oxidizing agent(s), when they are present in the dyeing process of the invention, are in very mild concentrations, i.e. less than or equal to 5% by weight and preferentially 1% by weight relative to the total weight of the mixture comprising the dye(s) bearing a disulfide, thiol or protected-thiol function in particular of formula (I) as defined above and the chemical oxidizing agent(s). According to one particular embodiment of the invention, the dyeing process does not involve any chemical oxidizing agent.

The application of the composition may be performed on dry hair or may be preceded by moistening of the hair.

The examples that follow serve to illustrate the invention without, however, being limiting in nature.

The direct thiol, protected-thiol or disulfide dyes of formula **(I)** that are useful in the present invention are compounds that may be prepared according to methods known to those skilled in the art, in particular from the methods described in patent applications WO 2009/034059, FR 290 780, EP 1 647 580, EP 2 004 759, WO 2007/110 541, WO 2007/110 540, WO 2007/110 539, WO 2007/110 538, WO 2007/110 537, WO 2007/110 536, WO 2007/110 535, WO 2007/110 534, WO 2007/110 533, WO 2007/110 532, WO 2007/110 531, EP 2 070 988 and WO 2009/040 354.

**EXAMPLE**

Preparation examples:

Example of the synthesis of a dye derivatives of disulfide (or thiol) fatty chain type:

Synthesis scheme:

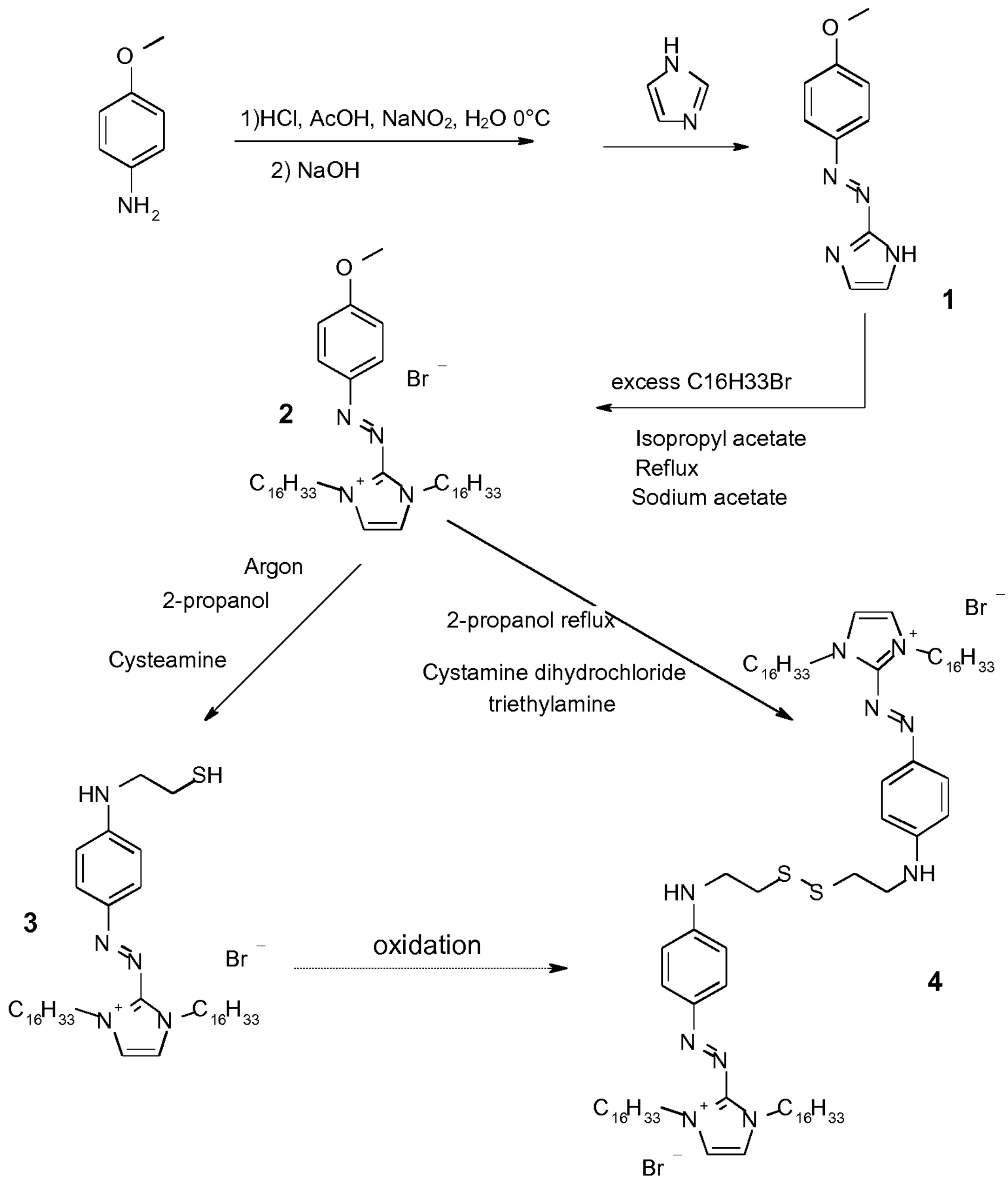


Synthesis of compound 1:

In a 250 ml three-necked flask equipped with a condenser, a thermometer, a nitrogen feed, a dropping funnel and a magnetic stirrer, 12.3 grams of 4-methoxyaniline (0.1 mol) were placed in 10 ml of acetic acid and 25 ml of 37% hydrochloric acid and 125 ml of water. The reaction medium was then cooled to 0°C by means of an ice bath, then a solution of 7.6 grams (0.11 mol) of sodium nitrite dissolved in 25 ml of water was added dropwise, in such a way as to

maintain a temperature of between 5°C and 0°C. After 30 minutes of stirring, 3 grams of urea were added in order to eliminate the excess sodium nitrite, then the mixture was kept stirring for 20 minutes at 0°C.

A solution of 6.8 grams of imidazole (0.1 mol) dissolved in 125 ml of water was added dropwise, in such a way as to maintain the temperature below 5° C, and also a 40% sodium hydroxide solution was added dropwise in such a way as to maintain the pH of the reaction medium between 9 and 8.5. The coupling reaction was thus obtained after having maintained the reaction medium at 10°C-15°C for 2 hours.

The reaction medium was then concentrated until a precipitate was obtained, which was filtered off over sintered glass, washed with water and then not totally dried.

The product obtained was then recrystallized from a dichloromethane/tetrachloromethane (5/1) mixture so as to obtain the pure expected product.

The NMR and mass spectra are in accordance with the expected chemical structure.

Synthesis of compound 2:

5.6 grams of compound 1 (0.0277 mol) were introduced into a 500 ml three-necked flask equipped with a condenser, a thermometer, a nitrogen feed and a magnetic stirrer, and then 4.5 grams of sodium acetate (0.0548 mol) and 100 ml of isopropyl acetate were added. 50 grams of bromohexadecane (0.164 mol) were then added and the reaction medium was then brought to reflux for 12 hours.

The progression of the reaction was monitored by thin-layer chromatography in order to monitor the disappearance of the starting product (compound 1) and the formation of the expected compound 2.

The mixture was allowed to return to ambient temperature and was then evaporated under reduced pressure. The residue was then taken up with acetone and the salts were then removed from the medium. This operation was performed 3 times. The acetone phase was again evaporated under reduced pressure, and then the residue was taken up with ethyl ether so as to cause the expected product to precipitate. A yellow powder was thus obtained after filtration and drying.

The NMR and mass spectra are in accordance with the expected chemical structure.

Synthesis of compound 3:

5 grams of compound 2 (0.00683 mol) were introduced into a 100 ml three-necked flask equipped with a condenser, a thermometer, a nitrogen feed and a magnetic stirrer, and then 0.8 gram of cysteamine (0.01037 mol) and 100 ml of 2-propanol were added. The reaction medium was maintained under an argon stream and was then brought to a T of 60°C. The progression of the reaction was monitored by thin-layer chromatography in order to monitor the disappearance of the starting product (yellow spot) and the formation of the expected product (red spot).

The mixture was allowed to return to ambient temperature and was then evaporated under reduced pressure so as to sublimate the excess cysteamine involved. The medium was always kept under argon. A red powder was thus obtained after filtration and drying.

The NMR and mass spectra are in accordance with the expected chemical structure.

Synthesis of compound 4:

0.77 gram of cystamine dihydrochloride (0.00342 mol) and 50 ml of 2-propanol were introduced into a 100 ml three-necked flask equipped with a condenser, a thermometer, a nitrogen feed and a magnetic stirrer. 0.7 gram of triethylamine (0.0069 mol) was then added and the mixture was kept stirring for 30 minutes at ambient temperature. 5 grams of compound 2 (0.00683 mol) were then added. The medium was then brought to reflux for 4 hours. The progression of the reaction was monitored by thin-layer chromatography in order to monitor the disappearance of the starting product (yellow spot) and the formation of the expected product (red spot).

The mixture was allowed to return to ambient temperature and was then evaporated under reduced pressure so as to obtain a red paste. This paste was subsequently dissolved in 50 ml of acetone, then cooled so as to cause the mineral salts (triethylamine hydrochloride) to precipitate and then again evaporated under reduced pressure. This operation was thus repeated 3 times, in order to eliminate the maximum amount of mineral salts.

Evaporation under reduced pressure was subsequently carried out so as to obtain a paste that was taken up with cold ethyl ether in order to cause the desired compound to precipitate.

The NMR and mass spectra are in accordance with the expected chemical structure.

<u>Evaluation of the compounds of the invention with regard to the care and repair of keratin fibres</u>

Dyeing of the fibre + Long-lasting care of the hair fibre. Dyeing of the hair fibre and Repair of the surface

condition of damaged hair, protection of the hair fibre with respect to high temperatures with long-lasting cosmetic effects.

*Application examples:*

The locks treated are locks of 2 grams of bleached Caucasian natural type which have a reducing solubility of 40; i.e. very damaged locks.

The products are dissolved in an amount of from 0.5% to 1% by weight in a mixture of ethanol (15%), benzyl alcohol (5%) and benzoic acid (0.5%), the remainder being made up (79.5%) with water.

The lock of hair is placed in an inclined channel to which 10 ml of a 1% solution of the compound to be studyed are added. The lock is thus maintained at ambient temperature for 20 minutes. It is then rinsed with water, then blow drying is applied, followed by 10 passes with a straightening iron at 210°C. A shampooing operation and 5 shampooing operations are carried out with a sensory evaluation after each shampooing operation.

The compounds studied are the following:

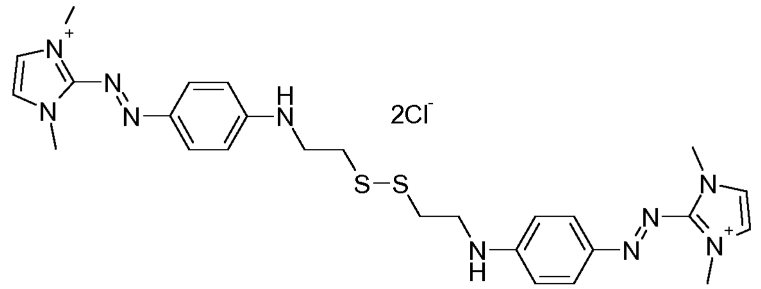

Comparatif A

vs.

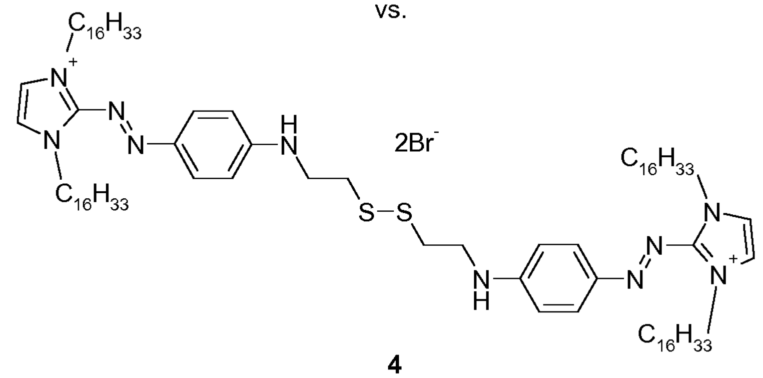

**4**

*Sensory evaluation:*

The locks treated with the compound according to the invention **4** with a straightening iron show greater coating than the locks treated with the comparative A and exhibit, when dry, a softness that is much more pronounced after 1 shampooing operation and all the greater after 5 shampooing operations, without however modifying the colouration of the hair in a visually observable manner.

The comparative carried out with a lock treated with A compared with a lock treated with the compound according to the invention **4,** optionally followed by a treatment with the iron, shows the importance of the presence of a fatty chain on the dye, and the importance of the straightening iron on the cosmetic effect observed and on the persistence of the effect. This was confirmed by the spectrocolorimetric measurements.

*Spectrocolorimetric evaluation:*

The colour of the locks was evaluated in the CIE L* a* b* system using a Minolta Spectrophotometer CM3610D colorimeter. In this L* a* b* system, the three parameters denote, respectively, the colour intensity (L*), the green/red colour axis (a*) and the blue/yellow colour axis (b*).

*Build-up of the colour:*

The variation in colouration between the non-dyed and dyed locks of hair are defined by (ΔE*) according to the following equation:

$$\Delta E^* = \sqrt{(L^* - L_o^*)^2 + (a^* - a_o^*)^2 + (b^* - b_o^*)^2}$$

In this equation, L*, a* and b* represent the values measured on locks of hair after dyeing and $L_0$*, $a_0$* and $b_0$* represent the values measured on locks of hair before dyeing. The higher the value of ΔE*, the greater the colour build-up.

*Chromaticity of the colour:*

The chromaticity is given by the following equation: $C^* = \sqrt{(a^*)^2 + (b^*)^2}$; the higher the value, the more chromatic the colour of the fibre.

Chromaticity C*

| Process | a* (D65) | b* (D65) | C* (D65) |
|---|---|---|---|
| **A** (comparative) | 19.64 | 10.98 | 22.50 |
| **4** | 37.70 | 23.14 | 44.23 |

It appears that the composition according to the invention which contains a disulfide dye bearing a $C_{10}$-$C_{30}$ aliphatic chain makes it possible to very significantly increase the chromaticity of the colour of keratin fibres.

Build-up of the colour: ΔE*build-up and power

| Process | L* (D65) | a* (D65) | b* (D65) | ΔE* build-up |
|---|---|---|---|---|
| **4** | 31.42 | 37.70 | 23.14 | 36.54 |
| **4** + steam iron | 27.97 | 32.84 | 19.52 | 36.85 |

It appears that the keratin fibres treated with a disulfide dye of the invention which contains a $C_{10}$-$C_{30}$ aliphatic chain makes it possible to obtain very intense colourations and with a very good build-up of the colour. Furthermore, the build-up does not significantly vary even after a steam iron and 5 shampooing operations. Specifically, a ΔE of 36.30 after 5 shampooing operations was obtained, while it is 36.54 without steam iron and 36.85 with steam iron.

## Claims

**1.** Process for dyeing keratin fibres, consisting in applying to said fibres one or more cationic direct dye(s) bearing a disulfide, thiol or protected-thiol function of formula **(I):**

$$\mathbf{A\text{-}(X)_p\text{-}C_{sat}\text{-}S\text{-}U} \qquad \mathbf{(I)}$$

salts thereof with an organic or mineral acid, optical or geometric isomers thereof, tautomers thereof, and solvates thereof;
in which formula **(I):**

- **U** represents a radical chosen from:

  *a)* **-S-C'$_{sat}$-(X')$_{p'}$-A';** and
  *b)* **-Y;**

- **A** and **A',** which may be identical or different, represent a cationic or non-cationic chromophore, preferably a

cationic chromophore, it being understood that at least one of the two chromophores bears at least one group bearing a linear or branched, saturated or unsaturated, $C_{10}$-$C_{30}$ aliphatic chain;
• **Y** represents i) a hydrogen atom or ii) a thiol-function protecting group;
• **X** and **X'**, which may be identical or different, represent a linear or branched, saturated or unsaturated, divalent $C_1$-$C_{30}$ hydrocarbon-based chain, optionally interrupted and/or optionally terminated at one or both of its ends with one or more divalent groups or combinations thereof chosen from:

➢ -N(R)-, $-N^+(R)(R)-$, -O-, -S-, -CO-, $-SO_2-$ with R, which may be identical or different, chosen from a hydrogen and a $C_1$-$C_4$ alkyl, hydroxyalkyl or aminoalkyl radical;

➢ an aromatic or non-aromatic, saturated or unsaturated, fused or non-fused (hetero)cyclic radical optionally comprising one or more heteroatoms, identical or different, optionally substituted;

• **p** and **p'**, which may be identical or different, are equal to 0 or 1;
• $\mathbf{C_{sat}}$ and $\mathbf{C'_{sat}}$, identical or different, represent an optionally cyclic, optionally substituted, linear or branched, $C_1$-$C_{18}$ alkylene chain.

2. Process according to the preceding claim, in which the dye(s) of formula **(I)** are disulfide dyes with **U** representing a radical *a)* $\mathbf{-S-C'_{sat}-(X')_{p'}-A'}$; in particular, the dyes of formula **(I)** according to the invention are disulfides and symmetrical, i.e. formula **(I)** has the following formula:

$$\mathbf{A-(X)_p-C_{sat}-S-S-C'_{sat}-(X')_{p'}-A'}$$

with $\mathbf{A = A', X = X', p = p', C_{sat} = C'_{sat}}$.

3. Process according to Claim 1, in which the dye(s) of formula **(I)** are dyes bearing a thiol or protected-thiol function, i.e. **U** representing the radical *b)* **Y** as defined in the preceding claim, in particular **Y** represents a hydrogen atom.

4. Dyeing process according to any one of the preceding claims, in which the dye(s) of formula **(I)** are dyes with $C_{sat}$ and $C'_{sat}$, which may be identical or different, representing a chain $-(CH_2)_k-$ with k being an integer between 1 and 8 inclusive.

5. Process according to any one of the preceding claims, in which the dye(s) of formula **(I)** are dyes which, when **p** and **p'** is equal to 1, **X** and **X',** which may be identical or different, represent the following sequence:

$$\mathbf{-(T)_t-(Z)_z-(T)_{t'}-}$$

said sequence being linked in formula **(I)** symmetrically as follows:

$$\mathbf{-\ C_{sat}(or\ C'_{sat})-(T)_t-(Z)_z-(A\ or\ A');}$$

in which:

• **T** and **T'**, which may be identical or different, represent one or more radicals or combinations thereof chosen from: -O-; -S-; -N(R)-; $-N^+(R)(R^\circ)-$; -S(O)-; $-S(O)_2-$;-C(O)-; with R, R°, which may be identical or different, representing a hydrogen atom, a $C_1$-$C_4$ alkyl radical, $C_1$-$C_4$ hydroxyalkyl radical or an aryl($C_1$-$C_4$)alkyl radical; and a cationic or non-cationic, preferentially monocyclic heterocycloalkyl or heteroaryl radical, preferentially containing two heteroatoms (more preferentially two nitrogen atoms) and preferentially being 5- to 7-membered, more preferentially imidazolium;
the indices **t and t',** which may be identical or different, are equal to 0 or 1;
• **Z** represents:

➢ $-(CH_2)_m-$ with m being an integer between 1 and 8;

➢ $-(CH_2CH_2O)_q-$ or $-(OCH_2CH_2)_q-$ in which q is an integer between 1 and 5 inclusive;

➢ an aryl, alkylaryl or arylalkyl radical in which the alkyl radical is $C_1$-$C_4$ and the aryl radical is preferably $C_6$, being optionally substituted with at least one group $SO_3M$ with M representing a hydrogen atom, an

alkali metal or an ammonium group substituted with one or more identical or different, linear or branched $C_1$-$C_{18}$ alkyl radicals optionally bearing at least one hydroxyl;

• **z** is 0 or 1.

6. Process for dyeing keratin fibres according to any one of the preceding claims, in which the dye(s) of formula **(I)** comprise a chromophore **A** and/or **A',** which may be identical or different, derived from the following acridine dyes:

acridones; anthranthrones; anthrapyrimidines; anthraquinones; azines; (poly)azos, hydrazono or hydrazones, in particular arylhydrazones; azomethines; benzanthrones; benzimidazoles; benzimidazolones; benzindoles; benzoxazoles; benzopyrans; benzothiazoles; benzoquinones; bisazines; bis-isoindolines; carboxanilides; coumarins; cyanins such as azacarbocyanins, diazacarbocyanins, diazahemicyanins, tetraazacarbocyanins or (poly)methines; diazines; diketopyrrolopyrroles; dioxazines; diphenylamines; diphenylmethanes; dithiazines; flavonoids such as flavanthrones and flavones; fluorindines; formazans; indamines; indanthrones; indigoids and pseudo-indigoids; indophenols; indoanilines; isoindolines; isoindolinones; isoviolanthrones; lactones; naphthoquinones; nitro, in particular nitro(hetero)aromatics; oxadiazoles; oxazines; perilones; perinones; perylenes; phenazines; phenoxazine; phenothiazines; phthalocyanin; polyenes/carotenoids; porphyrins; pyranthrones; pyrazolanthrones; pyrazolones; pyrimidinoanthrones; pyronines; quinacridones; quinolines; quinophthalones; squaranes; tetrazoliums; thiazines, thioindigo; thiopyronines; triarylmethanes, or xanthenes; preferentially, the chromophores are chosen from (poly)azo, hydrazono and azomethine chromophores, more preferentially from azo chromophores.

7. Process according to any one of the preceding claims, in which the dye(s) of formula **(I)** comprise a chromophore **A** and/or **A',** which may be identical or different, chosen from the following cationic chromophores:

**(*)-Het$^+$-C(R$^a$)=N-N(R$^b$)-Ar, Q$^-$** **(II)**

**Q$^-$, Het$^+$-C(R$^a$)=N-N(R$^b$)-Ar'-(*),** **(II')**

**(*)-Het$^+$-N(R$^a$)-N=C(R$^b$)-Ar, Q$^-$** **(III)**

**Q$^-$, Het$^+$-N(R$^a$)-N=C(R$^b$)-Ar'-(*),** **(III')**

**(*)-Het$^+$-N=N-Ar, Q$^-$** **(IV)**

**Q$^-$, Het$^+$-N=N-Ar'-(*),** **(IV')**

**(*)-Ar$^+$-N=N-Ar", Q$^-$** **(V)**

**Q$^-$, Ar$^+$-N=N-Ar"-(*)** **(V')**

formulae **(II)** to **(V')** with:

- **Het$^+$** representing a cationic heteroaryl radical, preferentially bearing an endocyclic cationic charge, such as imidazolium, indolium or pyridinium, optionally substituted, preferentially with at least one ($C_1$-$C_8$) alkyl group such as methyl;
- **Ar$^+$** representing an aryl radical, such as phenyl or naphthyl, bearing an exocyclic cationic charge, preferentially ammonium, particularly tri($C_1$-$C_8$)alkylammonium such as trimethylammonium;
- **Ar** represents an aryl group, in particular phenyl, which is optionally substituted, preferentially with one or more electron-donating groups such as i) optionally substituted ($C_1$-$C_8$)alkyl, ii) optionally substituted ($C_1$-$C_8$)alkoxy, iii) (di)($C_1$-$C_8$)(alkyl)amino optionally substituted on the alkyl group(s) with a hydroxyl group, iv) aryl($C_1$-$C_8$)alkylamino, v) optionally substituted *N*-($C_1$-$C_8$)alkyl-*N*-aryl($C_1$-$C_8$)alkylamino or alternatively **Ar** represents a julolidine group;
- **Ar'** is an optionally substituted divalent (hetero)arylene group such as phenylene, particularly para-phenylene, or naphthalene, which are optionally substituted, preferentially with one or more groups ($C_1$-$C_8$)alkyl, hydroxyl or ($C_1$-$C_8$)alkoxy;
- **Ar"** is an optionally substituted (hetero)aryl group such as phenyl or pyrazolyl, which are optionally substituted, preferentially with one or more groups ($C_1$-$C_8$)alkyl, hydroxyl, (di)($C_1$-$C_8$)(alkyl)amino, ($C_1$-$C_8$)alkoxy or phenyl;

- **$R^a$** and **$R^b$,** which may be identical or different, represent a hydrogen atom or a ($C_1$-$C_8$)alkyl group, which is optionally substituted, preferentially with a hydroxyl group;
or else the substituent **$R^a$** with a substituent of **$Het^+$** and/or **$R^b$** with a substituent of **Ar** form, together with the atoms that bear them, a (hetero)cycloalkyl;
particularly, **$R^a$** and **$R^b$** represent a hydrogen atom or a ($C_1$-$C_4$)alkyl group, which is optionally substituted with a hydroxyl group;
- **$Q^-$** represents an organic or mineral anionic counterion such as a halide or an alkyl sulfate;
- **(*)** represents the part of the chromophore linked to the rest of the molecule of formula **(I);**
- it being understood that at least one of the $Het^+$, $Ar^+$, Ar and Ar' groups bears at least one linear or branched, saturated or unsaturated $C_{10}$-$C_{30}$ aliphatic chain; preferably a linear or branched ($C_{10}$-$C_{30}$)alkyl group or a linear or branched ($C_{10}$-$C_{30}$)alkenyl group;

**$Het^+$-C($R^a$)=N-N($R^b$)-Ar, $Q^-$** **(II)**

**$Q^-$, $Het^+$-C($R^a$)=N-N($R^b$)-Ar'-(*),** **(II')**

**(*)-$Het^+$-N($R^a$)-N=C($R^b$)-Ar, $Q^-$** **(III)**

**$Q^-$, $Het^+$-N($R^a$)-N=C($R^b$)-Ar'-(*),** **(III')**

**(*)-$Het^+$-N=N-Ar, $Q^-$** **(IV)**

**$Q^-$, $Het^+$-N=N-Ar'-(*),** **(IV')**

**(*)-$Ar^+$-N=N-Ar", $Q^-$** **(V)** **(VI)**

**$Q^-$, $Ar^+$-N=N-Ar"-(*)** **(V)** **(VI)**

formulae **(II)** to **(V')** with:

- **$Het^+$** representing a cationic heteroaryl radical, preferentially bearing an endocyclic cationic charge, such as imidazolium, indolium or pyridinium, optionally substituted, preferentially with at least one ($C_1$-$C_8$) alkyl group such as methyl;
- **$Ar^+$** representing an aryl radical, such as phenyl or naphthyl, bearing an exocyclic cationic charge, preferentially ammonium, particularly tri($C_1$-$C_8$)alkylammonium such as trimethylammonium;
- **Ar** represents an aryl group, in particular phenyl, which is optionally substituted, preferentially with one or more electron-donating groups such as i) optionally substituted ($C_1$-$C_8$)alkyl, ii) optionally substituted ($C_1$-$C_8$)alkoxy, iii) (di)($C_1$-$C_8$)(alkyl)amino optionally substituted on the alkyl group(s) with a hydroxyl group, iv) aryl($C_1$-$C_8$)alkylamino, v) optionally substituted *N*-($C_1$-$C_8$)alkyl-*N*-aryl($C_1$-$C_8$)alkylamino or alternatively **Ar** represents a julolidine group;
- **Ar'** is an optionally substituted divalent (hetero)arylene group such as phenylene, particularly para-phenylene, or naphthalene, which are optionally substituted, preferentially with one or more groups ($C_1$-$C_8$)alkyl, hydroxyl or ($C_1$-$C_8$)alkoxy;
- **Ar"** is an optionally substituted (hetero)aryl group such as phenyl or pyrazolyl, which are optionally substituted, preferentially with one or more groups ($C_1$-$C_8$)alkyl, hydroxyl, (di)($C_1$-$C_8$)(alkyl)amino, ($C_1$-$C_8$)alkoxy or phenyl;
- **$R^a$** and **$R^b$,** which may be identical or different, represent a hydrogen atom or a ($C_1$-$C_8$)alkyl group, which is optionally substituted, preferentially with a hydroxyl group;
or else the substituent **$R^a$** with a substituent of **$Het^+$** and/or **$R^b$** with a substituent of **Ar** form, together with the atoms that bear them, a (hetero)cycloalkyl;
particularly, **$R^a$** and **$R^b$** represent a hydrogen atom or a ($C_1$-$C_4$)alkyl group, which is optionally substituted with a hydroxyl group;
- **$Q^-$** represents an organic or mineral anionic counterion such as a halide or an alkyl sulfate;
- **(*)** represents the part of the chromophore linked to the rest of the molecule of formula **(I);**
- it being understood that at least one of the $Het^+$, $Ar^+$, Ar and Ar' groups bears at least one linear or branched, saturated or unsaturated $C_{10}$-$C_{30}$ aliphatic chain; preferably a linear or branched ($C_{10}$-$C_{30}$)alkyl group or a linear or branched ($C_{10}$-$C_{30}$)alkenyl group;

in particular the cationic chromophores **(A)** and **(A')** bear azo and hydrazono endocyclic cationic charges of formula **(II)** to **(IV')** as defined above, more particularly the cationic chromophores **(A)** and **(A')** have the following formulae:

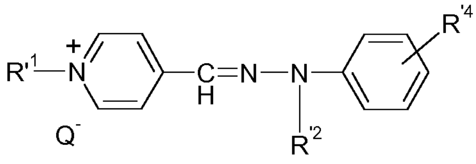

**(II-1)**

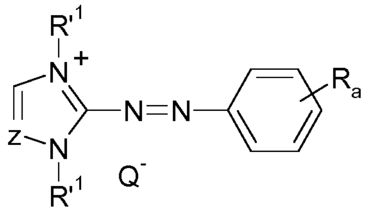

**(IV-1)**

formulae **(II-1)** and **(IV-1)** with:

- **R'1** and **R'2**, which may be identical or different, represent a linear or branched $(C_1\text{-}C_{30})$alkyl or linear or branched $(C_2\text{-}C_{30})$alkenyl group;
- $\mathbf{R_a}$ represents an amino group $NR'_2R'_3$ with $\mathbf{R'_2}$ and $\mathbf{R'_3}$, which may be identical or different, representing a hydrogen atom or a linear or branched $(C_1\text{-}C_{30})$alkyl or linear or branched $(C_2\text{-}C_{30})$alkenyl group; and
- **R'4** represents a hydrogen atom or an electron-donating group such as optionally substituted $(C_1\text{-}C_{30})$alkyl, optionally substituted $(C_1\text{-}C_{30})$alkoxy, or (di)$(C_1\text{-}C_{30})$(alkyl)amino optionally substituted on the alkyl group(s) with a hydroxyl group;
- **Z** represents a CH group or a nitrogen atom, preferentially CH,
- **Q'** is as defined above, particularly a halide such as chloride or an alkyl sulfate such as methyl sulfate or mesityl;

it being understood that:

- the chromophore **(II-1)** or **(IV-1)** is linked to the rest of the molecule of formula **(I)** by $\mathbf{R'^2}$, $\mathbf{R'^1}$, $\mathbf{R'^4}$ or $\mathbf{R_a}$, in which case one of the hydrogen atoms of $\mathbf{R'^2}$, $\mathbf{R'^1}$ or $\mathbf{R'^4}$ is substituted with **X** or **X'** if p = 1 or p'=1 or else with $\mathbf{C_{sat}}$ or $\mathbf{C_{sat'}}$ if p = 0 or p'=0, and
- at least one of the $R'^1$, $R'^2$, $R'^3$, $R'^4$ and $R_a$ groups bears at least one linear or branched, saturated or unsaturated $C_{10}\text{-}C_{30}$ aliphatic chain; preferably $R'^1$ represents a linear or branched $(C_{10}\text{-}C_{30})$alkyl group or a linear or branched $(C_{10}\text{-}C_{30})$alkenyl group;

more particularly, the chromophores **(A)** and **(A')** are derived from the following dyes:

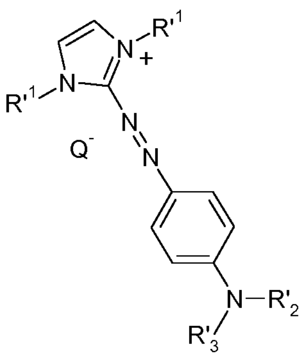

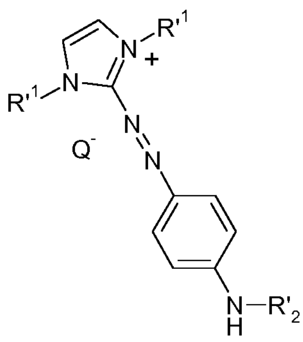

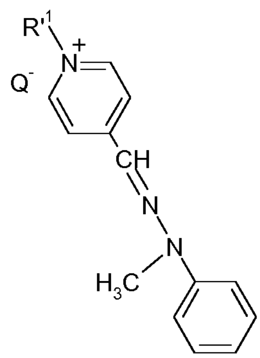

with:

- **Q'** being an anionic counterion as defined above, particularly a halide such as chloride or an alkyl sulfate such as methyl sulfate or mesityl,
- with $R'^1$ representing a linear or branched $(C_{10}\text{-}C_{30})$alkyl group or a linear or branched $(C_{10}\text{-}C_{30})$alkenyl group, preferably a linear $(C_{10}\text{-}C_{30})$alkyl group.

**8.** Process according to any one of the preceding claims, in which the dye(s) of formula **(I)** are disulfide dyes chosen from those having the following formulae:

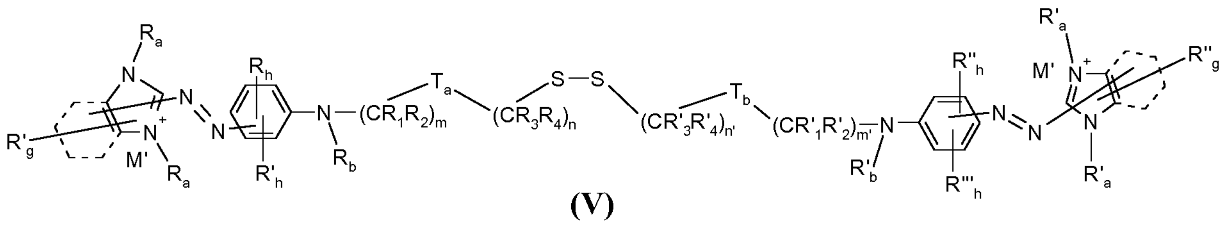

**(V)**

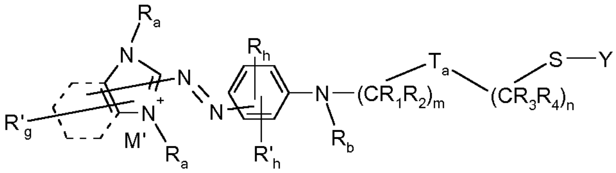

**(V')**

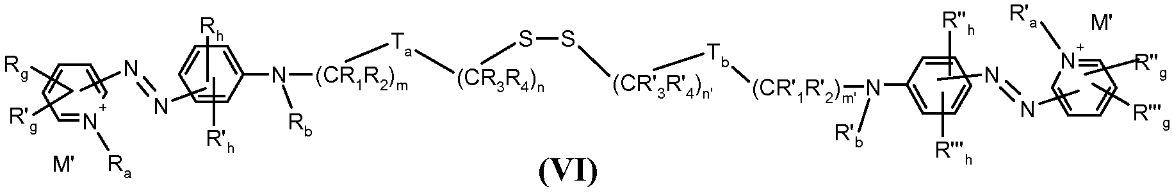

**(VI)**

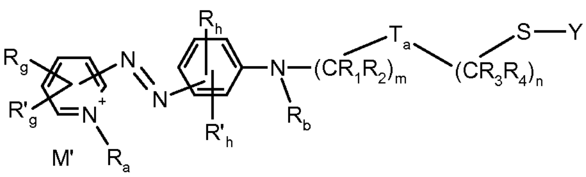

**(VI')**

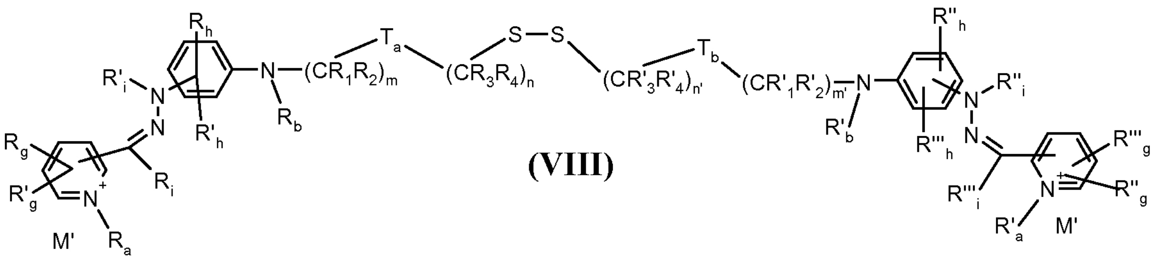

**(VIII)**

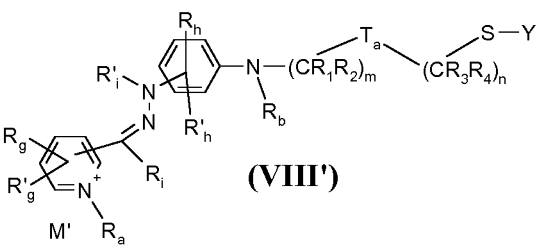

**(VIII')**

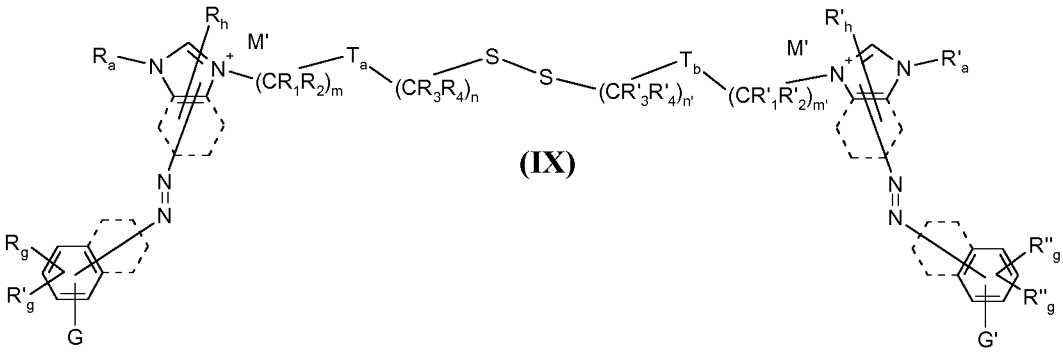

(IX)

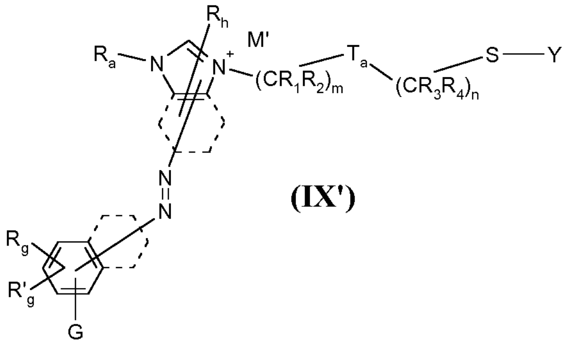

(IX')

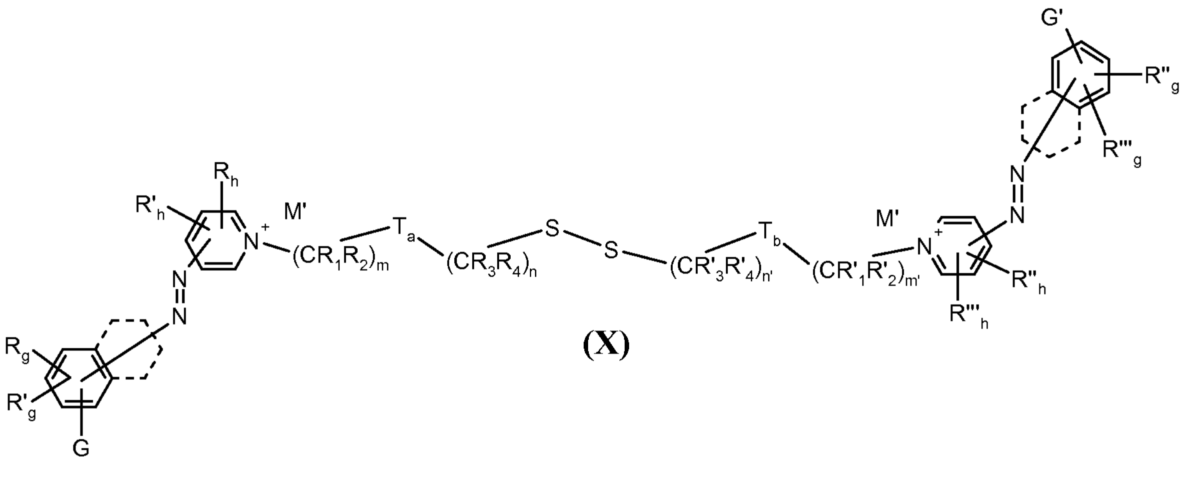

(X)

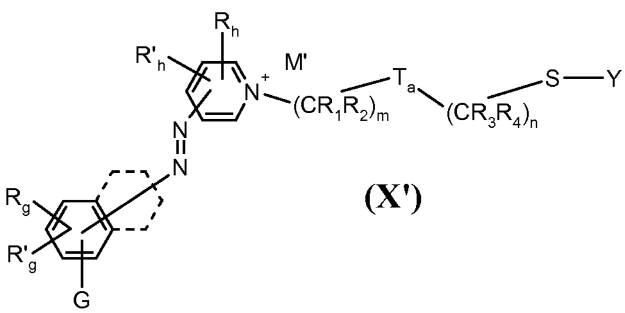

(X')

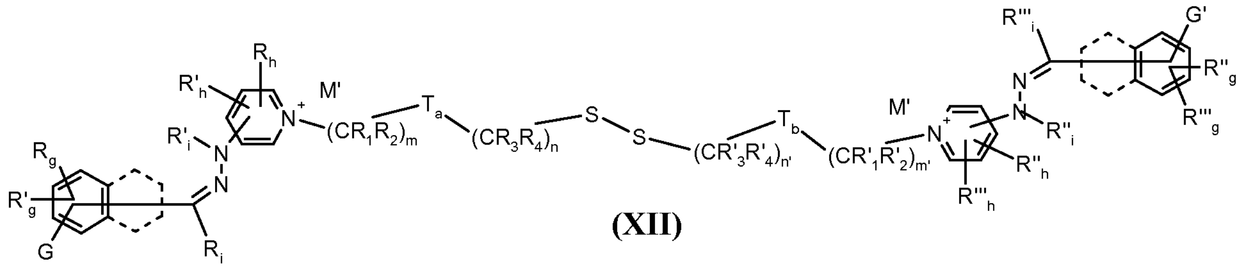

(XII)

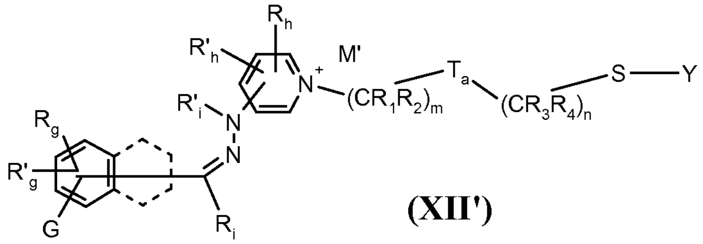

(XII')

in which formulae **(V)** to **(XII)** and **(V')** to **(XII')**:

- G and G', which may be identical or different, represent a hydrogen atom, or i) an-$NR_cR_d$ or $-NR'_cR'_d$ group; ii) optionally substituted, preferentially unsubstituted, $C_1$-$C_6$ alkoxy; or iii) a linear or branched ($C_{10}$-$C_{30}$)alkoxy group or a linear or branched ($C_{10}$-$C_{30}$)alkenyloxy group;
- $R_a$ and $R'_a$, which may be identical or different, represent an aryl($C_1$-$C_4$)alkyl group or a $C_1$-$C_6$ alkyl group optionally substituted with a hydroxyl or amino group, a linear or branched ($C_{10}$-$C_{30}$)alkyl group, or a linear or branched ($C_{10}$-$C_{30}$)alkenyl group;
preferentially, $R_a$ and $R'_a$ represent a linear or branched ($C_{10}$-$C_{30}$)alkyl group;
- $R_b$, and $R'_b$, which may be identical or different, represent a hydrogen atom, an aryl($C_1$-$C_4$)alkyl group, or an optionally substituted $C_1$-$C_6$ alkyl group;
- $R_c$, $R'_c$, $R_d$ and $R'_d$, which may be identical or different, represent a hydrogen atom, an aryl($C_1$-$C_4$)alkyl group, an optionally substituted $C_1$-$C_6$ alkyl group or a linear or branched ($C_{10}$-$C_{30}$)alkyl group, or a linear or branched ($C_{10}$-$C_{30}$)alkenyl group; $R_c$, $R'_c$, $R_d$ and $R'_d$ preferentially represent a hydrogen atom, an optionally substituted $C_1$-$C_6$ alkyl group or a linear or branched ($C_{10}$-$C_{30}$)alkyl group;
or alternatively two adjacent radicals $R_c$ and $R_d$, $R'_c$ and $R'_d$ borne by the same nitrogen atom together form a heterocyclic or heteroaryl group; preferentially, the heterocycle or heteroaryl is monocyclic and 5- to 7-membered; more preferentially, the groups are chosen from imidazolyl and pyrrolidinyl;
- $R_g$, $R'_g$, $R''_g$, $R'''_g$, $R_h$, $R'_h$, $R''_h$, and $R'''_h$, which may be identical or different, represent a hydrogen atom, a halogen atom, an amino, $C_1$-$C_4$ alkylamino, $C_1$-$C_4$ dialkylamino, cyano, carboxyl, hydroxyl or trifluoromethyl group, an acylamino, $C_1$-$C_4$ alkoxy, (poly)hydroxy($C_2$-$C_4$)alkoxy, alkylcarbonyloxy, alkoxycarbonyl or alkylcarbonylamino radical, an acylamino, carbamoyl or alkylsulfonylamino radical, an aminosulfonyl radical, or a $C_1$-$C_{16}$ alkyl radical optionally substituted with a group chosen from $C_1$-$C_{12}$ alkoxy, hydroxyl, cyano, carboxyl, amino, $C_1$-$C_4$ alkylamino and $C_1$-$C_4$ dialkylamino, or alternatively the two alkyl radicals borne by the nitrogen atom of the amino group form a 5- to 7-membered heterocycle optionally comprising another heteroatom identical to or different from that of the nitrogen atom; preferentially, $R_g$, $R'_g$, $R''_g$, $R'''_g$, $R_h$, $R'_h$, $R''_h$, and $R'''_h$ represent a hydrogen or halogen atom or a $C_1$-$C_3$ alkyl group;
- or alternatively two groups $R_g$ and $R'_g$; $R''_g$ and $R'''_g$; $R_h$ and $R'_h$; $R''_h$ and $R'''_h$ borne by two adjacent carbon atoms together form a benzo or indeno ring, a fused heterocycloalkyl or fused heteroaryl group; the benzo, indeno, heterocycloalkyl or heteroaryl ring being optionally substituted with a halogen atom, an amino, $C_1$-$C_4$ alkylamino, $C_1$-$C_4$ dialkylamino, nitro, cyano, carboxyl, hydroxyl or trifluoromethyl group, an acylamino, $C_1$-$C_4$ alkoxy, (poly)hydroxy($C_2$-$C_4$)alkoxy, alkylcarbonyloxy, alkoxycarbonyl or alkylcarbonylamino radical, an acylamino, carbamoyl or alkylsulfonylamino radical, an aminosulfonyl radical, or a $C_1$-$C_{16}$ alkyl radical optionally substituted with: a group chosen from $C_1$-$C_{12}$ alkoxy, hydroxyl, cyano, carboxyl, amino, $C_1$-$C_4$ alkylamino, $C_1$-$C_4$ dialkylamino, or alternatively two alkyl radicals borne by the nitrogen atom of the amino group form a 5- to 7-membered heterocycle optionally comprising another heteroatom identical to or different from that of the nitrogen atom; preferentially, $R_g$ and $R'_g$; $R''_g$ and $R'''_g$ together form a benzo group;
- or alternatively when G represents $-NR_cR_d$ and G' represents $-NR'_cR'_d$, two groups $R_c$ and $R'_g$; $R'_c$ and $R''_g$; $R_d$ and $R_g$; $R'_d$ and $R'''_g$ together form a saturated heteroaryl or heterocycle, optionally substituted with one or

more groups $(C_1-C_6)$alkyl, preferentially a 5- to 7-membered heterocycle containing one or two heteroatoms chosen from nitrogen and oxygen; more preferentially the heterocycle is chosen from morpholinyl, piperazinyl, piperidyl and pyrrolidinyl groups;
• $R_i$, $R'_i$, $R''_i$, and $R'''_i$, which may be identical or different, represent a hydrogen atom or a $C_1-C_4$ alkyl group;
• $R_1$, $R_2$, $R_3$, $R_4$, $R'_1$, $R'_2$, $R'_3$, and $R'_4$, which may be identical or different, represent a hydrogen atom or a $C_1-C_4$ alkyl, $C_1-C_4$ alkoxy, hydroxyl, cyano, carboxyl, amino, $C_1-C_4$ alkylamino or $C_1-C_4$ dialkylamino group, said alkyl radicals possibly forming with the nitrogen atom that bears them a 5- to 7-membered heterocycle optionally comprising another nitrogen or non-nitrogen heteroatom; preferentially, $R_1$, $R_2$, $R_3$, $R_4$, $R'_1$, $R'_2$, $R'_3$, and $R'_4$ are hydrogen atoms or an amino group; more preferentially, $R_1$, $R_2$, $R_3$, $R_4$, $R'_1$, $R'_2$, $R'_3$, and $R'_4$ represent a hydrogen atom;
• $T_a$, $T_b$, which may be identical or different, represent i) either a covalent bond σ, ii) or one or more radicals or combinations thereof chosen from $-SO_2-$, -O-, -S-, -N(R)-, $-N^+(R)(R^\circ)-$, -C(O)-, with R, R°, which may be identical or different, representing a hydrogen atom, a $C_1-C_4$ alkyl radical, a $C_1-C_4$ hydroxyalkyl radical; or an aryl$(C_1-C_4)$alkyl; preferentially, $T_a$ is identical to $T_b$ and represents a covalent bond σ or a group chosen from -N(R)-, -C(O)-N(R)-, -N(R)-C(O)-, -O-C(O)-, -C(O)-O- and$-N^+(R)(R^\circ)-$, $Q^-$, with R, R°, which may be identical or different, representing a hydrogen atom or a $C_1-C_4$ alkyl group; an optionally substituted $C_1-C_6$ alkyl group or a linear or branched $(C_{10}-C_{30})$alkyl group, and $Q^-$ representing an anionic counterion; more preferentially, $T_a$ and $T_b$ represent a bond σ or -C(O)-N(R)- or-N(R)-C(O)-; iii) or a cationic or non-cationic, preferentially monocyclic, preferentially identical, heterocycloalkyl or heteroaryl radical, preferentially containing two heteroatoms (more preferentially two nitrogen atoms) and preferentially comprising from 5 to 7 ring members, such as imidazolium;
•

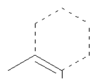

represents an aryl or heteroaryl group fused to the imidazolium or phenyl ring; or alternatively is absent from the imidazolium or phenyl ring; in particular, when the ring is present, the ring is a benzo; preferentially is absent from the imidazolium or phenyl ring;
• m, m', n and n', which may be identical or different, represent an integer between 0 and 6 inclusive, with m+n and m'+n', which may be identical or different, representing an integer between 1 and 10 inclusive; preferentially, m+n = m'+n' = an integer between 2 and 4 inclusive; more preferentially, m+n = m'+n' = an integer equal to 2;
• **Y** is as defined in Claim 1; in particular, Y represents a hydrogen atom or a protective group such as:

- $(C_1-C_4)$alkylcarbonyl, for instance methylcarbonyl or ethylcarbonyl;
- arylcarbonyl such as phenylcarbonyl;
- $(C_1-C_4)$alkoxycarbonyl;
- aryloxycarbonyl;
- aryl$(C_1-C_4)$alkoxycarbonyl;
- (di) $(C_1-C_4)$ (alkyl)aminocarbonyl such as dimethylaminocarbonyl;
- $(C_1-C_4)$(alkyl)arylaminocarbonyl;
- optionally substituted aryl such as phenyl;
- 5- or 6-membered monocyclic heteroaryl such as imidazolyl or pyridyl;
- cationic 5- or 6-membered monocyclic heteroaryl such as pyrylium, pyridinium, pyrimidinium, pyrazinium, pyridazinium, triazinium, imidazolium; these groups being optionally substituted with one or more identical or different $(C_1-C_4)$alkyl groups such as methyl;
- cationic 8- to 11-membered bicyclic heteroaryl such as benzimidazolium or benzoxazolium; these groups being optionally substituted with one or more identical or different $(C_1-C_4)$alkyl groups such as methyl;
- cationic heterocycle of formula below:

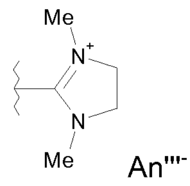

- $-C(NH_2)=N^+H_2$; An'"-; with An'"- being an anionic counterion as defined above;
- $-C(NH_2)=NH$;
- $SO_3^-$, $M^+$ with $M^+$ representing an alkali metal such as sodium or potassium;

preferentially, Y represents a hydrogen atom; and

• **M'** representing an anionic counterion, derived from a salt of an organic or mineral acid, or from an organic or mineral base that ensures the electrical neutrality of the molecule;
• it being understood that at least one of the G, G', $R_a$, $R'_a$, $R_c$, $R'_c$, $R_d$ and $R'_d$ groups bears at least one linear or branched ($C_{10}$-$C_{30}$)alkyl group, or a linear or branched ($C_{10}$-$C_{30}$)alkenyl group, preferably at least one linear or branched, more preferentially linear, ($C_{10}$-$C_{30}$)alkyl group;

in particular, the dyes of formula **(I)** are chosen from disulfide, thiol or thiol-protected azo chromophore dyes, preferably chosen from formulae **(V)** and **(V')**, as defined above, preferably with $\mathbf{R_a}$ and $\mathbf{R'}_a$, which are identical, representing a linear or branched, more preferentially linear, ($C_{10}$-$C_{30}$)alkyl group.

**9.** Dyeing process according to any one of the preceding claims, in which the dye(s) of formula **(I)** are dyes chosen from those of formulae **(XIII)** to **(XIII')** below:

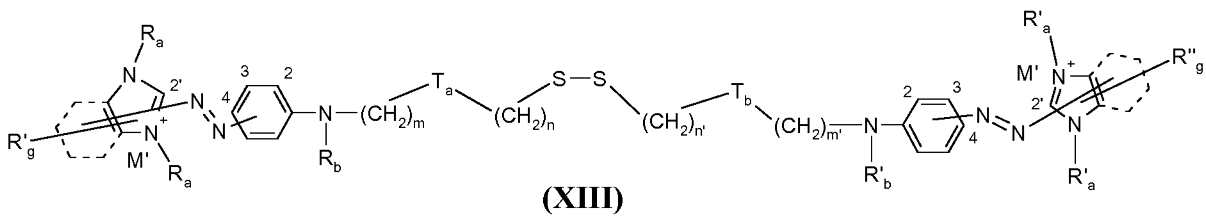

**(XIII)**

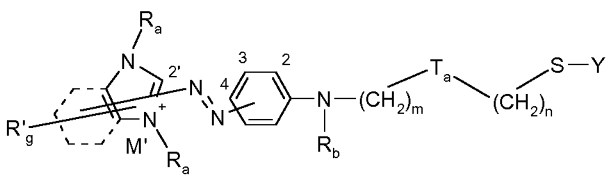

**(XIII')**

salts thereof with organic or mineral acid salts, optical isomers and geometrical isomers thereof, and solvates such as hydrates;
in which formulae **(XIII)** to **(XIII'):**

• $\mathbf{R_a}$ and $\mathbf{R'_a}$, which may be identical or different, represent a linear or branched ($C_{10}$-$C_{30}$)alkyl group, or a linear or branched ($C_{10}$-$C_{30}$)alkenyl group; preferably, $R_a$ and $R'_a$ are identical and represent a linear or branched, in particular linear, ($C_{10}$-$C_{30}$)alkyl group;
• $\mathbf{R_b}$ and $\mathbf{R'_b}$, which may be identical or different, represent a hydrogen atom or a $C_1$-$C_6$ alkyl group;
• $\mathbf{R'_g}$ and $\mathbf{R''_g}$, which may be identical or different, represent a hydrogen atom, a halogen group, an amino, $C_1$-$C_4$ alkylamino, $C_1$-$C_4$ dialkylamino, cyano, carboxy or hydroxyl group, an acylamino, $C_1$-$C_4$ alkoxy, alkylcarbonyloxy, alkoxycarbonyl or alkylcarbonylamino radical, an acylamino, carbamoyl or alkylsulfonylamino radical, an aminosulfonyl radical, or a $C_1$-$C_6$ alkyl radical; in particular, $R'_g$ and $R''_g$ represent a hydrogen atom or a ($C_1$-$C_4$)alkyl group, preferably hydrogen;

• $\mathbf{T_a}$ and $\mathbf{T_b}$, which may be identical or different, represent i) either a covalent bond σ, ii) or one or more radicals or combinations thereof chosen from -O-, -N(R)-,-$N^+$(R)(R°)-, $Q^-$ and -C(O)-, with R, R°, which may be identical or different, representing a hydrogen atom or a $C_1$-$C_4$ alkyl radical; preferentially $T_a$ is identical to $T_b$ and they represent a covalent bond σ or a group chosen from -N(R)-, -C(O)-N(R)- and -N(R)-C(O)-, with R representing a hydrogen atom or a $C_1$-$C_4$ alkyl group; more preferentially, $T_a$ and $T_b$ represent a bond σ;

•

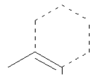

is as defined in the preceding claim; preferentially is absent from the imidazolium or phenyl ring;

• **m, m', n** and **n',** which may be identical or different, represent an integer between 0 and 6 inclusive, with m+n and m'+n', which may be identical or different, representing an integer between 1 and 10 inclusive; preferentially, m+n = m'+n' = an integer between 2 and 4 inclusive; more preferentially, m+n = m'+n' = an integer equal to 2;

• **Y** represents a hydrogen atom, or a protective group chosen from:

- ($C_1$-$C_4$)alkylcarbonyl, for instance methylcarbonyl or ethylcarbonyl;
- arylcarbonyl such as phenylcarbonyl;
- ($C_1$-$C_4$)alkoxycarbonyl;
- aryloxycarbonyl;
- aryl($C_1$-$C_4$)alkoxycarbonyl;
- (di) ($C_1$-$C_4$) (alkyl)aminocarbonyl such as dimethylaminocarbonyl;
- ($C_1$-$C_4$)(alkyl)arylaminocarbonyl;
- optionally substituted aryl such as phenyl;
- 5- or 6-membered monocyclic heteroaryl such as imidazolyl or pyridyl;
- cationic 5- or 6-membered monocyclic heteroaryl such as pyrylium, pyridinium, pyrimidinium, pyrazinium, pyridazinium, triazinium, imidazolium; these groups being optionally substituted with one or more identical or different ($C_1$-$C_4$)alkyl groups such as methyl;
- cationic 8- to 11-membered bicyclic heteroaryl such as benzimidazolium or benzoxazolium; these groups being optionally substituted with one or more identical or different ($C_1$-$C_4$)alkyl groups such as methyl;
- cationic heterocycle of formula below:

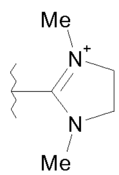

An'''-

- -C($NH_2$)=$N^+H_2$; An'''-; with An'''- being an anionic counterion as defined above;
- -C($NH_2$)=NH;
- $SO_3^-$, $M^+$ with $M^+$ representing an alkali metal such as sodium or potassium; preferably, **Y** represents a hydrogen atom; and

• **M'** representing an anionic counterion, derived from a salt of an organic or mineral acid, or from an organic or mineral base that ensures the electrical neutrality of the molecule.

in particular the azo group linking the (benzo)imidazolium part to the phenyl is in the ortho-or para-position with respect to the pyridinium, i.e. in the 2'-4, 4-2', 2'-4 and 4-2' position, preferably in the 2'-4 and 4-2' para position.

**10.** Dyeing process according to any one of the preceding claims, in which the dye(s) of formula **(I)** are dyes chosen from those of having the following formulae:

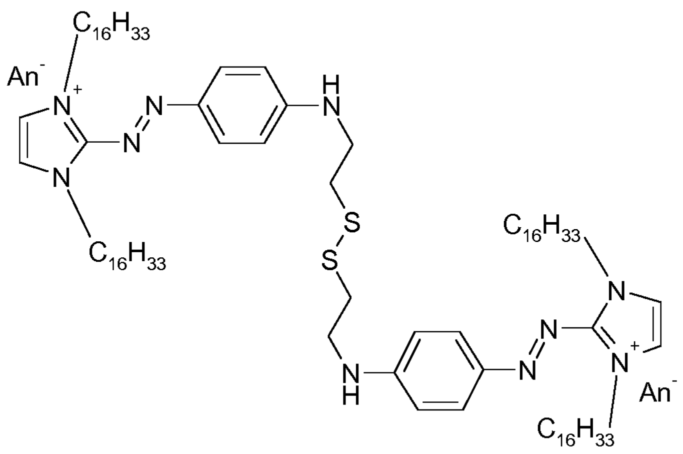

**1**

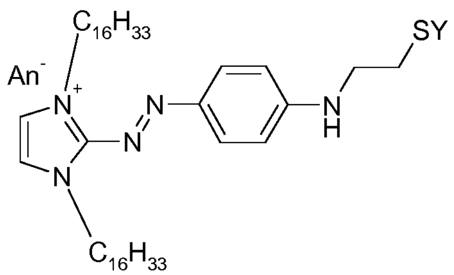

**2**

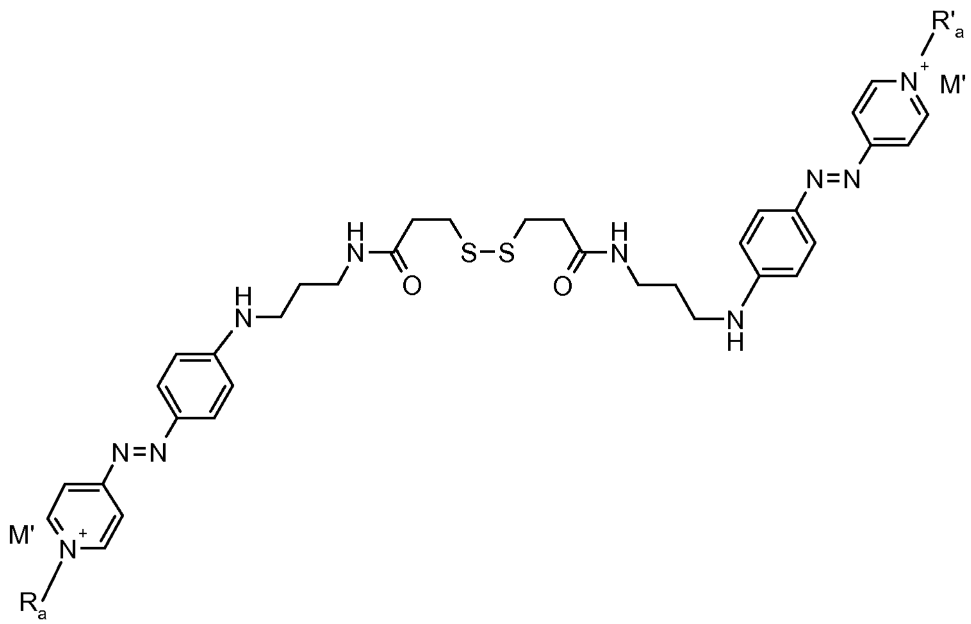

**3**

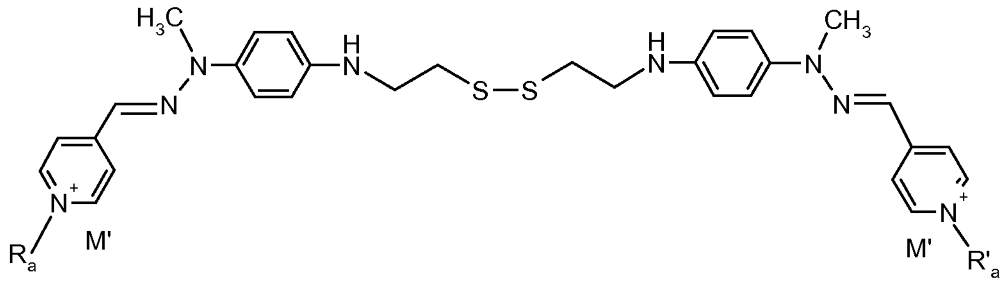

4

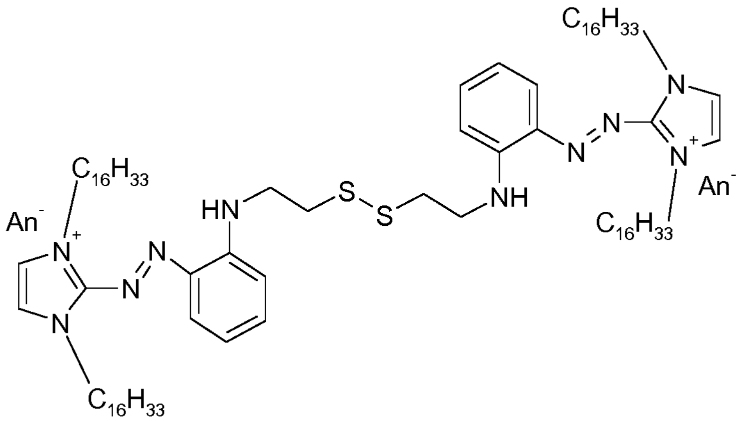

5

with **$An^-$, M'**, which may be identical or different, preferentially identical, representing anionic counterions; more particularly, the anionic counterion is chosen from halides such as chloride, alkyl sulfate such as methyl sulfate and mesylate; **Y** is as defined in one of Claims 1, 8 and 9, and preferably represents

- $(C_1-C_4)$alkylcarbonyl, for instance methylcarbonyl or ethylcarbonyl;
- arylcarbonyl such as phenylcarbonyl;
- $(C_1-C_4)$alkoxycarbonyl;
- aryloxycarbonyl;
- aryl$(C_1-C_4)$alkoxycarbonyl;
- optionally substituted aryl such as phenyl;
- 5- or 6-membered monocyclic heteroaryl such as imidazolyl or pyridyl;
- $-C(NH_2)=N^+H_2$; $An'''^-$; with $An'''^-$ an anionic counterion as defined above; more preferentially, R is a hydrogen atom; and

**$R'_a$** represents an n-$(C_{14}-C_{20})$alkyl group such as an n-($C_{16}$ alkyl) group.

**11.** Process according to any one of the preceding claims, which does not use a reducing agent.

**12.** Process according to any one of the preceding claims, which does not use a chemical oxidizing agent.

**13.** Use of at least one direct dye bearing a disulfide, thiol or protected-thiol function of formula **(I)** as defined in any one of Claims 1 to 12, for protecting said fibres against heat treatments at a temperature greater than or equal to 80°C, provided in particular by steam and/or a straightening iron, in particular originating from a steam iron.

**14.** Direct dye bearing a disulfide, thiol or protected-thiol function of formula **(I)** as defined in any one of Claims 1 to 10.

**15.** Composition comprising one or more direct dyes bearing a disulfide, thiol or protected-thiol function of formula **(I)** as defined in any one of Claims 1 to 12.

**16.** Process for preparing dyes of formula **(I)** as defined in any one of Claims 1 to 10, which consists:

- either using two equivalents of reagents comprising a chromophore and at least one nucleofugal group such as halogeno **(a)** and one equivalent of amino disulfide compound **(b):**

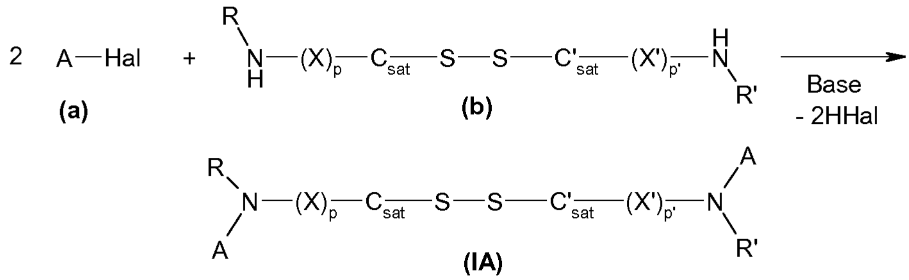


(a) (b) (IA)

with **Hal** representing a nucleofugal group such as halogeno, (poly)halogeno($C_1$-$C_4$)alkoxy, (poly)halogeno($C_1$-$C_4$)sulfoxy, **X**, **X'**, **p**, **p'**, $\mathbf{C_{sat}}$ and $\mathbf{C'_{sat}}$ being as defined for the compound **(I)** in any one of Claims 1 to 10, **R** and **R'** representing a hydrogen atom or an optionally substituted ($C_1$-$C_4$)alkyl group; **A** representing a chromophore as defined above for **(I)** in any one of Claims 1 to 10, **R** and **R'** or else **A** represents a chromophore comprising a nucleofugal group as defined, which reacts with one or two equivalents of aminoalkylene followed by an alkylation reaction using for example ($C_{10}$-$C_{30}$)alkyl halide, preferably **A** represents a hydrazono cationic chromophore of formulae **(II)** and **(III')**, or azo cationic chromophore **(IV)**, **(IV')**, **(V)** and **(V')** as defined in Claim 7; this reaction preferably being carried out in a polar protic solvent, in particular at the reflux of the solvent, preferably the solvent is an alcohol such as ethanol;
- or the first step consists in preparing the disulfide reagent **(c)** onto which will be grafted a heteroaryl substituted with at least one ($C_1$-$C_6$)alkyl group such as a pyridine substituted with a hydrazino group **(d)** which in turn reacts with an aryl(thio)aldehyde or aryl(thio)ketone reagent comprising at least one group bearing a linear or branched, saturated or unsaturated $C_{10}$-$C_{30}$ aliphatic chain; with elimination of $H_2O$ or $H_2S$ so as to give a disulfide dye bearing a hydrazono chromophore **(I')**, and also optical and geometric isomers thereof, belonging to formula (I) according to the invention:

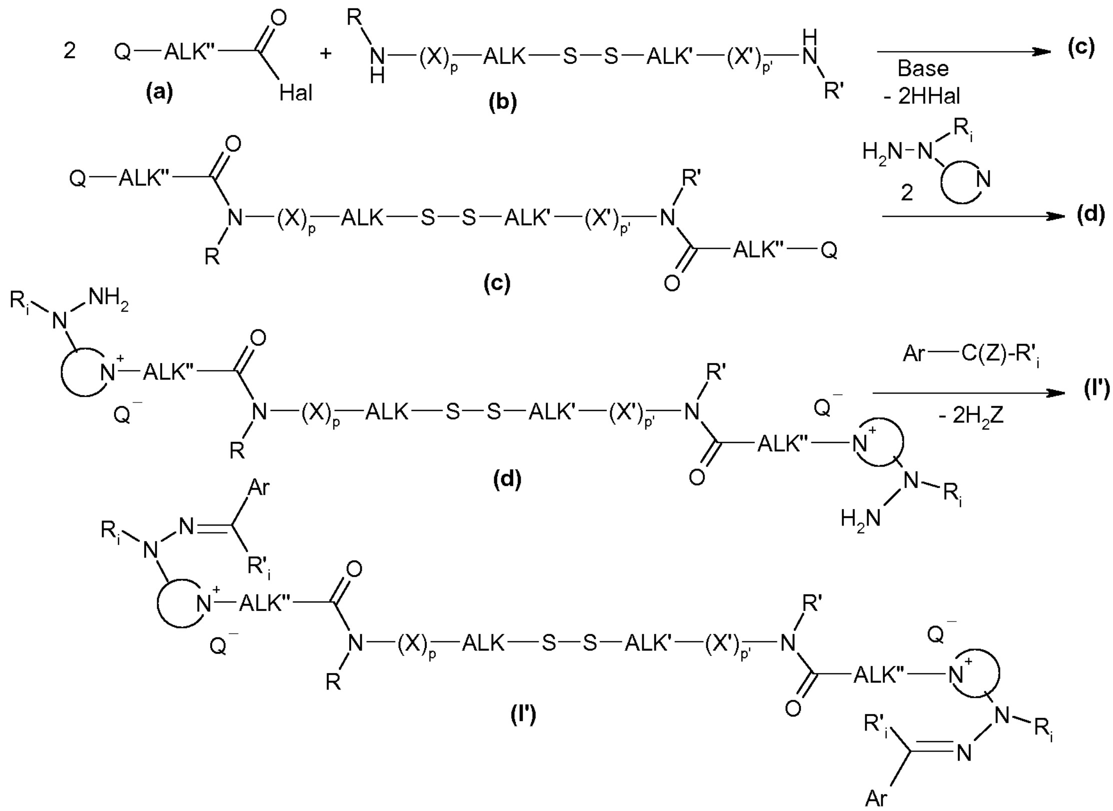


(a) (b) (c) (c) (d) (d) (I') (I')

Compounds **(a), (b), (c), (d)** and **(I')** with:

- **Q** representing an electrofugal group such as halogeno, (poly)halo($C_1$-$C_6$)-$S(O)_2$-O-, alkyl sulfonyl ($C_1$-$C_6$)Alk-$S(O)_2$O- such as methylsulfonyl and ethylsulfonyl; arylsulfonyls: Ar-$S(O)_2O^-$ such as benzenesulfonyl and toluenesulfonyl; (poly)(hydroxy)($C_1$-$C_6$)alkylcarbonyloxy, alkylsulfonyl: Alk-O-S(O)O-; arylsulfonyls: Ar-O-S(O)O- such as benzenesulfonyl and toluenesulfonyl; alkoxysulfonyls: Alk-O-$S(O)_2$O-; aryloxysulfates: Ar-O-$S(O)_2$O-, phosphonyl; and borates such as tetrahaloborate;
- **$Q^-$** representing an anionic counterion which derives from Q as defined in Claim 7;
- **ALK, ALK', ALK"**, which may be identical or different, represent an optionally substituted linear or branched ($C_1$-$C_6$)alkylene group, such as ethylene or propylene;
- **Hal, X, X', p** and **p'** are as defined above;
- **Z** represents an oxygen or sulfur, preferably oxygen, atom;
- **$R_i$** and **$R'_i$** are as defined above;
- 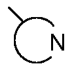

  represents a heteroaryl group comprising at least one nitrogen atom substituted with at least one ($C_1$-$C_6$)alkyl group, preferably methyl;
- 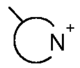

  represents a cationic heteroaryl group substituted with at least one ($C_1$-$C_6$)alkyl group, preferably methyl, and comprising at least one ammonium;
- **Ar** represents an optionally substituted (hetero)aryl group, preferably phenyl, comprising at least one group bearing a linear or branched, saturated or unsaturated $C_{10}$-$C_{30}$ aliphatic chain;

- or when the disulfide compound has an alkylene linker which is uninterrupted between the chromophore and the disulfide group via a heteroatom or an -N(R)-,-$N^+$(R)(R)-, -O-, -S-, -C(O)-, -$SO_2$- group or a combination thereof with R as defined above and can be prepared according to the following synthesis scheme:

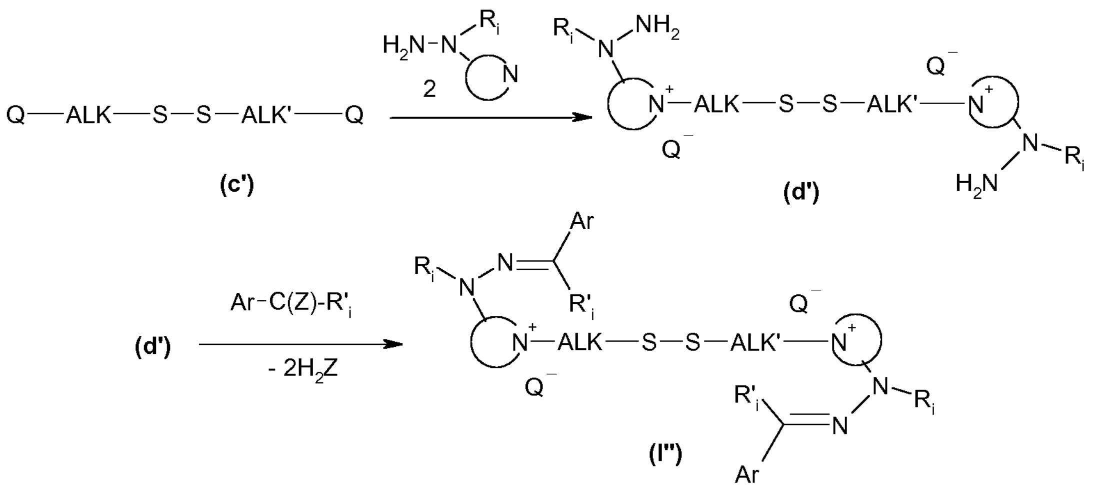


(c') (d')

(d') (I")

with ALK, ALK', Q, $Q^-$, $R_1$, Z, $R_1$, $R'_1$, Ar as defined above; and

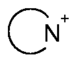

represents a cationic heteroaryl group comprising at least one ammonium;

- or using the azo chromophore reagent **(f)** comprising a leaving group GP such as halide, mesylate or tosylate, a (hetero)aromatic part Ar and a heteroaryl group comprising at least one nitrogen atom, such as a (benzo)imidazolyl which is itself prepared from a (hetero)aromatic compound (e) comprising at least one GP and an amino group in the presence i) of an acid, in particular in a mixture of organic and inorganic acid, preferably in a mixture with a carboxylic acid and of hydrochloric acid, and of $MNO_2$ with M representing an alkali or alkaline-earth metal preferably in water and at a temperature between 0°C et 5°C, then ii) of a base such as sodium hydroxide or potassium hydroxide, then iii) of a heteroaryl comprising at least one nitrogen atom, such as (benzo)imidazole, preferably imidazole.; the compound (f) can then be alkylated in particular by $R^a$ a linear or branched, saturated or unsaturated $C_{10}$-$C_{30}$ aliphatic chain using a reagent $R_a$-Q with Q representing a leaving group such as halide so as to give the intermediate **(g),** the latter can then react with the diamino sulfide reagent (b) so as to give the azo compound **(I"")** according to the invention:

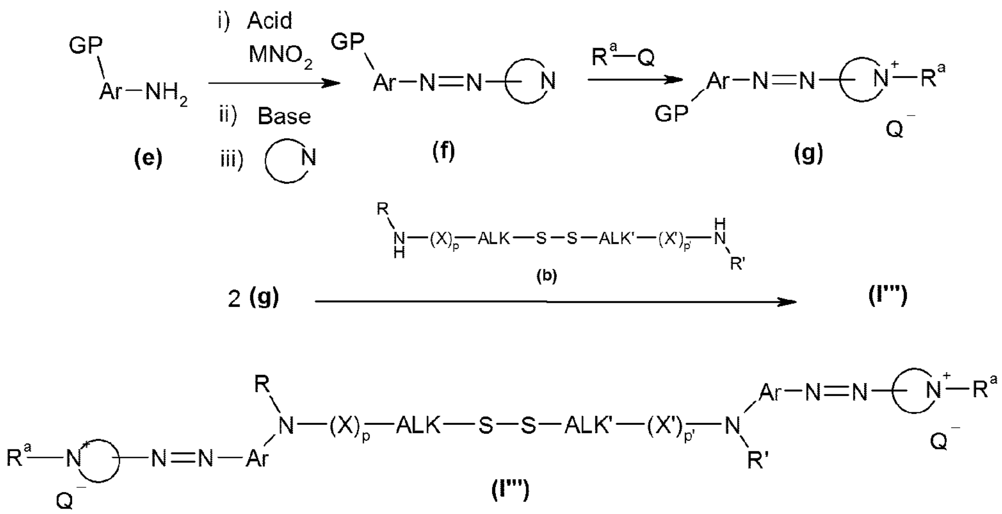


with ALK, ALK', X, X', p, p', Q, $Q^-$, Ar, GP and

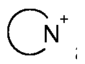

as defined above;

R and R', which may be identical different, represent a hydrogen atom or a ($C_1$-$C_6$)alkyl group;

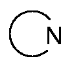

represents a heteroaryl group comprising at least one nitrogen atom.

**17.** Process for preparing, according to the preceding claim, the dyes of formulae **(Va)** and **(V')** which consists in reacting an aniline substituted with an alkoxy or benzyloxy group (h) with i) an acid, preferably a mineral acids such as hydrochloric acid or a carboxylic acid such as acetic acid, in the presence of a nitrogenous compound $MNO_2$ with M representing a cationic counterion such as Na or K, then ii) with an organic or mineral base, preferably a mineral base, such as sodium hydroxide, then iii) an imidazolyl derivative, optionally fused with a (hetero)aryl group, preferably a benzo group, so as to give the azo compound (i); the latter reacting with at least 2 molar equivalents of an alkylating agent such as $R^a$-Q with $R_a$ and Q as defined in the preceding claim, so as to give the compound (j);

- either two equivalents of (j) reacting with an amino disulfide compound (d), so as to give the disulfide dye **(Va);**
- or one molar equivalent of (j) reacting with a thiol or protected-thiol compound (d') so as to give the thiol or protected-thiol dye **(V')** as defined in Claim 8;

according to the following scheme:

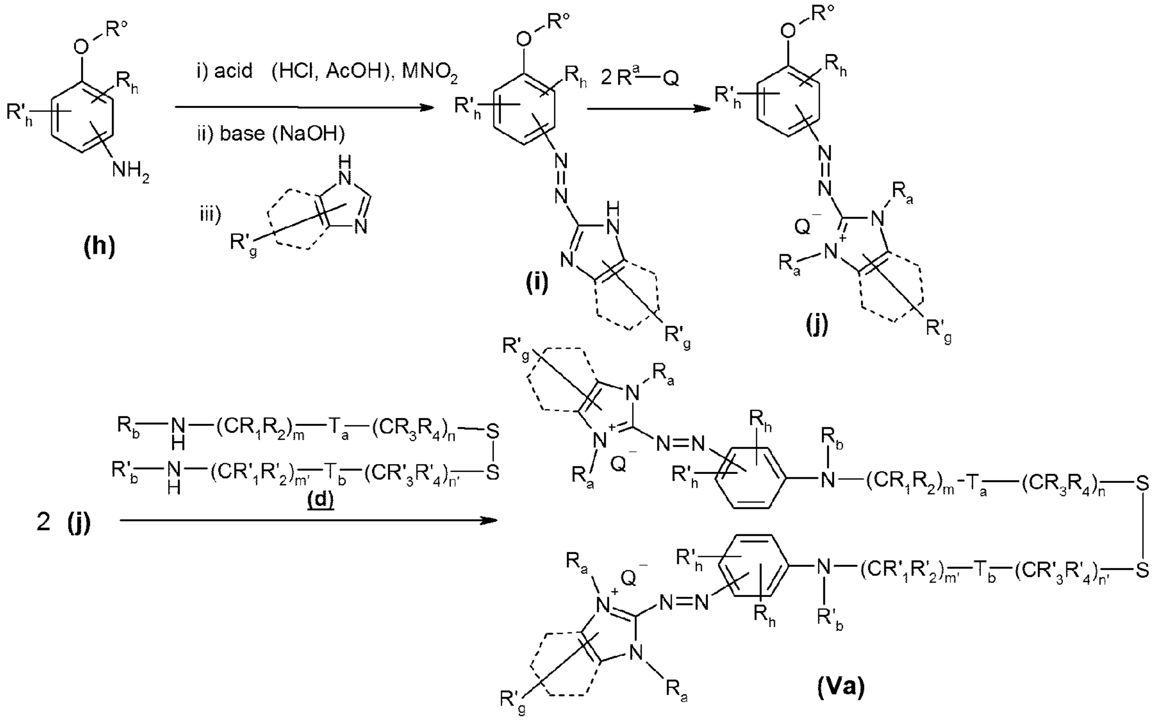


(h) (i) (j) (Va)

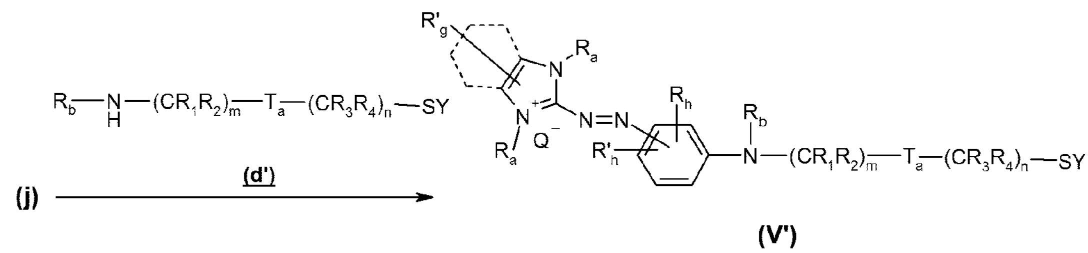


(V')

with $R_a$, $R_b$, $R'_b$, $R'_g$, $R_h$, $R'_h$, $R_1$, $R_2$, $R_3$, $R_4$, $R'_1$, $R'_2$, $R'_3$, $R'_4$, Y, m, m', n, n', $T_a$, $T_b$, and

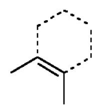

being as defined in Claim 8 or 9, Q being as defined in the preceding claim, and R° representing a ($C_1$-$C_6$)alkyl, (hetero)aryl or benzyl group.

## Patentansprüche

1. Verfahren zum Färben von Keratinfasern, bei dem man auf die Keratinfasern einen oder mehrere kationische Direktfarbstoffe mit einer Disulfid-, Thiol- oder geschützten Thiolfunktion der Formel **(I)**:

$$A\text{-}(X)_p\text{-}C_{sat}\text{-}S\text{-}U \qquad (I),$$

Salze davon mit einer organischen Säure oder Mineralsäure, optische oder geometrische Isomere davon, Tautomere davon, und Solvate davon aufbringt;
wobei in Formel **(I)**:

• **U** für einen Rest steht, der ausgewählt ist aus:

*a)* **-S-C'$_{sat}$-(X')$_{p'}$-A'** und

*b)* - **Y;**

- **A** und **A',** die gleich oder verschieden sein können, für einen kationischen oder nichtkationischen Chromophor, vorzugsweise einen kationischen Chromophor, stehen, mit der Maßgabe, dass mindestens einer der beiden Chromophore mindestens eine Gruppe mit einer linearen oder verzweigten, gesättigten oder ungesättigten aliphatischen $C_{10}$-$C_{30}$-Kette trägt;
- **Y** für i) ein Wasserstoffatom oder ii) eine Schutzgruppe für die Thiolfunktion steht;
- **X** und **X',** die gleich oder verschieden sein können, für eine lineare oder verzweigte, gesättigte oder ungesättigte zweiwertige $C_1$-$C_{30}$-Kette auf Kohlenwasserstoffbasis, die gegebenenfalls durch eine oder mehrere zweiwertige Gruppen oder Kombinationen davon, die aus:

  ➢ -N(R)-, $-N^+(R)(R)^-$, -O-, -S-, -CO- oder $-SO_2-$, wobei die Reste R, die gleich oder verschieden sein können, aus Wasserstoff und einem $C_1$-$C_4$-Alkyl-, Hydroxyalkyl- oder Aminoalkylrest ausgewählt sind;

  ➢ einem aromatischen oder nichtaromatischen, gesättigten oder ungesättigten, anellierten oder nichtanellierten (hetero)cyclischen Rest, der gegebenenfalls ein oder mehrere gleiche oder verschiedene, gegebenenfalls substituierte Heteroatome umfasst; ausgewählt sind, unterbrochen und/oder an einem oder beiden ihrer Enden terminiert ist, stehen;

- **p** und **p',** die gleich oder verschieden sein können, gleich 0 oder 1 sind;
- $\mathbf{C_{sat}}$ und $\mathbf{C'_{sat}}$, die gleich oder verschieden sein können, für eine gegebenenfalls cyclische, gegebenenfalls substituierte lineare oder verzweigte $C_1$-$C_{18}$-Alkylenkette stehen.

**2.** Verfahren nach dem vorhergehenden Anspruch, wobei es sich bei dem Farbstoff bzw. den Farbstoffen der Formel **(I)** um Disulfid-Farbstoffe handelt, wobei **U** für einen Rest a) $\mathbf{-S\text{-}C'_{sat}\text{-}(X')_{p'}\text{-}A'}$ steht; es sich bei den erfindungsgemäßen Farbstoffen der Formel **(I)** insbesondere um symmetrische Disulfide handelt, d.h. die Formel **(I)** die folgende Formel aufweist:

$$\mathbf{A\text{-}(X)_p\text{-}C_{sat}\text{-}S\text{-}S\text{-}C'_{sat}\text{-}(X')_{p'}\text{-}A'},$$

wobei **A = A', X = X', p = p',** $\mathbf{C_{sat} = C'_{sat}}$.

**3.** Verfahren nach Anspruch 1, wobei es sich bei dem Farbstoff bzw. den Farbstoffen der Formel **(I)** um Farbstoffe mit einer Thiolfunktion oder geschützten Thiolfunktion handelt, d.h. **U** für den Rest *b)* **Y** steht, insbesondere **Y** für ein Wasserstoffatom steht.

**4.** Färbeverfahren nach einem der vorhergehenden Ansprüche, wobei es sich bei dem Farbstoff bzw. den Farbstoffen der Formel **(I)** um Farbstoffe handelt, wobei $C_{sat}$ und $C'_{sat}$, die gleich oder verschieden sein können, für eine Kette $-(CH_2)_k$-stehen, wobei k eine ganze Zahl zwischen 1 und 8 inklusive ist.

**5.** Verfahren nach einem der vorhergehenden Ansprüche, wobei es sich bei dem Farbstoff bzw. den Farbstoffen der Formel **(I)** um Farbstoffe handelt, in denen dann, wenn **p** und **p'** gleich 1 sind, **X** und **X',** die gleich oder verschieden sein können, für die folgende Sequenz stehen:

$$\mathbf{-(T)_t\text{-}(Z)_z\text{-}(T')_{t'}\text{-}}$$

wobei die Sequenz in Formel **(I)** folgendermaßen symmetrisch verknüpft ist:

$$- \mathbf{C_{sat}}\ (\text{oder } \mathbf{C'}_{sat})\mathbf{\text{-}(T)_t\text{-}(Z)_z\text{-}(A}\ \text{oder}\ \mathbf{A');}$$

wobei:

- **T** und **T',** die gleich oder verschieden sein können, für einen oder mehrere Reste oder Kombinationen davon stehen, die ausgewählt sind aus: -O-; -S-; -N(R)-; $-N^+(R)(R^\circ)-$; -S(O)-; $-S(O)_2-$; -C(O)-; wobei R und R°, die gleich oder verschieden sein können, für ein Wasserstoffatom oder einen $C_1$-$C_4$-Alkyl-, $C_1$-$C_4$-Hydroxyalkyl- oder Aryl ($C_1$-$C_4$) alkylrest stehen; und einem kationischen oder nichtkationischen, vorzugsweise monocyclischen Heterocycloalkyl- oder Heteroarylrest, der vorzugsweise zwei Heteroatome (weiter bevorzugt zwei Stickstoffatome) umfasst und vorzugsweise 5- bis 7-gliedrig ist, weiter bevorzugt Imidazolium;

die Indices **t** und **t',** die gleich oder verschieden sein können, gleich 0 oder 1 sind;

• **Z** für:

> ➢ $-(CH_2)_m-$, wobei m eine ganze Zahl zwischen 1 und 8 inklusive ist;
>
> ➢ $-(CH_2CH_2O)_q-$ oder $-(OCH_2CH_2)_q-$, wobei q eine ganze Zahl zwischen 1 und 5 inklusive ist;
>
> ➢ einen Aryl-, Alkylaryl- oder Arylalkylrest, wobei der Alkylrest davon ein $C_1$-$C_4$-Alkylrest ist und der Arylrest davon vorzugsweise ein $C_6$-Arylrest ist, der gegebenenfalls durch mindestens eine Gruppe $SO_3M$ substituiert ist, wobei M für ein Wasserstoffatom, ein Alkalimetall oder eine Ammoniumgruppe, die durch einen oder mehrere gleiche oder verschiedene und lineare oder verzweigte $C_1$-$C_{18}$-Alkylreste, die gegebenenfalls mindestens ein Hydroxyl tragen, substituiert ist, steht; steht;

• **z** für 0 oder 1 steht.

**6.** Verfahren zum Färben von Keratinfasern nach einem der vorhergehenden Ansprüche, wobei der Farbstoff bzw. die Farbstoffe der Formel **(I)** einen Chromophor **A** und/oder **A'** umfasst bzw. umfassen, wobei **A** und **A'** gleich oder verschieden sein können und von den folgenden Acridinfarbstoffen abgeleitet sind:
Acridonen; Anthranthronen; Anthrapyrimidinen; Anthrachinonen; Azinen; (Poly)azo-Farbstoffen, Hydrazono-Farbstoffen oder Hydrazonen, insbesondere Arylhydrazonen; Azomethinen; Benzanthronen; Benzimidazolen; Benzimidazolonen; Benzindolen; Benzoxazolen; Benzopyranen; Benzothiazolen; Benzochinonen; Bisazinen; Bisisoindolinen; Carboxaniliden; Cumarinen; Cyaninen wie Azacarbocyaninen, Diazocarbocyaninen, Diazahemicyaninen, Tetraazacarbocyaninen oder (Poly)methinen; Diazinen; Diketopyrrolopyrrolen; Dioxazinen; Diphenylaminen; Diphenylmethanen; Dithiazinen; Flavonoiden wie Flavanthronen und Flavonen; Fluorindinen; Formazanen; Indaminen; Indanthronen; Indigoiden und Pseudoindigoiden; Indophenolen; Indoanilinen; Isoindolinen; Isoindolinonen; Isoviolanthronen; Lactonen; Naphthochinonen; Nitro-Farbstoffen, insbesondere Nitro(hetero)aromaten; Oxadiazolen; Oxazinen; Perilonen; Perinonen; Perylenen; Phenazinen; Phenoxazinen; Phenothiazinen; Phthalocyaninen; Polyenen/Carotinoiden; Porphyrinen; Pyranthronen; Pyrazolanthronen; Pyrazolonen; Pyrimidinoanthronen; Pyroninen; Chinacridonen; Chinolinen; Chinophthalonen; Squaranen; Tetrazolium-Farbstoffen; Thiazinen, Thioindigo-Farbstoffen; Thiopyroninen; Triarylmethanen oder Xanthenen; vorzugsweise die Chromophore aus (Poly)azo-, Hydrazono- und Azomethin-Chromophoren, weiter bevorzugt aus Azo-Chromophoren, ausgewählt sind.

**7.** Verfahren nach einem der vorhergehenden Ansprüche, wobei der Farbstoff bzw. die Farbstoffe der Formel **(I)** einen Chromophor **A** und/oder **A'** umfasst bzw. umfassen, wobei **A** und **A'** gleich oder verschieden sein können und aus den nachstehenden kationischen Chromophoren ausgewählt sind:

**(*) -Het⁺-C(Rᵃ)=N-N(Rᵇ)-Ar, Q⁻** **(II)**

**Q⁻, Het⁺-C (Rᵃ)=N-N(Rᵇ)-Ar'- (*),** **(II')**

**(*) -Het⁺-N(Rᵃ)-N=C(Rᵇ)-Ar, Q⁻** **(III)**

**Q⁻, Het⁺-N (Rᵃ)-N=C(Rᵇ)-Ar'- (*),** **(III')**

**(*)-Het⁺-N=N-Ar, Q⁻** **(IV)**

**Q⁻, Het⁺-N=N-Ar'-(*),** **(IV')**

**(*) -Ar⁺-N=N-Ar", Q⁻** **(V)**

**Q⁻, Ar⁺-N=N-Ar"-(*)** **(V')**

wobei in den Formeln **(II)** bis **(V')**:

- **Het⁺** für einen kationischen Heteroarylrest, der vorzugsweise eine endocyclische kationische Ladung trägt, wie Imidazolium, Indolium oder Pyridinium, der gegebenenfalls insbesondere durch mindestens eine $(C_1$-$C_8)$Alkylgruppe wie Methyl substituiert ist, steht;
- **Ar⁺** für einen Arylrest, wie Phenyl oder Naphthyl, mit einer exocyclischen kationischen Ladung, vorzugsweise

Ammonium, insbesondere Tri($C_1$-$C_8$)alkylammonium wie Trimethylammonium, steht;

- **Ar** für eine Arylgruppe, insbesondere Phenyl, die gegebenenfalls substituiert ist, vorzugsweise durch einen oder mehrere elektronenspendende Gruppen wie i) gegebenenfalls substituiertes ($C_1$-$C_8$)Alkyl, ii) gegebenenfalls substituiertes ($C_1$-$C_8$)Alkoxy, iii) (Di)($C_1$-$C_8$)(alkyl)amino, das gegebenenfalls an der Alkylgruppe bzw. den Alkylgruppen durch eine oder mehrere Hydroxylgruppen substituiert ist, iv) Aryl($C_1$-$C_8$)alkylamino, v) gegebenenfalls substituiertes *N*-($C_1$-$C_8$)Alkyl-*N*-aryl($C_1$-$C_8$)alkylamino, steht oder alternativ dazu **Ar** für eine Julolidingruppe steht;
- **Ar'** für eine gegebenenfalls substituierte zweiwertige (Hetero)arylengruppe wie Phenylen, insbesondere para-Phenylen, oder Naphthalin, die gegebenenfalls substituiert ist, insbesondere durch eine oder mehrere Gruppen ($C_1$-$C_8$)Alkyl, Hydroxy oder ($C_1$-$C_8$)Alkoxy, steht;
- **Ar"** für eine gegebenenfalls substituierte (Hetero)arylgruppe wie Phenyl oder Pyrazolyl, die gegebenenfalls substituiert ist, insbesondere durch eine oder mehrere Gruppen ($C_1$-$C_8$)Alkyl, Hydroxy, (Di)($C_1$-$C_8$)(alkyl)amino, ($C_1$-$C_8$)Alkoxy oder Phenyl, steht;
- $\mathbf{R^a}$ und $\mathbf{R^b}$**,** die gleich oder verschieden sind, für ein Wasserstoffatom oder eine ($C_1$-$C_8$)Alkylgruppe, die gegebenenfalls substituiert ist, insbesondere durch eine Hydroxygruppe, stehen; oder auch der Substituent $\mathbf{R^a}$ mit einem Substituenten von $\mathbf{Het^+}$ und/oder $\mathbf{R^b}$ mit einem Substituenten von **Ar** zusammen mit dem Atom, das sie trägt, ein (Hetero)cycloalkyl bilden; insbesondere $\mathbf{R^a}$ und $\mathbf{R^b}$ für ein Wasserstoffatom oder eine ($C_1$-$C_4$)Alkylgruppe, die gegebenenfalls durch eine Hydroxygruppe substituiert ist, stehen;
- $\mathbf{Q^-}$ für ein organisches oder mineralisches anionisches Gegenion wie ein Halogenid oder ein Alkylsulfat steht;
- **(*)** für den an den Rest des Moleküls der Formel **(I)** gebundenen Teil des Chromophors steht;

mit der Maßgabe, dass mindestens eine der $Het^+$-, $Ar^+$-, Ar- und Ar'-Gruppen mindestens eine lineare oder verzweigte, gesättigte oder ungesättigte aliphatische $C_{10}$-$C_{30}$-Kette; vorzugsweise eine lineare oder verzweigte ($C_{10}$-$C_{30}$)Alkylgruppe oder eine lineare oder verzweigte ($C_{10}$-$C_{30}$)Alkenylgruppe, trägt;

**$Het^+$-C($R^a$)=N-N($R^b$)-Ar, $Q^-$**
**(II)**

**$Q^-$, $Het^+$-C ($R^a$)=N-N($R^b$)-Ar'- (*),**
**(II')**

**(*) -$Het^+$-N($R^a$)-N=C($R^b$)-Ar, $Q^-$**
**(III)**

**$Q^-$, $Het^+$-N ($R^a$)-N=C($R^b$)-Ar'- (*),**
**(III')**

**(*)-$Het^+$-N=N-Ar, $Q^-$** **(IV)**

**$Q^-$, $Het^+$-N=N-Ar'-(*),** **(IV')**

**(*) -$Ar^+$-N=N-Ar", $Q^-$** **(V)**
**(VI)**

**$Q^-$, $Ar^+$-N=N-Ar" -(*)** **(V) (VI)**

wobei in den Formeln **(II)** bis **(V'):**

- $\mathbf{Het^+}$ für einen kationischen Heteroarylrest, der vorzugsweise eine endocyclische kationische Ladung trägt, wie Imidazolium, Indolium oder Pyridinium, der gegebenenfalls insbesondere durch mindestens eine ($C_1$-$C_8$)Alkylgruppe wie Methyl substituiert ist, steht;
- $\mathbf{Ar^+}$ für einen Arylrest, wie Phenyl oder Naphthyl, mit einer exocyclischen kationischen Ladung, vorzugsweise Ammonium, insbesondere Tri($C_1$-$C_8$)alkylammonium wie Trimethylammonium, steht;
- **Ar** für eine Arylgruppe, insbesondere Phenyl, die gegebenenfalls substituiert ist, vorzugsweise durch einen oder mehrere elektronenspendende Gruppen wie i) gegebenenfalls substituiertes ($C_1$-$C_8$)Alkyl, ii) gegebenenfalls substituiertes ($C_1$-$C_8$)Alkoxy, iii) (Di)($C_1$-$C_8$)(alkyl)amino, das gegebenenfalls an der Alkylgruppe bzw. den Alkylgruppen durch eine oder mehrere Hydroxylgruppen substituiert ist, iv) Aryl($C_1$-$C_8$)alkylamino, v) gegebenenfalls substituiertes *N*-($C_1$-$C_8$)Alkyl-*N*-aryl($C_1$-$C_8$)alkylamino, steht oder alternativ dazu **Ar** für eine Julolidingruppe steht;
- **Ar'** für eine gegebenenfalls substituierte zweiwertige (Hetero)arylengruppe wie Phenylen, insbesondere para-Phenylen, oder Naphthalin, die gegebenenfalls substituiert ist, insbesondere durch eine oder mehrere Gruppen ($C_1$-$C_8$)Alkyl, Hydroxy oder ($C_1$-$C_8$)Alkoxy, steht;

- **Ar"** für eine gegebenenfalls substituierte (Hetero)arylgruppe wie Phenyl oder Pyrazolyl, die gegebenenfalls substituiert ist, insbesondere durch eine oder mehrere Gruppen $(C_1-C_8)$Alkyl, Hydroxy, (Di)$(C_1-C_8)$(alkyl)amino, $(C_1-C_8)$Alkoxy oder Phenyl, steht;
- **$R^a$** und **$R^b$,** die gleich oder verschieden sind, für ein Wasserstoffatom oder eine $(C_1-C_8)$Alkylgruppe, die gegebenenfalls substituiert ist, insbesondere durch eine Hydroxygruppe, stehen; oder auch der Substituent **$R^a$** mit einem Substituenten von **$Het^+$** und/oder **$R^b$** mit einem Substituenten von **Ar** zusammen mit dem Atom, das sie trägt, ein (Hetero)cycloalkyl bilden; insbesondere **$R^a$** und **$R^b$** für ein Wasserstoffatom oder eine $(C_1-C_4)$Alkylgruppe, die gegebenenfalls durch eine Hydroxygruppe substituiert ist, stehen;
- **$Q^-$** für ein organisches oder mineralisches anionisches Gegenion wie ein Halogenid oder ein Alkylsulfat steht;
- **(*)** für den an den Rest des Moleküls der Formel **(I)** gebundenen Teil des Chromophors steht;

mit der Maßgabe, dass mindestens eine der $Het^+$-, $Ar^+$-, Ar- und Ar'-Gruppen mindestens eine lineare oder verzweigte, gesättigte oder ungesättigte aliphatische $C_{10}-C_{30}$-Kette; vorzugsweise eine lineare oder verzweigte $(C_{10}-C_{30})$Alkylgruppe oder eine lineare oder verzweigte $(C_{10}-C_{30})$Alkenylgruppe, trägt;
insbesondere die kationischen Chromophore **(A)** und **(A')** endocyclische kationische Azo- und Hydrazonoladungen der oben definierten Formeln **(II)** bis **(IV')** gemäß obiger Definition tragen, spezieller die kationischen Chromophore **(A)** und **(A')** die folgenden Formeln aufweisen:

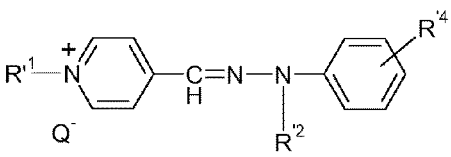

**(II-1)**

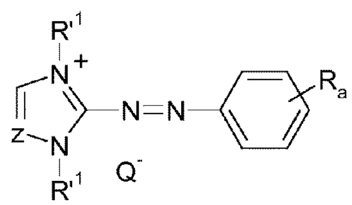

**(IV-1)**

wobei in den Formeln **(II-1)** und **(IV-1):**

- **$R'^1$** und **$R'^2$,** die gleich oder verschieden sein können, für eine lineare oder verzweigte $(C_1-C_{30})$Alkyl- oder lineare oder verzweigte $(C_2-C_{30})$Alkenylgruppe stehen;
- **$R_a$** für eine Aminogruppe $NR'_2R'_3$ steht, wobei **$R'_2$** und **$R'_3$,** die gleich oder verschieden sein können, für ein Wasserstoffatom oder eine lineare oder verzweigte $(C_1-C_{30})$Alkyl- oder lineare oder verzweigte $(C_2-C_{30})$Alkenylgruppe stehen;
- **$R'^4$** für ein Wasserstoffatom oder eine elektronenspendende Gruppe wie gegebenenfalls substituiertes $(C_1-C_{30})$Alkyl, gegebenenfalls substituiertes $(C_1-C_{30})$Alkoxy oder (Di)$(C_1-C_{30})$(alkyl)amino, das gegebenenfalls an der Alkylgruppe bzw. den Alkylgruppen durch eine Hydroxylgruppe substituiert ist, steht;
- **Z** für eine CH-Gruppe oder ein Stickstoffatom, vorzugsweise CH, steht;
- **Q'** wie oben definiert ist und insbesondere für ein Halogenid wie Chlorid oder ein Alkylsulfat wie Methylsulfat oder Mesityl steht;

mit der Maßgabe, dass:

- der Chromophor **(II-1)** oder **(IV-1)** über **$R'^2$, $R'^1$, $R'^4$** oder **$R_a$** an den Rest des Moleküls der Formel **(I)** gebunden ist, wobei in diesem Fall eines der Wasserstoffatome von **$R'^2$, $R'^1$** oder **$R'^4$** im Fall von p = 1 oder p' = 1 durch **X** oder **X'** oder alternativ dazu im Fall von p = 0 oder p' = 0 durch **$C_{sat}$** oder **$C_{sat'}$** ersetzt ist und
- mindestens eine der $R'^1$-, $R'^2$-, $R'^3$-, $R'^4$- und $R_a$-Gruppen mindestens eine lineare oder verzweigte, gesättigte oder ungesättigte aliphatische $C_{10}-C_{30}$-Kette trägt; vorzugsweise $R'^1$ für eine lineare oder verzweigte $(C_{10}-C_{30})$Alkylgruppe oder eine lineare oder verzweigte $(C_{10}-C_{30})$Alkenylgruppe steht;

spezieller die Chromophore **(A)** und **(A')** von den folgenden Farbstoffen abgeleitet sind:

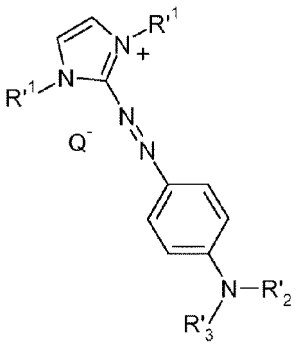 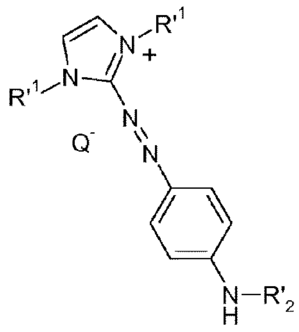 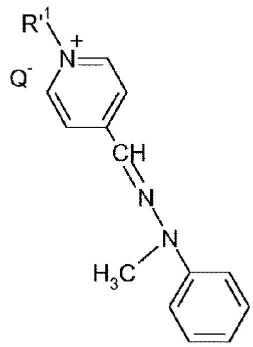

wobei:

- **Q'** wie oben definiert ist und insbesondere für ein Halogenid wie Chlorid oder ein Alkylsulfat wie Methylsulfat oder Mesityl steht,
- wobei R'[1] für eine lineare oder verzweigte ($C_{10}$-$C_{30}$)Alkylgruppe oder eine lineare oder verzweigte ($C_{10}$-$C_{30}$)Alkenylgruppe,vorzugsweise eine lineare ($C_{10}$-$C_{30}$)Alkylgruppe, steht.

**8.** Verfahren nach einem der vorhergehenden Ansprüche, bei dem es sich bei dem Farbstoff bzw. den Farbstoffen der Formel **(I)** um Disulfidfarbstoffe handelt, die aus denjenigen der folgenden Formeln ausgewählt sind:

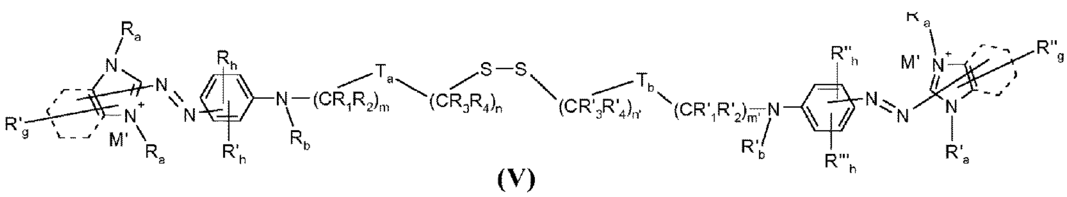

**(V)**

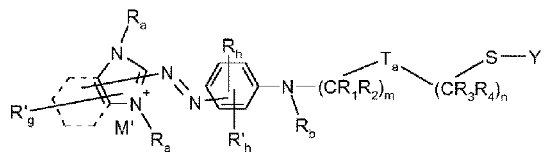

**(V')**

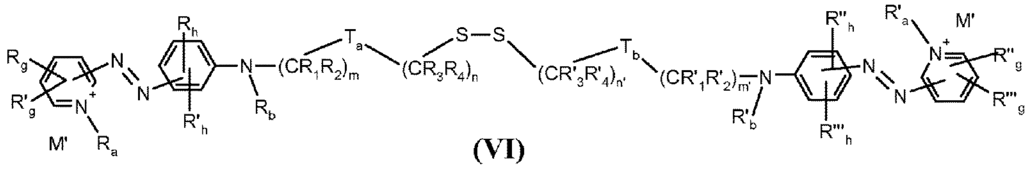

**(VI)**

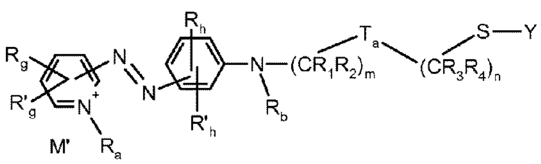

**(VI')**

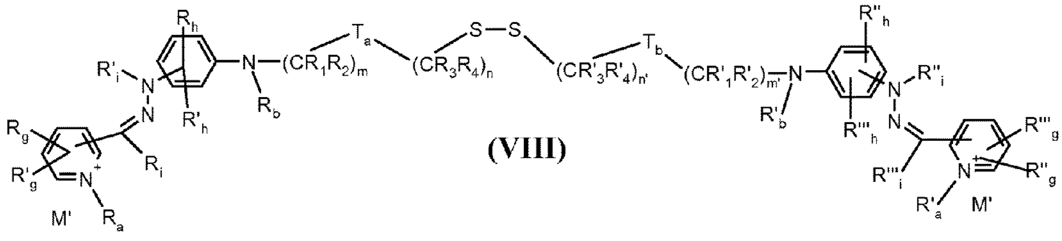

(VIII)

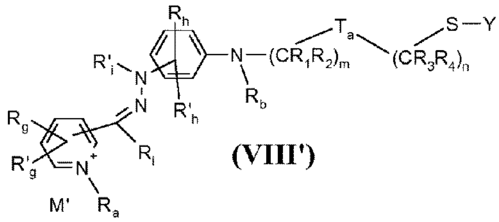

(VIII')

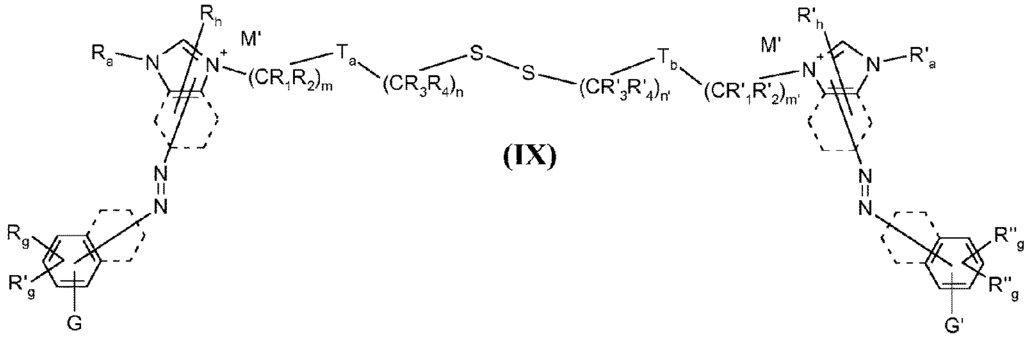

(IX)

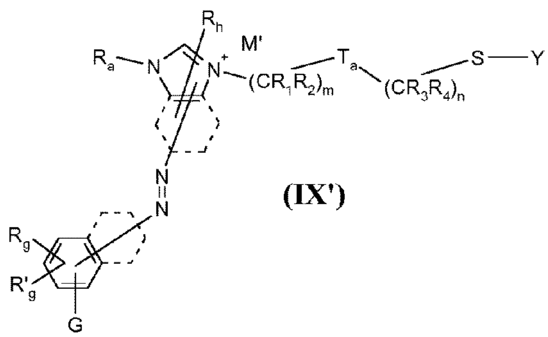

(IX')

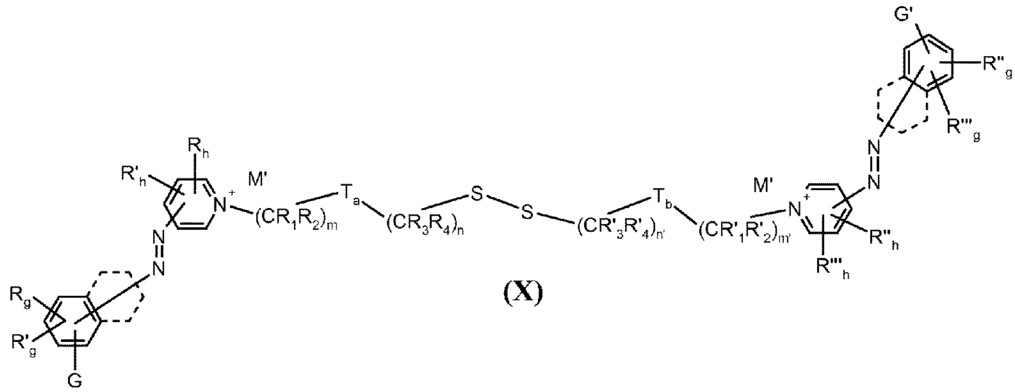

(X)

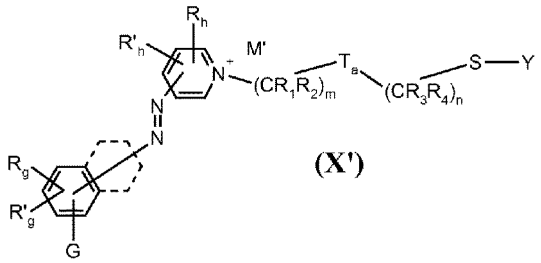

(X')

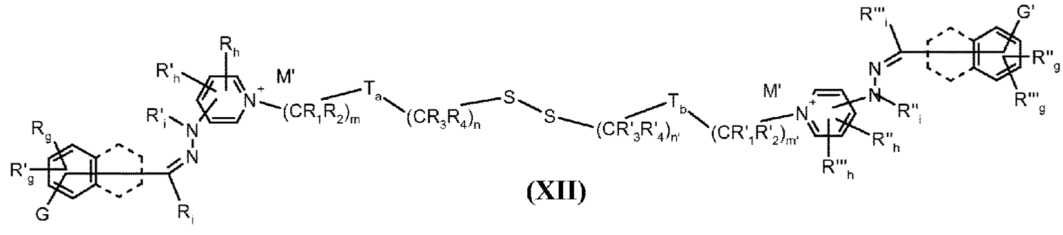

(XII)

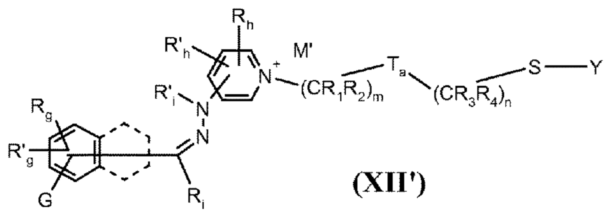

(XII')

wobei in den Formeln **(V)** bis **(XII)** und **(V')** bis **(XII')**:

- G und G', die gleich oder verschieden sein können, für ein Wasserstoffatom oder i) eine-$NR_cR_d$- oder -$NR'_cR'_d$-Gruppe; ii) gegebenenfalls substituiertes, vorzugsweise unsubstituiertes, $C_1$-$C_6$-Alkoxy oder iii) eine lineare oder verzweigte ($C_{10}$-$C_{30}$) Alkoxygruppe oder eine lineare oder verzweigte ($C_{10}$-$C_{30}$) Alkenyloxygruppe stehen;
- $R_a$ und $R'_a$, die gleich oder verschieden sein können, für eine Aryl ($C_1$-$C_4$)alkylgruppe oder eine $C_1$-$C_6$-Alkylgruppe, die gegebenenfalls durch eine Aminogruppe substituiert ist, eine lineare oder verzweigte ($C_{10}$-$C_{30}$) Alkylgruppe oder eine lineare oder verzweigte ($C_{10}$-$C_{30}$) Alkenylgruppe stehen; vorzugsweise $R_a$ und $R'_a$ für eine lineare oder verzweigte ($C_{10}$-$C_{30}$) Alkylgruppe stehen;
- $R_b$ und $R'_b$, die gleich oder verschieden sein können, für ein Wasserstoffatom, eine Aryl($C_1$-$C_4$)alkylgruppe oder eine gegebenenfalls substituierte $C_1$-$C_6$-Alkylgruppe stehen;
- $R_c$, $R'_c$, $R_d$ und $R'_d$, die gleich oder verschieden sein können, für ein Wasserstoffatom, eine Aryl($C_1$-$C_4$)alkylgruppe, eine gegebenenfalls substituierte $C_1$-$C_6$-Alkylgruppe oder eine lineare oder verzweigte ($C_{10}$-$C_{30}$)Alkylgruppe oder eine lineare oder verzweigte ($C_{10}$-$C_{30}$)Alkenylgruppe stehen; $R_c$, $R'_c$, $R_d$ und $R'_d$ vorzugsweise für ein Wasserstoffatom, eine gegebenenfalls substituierte $C_1$-$C_6$-Alkylgruppe oder eine lineare oder verzweigte ($C_{10}$-$C_{30}$)Alkylgruppe stehen;
oder alternativ dazu zwei benachbarte Reste $R_c$ und $R_d$, $R'_c$ und $R'_d$, die von demselben Stickstoffatom getragen werden, zusammen eine heterocyclische Gruppe oder Heteroarylgruppe bilden; vorzugsweise der Heterocyclus bzw. das Heteroaryl monocyclisch und 5- bis 7-gliedrig ist; weiter bevorzugt die Gruppen aus Imidazolyl und Pyrrolidinyl ausgewählt sind;
- $R_g$, $R'_g$, $R''_g$, $R'''_g$, $R_h$, $R'_h$, $R''_h$ und $R'''_h$, die gleich oder verschieden sein können, für ein Wasserstoffatom, ein Halogenatom, eine Amino-, $C_1$-$C_4$-Alkylamino-, $C_1$-$C_4$-Dialkylamino-, Cyano-, Carboxyl-, Hydroxyl- oder Trifluormethylgruppe, einen Acylamino-, $C_1$-$C_4$-Alkoxy-, (Poly)hydroxy-($C_2$-$C_4$)alkoxy-, Alkylcarbonyloxy-, Alkoxycarbonyl- oder Alkylcarbonylaminorest, einen Acylamino-, Carbamoyl- oder Alkylsulfonylaminorest, einen Aminosulfonylrest oder einen $C_1$-$C_{16}$-Alkylrest, der gegebenenfalls durch eine Gruppe, die aus $C_1$-$C_{12}$-Alkoxy, Hydroxyl, Cyano, Carboxyl, Amino, $C_1$-$C_4$-Alkylamino und $C_1$-$C_4$-Dialkylamino ausgewählt ist, substituiert ist oder alternativ dazu zwei Alkylreste, die von dem Stickstoffatom der Aminogruppe getragen werden, einen 5- bis 7-gliedrigen Heterocyclus bilden, der gegebenenfalls ein weiteres Heteroatom, das mit dem Stickstoffatom identisch oder davon verschieden ist, umfasst, stehen; vorzugsweise $R_g$, $R'_g$, $R''_g$, $R'''_g$, $R_h$, $R'_h$, $R''_h$ und $R'''_h$ für ein Wasserstoff- oder Halogenatom oder eine $C_1$-$C_3$-Alkylgruppe stehen;

• oder alternativ dazu zwei Gruppen $R_g$ und $R'_g$; $R''_g$ und $R'''_g$; $R_h$ und $R'_h$; $R''_h$ und $R'''_h$, die von zwei benachbarten Kohlenstoffatomen getragen werden, zusammen einen Benzo- oder Indenoring, eine anellierte Heterocycloalkylgruppe oder eine anellierte Heteroarylgruppe bilden; wobei der Benzo-, Indeno-, Heterocycloalkyl- oder Heteroarylring gegebenenfalls durch ein Halogenatom, eine Amino-, $C_1$-$C_4$-Alkylamino-, $C_1$-$C_4$-Dialkylamino-, Nitro-, Cyano-, Carboxyl-, Hydroxyl- oder Trifluormethylgruppe, einen Acylamino-, $C_1$-$C_4$-Alkoxy-, (Poly)hydroxy($C_2$-$C_4$)alkoxy-, Alkylcarbonyloxy-, Alkoxycarbonyl- oder Alkylcarbonylaminorest, einen Acylamino-, Carbamoyl- oder Alkylsulfonylaminorest, einen Aminosulfonylrest oder einen $C_1$-$C_{16}$-Alkylrest, der gegebenenfalls durch eine Gruppe, die aus $C_1$-$C_{12}$-Alkoxy, Hydroxyl, Cyano, Carboxyl, Amino, $C_1$-$C_4$-Alkylamino und Di($C_1$-$C_4$)alkylamino ausgewählt ist, substituiert ist, substituiert ist oder alternativ dazu zwei Alkylreste, die von dem Stickstoffatom der Aminogruppe getragen werden, einen 5- bis 7-gliedrigen Heterocyclus bilden, der gegebenenfalls ein weiteres Heteroatom, das mit dem Stickstoffatom identisch oder davon verschieden ist, umfasst; vorzugsweise $R_g$ und $R'_g$; $R''_g$ und $R'''_g$ zusammen eine Benzogruppe bilden;

• oder alternativ dazu dann, wenn G für $-NR_cR_d$ steht und G' für $-NR'_cR'_d$ steht, zwei Gruppen $R_c$ und $R'_g$; $R'_c$ und $R''_g$; $R_d$ und $R_g$; $R'_d$ und $R'''_g$ zusammen ein gesättigtes Heteroaryl oder einen gesättigten Heterocyclus, das bzw. der gegebenenfalls durch eine oder mehrere Gruppen ($C_1$-$C_6$)Alkyl substituiert ist, vorzugsweise einen 5- bis 7-gliedrigen Heterocyclus mit einem oder zwei Heteroatomen, die aus Stickstoff und Sauerstoff ausgewählt sind, bilden; weiter bevorzugt der Heterocyclus aus Morpholinyl-, Piperazinyl-, Piperidyl- und Pyrrolidinylgruppen ausgewählt ist;

• $R_i$, $R'_i$, $R''_i$, und $R'''_i$, die gleich oder verschieden sein können, für ein Wasserstoffatom oder eine $C_1$-$C_4$-Alkylgruppe stehen;

• $R_1$, $R_2$, $R_3$, $R_4$, $R'_1$, $R'_2$, $R'_3$ und $R'_4$, die gleich oder verschieden sein können, für ein Wasserstoffatom oder eine $C_1$-$C_4$-Alkyl-, $C_1$-$C_4$-Alkoxy-, Hydroxyl-, Cyano-, Carboxyl-, Amino-, $C_1$-$C_4$-Alkylamino- oder $C_1$-$C_4$-Dialkylaminogruppe stehen, wobei die Alkylreste mit dem Stickstoffatom, das sie trägt, einen 5- bis 7-gliedrigen Heterocyclus, der gegebenenfalls ein weiteres Heteroatom, das mit dem Stickstoffatom identisch oder davon verschieden ist, umfasst, bilden können; vorzugsweise $R_1$, $R_2$, $R_3$, $R_4$, $R'_1$, $R'_2$, $R'_3$ und $R'_4$ für Wasserstoffatome oder eine Aminogruppe stehen; weiter bevorzugt $R_1$, $R_2$, $R_3$, $R_4$, $R'_1$, $R'_2$, $R'_3$ und $R'_4$ für ein Wasserstoffatom stehen;

• $T_a$ und $T_b$, die gleich oder verschieden sein können, für i) entweder eine kovalente $\sigma$-Bindung, ii) oder einen oder mehrere Reste oder Kombinationen davon, die aus $-SO_2-$, -O-, -S-, -N(R)-, $-N^+(R)(R^\circ)-$ und -C(O)- ausgewählt sind, wobei R, R°, die gleich oder verschieden sein können, für ein Wasserstoffatom, einen $C_1$-$C_4$-Alkylrest, $C_1$-$C_4$-Hydroxyalkylrest oder einen Aryl($C_1$-$C_4$)alkylrest stehen; vorzugsweise $T_a$ mit $T_b$ identisch ist und für eine kovalente $\sigma$-Bindung oder eine Gruppe, die aus -N(R)-, -C(O)-N(R)-, -N(R)-C(O)-, -O-C(O)-, -C(O)-O-und $-N^+(R)(R^\circ)-$, $Q^-$ ausgewählt ist, stehen, wobei R, R°, die gleich oder verschieden sein können, für ein Wasserstoffatom oder eine $C_1$-$C_4$-Alkylgruppe; eine gegebenenfalls substituierte $C_1$-$C_6$-Alkylgruppe oder eine lineare oder verzweigte ($C_{10}$-$C_{30}$)Alkylgruppe stehen und $Q^-$ für ein anionisches Gegenion steht; weiter bevorzugt $T_a$ und $T_b$ für eine $\sigma$-Bindung oder -C(O)-N(R)- oder -N(R)-C(O)- stehen; iii) einen kationischen oder nichtkationischen, vorzugsweise monocyclischen, vorzugsweise identischen, Heterocycloalkyl- oder Heteroarylrest, der vorzugsweise zwei Heteroatome (weiter bevorzugt zwei Stickstoffatome) enthält und vorzugsweise 5 bis 7 Ringglieder umfasst, wie Imidazolium, stehen;

•

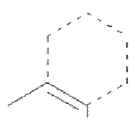

für eine Aryl- oder Heteroarylgruppe, die an den Imidazolium- oder Phenylring anelliert ist, steht oder alternativ dazu bei dem Imidazolium- oder Phenylring fehlt; insbesondere der Ring dann, wenn er vorhanden ist, ein Benzo ist; vorzugsweise bei dem Imidazolium- oder Phenylring fehlt;

• m, m', n und n', die gleich oder verschieden sein können, für eine ganze Zahl zwischen 0 und 6 inklusive stehen, wobei m+n und m'+n' für eine ganze Zahl zwischen 1 und 10 inklusive stehen; vorzugsweise m+n = m'+n' = eine ganze Zahl zwischen 2 und 4 inklusive; weiter bevorzugt m+n = m'+n' = eine ganze Zahl mit einem Wert von 2;

• **Y** wie in Anspruch 1 definiert ist; insbesondere Y für ein Wasserstoffatom oder eine Schutzgruppe wie:

- ($C_1$-$C_4$)Alkylcarbonyl, beispielsweise Methylcarbonyl oder Ethylcarbonyl;
  - Arylcarbonyl wie Phenylcarbonyl;

➢ $(C_1-C_4)$Alkoxycarbonyl;

➢ Aryloxycarbonyl;

➢ Aryl$(C_1-C_4)$alkoxycarbonyl;

➢ (Di)$(C_1-C_4)$(alkyl)aminocarbonyl wie Dimethylaminocarbonyl;

➢ $(C_1-C_4)$(Alkyl)arylaminocarbonyl;

➢ gegebenenfalls substituiertes Aryl wie Phenyl;

➢ 5- oder 6-gliedriges monocyclisches Heteroaryl wie Imidazolyl oder Pyridyl;

➢ kationisches 5- oder 6-gliedriges monocyclisches Heteroaryl wie Pyrylium, Pyridinium, Pyrimidinium, Pyrazinium, Pyridazinium, Triazinium, Imidazolium; wobei diese Gruppen gegebenenfalls durch eine oder mehrere gleiche oder verschiedene $(C_1-C_4)$Alkylgruppen wie Methyl substituiert sind;

➢ kationisches 8- bis 11-gliedriges bicyclisches Heteroaryl wie Benzimidazolium oder Benzoxazolium; wobei diese Gruppen gegebenenfalls durch eine oder mehrere gleiche oder verschiedene $(C_1-C_4)$Alkylgruppen wie Methyl substituiert sind;

➢ kationischer Heterocyclus der nachstehenden Formel:

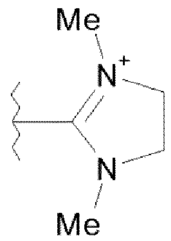

An'''-

➢ $-C(NH_2)=N^+H_2$; An'''- ; wobei An'''- für ein anionisches Gegenion gemäß obiger Definition steht;

➢ $-C(NH_2)=NH$;

➢ $SO_3^-$, $M^+$, wobei $M^+$ für ein Alkalimetall wie Natrium oder Kalium steht;

steht; vorzugsweise Y für ein Wasserstoffatom steht; und

• **M'** für ein anionisches Gegenion steht, das sich von einem Salz einer organischen Säure oder Mineralsäure oder von einer organischen Base oder Mineralbase ableitet und die elektrische Neutralität des Moleküls gewährleistet;

• mit der Maßgabe, dass mindestens eine der G-, G'-, $R_a$-, $R'_a$-, $R_c$-, $R'_c$-, $R_d$- und $R'_d$- Gruppen mindestens eine lineare oder verzweigte $(C_{10}-C_{30})$Alkylgruppe oder eine lineare oder verzweigte $(C_{10}-C_{30})$Alkenylgruppe, vorzugsweise mindestens eine lineare oder verzweigte, weiter bevorzugt lineare, $(C_{10}-C_{30})$Alkylgruppe trägt; insbesondere die Farbstoffe der Formel **(I)** aus Disulfid-, Thiol- oder geschützten Thiol-Azochromophor-Farbstoffen, vorzugsweise aus den Formeln **(V)** und **(V'),** gemäß obiger Definition ausgewählt sind, vorzugsweise wobei $\mathbf{R_a}$ und $\mathbf{R'_a}$, die gleich oder verschieden sind, für eine lineare oder verzweigte, weiter bevorzugt lineare, $(C_{10}-C_{30})$Alkylgruppe stehen.

**9.** Färbeverfahren nach einem der vorhergehenden Ansprüche, bei dem es sich bei dem Farbstoff bzw. den Farbstoffen der Formel **(I)** um Farbstoffe handelt, die aus denjenigen der nachstehenden Formeln **(XIII)** bis **(XIII')** ausgewählt sind:

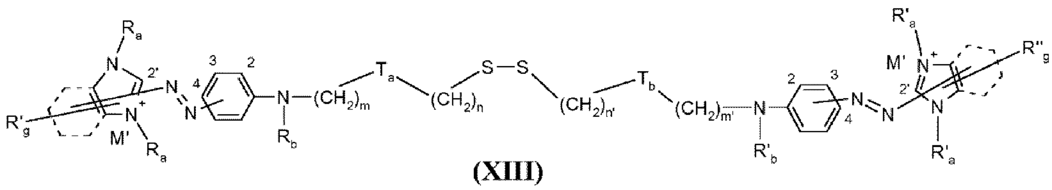

**(XIII)**

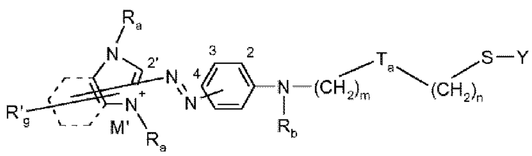

**(XIII')**,

Salzen davon mit organischen Säuren oder Mineralsäuren, optischen Isomeren und geometrischen Isomeren davon und Solvaten wie Hydraten ausgewählt sind;
wobei in den Formeln **(XIII)** bis **(XIII')**:

- **$R_a$** und **$R'_a$,** die gleich oder verschieden sein können, für eine lineare oder verzweigte ($C_{10}$-$C_{30}$)Alkylgruppe oder eine lineare oder verzweigte ($C_{10}$-$C_{30}$) Alkenylgruppe stehen; vorzugsweise $R_a$ und $R'_a$ gleich sind und für eine lineare oder verzweigte, insbesondere lineare, ($C_{10}$-$C_{30}$) Alkylgruppe stehen;
- **$R_b$** und **$R'_b$,** die gleich oder verschieden sein können, für ein Wasserstoffatom oder eine $C_1$-$C_6$-Alkylgruppe stehen;
- **$R'_g$** und **$R''_g$,** die gleich oder verschieden sein können, für ein Wasserstoffatom, ein Halogenatom, eine Amino-, $C_1$-$C_4$-Alkylamino-, $C_1$-$C_4$-Dialkylamino-, Cyano-, Carboxy- oder Hydroxylgruppe, einen Acylamino-, $C_1$-$C_4$-Alkoxy-, Alkylcarbonyloxy-, Alkoxycarbonyl- oder Alkylcarbonylaminorest, einen Acylamino-, Carbamoyl- oder Alkylsulfonylaminorest, einen Aminosulfonylrest oder einen $C_1$-$C_6$-Alkylrest stehen; insbesondere $R'_g$ und $R''_g$ für ein Wasserstoffatom oder eine ($C_1$-$C_4$)Alkylgruppe, vorzugsweise Wasserstoff, stehen;
- **$T_a$** und **$T_b$,** die gleich oder verschieden sein können, für i) entweder eine kovalente $\sigma$-Bindung, ii) oder einen oder mehrere Reste oder Kombinationen davon, die aus -O-, -N(R)-, -$N^+$(R)(R°)-, $Q^-$ und -C(O)- ausgewählt sind, wobei R, R°, die gleich oder verschieden sein können, für ein Wasserstoffatom oder einen $C_1$-$C_4$-Alkylrest stehen, stehen; vorzugsweise $T_a$ mit $T_b$ identisch ist und für eine kovalente $\sigma$-Bindung oder eine Gruppe, die aus -N(R)-, -C(O)-N(R)- und -N(R)-C(O)- ausgewählt ist, stehen, wobei R für ein Wasserstoffatom oder eine $C_1$-$C_4$-Alkylgruppe steht; weiter bevorzugt $T_a$ und $T_b$ für eine $\sigma$-Bindung stehen;
- 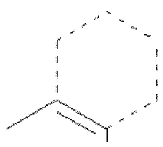

wie im vorhergehenden Anspruch definiert ist; vorzugsweise bei dem Imidazolium- oder Phenylring fehlt;
- **m, m', n** und **n',** die gleich oder verschieden sein können, für eine ganze Zahl zwischen 0 und 6 inklusive stehen, wobei m+n und m'+n' für eine ganze Zahl zwischen 1 und 10 inklusive stehen; vorzugsweise m+n = m'+n' = eine ganze Zahl zwischen 2 und 4 inklusive; weiter bevorzugt m+n = m'+n' = eine ganze Zahl mit einem Wert von 2;
- **Y** für ein Wasserstoffatom oder eine Schutzgruppe steht, die aus:

➢ ($C_1$-$C_4$)Alkylcarbonyl, beispielsweise Methylcarbonyl oder Ethylcarbonyl;

➢ Arylcarbonyl wie Phenylcarbonyl;

➢ ($C_1$-$C_4$)Alkoxycarbonyl;

➢ Aryloxycarbonyl;

- Aryl($C_1$-$C_4$)alkoxycarbonyl;
  - (Di)($C_1$-$C_4$)(alkyl)aminocarbonyl wie Dimethylaminocarbonyl;
  - ($C_1$-$C_4$)(Alkyl)arylaminocarbonyl;
    - gegebenenfalls substituiertem Aryl wie Phenyl;
    - 5- oder 6-gliedrigem monocyclischem Heteroaryl wie Imidazolyl oder Pyridyl;

➤ kationischem 5- oder 6-gliedrigem monocyclischem Heteroaryl wie Pyrylium, Pyridinium, Pyrimidinium, Pyrazinium, Pyridazinium, Triazinium, Imidazolium; wobei diese Gruppen gegebenenfalls durch eine oder mehrere gleiche oder verschiedene ($C_1$-$C_4$)Alkylgruppen wie Methyl substituiert sind;

➤ kationischem 8- bis 11-gliedrigem bicyclischem Heteroaryl wie Benzimidazolium oder Benzoxazolium; wobei diese Gruppen gegebenenfalls durch eine oder mehrere gleiche oder verschiedene ($C_1$-$C_4$)Alkylgruppen wie Methyl substituiert sind;

➤ einem kationischen Heterocyclus der nachstehenden Formel:

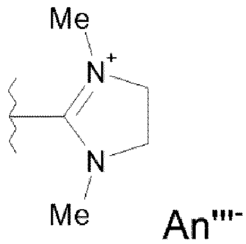

➤ $-C(NH_2)=N^+H_2$ ; An'''⁻; wobei An'''⁻ für ein anionisches Gegenion gemäß obiger Definition steht;

➤ $-C(NH_2)=NH$;

➤ $SO_3^-$, $M^+$, wobei $M^+$ für ein Alkalimetall wie Natrium oder Kalium steht;

ausgewählt ist; vorzugsweise **Y** für ein Wasserstoffatom steht; und

- **M'** für ein anionisches Gegenion steht, das sich von einem Salz einer organischen Säure oder Mineralsäure oder von einer organischen Base oder Mineralbase ableitet und die elektrische Neutralität des Moleküls gewährleistet;

  insbesondere die Azogruppe, die den (Benzo)-imidazolium-Teil mit dem Phenyl verknüpft, in der ortho- oder para-Position zum Pyridinium, d.h. in der 2'-4-, 4-2'-, 2'-4- und 4-2'-Position, vorzugsweise in der 2'-4- und 4-2'-para-Position, steht.

**10.** Färbeverfahren nach einem der vorhergehenden Ansprüche, wobei es sich bei dem Farbstoff bzw. den Farbstoffen der Formel **(I)** um Farbstoffe handelt, die aus denjenigen mit den folgenden Formeln ausgewählt sind:

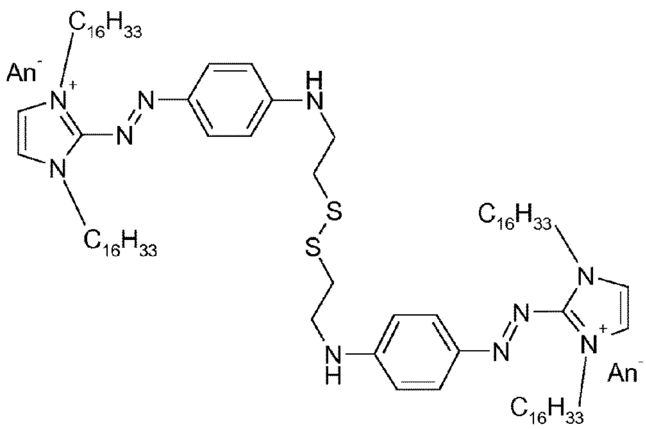

<u>**1**</u>

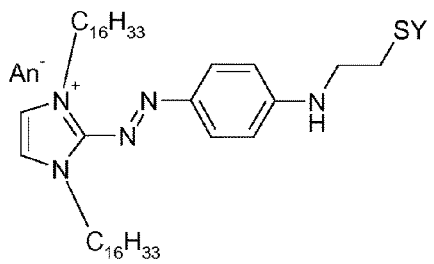

2

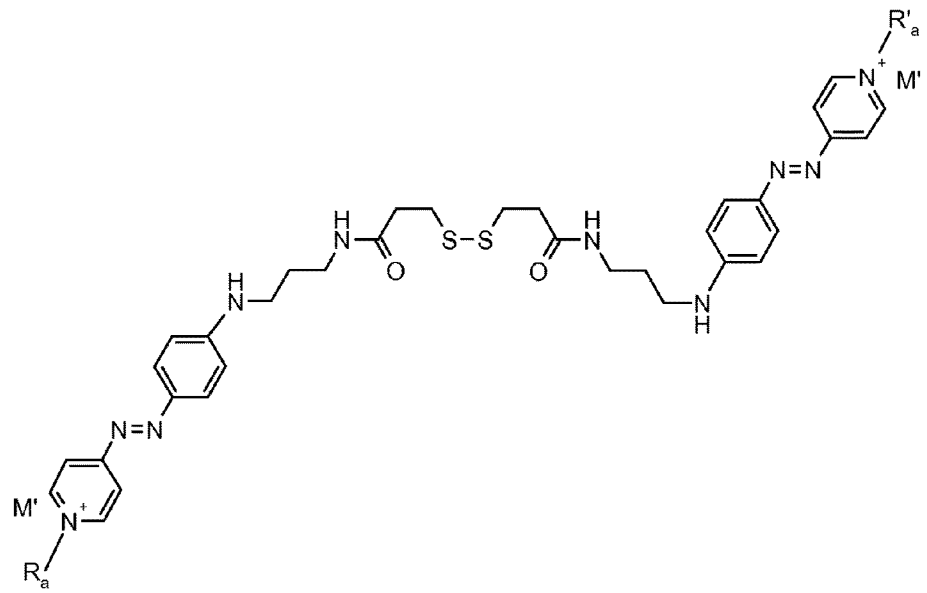

3

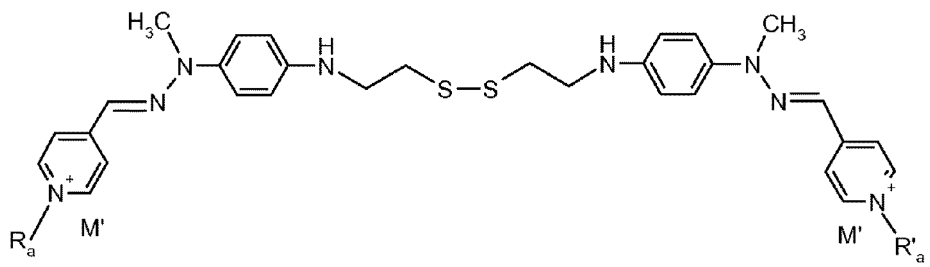

4

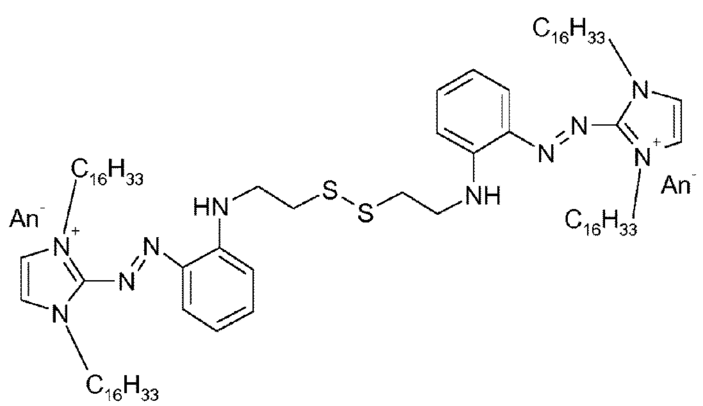

5

wobei **An⁻, M'**, die gleich oder verschieden, vorzugsweise gleich, sein können, für anionische Gegenionen stehen; spezieller das anionische Gegenion aus Halogeniden wie Chlorid, Alkylsulfaten wie Methylsulfat und Mesylat ausgewählt ist; **Y** wie in einem der Ansprüche 1, 8 und 9 definiert ist und vorzugsweise für

- $(C_1-C_4)$Alkylcarbonyl, beispielsweise Methylcarbonyl oder Ethylcarbonyl;
  - Arylcarbonyl wie Phenylcarbonyl; ➢ $(C_1-C_4)$Alkoxycarbonyl;
    - Aryloxycarbonyl;
  - Aryl$(C_1-C_4)$alkoxycarbonyl;
    - gegebenenfalls substituiertes Aryl wie Phenyl;
    - 5- oder 6-gliedriges monocyclisches Heteroaryl wie Imidazolyl oder Pyridyl;
  - $-C(NH_2)=N^+H_2$; An"'-; wobei An'"- für ein anionisches Gegenion gemäß obiger Definition steht; weiter bevorzugt R für ein Wasserstoffatom steht;

steht und
$R'_a$ für eine n-$(C_{14}-C_{20})$Alkylgruppe wie eine n($C_{16}$-Alkylgruppe steht.

**11.** Verfahren nach einem der vorhergehenden Ansprüche, bei dem kein Reduktionsmittel verwendet wird.

**12.** Verfahren nach einem der vorhergehenden Ansprüche, bei dem kein chemisches Oxidationsmittel verwendet wird.

**13.** Verwendung mindestens eines Direktfarbstoffs mit einer Disulfid-, Thiol- oder geschützten Thiolfunktion der Formel **(I)** gemäß einem der Ansprüche 1 bis 12 zum Schützen der Fasern gegen Wärmebehandlungen bei einer Temperatur größer oder gleich 80 °C, die insbesondere durch Wasserdampf und/oder ein Glätteeisen bereitgestellt wird und insbesondere aus einem Dampfeisen stammt.

**14.** Direktfarbstoff mit einer Disulfid-, Thiol- oder geschützten Thiolfunktion der Formel **(I)** gemäß einem der Ansprüche 1 bis 10.

**15.** Zusammensetzung, umfassend einen oder mehrere Direktfarbstoffe mit einer Disulfid-, Thiol- oder geschützten Thiolfunktion der Formel **(I)** gemäß einem der Ansprüche 1 bis 12.

**16.** Verfahren zur Herstellung von Farbstoffen der Formel **(I)** gemäß einem der Ansprüche 1 bis 10, das aus Folgendem besteht:

- entweder Verwenden von zwei Äquivalenten von Reagenzien, die ein Chromophor und mindestens eine nukleofuge Gruppe wie Halogen umfassen **(a),** und einem Äquivalent Aminodisulfid-Verbindung **(b):**

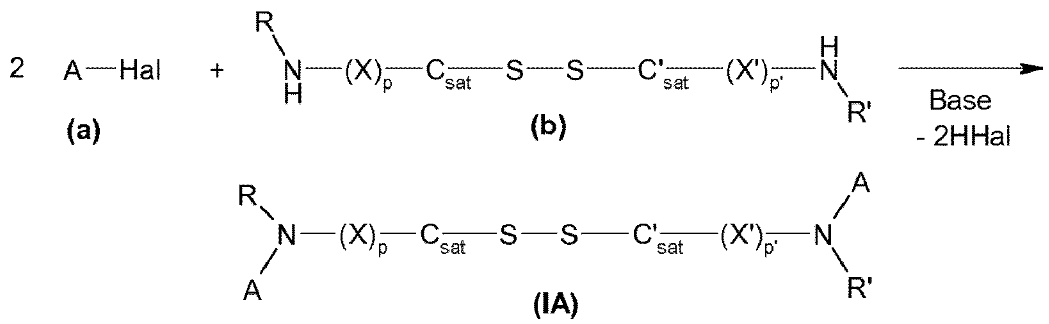

(a) (b) (IA)

wobei **Hal** für eine nukleofuge Gruppe wie Halogen, (Poly)halogen$(C_1-C_4)$alkoxy, (Poly)-halogen$(C_1-C_4)$sulfoxy steht, **X, X', p, p', $C_{sat}$** und **$C'_{sat}$** wie für die Verbindung **(I)** in einem der Ansprüche 1 bis 10 definiert sind, **R** und **R',** für ein Wasserstoffatom oder eine gegebenenfalls substituierte $(C_1-C_4)$Alkylgruppe steht; **A** für einen Chromophor wie oben für **(I)** gemäß einem der Ansprüche 1 bis 10 definiert steht, **R** und **R'** oder auch **A** für einem Chromophor steht, der eine nukleofuge Gruppe wie definiert umfasst, welche mit einem oder zwei Aminoalkylen-Äquivalenten reagiert, gefolgt von einer Alkylierungsreaktion, beispielsweise unter Verwendung von $(C_{10}-C_{30})$Alkylhalogenid, vorzugsweise **A** für einen kationischen Hydrazono-Chromophor der Formeln **(II)** und **(III')** oder kationischen AzoChromophor der Formeln **(IV), (IV'), (V)** und **(V')** gemäß Anspruch 7 steht; wobei diese Reaktion vorzugsweise in einem polaren aprotischen Lösungsmittel, insbesondere am Rückfluss des

Lösungsmittels, durchgeführt wird, vorzugsweise das Lösungsmittel ein Alkohol wie Ethanol ist;
- oder der erste Schritt aus der Herstellung des Disulfid-Reagenz **(c)** besteht, auf das ein Heteroaryl, das durch mindestens eine $(C_1-C_6)$Alkylgruppe substituiert ist, wie ein Pyridin, das durch eine Hydrazonogruppe substituiert ist, **(d)** aufgepfropft wird, welche wiederum mit einem Aryl(thio)aldehyd- oder Aryl(thio)keton-Reagenz, das mindestens eine Gruppe umfasst, die eine lineare oder verzweigte, gesättigte oder ungesättigte aliphatische $C_{10}$-$C_{30}$-Kette trägt, unter Eliminierung von $H_2O$ bzw. $H_2S$ reagiert, was einen Disulfid-Farbstoff mit einem Hydrazono-Chromophor **(I')** sowie optische und geometrische Isomere davon, die zur erfindungsgemäßen Formel (I) gehören, ergibt:

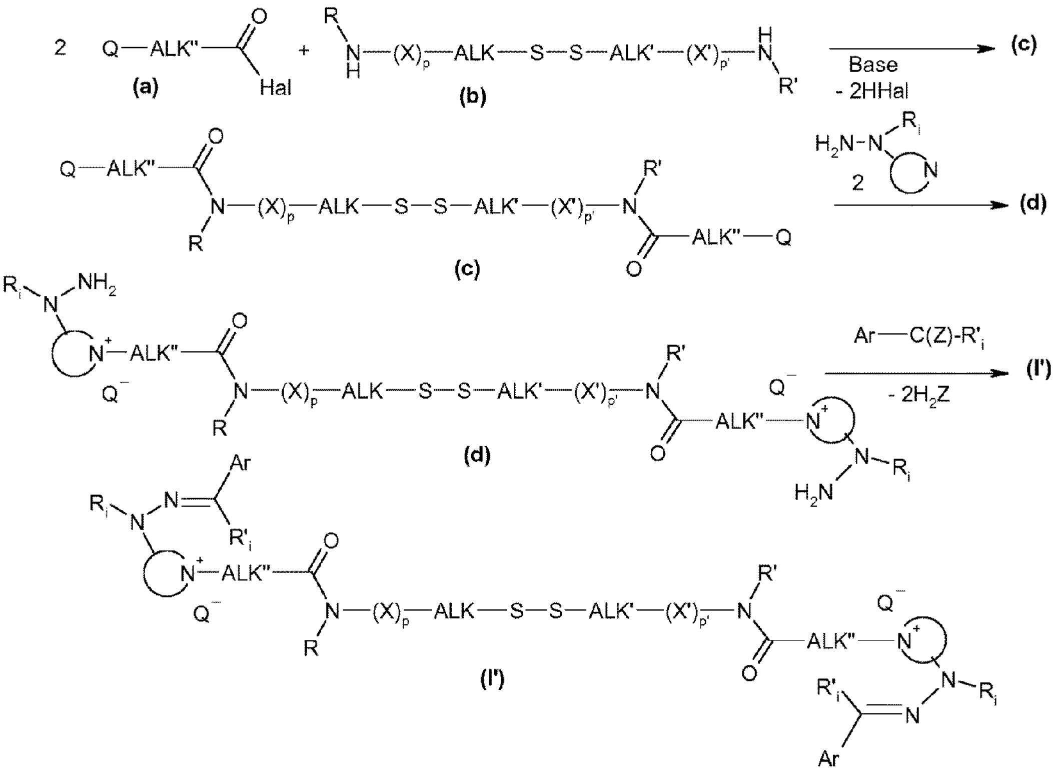


wobei in den Verbindungen **(a)**, **(b)**, **(c)**, **(d)** und **(I')**:

- **Q** für eine elektrofuge Gruppe wie Halogen, (Poly)halogen$(C_1-C_6)$-$S(O)_2$-O-, Alkylsulfonyl $(C_1-C_6)$Alk-$S(O)_2O$- wie Methylsulfonyl und Ethylsulfonyl; Arylsulfonyl Ar-$S(O)_2O$- wie Benzolsulfonyl und Toluolsulfonyl; (Poly)-(hydroxy)$(C_1-C_6)$alkylcarbonyloxy, Alkylsulfonyl Alk-O-S(O)O-; Arylsulfonyl Ar-O-S(O)O- wie Benzolsulfonyl und Toluolsulfonyl; Alkoxysulfonyl Alk-O-$S(O)_2O$-; Aryloxysulfat Ar-O-$S(O)_2O$-, Phosphonyl und Borate wie Tetrahalogenborat steht;
- **Q⁻** für ein anionisches Gegenion steht, das sich von Q gemäß Anspruch 7 ableitet;
- **ALK, ALK', ALK"**, die gleich oder verschieden sein können, für eine gegebenenfalls substituierte lineare oder verzweigte $(C_1-C_6)$Alkylengruppe, wie Ethylen oder Propylen, stehen;
- **Hal**, **X, X', p** und **p'** wie oben definiert sind;
- **Z** für ein Sauerstoff- oder Schwefelatom, vorzugsweise ein Sauerstoffatom, steht;
- $\mathbf{R_i}$ und $\mathbf{R'_i}$ wie oben definiert sind;
- 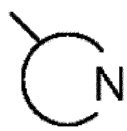

  für eine Heteroarylgruppe mit mindestens einem Stickstoffatom, die durch mindestens eine $(C_1-C_6)$Alkylgruppe, vorzugsweise Methyl, substituiert ist, steht;
-

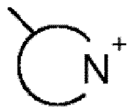

für eine kationische Heteroarylgruppe, die durch mindestens eine ($C_1$-$C_6$)Alkylgruppe, vorzugsweise Methyl, substituiert ist, und mindestens ein Ammonium umfasst, steht;

- **Ar** für eine gegebenenfalls substituierte (Hetero)arylgruppe, vorzugsweise Phenyl, mit mindestens einer Gruppe, die eine lineare oder verzweigte, gesättigte oder ungesättigte aliphatische $C_{10}$-$C_{30}$-Kette trägt, steht;

- oder dann, wenn die Disulfidverbindung einen Alkylen-Linker aufweist, der zwischen dem Chromophor und der Disulfidgruppe nicht durch ein Heteroatom oder eine -N(R)-, -$N^+$(R)(R)-, -O-, -S-, -C(O)-, -$SO_2$-Gruppe oder eine Kombination davon unterbrochen ist, wobei R wie oben definiert ist, und gemäß dem folgenden Syntheseschema hergestellt werden kann:

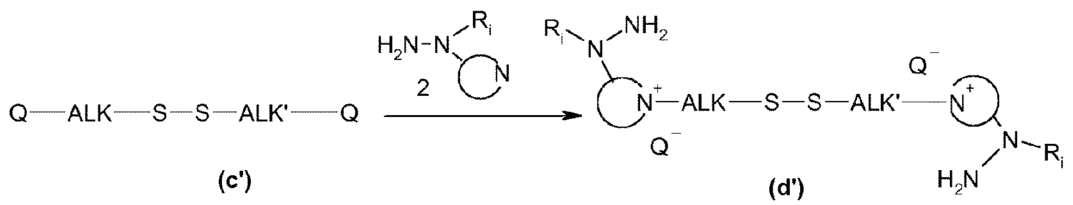
(c') (d')

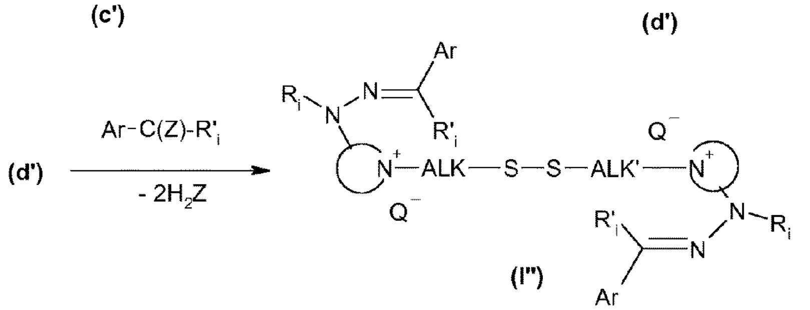

(d') (I'')

wobei ALK, ALK', Q, $Q^-$, $R_1$, Z, $R_1$, $R'_1$, Ar wie oben definiert sind und

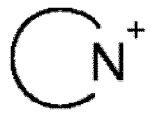

für eine kationische Heteroarylgruppe, die mindestens ein Ammonium umfasst, steht;

- oder Verwenden des Azochromophor-Reagenz **(f)**, das eine Abgangsgruppe GP wie Halogenid, Mesylat oder Tosylat, einen (hetero)aromatischen Teil Ar und eine Heteroarylgruppe mit mindestens einem Stickstoffatom, wie ein (Benzo)imidazolyl, umfasst, das selbst aus einer (hetero)aromatischen Verbindung (e) mit mindestens einer GP und einer Aminogruppe in Gegenwart i) einer Säure, insbesondere in einer Mischung von organischer und anorganischer Säure, vorzugsweise in einer Mischung mit einer Carbonsäure und Salzsäure, und $MNO_2$, wobei M für ein Alkali- oder Erdalkalimetall steht, vorzugsweise in Wasser und bei einer Temperatur zwischen 0 °C und 5 °C, dann ii) einer Base wie Natriumhydroxid oder Kaliumhydroxid, dann iii) eines Heteroaryls mit mindestens einem Stickstoffatom, wie (Benzo)imidazol, vorzugsweise Imidazol, hergestellt wird; wonach die Verbindung **(f)** insbesondere durch $R^a$ eine lineare oder verzweigte, gesättigte oder ungesättigte aliphatische $C_{10}$-$C_{30}$-Kette unter Verwendung eines Reagenz $R_a$-Q, wobei Q für eine Abgangsgruppe wie Halogenid steht, alkyliert werden kann, was das Zwischenprodukt **(g)** ergibt, wonach Letzteres mit dem Diaminosulfid-Reagenz (b) reagieren kann, was die erfindungsgemäße Azoverbindung **(I''')** ergibt:

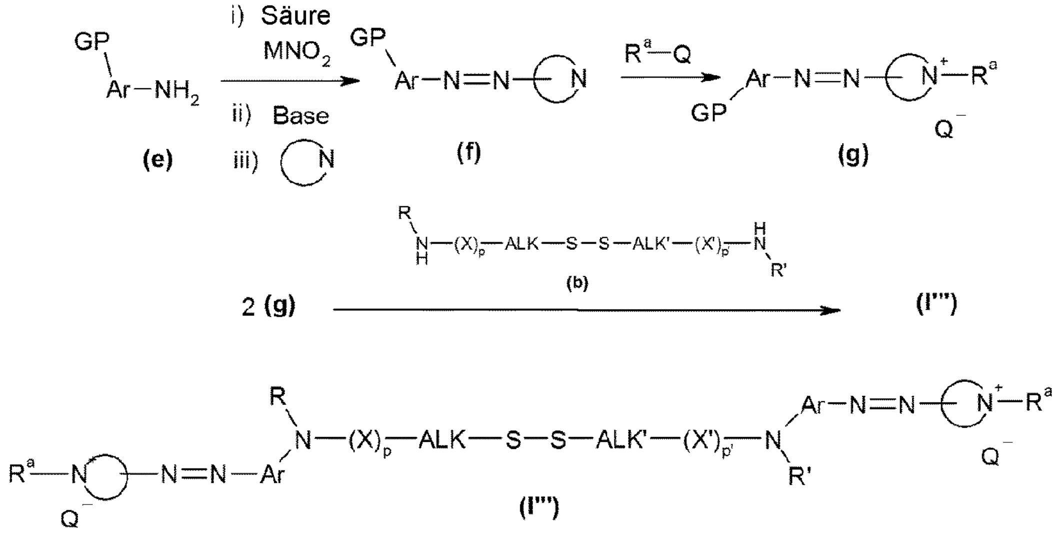


(e) (f) (g)

(b)

2 (g) (I''')

(I''')

wobei ALK, ALK', X, X', p, p', Q, Q⁻, Ar, GP und

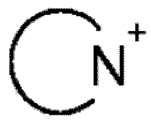

wie oben definiert sind;

R und R', die gleich oder verschieden sein können, für ein Wasserstoffatom oder eine ($C_1$-$C_6$)Alkylgruppe stehen;

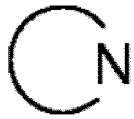

für eine Heteroarylgruppe mit mindestens einem Stickstoffatom steht.

**17.** Verfahren zur Herstellung der Farbstoffe der Formel **(Va)** und **(V')** nach dem vorhergehenden Anspruch, das darin besteht, dass ein durch eine Alkoxy- oder Benzyloxygruppe substituiertes Anilin (h) mit i) einer Säure, vorzugsweise einer Mineralsäure wie Salzsäure oder einer Carbonsäure wie Essigsäure, in Gegenwart einer stickstoffhaltigen Verbindung $MNO_2$, wobei M für ein kationisches Gegenion wie Na oder K steht, dann ii) mit einer organischen Base oder Mineralbase, vorzugsweise eine Mineralbase, wie Natriumhydroxid, dann iii) einem Imidazolylderivat, das gegebenenfalls mit einer (Hetero)arylgruppe, vorzugsweise einer Benzogruppe, anelliert ist, umgesetzt wird, was die Azoverbindung (i) ergibt; wobei Letztere mit mindestens 2 Moläquivalenten eines Alkylierungsmittels wie $R^a$-Q, wobei $R_a$ und Q wie im vorhergehenden Anspruch definiert sind, reagiert, was die Verbindung (j) ergibt;

- wobei entweder zwei Äquivalente von (j) mit einer Aminodisulfid-Verbindung (d) reagieren, was den Disulfid-Farbstoff **(Va)** ergibt;
- oder ein Moläquivalent von (j) mit einer Thiol- oder geschützten Thiolverbindung (d') reagiert, was den Thiol- oder geschützten Thiolen-Farbstoff **(V')** gemäß Anspruch 8 ergibt;

gemäß dem folgenden Schema:

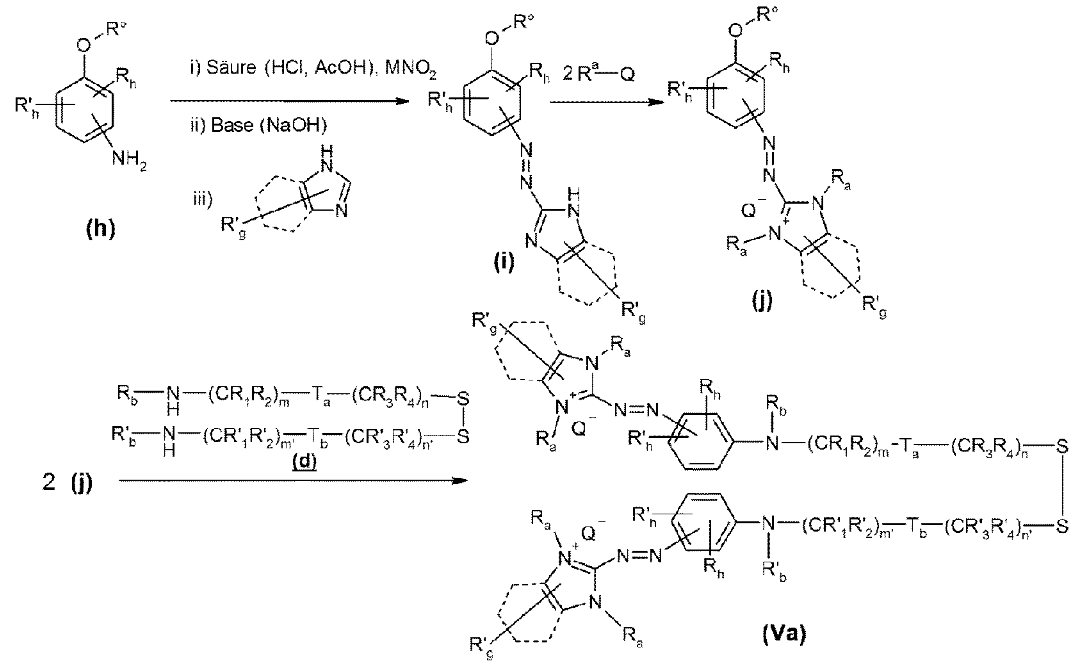


(h) (i) (j)

(Va)

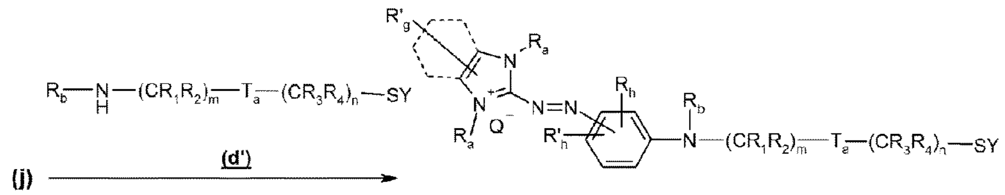


(V')

wobei $R_a$, $R_b$, $R'_b$, $R'_g$, $R_h$, $R'_h$, $R_1$, $R_2$, $R_3$, $R_4$, $R'_1$, $R'_2$, $R'_3$, $R'_4$, Y, m, m', n, n', $T_a$, $T_b$ und

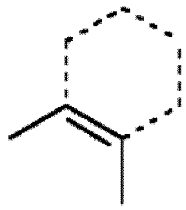

wie in Anspruch 8 oder 9 definiert sind, Q wie im vorhergehenden Anspruch definiert ist und R° für eine $(C_1$-$C_6)$Alkyl-, (Hetero) aryl- oder Benzylgruppe steht.

## Revendications

1. Procédé de coloration des fibres kératiniques consistant à appliquer sur lesdites fibres un ou plusieurs colorant(s) direct(s) cationique(s) à fonction disulfure, à fonction thiol ou à fonction thiol protégé de formule **(I)** :

$$\mathbf{A - (X)_p - C_{sat} - S - U} \qquad \mathbf{(I)}$$

ses sels d'acide organique ou minéral, ses isomères optiques ou géométriques, ses tautomères et leurs solvates ; formule **(I),** dans laquelle :

• **U** représente un radical choisi parmi :

*a)* - **S** - $\mathbf{C'_{sat}}$ - $\mathbf{(X')_{p'}}$ - **A'** ; et

*b)* **- Y ;**

• **A** et **A'**, identiques ou différents, représentent un chromophore cationique ou non cationique, de préférence un chromophore cationique, étant entendu qu'au moins un des deux chromophores porte au moins un groupe à chaîne aliphatique, linéaire ou ramifiée, saturée ou insaturée, en $C_{10}$-$C_{30}$ ;
• **Y** représente i) un atome d'hydrogène ou ii) un groupe protecteur de fonction thiol ;
• **X** et **X'**, identiques ou différents, représentent une chaîne hydrocarbonée divalente en $C_1$-$C_{30}$, linéaire ou ramifiée, saturée ou insaturée, éventuellement interrompue et/ou éventuellement terminée à l'une ou deux de ses extrémités par un ou plusieurs groupes divalents ou leurs combinaisons choisis parmi :

➢ -N(R)-, $-N^+(R)(R)-$, -O-, -S-, -CO-, $-SO_2-$ avec R, identiques ou différents, choisis parmi un hydrogène et un radical alkyle en $C_1$-$C_4$, hydroxyalkyle ou aminoalkyle ;

➢ un radical (hétéro)cyclique aromatique ou non, saturé ou insaturé, condensé ou non comprenant éventuellement un ou plusieurs hétéroatomes identiques ou non, éventuellement substitué ;

• **p** et **p'**, identiques ou différents, valent 0 ou 1 ;
• $\mathbf{C_{sat}}$ et $\mathbf{C'_{sat}}$, identiques ou différents représentent une chaîne alkylène en $C_1$-$C_{18}$, linéaire ou ramifiée, éventuellement substituée, éventuellement cyclique.

**2.** Procédé selon la revendication précédente dans lequel le ou les colorants de formule **(I)** sont des colorants disulfures avec **U** représentant un radical a) - $\mathbf{S\text{-}C'_{sat}\text{-}(X')_{p'}\text{-} A'}$ ; particulièrement les colorants de formule **(I)** selon l'invention sont disulfures et symétriques i.e. la formule **(I)** est de formule suivante :

$$\mathbf{A\text{-}(X)_p\text{-}C_{sa}t\text{-}S\text{-}S\text{-}\ C'_{sat}\ \text{-}\ (X')_{p'}\text{-}\ A'}$$

avec **A = A', X = X', p = p',** $\mathbf{C_{sat} = C'_{sat}}$.

**3.** Procédé selon la revendication 1 dans lequel le ou les colorants de formule **(I)** sont des colorants à fonction thiol ou thiol protégé i.e. **U** représentant le radical *b)* **Y** tel que défini dans la revendication précédente, particulièrement **Y** représente un atome d'hydrogène.

**4.** Procédé de coloration selon l'une quelconque des revendications précédentes dans lequel le ou les colorants de formule **(I)** sont des colorants avec $C_{sat}$ et $C'_{sat}$, identiques ou différents, représentant une chaîne $-(CH_2)_k-$ avec k entier, compris inclusivement entre 1 et 8.

**5.** Procédé selon l'une quelconque des revendications précédentes dans lequel le ou les colorants de formule **(I)** sont des colorants qui, lorsque **p** et **p'** valent 1, **X** et **X'**, identiques ou différents, représentent la séquence suivante :

$$\mathbf{-(T)_t\text{-}(Z)_z\text{-}(T')_{t'}\text{-}}$$

ladite séquence étant reliée dans la formule **(I)** de façon symétrique comme suit :

$$-\ \mathbf{C_{sat}}\ (\text{ou}\ \mathbf{C'_{sat}})\mathbf{\text{-}(T)_t\text{-}(Z)_z\text{-}(A}\ \text{ou}\ \mathbf{A')}\ ;$$

dans laquelle :

• **T** et **T'**, identiques ou différents, représentent un ou plusieurs radicaux ou leurs combinaisons choisis parmi : -O- ; -S- ; -N(R)- ; $-N^+(R)(R^\circ)-$; -S(O)- ; $-S(O)_2-$ ; -C(O)- ; avec R, R°, identiques ou différents, représentant un atome d'hydrogène, un radical alkyle en $C_1$-$C_4$, hydroxyalkyle en $C_1$-$C_4$ ou un aryl($C_1$-$C_4$)alkyle ; et un radical hétérocycloalkyle ou hétéroaryle, cationique ou non, préférentiellement monocyclique, contenant préférentiellement deux hétéroatomes (plus préférentiellement deux atomes d'azote) et comportant préférentiellement de 5 à 7 chaînons, plus préférentiellement imidazolium ;
les indices **t** et **t'**, identiques ou différents, valent 0 ou 1 ;
• **Z** représente :

➢ $-(CH_2)_m-$ avec m entier compris entre 1 et 8 ;

➢ $-(CH_2CH_2O)_q-$ ou $-(OCH_2CH_2)_q-$ dans lesquelles q est un entier compris inclusivement entre 1 et 5 ;

➢ un radical aryle, alkylaryle ou arylalkyle dont le radical alkyle est en $C_1$-$C_4$ et le radical aryle est de préférence en $C_6$, étant éventuellement substitué par au moins un groupe $SO_3M$ avec M représentant un atome d'hydrogène, un métal alcalin ou un groupe ammonium substitué par un ou plusieurs radicaux alkyle, identiques ou non, linéaires ou ramifiés, en $C_1$-$C_{18}$ éventuellement porteurs d'au moins un hydroxy ;

• **z** vaut 0 ou 1.

6. Procédé de coloration des fibres kératiniques selon l'une quelconque des revendications précédentes dans lequel le ou les colorants de formule **(I)** comprennent un chromophore **A** et/ou **A'**, identiques ou différents, issus des colorants acridines suivants :
acridones ; anthranthrones ; anthrapyrimidines ; anthraquinones ; azines ; (poly)azoïques, hydrazono ou hydrazones, en particulier arylhydrazones ; azométhines ; benzanthrones ; benzimidazoles ; benzimidazolones ; benzindoles ; benzoxazoles ; benzopyranes ; benzothiazoles ; benzoquinones ; bisazines ; bis-isoindolines ; carboxanilides ; coumarines ; cyanines telles que les azacarbocyanines, diazacarbocyanines, diazahémicyanines, tétraazacarbocyanines ou (poly)méthines ; diazines ; dicétopyrrolopyrroles ; dioxazines ; diphénylamines ; diphénylméthanes ; dithiazines ; flavonoïdes tels que flavanthrones et flavones ; fluorindines ; formazans ; indamines ; indanthrones ; indigoïdes et pseudo-indigoïdes ; indophénols ; indoanilines ; isoindolines ; isoindolinones ; isoviolanthrones ; lactones ; naphtoquinones ; nitro, notamment les nitro(hétéro)aromatiques ; oxadiazoles ; oxazines ; périlones ; périnones ; pérylènes ; phénazines ; phénoxazine ; phénothiazines ; phthalocyanine ; polyènes/caroténoïdes ; porphyrines ; pyranthrones ; pyrazolanthrones ; pyrazolones ; pyrimidinoanthrones ; pyronines ; quinacridones ; quinolines ; quinophthalones ; squaranes ; tétrazoliums ; thiazines, thioindigo ; thiopyronines ; triarylméthanes, ou xanthènes ; préférentiellement les chromophores sont choisis parmi les chromophores (poly)azoïques, hydrazonos et azométhines ; plus préférentiellement parmi les azoïques.

7. Procédé selon l'une quelconque des revendications précédentes dans lequel le ou les colorants de formule **(I)** comprennent un chromophore **A** et/ou **A'**, identique ou différent, choisi parmi les chromophores cationiques suivants :

**(*) -Hét$^+$-C(R$^a$)=N-N(R$^b$)-Ar, Q$^-$** **(II)**

**Q$^-$, Hét$^+$-C(R$^a$)=N-N(R$^b$)-Ar'- (*),** **(II')**

**(*) -Hét$^+$-N(R$^a$)-N=C(R$^b$)-Ar, Q$^-$** **(III)**

**Q$^-$, Hét$^+$-N(R$^a$)-N=C(R$^b$)-Ar'- (*),** **(III')**

**(*)-Hét$^+$-N=N-Ar, Q$^-$** **(IV)**

**Q$^-$, Hét$^+$-N=N-Ar'-(*),** **(IV')**

**(*) -Ar$^+$-N=N-Ar", Q$^-$** **(V)**

**Q$^-$, Ar$^+$-N=N-Ar"-(*)** **(V')**

formules **(II)** à **(V')** avec :

- **Hét$^+$** représentant un radical hétéroaryle cationique, préférentiellement à charge cationique endocyclique tel que imidazolium, indolium, ou pyridinium, éventuellement substitué, préférentiellement par au moins un groupe ($C_1$-$C_8$)alkyle tel que méthyle ;
- **Ar$^+$** représentant un radical aryle, tel que phényle ou naphtyle, à charge cationique exocyclique, préférentiellement ammonium, particulièrement tri ($C_1$-$C_8$) alkyl-ammonium tel que triméthylammonium ;
- **Ar** représentant un groupe aryle, notamment phényle, éventuellement substitué, préférentiellement par un ou plusieurs groupes électrodonneurs tels que i) ($C_1$-$C_8$)alkyle éventuellement substitué, ii) ($C_1$-$C_8$)alcoxy éventuellement substitué, iii) (di)($C_1$-$C_8$)(alkyl)amino éventuellement substitué sur le ou les groupes alkyle par un groupe hydroxyle, iv) aryl($C_1$-$C_8$)alkylamino, v) *N*-($C_1$-$C_8$)alkyl-*N*-aryl($C_1$-$C_8$)alkylamino éventuellement substitué ou alors **Ar** représentant un groupe julolidine ;
- **Ar'** étant un groupe divalent (hétéro)arylène éventuellement substitué tel que phénylène, particulièrement

para-phénylène, ou naphtalène, éventuellement substitués, préférentiellement par un ou plusieurs groupes $(C_1-C_8)$alkyle, hydroxyle, ou $(C_1-C_8)$alcoxy ;
- **Ar"** étant un groupe (hétéro)aryle éventuellement substitué tel que phényle ou pyrazolyle, éventuellement substitués, préférentiellement par un ou plusieurs groupes $(C_1-C_8)$alkyle, hydroxyle, (di)$(C_1-C_8)$(alkyl)amino, $(C_1-C_8)$alcoxy ou phényle ;
- $\mathbf{R^a}$ et $\mathbf{R^b}$, identiques ou différents, représentant un atome d'hydrogène ou un groupe $(C_1-C_8)$alkyle, éventuellement substitué, préférentiellement par un groupe hydroxyle ;
ou alors le substituant $\mathbf{R^a}$ avec un substituant de **Hét⁺** et/ou $\mathbf{R^b}$ avec un substituant de **Ar** forment ensemble avec les atomes qui les portent un (hétéro)cycloalkyle ;
particulièrement $\mathbf{R^a}$ et $\mathbf{R^b}$, représentant un atome d'hydrogène ou un groupe $(C_1-C_4)$alkyle, éventuellement substitué par un groupe hydroxyle ;
- $\mathbf{Q^-}$ représentant un contre-ion anionique organique ou minéral tel qu'un halogénure ou un alkylsulfate ;
- **(*)** représentant la partie du chromophore reliée au reste de la molécule de formule **(I)** ;
- étant entendu qu'au moins un des groupes $Hét^+$, $Ar^+$, Ar, Ar' porte au moins une chaîne aliphatique, linéaire ou ramifiée, saturée ou insaturée, en $C_{10}-C_{30}$ ; de préférence un groupe $(C_{10}-C_{30})$alkyle, linéaire ou ramifié, ou un groupe $(C_{10}-C_{30})$alcényle, linéaire ou ramifié ;.

**(*)-$Hét^+$-C($R^a$)=N-N($R^b$)-Ar, $Q^-$**
**(II)**

**$Q^-$, $Hét^+$-C($R^a$)=N-N($R^b$)-Ar'-(*),**
**(II')**

**(*)-$Hét^+$-N($R^a$)-N=C($R^b$)-Ar, $Q^-$**
**(III)**

**$Q^-$, $Hét^+$-N($R^a$)-N=C($R^b$)-Ar'-(*),**
**(III')**

**(*)-$Hét^+$-N=N-Ar, $Q^-$** **(IV)**

**$Q^-$, $Hét^+$-N=N-Ar'-(*),** **(IV')**

**(*) -$Ar^+$-N=N-Ar", $Q^-$** **(V)**
**(VI)**

**$Q^-$, $Ar^+$-N=N-Ar" -(*)** **(V) (VI)**

formules **(II)** à **(V')** avec :

- **Hét⁺** représentant un radical hétéroaryle cationique, préférentiellement à charge cationique endocyclique tel que imidazolium, indolium, ou pyridinium, éventuellement substitué, préférentiellement par au moins un groupe $(C_1-C_8)$alkyle tel que méthyle ;
- **Ar⁺** représentant un radical aryle, tel que phényle ou naphtyle, à charge cationique exocyclique, préférentiellement ammonium, particulièrement tri $(C_1-C_8)$ alkyl-ammonium tel que triméthylammonium ;
- **Ar** représentant un groupe aryle, notamment phényle, éventuellement substitué, préférentiellement par un ou plusieurs groupes électrodonneurs tels que i) $(C_1-C_8)$alkyle éventuellement substitué, ii) $(C_1-C_8)$alcoxy éventuellement substitué, iii) (di)$(C_1-C_8)$(alkyl)amino éventuellement substitué sur le ou les groupes alkyle par un groupe hydroxyle, iv) aryl $(C_1-C_8)$alkylamino, v) *N*-$(C_1-C_8)$alkyl-*N*-aryl$(C_1-C_8)$alkylamino éventuellement substitué ou alors **Ar** représente un groupe julolidine ;
- **Ar'** étant un groupe divalent (hétéro)arylène éventuellement substitué tel que phénylène, particulièrement para-phénylène, ou naphtalène, éventuellement substitués, préférentiellement par un ou plusieurs groupes $(C_1-C_8)$ alkyle, hydroxyle, ou $(C_1-C_8)$ alcoxy ;
- **Ar"** étant un groupe (hétéro)aryle éventuellement substitué tel que phényle ou pyrazolyle, éventuellement substitués, préférentiellement par un ou plusieurs groupes $(C_1-C_8)$alkyle, hydroxyle, (di)$(C_1-C_8)$(alkyl)amino, $(C_1-C_8)$alcoxy ou phényle ;
- $\mathbf{R^a}$ et $\mathbf{R^b}$, identiques ou différents, représentant un atome d'hydrogène ou un groupe $(C_1-C_8)$alkyle, éventuellement substitué, préférentiellement par un groupe hydroxyle ;
ou alors le substituant $\mathbf{R^a}$ avec un substituant de **Hét⁺** et/ou $\mathbf{R^b}$ avec un substituant de **Ar** forment ensemble avec les atomes qui les portent un (hétéro)cycloalkyle ;
particulièrement $\mathbf{R^a}$ et $\mathbf{R^b}$, représentant un atome d'hydrogène ou un groupe $(C_1-C_4)$alkyle, éventuellement

substitué par un groupe hydroxyle ;
- **Q⁻** représentant un contre-ion anionique organique ou minéral tel qu'un halogénure ou un alkylsulfate ;
- **(*)** représentant la partie du chromophore reliée au reste de la molécule de formule **(I)** ;
- étant entendu qu'au moins un des groupes $Hét^+$, $Ar^+$, Ar, Ar' porte au moins une chaîne aliphatique, linéaire ou ramifiée, saturée ou insaturée, en $C_{10}$-$C_{30}$ ; de préférence un groupe ($C_{10}$-$C_{30}$)alkyle, linéaire ou ramifié, ou un groupe ($C_{10}$-$C_{30}$)alcényle, linéaire ou ramifié ;

particulièrement les chromophores cationiques **(A)** et **(A')** sont à charges cationiques endocycliques azoïques et hydrazono de formule **(II)** à **(IV')** telles que définies précédemment, plus particulièrement les chromophores cationiques **(A)** et **(A')** sont de formules suivantes :

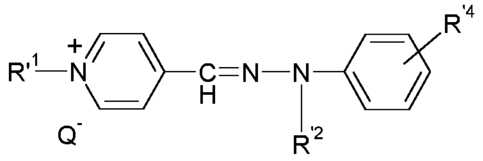

(II-1)

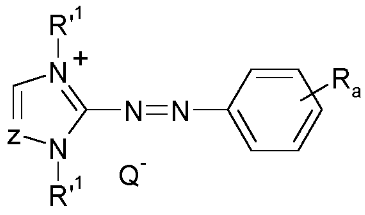

(IV-1)

formules **(II-1)** et **(IV-1)** avec :

- **R'¹** et **R'²,** identiques ou différents, représentant un groupe ($C_1$-$C_{30}$)alkyle linéaire ou ramifié ou ($C_2$-$C_{30}$)alcényle linéaire ou ramifié ;
- $\mathbf{R_a}$ représentant un groupe amino $NR'_2R'_3$ avec $\mathbf{R'_2}$ et $\mathbf{R'_3}$**,** identiques ou différents, représentant un atome d'hydrogène ou un groupe ($C_1$-$C_{30}$)alkyle linéaire ou ramifié ou ($C_2$-$C_{30}$)alcényle linéaire ou ramifié; et
- **R'⁴** représentant un atome d'hydrogène ou un groupe électrodonneur tels que ($C_1$-$C_{30}$)alkyle éventuellement substitué, ($C_1$-$C_{30}$)alcoxy éventuellement substitué, ou (di)($C_1$-$C_{30}$)(alkyl)amino éventuellement substitué sur le ou les groupes alkyle par un groupe hydroxyle ;
- **Z** représente un groupe CH ou un atome d'azote, préférentiellement CH,
- **Q'** étant tel que défini précédemment, particulièrement halogénure tel que chlorure ou un alkylsulfate tel que méthylsulfate ou mésytyle ;

étant entendu que :

- le chromophore **(II-1)** ou **(IV-1)** est relié au reste de la molécule de formule **(I)** par **R'², R'¹, R'⁴** ou $\mathbf{R_a}$ auquel cas un des atomes d'hydrogène de **R'², R'¹** ou **R'⁴** est substitué par **X** ou **X'** si p = 1 ou p'=1 ou alors par $\mathbf{C_{sat}}$ ou $\mathbf{C_{sat'}}$ si p = 0 ou p'=0, et
- au moins un des groupes R'¹, R'², R'³, R'⁴, $R_a$ porte au moins une chaîne aliphatique, linéaire ou ramifiée, saturée ou insaturée, en $C_{10}$-$C_{30}$ ; de préférence R'¹ représentant un groupe ($C_{10}$-$C_{30}$) alkyle, linéaire ou ramifié, ou un groupe ($C_{10}$-$C_{30}$) alcényle, linéaire ou ramifié ;
plus particulièrement les chromophores **(A)** et **(A')** sont issus des colorants suivants :

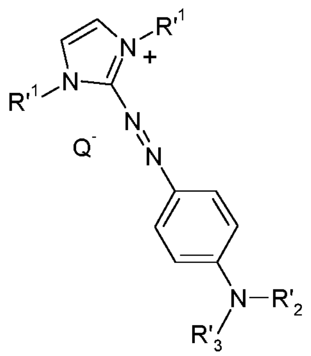

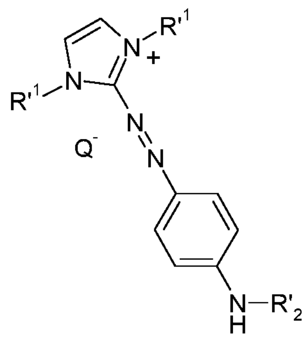

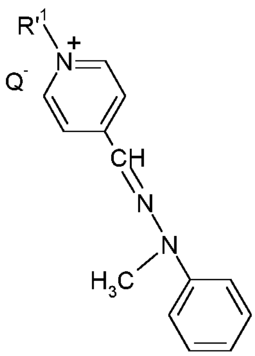

avec :

- **Q'** un contre-ion anionique tel que défini précédemment, particulièrement halogénure tel que chlorure ou un alkylsulfate tel que méthylsulfate ou mésytyle,
- avec $R'^1$ représentant un groupe ($C_{10}$-$C_{30}$) alkyle, linéaire ou ramifié, ou un groupe ($C_{10}$-$C_{30}$) alcényle linéaire ou ramifié, de préférence ($C_{10}$-$C_{30}$) alkyle linéaire.

**8.** Procédé selon l'une quelconque des revendications précédentes dans lequel le ou les colorants de formule **(I)** sont des colorants disulfures choisis parmi ceux de formules suivantes:

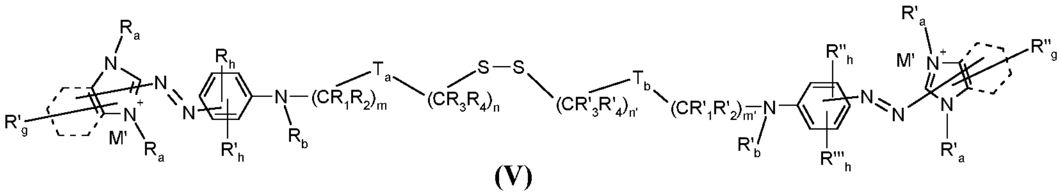

**(V)**

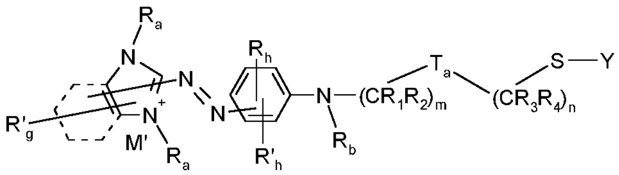

**(V')**

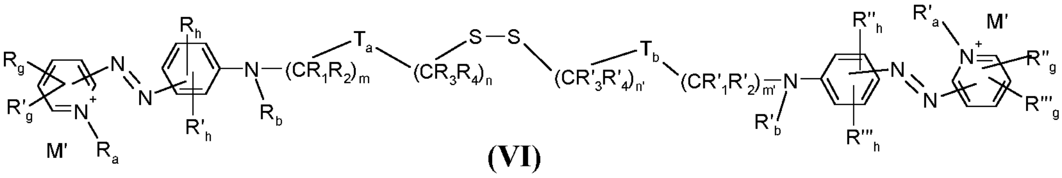

**(VI)**

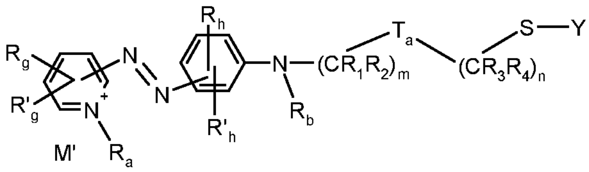

**(VI')**

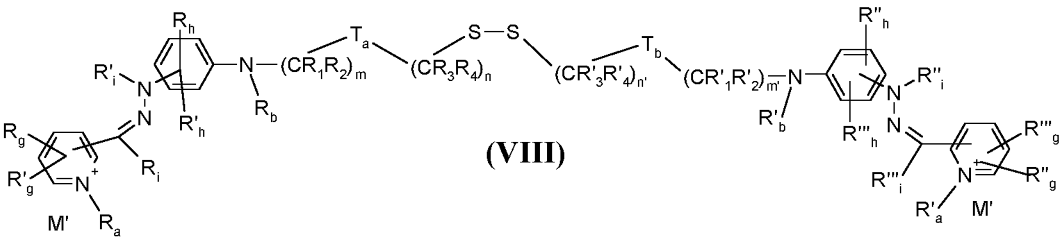

**(VIII)**

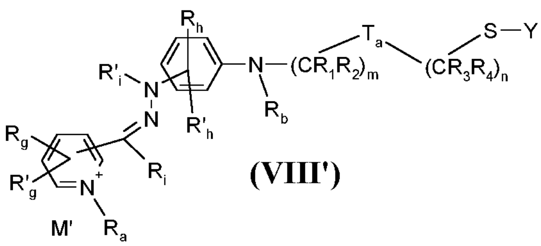

**(VIII')**

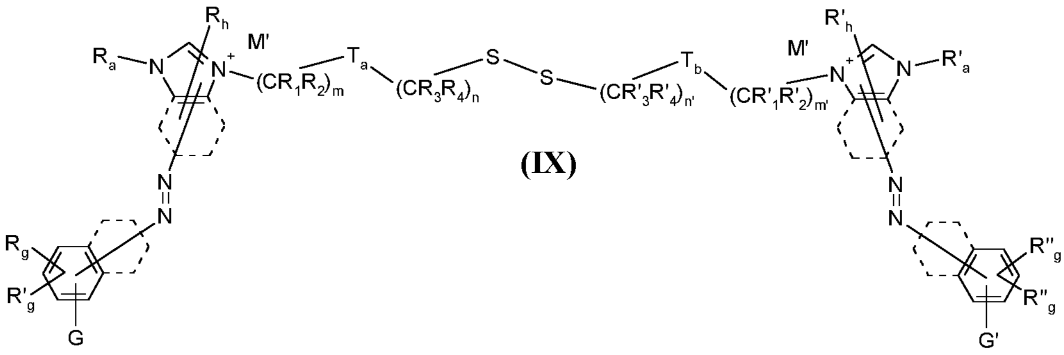

(IX)

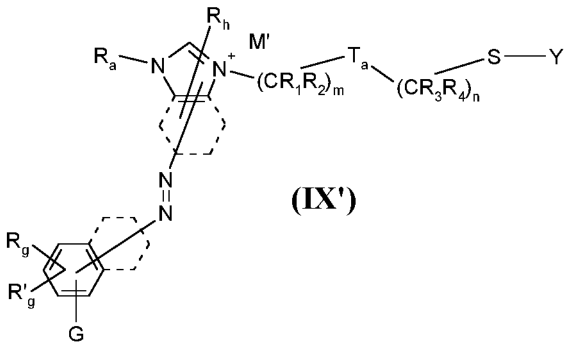

(IX')

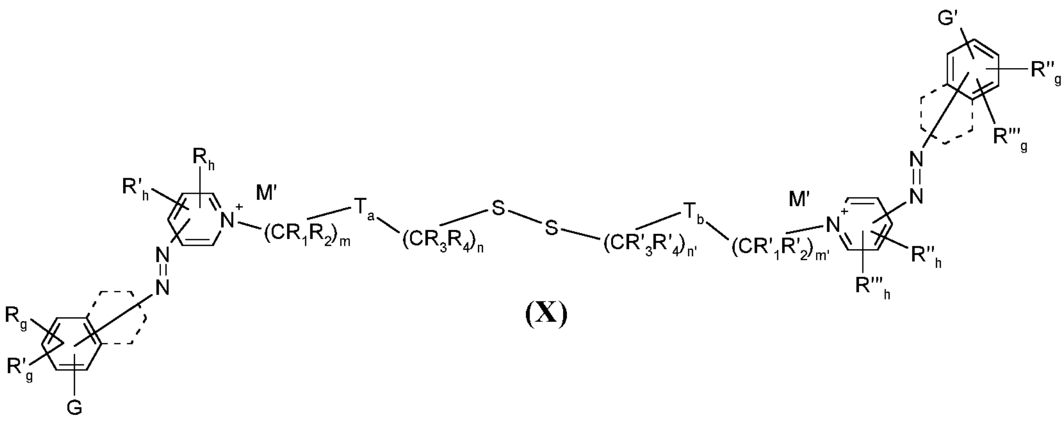

(X)

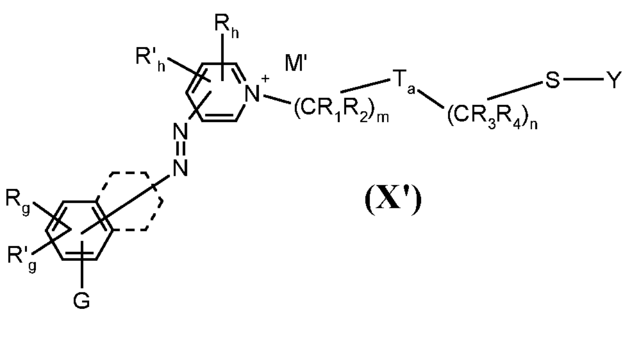

(X')

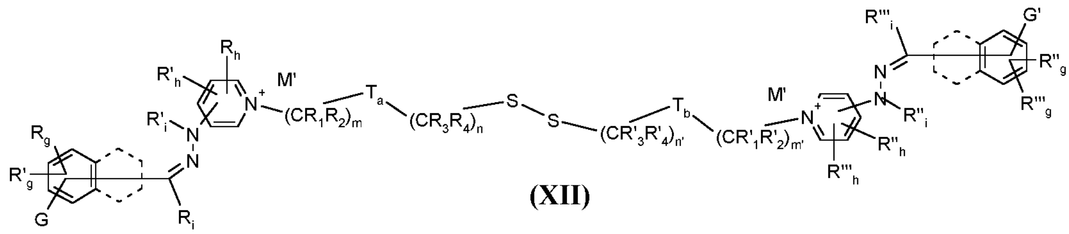

**(XII)**

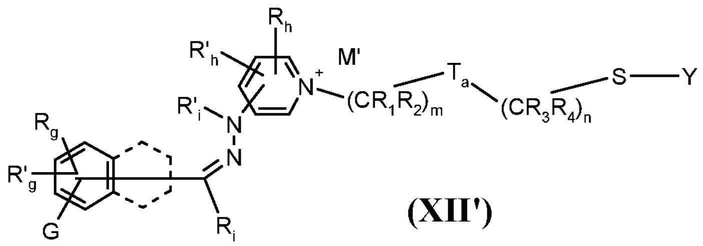

**(XII')**

formules **(V)** à **(XII)** et **(V')** à **(XII')** dans lesquelles :

- G et G', identiques ou différents, représentent un atome d'hydrogène, ou i) un groupe $-NR_cR_d$,$-NR'_cR'_d$, ii) $C_1$-$C_6$ alkoxy éventuellement substitué, préférentiellement non substitué ; ou iii) un groupe ($C_{10}$-$C_{30}$)alkoxy, linéaire ou ramifié, ou un groupe ($C_{10}$-$C_{30}$)alcényloxy, linéaire ou ramifié ;
- $R_a$ et $R'_a$, identiques ou différents, représentent un groupe aryl ($C_1$-$C_4$)alkyle ou un groupe $C_1$-$C_6$ alkyle éventuellement substitué par un groupe hydroxy ou amino, un groupe ($C_{10}$-$C_{30}$)alkyle, linéaire ou ramifié, ou un groupe ($C_{10}$-$C_{30}$)alcényle, linéaire ou ramifié ; préférentiellement $R_a$ et $R'_a$ représentent un groupe ($C_{10}$-$C_{30}$)alkyle, linéaire ou ramifié ;
- $R_b$, et $R'_b$, identiques ou différents, représentent un atome d'hydrogène, un groupe aryl($C_1$-$C_4$)alkyle ; ou un groupe $C_1$-$C_6$ alkyle éventuellement substitué ;
- $R_c$, $R'_c$, $R_d$ et $R'_d$, identiques ou différents, représentent un atome d'hydrogène, un groupe aryl($C_1$-$C_4$)alkyle, un groupe $C_1$-$C_6$ alkyle éventuellement substitué ou un groupe ($C_{10}$-$C_{30}$)alkyle, linéaire ou ramifié, ou un groupe ($C_{10}$-$C_{30}$)alcényle, linéaire ou ramifié ; $R_c$, $R'_c$, $R_d$ et $R'_d$ représentent préférentiellement un atome d'hydrogène, un groupe $C_1$-$C_6$ alkyle éventuellement substitué ou un groupe ($C_{10}$-$C_{30}$)alkyle, linéaire ou ramifié ;
ou alors deux radicaux adjacents $R_c$ et $R_d$, $R'_c$ et $R'_d$ portés par le même atome d'azote forment ensemble un groupe hétérocyclique ou hétéroaryle ;
préférentiellement l'hétérocycle ou l'hétéroaryle est monocyclique et comprend de 5 à 7 chaînons ; plus préférentiellement les groupes sont choisis parmi l'imidazolyle et le pyrrolidinyle ;
- $R_g$, $R'_g$, $R''_g$, $R'''_g$, $R_h$, $R'_h$, $R''_h$, et $R'''_h$, identiques ou différents, représentent un atome d'hydrogène, un atome d'halogène, un groupe amino, $C_1$-$C_4$ alkylamino, $C_1$-$C_4$ dialkylamino, cyano, carboxy, hydroxy ou trifluorométhyle, un radical acylamino, alcoxy en $C_1$-$C_4$, (poly)hydroxyalcoxy en $C_2$-$C_4$, alkylcarbonyloxy, alcoxycarbonyle ou alkylcarbonylamino, un radical acylamino, carbamoyle ou alkylsulfonylamino, un radical amino-sulfonyle, ou un radical $C_1$-$C_{16}$ alkyle éventuellement substitué par un groupe choisi parmi $C_1$-$C_{12}$ alcoxy, hydroxy, cyano, carboxy, amino, $C_1$-$C_4$ alkylamino et $C_1$-$C_4$ dialkylamino, ou alors les deux radicaux alkyle portés par l'atome d'azote du groupe amino forment un hétérocycle comprenant de 5 à 7 chaînons et comprenant éventuellement un autre hétéroatome identique à ou différent de celui de l'atome d'azote ; préférentiellement $R_g$, $R'_g$, $R''_g$, $R'''_g$, $R_h$, $R'_h$, $R''_h$, et $R'''_h$ représentent un atome d'hydrogène, d'halogène ou un groupe $C_1$-$C_3$ alkyle ;
- ou alors deux groupes $R_g$ et $R'_g$ ; $R''_g$ et $R'''_g$ ; $R_h$, et $R'_h$ ; $R''_h$ et $R'''_h$ portés par deux atomes de carbone adjacents, forment ensemble un cycle benzo ou indéno, un groupe hétérocycloalkyle fusionné ou hétéroaryle fusionné ; le cycle benzo, indéno, hétérocycloalkyle ou hétéroaryle étant éventuellement substitué par un atome d'halogène, un groupe amino, $C_1$-$C_4$ alkylamino, $C_1$-$C_4$ dialkylamino, nitro, cyano, carboxy, hydroxy ou trifluorométhyle, un radical acylamino, alcoxy en $C_1$-$C_4$, (poly) hydroxyalcoxy en $C_2$-$C_4$, alkylcarbonyloxy, alcoxycarbonyle ou alkylcarbonylamino, un radical acylamino, carbamoyle ou alkylsulfonylamino, un radical amino-sulfonyle, ou un radical $C_1$-$C_{16}$ alkyle éventuellement substitué par : un groupe choisi parmi $C_1$-$C_{12}$ alcoxy, hydroxy, cyano, carboxy, amino, $C_1$-$C_4$ alkylamino, $C_1$-$C_4$ dialkylamino, ou alors les deux radicaux alkyles portés par l'atome d'azote du groupe amino forment un hétérocycle comprenant de 5 à 7 chaînons et comprenant éventuellement un autre hétéroatome identique à ou différent de celui de l'atome d'azote ; préférentiellement $R_g$ et $R'_g$ ; $R''_g$ et $R'''_g$ forment ensemble un groupe benzo ;
- ou alors lorsque G représente $-NR_cR_d$ et G' représente $-NR'_cR'_d$ deux groupes $R_c$ et $R'_g$ ; $R'_c$ et $R''_g$ ; $R_d$ et

$R_g$ ; $R'_d$ et $R'''_g$ forment ensemble un hétéroaryle ou hétérocycle saturé, éventuellement substitué par un ou plusieurs groupes $C_1$-$C_6$ alkyle, préférentiellement un hétérocycle contenant un ou deux hétéroatomes choisis parmi l'azote et l'oxygène et comprenant de 5 à 7 chaînons ; plus préférentiellement l'hétérocycle est choisi parmi les groupes morpholinyle, pypérazinyle, pypéridinyle et pyrrolidinyle ;

• $R_i$, $R'_i$, $R''_i$, et $R'''_i$, identiques ou différents, représentent un atome d'hydrogène, ou un groupe $C_1$-$C_4$ alkyle ;

• $R_1$, $R_2$, $R_3$, $R_4$, $R'_1$, $R'_2$, $R'_3$, et $R'_4$, identiques ou différents, représentent un atome d'hydrogène ou un groupe $C_1$-$C_4$ alkyle, $C_1$-$C_4$ alkoxy, hydroxy, cyano, carboxy, amino, $C_1$-$C_4$ alkylamino ou $C_1$-$C_4$ dialkylamino, lesdits radicaux alkyle pouvant former avec l'atome d'azote qui les porte un hétérocyle comprenant de 5 à 7 chaînons, comprenant éventuellement un autre hétéroatome différent ou non de l'azote ; préférentiellement $R_1$, $R_2$, $R_3$, $R_4$, $R'_1$, $R'_2$, $R'_3$, et $R'_4$ sont des atomes d'hydrogène ou un groupe amino ; plus préférentiellement $R_1$, $R_2$, $R_3$, $R_4$, $R'_1$, $R'_2$, $R'_3$, et $R'_4$ représentent un atome d'hydrogène ;

• $T_a$, $T_b$, identiques ou différents, représentent i) soit une liaison covalente $\sigma$, ii) soit un ou plusieurs radicaux ou leurs combinaisons choisis parmi $-SO_2-$, -O-, -S-, -N(R)-, $-N^+(R)(R^\circ)-$, -C(O)-, avec R, R°, identiques ou différents, représentant un atome d'hydrogène, un radical alkyle en $C_1$-$C_4$, hydroxyalkyle en $C_1$-$C_4$ ; ou un aryl($C_1$-$C_4$)alkyle ; préférentiellement $T_a$ est identique à $T_b$ et représente une liaison covalente $\sigma$ ou un groupe choisi parmi -N(R)-, -C(O)-N(R)-, -N(R)-C(O)-, -O-C(O)-, -C(O)-O- et $-N^+(R)(R^\circ)-$, $Q^-$, avec R, R° identiques ou différents représentant un atome d'hydrogène ou un groupe $C_1$-$C_4$ alkyle ; un groupe $C_1$-$C_6$ alkyle éventuellement substitué ou un groupe ($C_{10}$-$C_{30}$) alkyle, linéaire ou ramifié et $Q^-$représentant un contre-ion anionique ; plus préférentiellement $T_a$ et $T_b$ représentent une liaison $\sigma$, -C(O)-N(R)- ou -N(R)-C(O)- ; iii) soit un radical hétérocycloalkyle ou hétéroaryle, cationique ou non, préférentiellement monocyclique, préférentiellement identique, contenant préférentiellement deux hétéroatomes (plus préférentiellement deux atomes d'azote) et comportant préférentiellement de 5 à 7 chaînons, tel que l'imidazolium;

•

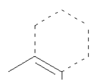

représente un groupe aryle ou hétéroaryle fusionné au cycle imidazolium ou phényle ; ou alors est absent du cycle imidazolium ou phényle ; particulièrement, lorsque le cycle est présent, le cycle est un benzo ; préférentiellement est absent du cycle imidazolium ou phényle ;

• m, m', n et n', identiques ou différents, représentent un entier compris inclusivement entre 0 et 6 avec m+n, m'+n', identiques ou différents, représentant un entier compris inclusivement entre 1 et 10 ; préférentiellement m+n=m'+n'=un entier compris inclusivement entre 2 et 4 ; plus préférentiellement m+n=m'+n'= un entier égal à 2;

• Y est tel que défini dans la revendication 1 ; particulièrement Y représente un atome d'hydrogène, ou un groupe protecteur tel que :

- ($C_1$-$C_4$)alkylcarbonyle comme méthylcarbonyle ou éthylcarbonyle ;
- arylcarbonyle comme phénylcarbonyle ;
- ($C_1$-$C_4$) alcoxycarbonyle ;
- aryloxycarbonyle ;
- aryl ($C_1$-$C_4$)alcoxycarbonyle ;
- (di) ($C_1$-$C_4$) (alkyl)aminocarbonyle comme diméthylaminocarbonyle ;
- ($C_1$-$C_4$) (alkyl)arylaminocarbonyle ;
- aryle éventuellement substitué tel que phényle ;
- hétéroaryle monocyclique à 5 ou 6 chaînons tels que imidazolyle ou pyridyle ;
- hétéroaryle monocyclique cationique à 5 ou 6 chaînons tels que pyrylium, pyridinium, pyrimidinium, pyrazinium, pyridazinium, triazinium, imidazolium ; ces groupes étant éventuellement substitués par un ou plusieurs, identiques ou différents, groupes ($C_1$-$C_4$)alkyle tels que méthyle ;
- hétéroaryle bicyclique cationique à 8 à 11 chaînons tel que benzo-imidazolium, ou benzoxazolium ; ces groupes étant éventuellement substitués par un ou plusieurs, identiques ou différents, groupes ($C_1$-$C_4$)alkyle

tels que méthyle ;

- hétérocycle cationique de formule suivante :

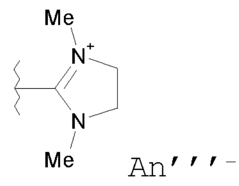

- $-C(NH_2)=N^+H_2$; An'''⁻ ; avec An'''⁻ un contre-ion anionique tel que défini précédemment ;
- $-C(NH_2)=NH$ ;
- $SO_3^-$, $M^+$ avec $M^+$ représentant un métal alcalin tel que le sodium ou le potassium ;

préférentiellement Y représente un atome d'hydrogène ; et

• M' représentant un contre-ion anionique, issu de sel d'acide organique ou minéral, ou d'une base organique ou minérale assurant l'électroneutralité de la molécule ;

• étant entendu qu'au moins un des groupes G, G', $R_a$, $R'_a$, $R_c$, $R'_c$, $R_d$ et $R'_d$, porte au moins un groupe $(C_{10}$-$C_{30})$ alkyle, linéaire ou ramifié, ou un groupe $(C_{10}$-$C_{30})$ alcényle, linéaire ou ramifié, de préférence au moins un groupe $(C_{10}$-$C_{30})$ alkyle, linéaire ou ramifié, plus préférentiellement linéaire ;

particulièrement les colorants de formule **(I)** sont choisis parmi les colorants à chromophore azoïques disulfures, thiols ou thiols protégés, de préférence choisis parmi les formules **(V)** et **(V')**, telles que définies précédemment, préférentiellement avec **$R_a$** et **$R'_a$**, identiques représentant un groupe $(C_{10}$-$C_{30})$ alkyle, linéaire ou ramifié, plus préférentiellement linéaire.

**9.** Procédé de coloration selon l'une quelconque des revendications précédentes dans lequel le ou les colorants de formule **(I)** sont des colorants choisis parmi ceux de formule **(XIII)** à **(XIII')** suivantes :

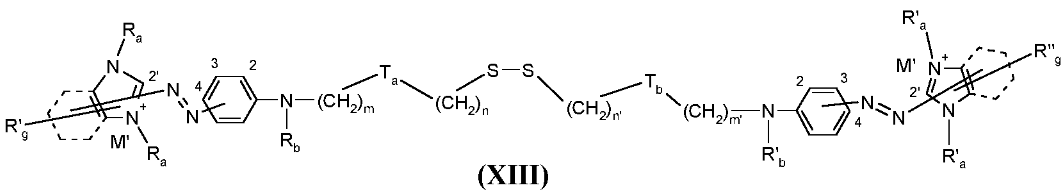

**(XIII)**

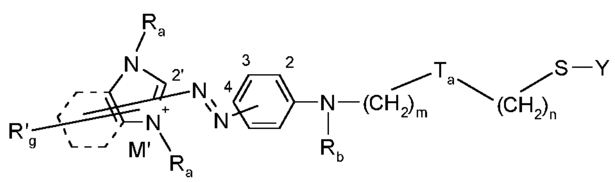

**(XIII')**

leurs sels d'acide organique ou minéral, isomères optiques, isomères géométriques, et les solvates tels que les hydrates ;

formules **(XIII)** à **(XIII')** dans lesquelles :

• **$R_a$** et **$R'_a$**, identiques ou différents, représentent un groupe $(C_{10}$-$C_{30})$alkyle, linéaire ou ramifié, ou un groupe $(C_{10}$-$C_{30})$alcényle, linéaire ou ramifié ; de préférence $R_a$ et $R'_a$ sont identiques et représentent un groupe $(C_{10}$-$C_{30})$ alkyle, linéaire ou ramifié, en particulier linéaire ;

• **$R_b$** et **$R'_b$**, identiques ou différents, représentent un atome d'hydrogène, ou un groupe $C_1$-$C_6$ alkyle ;

• **R'$_g$**, et **R"$_g$**, identiques ou différents, représentent un atome d'hydrogène, un groupe halogène, un groupe amino, $C_1$-$C_4$ alkylamino, $C_1$-$C_4$ dialkylamino, cyano, carboxy ou hydroxy, un radical acylamino, alcoxy en $C_1$-$C_4$, alkylcarbonyloxy, alcoxycarbonyle ou alkylcarbonylamino, un radical acylamino, carbamoyle ou alkylsulfonylamino, un radical amino-sulfonyle, ou un radical $C_1$-$C_6$ alkyle ; particulièrement R'$_g$, et R"$_g$, représentent un atome d'hydrogène, ou un groupe ($C_1$-$C_4$) alkyle, de préférence hydrogène ;

• **$T_a$**, et **$T_b$**, identiques ou différents, représentent

i) soit une liaison covalente σ,

ii) soit un ou plusieurs radicaux ou leurs combinaisons choisis parmi -O-, -N(R)-,-N$^+$(R) (R°)-, Q$^-$, -C(O)-, avec R, R°, identiques ou différents, représentant un atome d'hydrogène ou un radical alkyle en $C_1$-$C_4$, préférentiellement $T_a$ est identique à $T_b$ et ils représentent une liaison covalente σ ou un groupe choisi parmi-N(R)-, -C(O)-N(R)-, -N(R)-C(O)-, avec R représentant un atome d'hydrogène ou un groupe $C_1$-$C_4$ alkyle ; plus préférentiellement $T_a$ et $T_b$ représentent une liaison σ**;**

•

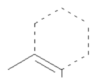

est tel que défini dans la revendication précédente ; préférentiellement est absent du cycle imidazolium ou phényle ;

• **m, m', n,** et **n',** identiques ou différents, représentent un entier compris inclusivement entre 0 et 6 avec m+n et m'+n', identiques ou différents, représentant un entier compris inclusivement entre 1 et 10; préférentiellement m+n=m'+n'=un entier compris inclusivement entre 2 et 4 ; plus préférentiellement m+n=m'+n'= un entier égal à 2 ;

• **Y** représente un atome d'hydrogène, ou un groupe protecteur choisi parmi :

- ($C_1$-$C_4$)alkylcarbonyle comme méthylcarbonyle ou éthylcarbonyle ;
- arylcarbonyle comme phénylcarbonyle ;
- ($C_1$-$C_4$) alcoxycarbonyle ;
- aryloxycarbonyle ;
- aryl ($C_1$-$C_4$) alcoxycarbonyle ;
- (di) ($C_1$-$C_4$) (alkyl)aminocarbonyle comme diméthylaminocarbonyle;
- ($C_1$-$C_4$) (alkyl)arylaminocarbonyle ;
- aryle éventuellement substitué tel que phényle ;
- hétéroaryle monocyclique à 5 ou 6 chaînons tel qu'imidazolyle ou pyridyle ;
- hétéroaryle monocyclique cationique à 5 ou 6 chaînons tel que pyrylium, pyridinium, pyrimidinium, pyrazinium, pyridazinium, triazinium, imidazolium ; ces groupes étant éventuellement substitués par un ou plusieurs, identiques ou différents, groupes ($C_1$-$C_4$)alkyle tel que méthyle ;
- hétéroaryle bicyclique cationique à 8 à 11 chaînons tels que benzo-imidazolium, ou benzoxazolium ; ces groupes étant éventuellement substitué par un ou plusieurs, identiques ou différents, groupes ($C_1$-$C_4$)alkyle tels que méthyle ;
- hétérocycle cationique de formule suivante :

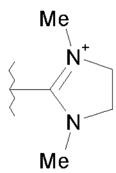

$An'''^-$

- $-C(NH_2)=N^+H_2$; $An'''^-$ ; avec $An'''^-$ un contre-ion anionique tels que défini précédemment ;
- $-C(NH_2)=NH$ ;
- $SO_3^-$, $M^+$ avec $M^+$ représentant un métal alcalin tel que le sodium ou le potassium ;

de préférence **Y** représente un atome d'hydrogène ; et
• **M'** représentant un contre-ion anionique, issu de sel d'acide organique ou minéral, ou d'une base organique ou minérale assurant l'électroneutralité de la molécule ; en particulier le groupe azoïque reliant la partie (benzo)imidazolium au phényle se trouve en position ortho ou para du pyridinium soit en position 2'-4, 4-2', 2'-4 et 4-2', de préférence en position para 2'-4 et 4-2' .

**10.** Procédé de coloration selon l'une quelconque des revendications précédentes dans lequel le ou les colorants de formule **(I)** sont des colorants choisis parmi ceux de formules suivantes :

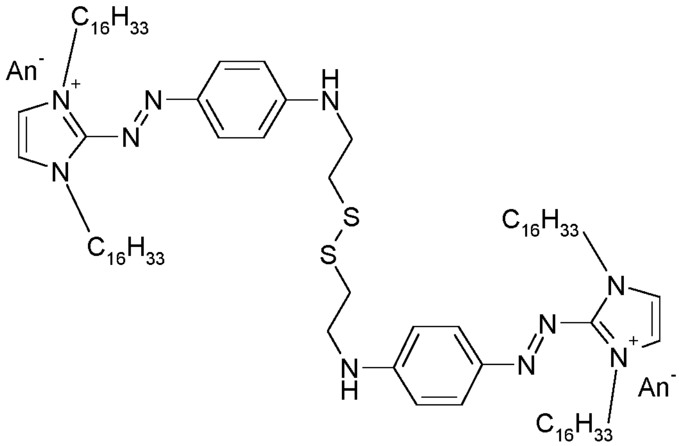

1

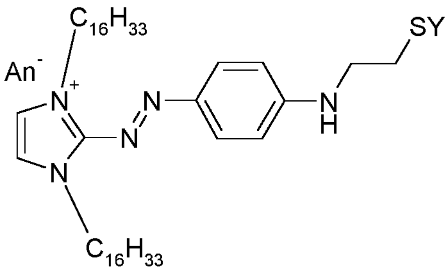

2

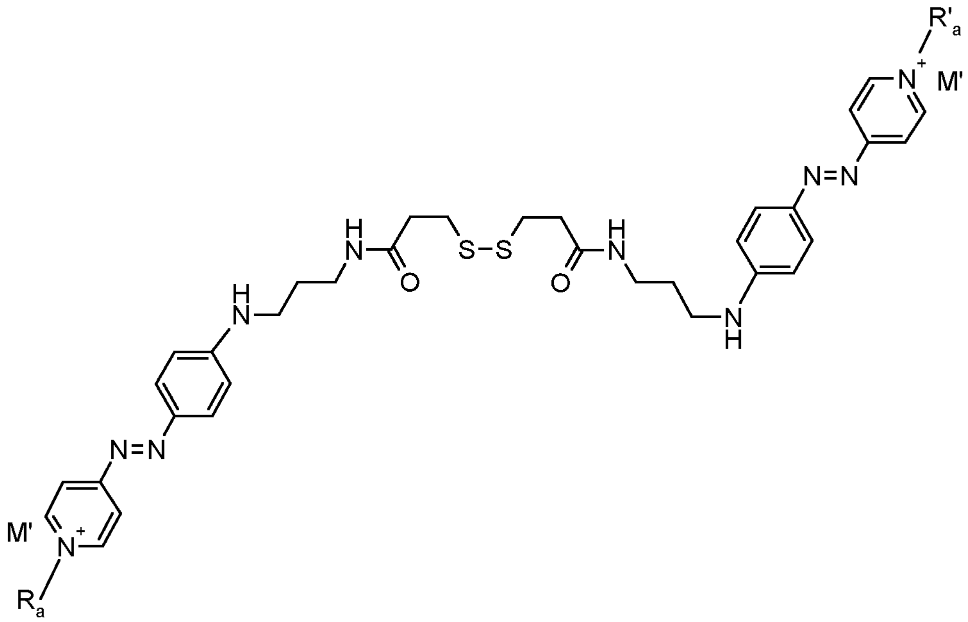

3

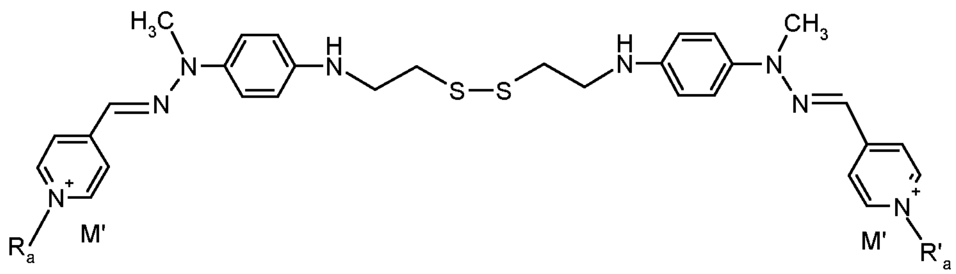

4

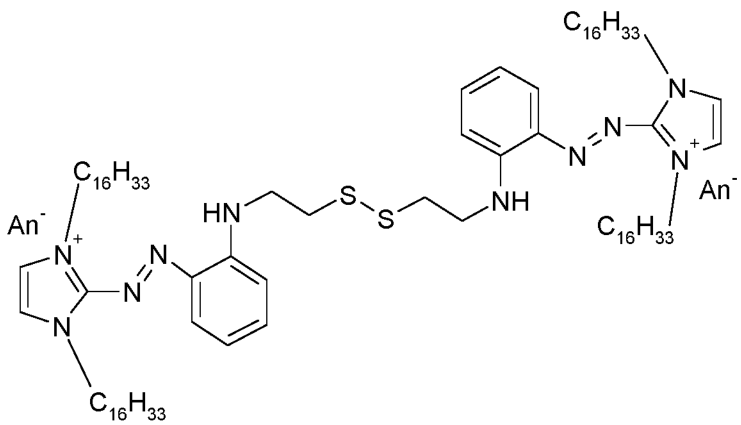

5

avec **An$^-$, M',** identiques ou différents, préférentiellement identiques, représentant des contre-ions anioniques, plus particulièrement le contre-ion anionique est choisi parmi les halogénures tels que le chlorure, alkylsulfate comme le méthylsulfate et le mésylate ; **Y** est tel que défini dans l'une des revendications 1, 8 et 9, de préférence représente

- ➢ ($C_1$-$C_4$)alkylcarbonyle comme méthylcarbonyle ou éthylcarbonyle ;
  - ➢ arylcarbonyle comme phénylcarbonyle ;➢ ($C_1$-$C_4$) alcoxycarbonyle ;
  - ➢ aryloxycarbonyle ;
- ➢ aryl ($C_1$-$C_4$) alcoxycarbonyle ;

➢ aryle éventuellement substitué tel que phényle ;

➢ hétéroaryle monocyclique à 5 ou 6 chaînons tel que imidazolyle ou pyridyle ;

➢ $-C(NH_2)=N^+H_2$; An'''- ; avec An'''- un contre-ion anionique tel que défini précédemment ; plus préférentiellement R est un atome d'hydrogène ; et
**R'$_a$** représente un groupe n-alkyle ($C_{14}$-$C_{20}$) tel qu'un groupe n-alkyle en $C_{16}$.

**11.** Procédé selon l'une quelconque des revendications précédentes qui ne met pas en œuvre d'agent réducteur.

**12.** Procédé selon l'une quelconque des revendications précédentes qui ne met pas en œuvre d'agent oxydant chimique.

**13.** Utilisation d'au moins un colorant direct à fonction disulfure, thiol ou thiol protégé de formule **(I)** tel que défini dans l'une quelconque des revendications 1 à 12 pour protéger lesdites fibres des traitements thermiques à une température supérieure ou égale à 80 °C, apportée notamment par de la vapeur d'eau et/ou un fer à lisser, en particulier provenant d'un fer vapeur.

**14.** Colorant direct à fonction disulfure, thiol ou thiol protégé de formule **(I)** tel que défini dans l'une quelconque des revendications 1 à 10.

**15.** Composition comprenant un ou plusieurs colorants directs à fonction disulfure, thiol ou thiol protégé de formule **(I)** tel que défini dans l'une quelconque des revendications 1 à 12.

**16.** Procédé de préparation des colorants de formule **(I)** tels que définis selon l'une quelconque des revendications 1 à 10 qui consiste :

- soit à partir de deux équivalents de réactifs comprenant un chromophore et au moins un groupe nucléofuge tel que halogéno **(a)** et d'un équivalent de composé disulfure aminé **(b)** :

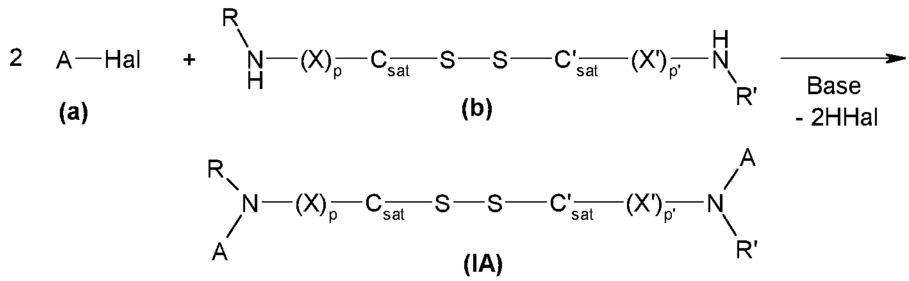

(a) (b) (IA)

avec **Hal** représentant un groupe nucléofuge tel que halogéno, (poly) halogéne ($C_1$-$C_4$) alcoxy, (poly) halogéno ($C_1$-$C_4$) sulfoxy, **X, X', p, p', C$_{sat}$** et **C'$_{sat}$,** étant tels que définis pour le composé **(I)** dans l'une quelconque des revendications 1 à 10, **R** et **R'** représentant un atome d'hydrogène ou un groupe ($C_1$-$C_4$)alkyle éventuellement substitué ; **A** représentant un chromophore tel que défini précédemment pour **(I)** dans l'une quelconque des revendications 1 à 10, **R** et **R'** ou alors **A** représente un chromophore comprenant un groupe nucléofuge tel que défini qui réagit avec un ou deux équivalents d'aminoalkylène suivi d'une réaction d'alkylation à partir par exemple d'halogénure de ($C_{10}$-$C_{30}$)alkyle, de préférence **A** représente un chromophore cationique hydrazono de formule **(II)** et **(III'),** ou azoïque **(IV), (IV'), (V)** et **(V')** tels que définis dans la revendication 7 ; cette réaction se faisant de préférence dans un solvant protique polaire, en particulier au reflux de solvant, de préférence le solvant est un alcool tel que l'éthanol ;
- soit la première étape consiste à préparer le réactif disulfure **(c)** sur lequel va venir se greffer un hétéroaryle substitué par au moins un groupe ($C_1$-$C_6$)alkyle tel qu'une pyridine substituée par un groupe hydrazino **(d)** qui à son tour réagit avec un réactif aryl(thio)aldéhyde ou aryl(thio)cétone comprenant au moins un groupe à chaîne aliphatique, linéaire ou ramifiée, saturée ou insaturée, en $C_{10}$-$C_{30}$ ; avec élimination de $H_2O$ ou $H_2S$ pour conduire à un colorant disulfure à chromophore hydrazono **(I'),** ainsi que ses isomères optiques et géométriques, appartenant à la formule (I) selon l'invention :

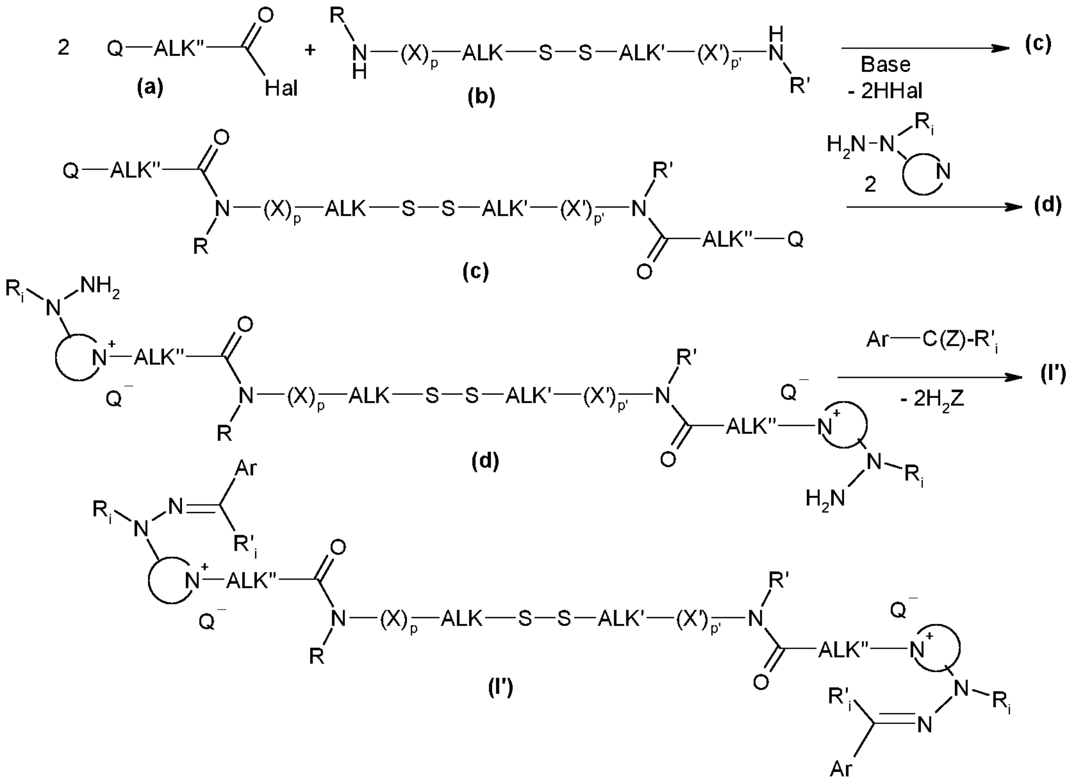


Composés **(a), (b), (c), (d)** et **(I')** avec :

- **Q** représentant un groupe électrofuge tel qu'halogéno, (poly)halogéno($C_1$-$C_6$)-$S(O)_2$-O-, alkyl sulfonyle ($C_1$-$C_6$)Alk-$S(O)_2O$- tels que le méthylsulfonyle et l'éthylsulfonyle ; les arylsulfonyles : Ar-$S(O)_2O^-$ tels que le benzènesulfonyle et le toluènesulfonyle ; (poly) (hydroxy) ($C_1$-$C_6$) alkylcarbonyloxy, les alkylsulfonyles : Alk-O-S(O)O- ; les arylsulfonyles : Ar-O-S(O)O- tels que le benzènesulfonyle et le toluènesulfonyle ; les alcoxysulfonyles : Alk-O-$S(O)_2O$- ; les aryloxysulfates : Ar-O-$S(O)_2O$-, le phosphonyle ; et les borates tels que le tétrahalogénoborate ;
- **$Q^-$** représentant un contre-ion anionique qui est dérivé de Q tel que défini dans la revendication 7 ;
- **ALK, ALK', ALK",** identiques ou différents, représentant un groupe ($C_1$-$C_6$)alkylène, linéaire ou ramifié, éventuellement substitué, tel qu'éthylène ou propylène ;
- **Hal, X, X', p** et **p'** étant tels que définis précédemment ;
- Z représentant un atome d'oxygène ou de soufre, de préférence oxygène ;
- **$R_i$** et **$R'_i$** étant tels que définis précédemment ;
- 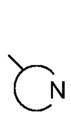

  représentant un groupe hétéroaryle comprenant au moins un atome d'azote substitué par au moins un groupe ($C_1$-$C_6$)alkyle, de préférence méthyle ;
- 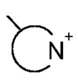

  représentant un groupe hétéroaryle cationique substitué par au moins un groupe ($C_1$-$C_6$) alkyle, de préférence méthyle, et comprenant au moins un ammonium;
- Ar représentant un groupe (hétéro)aryle éventuellement substitué, de préférence phényle, comprenant au moins un groupe à chaîne aliphatique, linéaire ou ramifiée, saturée ou insaturée, en $C_{10}$-$C_{30}$ ;

- soit lorsque le composé disulfure présente un lieur alkylène non interrompu entre le chromophore et le groupe disulfure par un hétéroatome ou un groupe-N(R)-, $-N^{+}(R)(R)-$, -O-, -S-, -C(O)-, $-SO_2-$ ou leur combinaison avec R tel que défini précédemment et peut être réalisé selon le schéma de synthèse suivant :

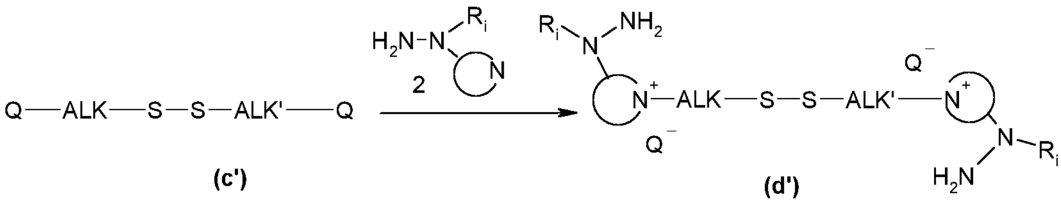

(c') (d')

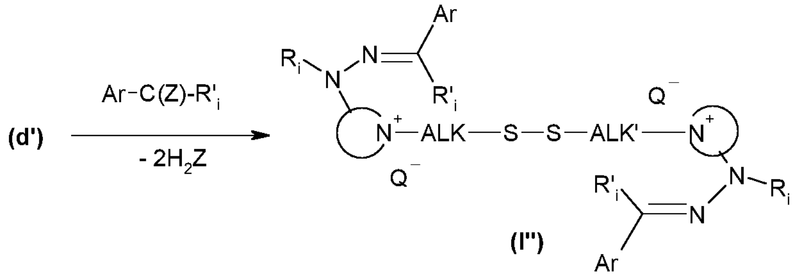


(d') (I'')

avec ALK, ALK', Q, $Q^-$, $R_1$, Z, $R_1$, $R'_1$, Ar tels que définis précédemment ; et

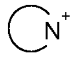

représente un groupe hétéroaryle cationique comprenant au moins un ammonium ;

- soit à partir du réactif chromophore **(f)** azoïque comprenant un groupe partant GP tel que halogénure, mésylate ou tosylate, une partie Ar (hétéro)aromatique et un groupe hétéroaryle comprenant au moins un atome d'azote, tel qu'un (benzo)imidazolyle qui est préparé lui-même à partir d'un composé (hétéro)aromatique (e) comprenant au moins un GP et un groupe amino en présence i) d'acide, en particulier dans un mélange d'acide organique et inorganique, de préférence en mélange avec un acide carboxylique et d'acide chlorhydrique, et de $MNO_2$ avec M représentant un métal alcalin ou alcalino-terreux de préférence dans l'eau et à une température comprise entre 0 °C et 5°C, puis ii) d'une base telle que la soude ou potasse, puis iii) d'un hétéroaryle comprenant au moins un atome d'azote tel que (benzo)imidazole, de préférence imidazole ; le composé **(f)** peut ensuite être alkylé en particulier par $R^a$ une chaîne aliphatique, linéaire ou ramifiée, saturée ou insaturée, en $C_{10}$-$C_{30}$ à l'aide d'un réactif $R_a$-Q avec Q représentant un groupe partant tel qu'halogénure pour conduire à l'intermédiaire **(g),** ce dernier peut ensuite réagir avec le réactif diamino sulfure (b) pour conduire au composé **(I'''')** azoïque selon l'invention :

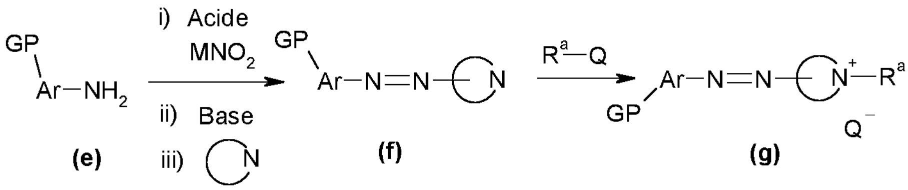


(e) (f) (g)

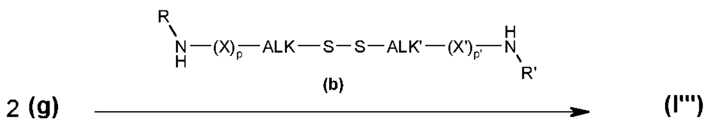

(b)

2 (g) (I''')

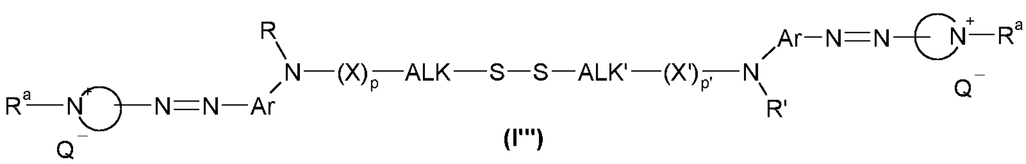

(I''')

avec ALK, ALK', X, X', p, p', Q, $Q^-$, Ar, GP et

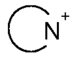

tels que définis précédemment ;

R et R', identiques ou différents, représentant un atome d'hydrogène ou un groupe ($C_1$-$C_6$) alkyle ;

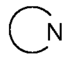

représente un groupe hétéroaryle comprenant au moins un atome d'azote.

**17.** Procédé de préparation selon la revendication précédente des colorants de formule **(Va)** et **(V')** qui consiste à faire réagir une aniline substituée par un groupe alcoxy ou benzyloxy (h) avec i) un acide de préférence acide minéral tel que l'acide chlorhydrique ou un acide carboxylique tel que l'acide acétique, en présence d'un composé nitré $MNO_2$ avec M représentant un contre-ion cationique tel que Na ou K, puis ii) avec une base organique ou minérale, de préférence minérale telle que la soude, puis iii) un dérivé imidazolyle, fusionné ou pas par un groupe (hétéro)aryle de préférence benzo, pour conduire au composé azoïque (i) ; ce dernier réagissant avec au moins 2 équivalents molaires d'un agent alkylant tel que $R^a$-Q avec $R_a$ et Q tels que définis dans la revendication précédente, pour conduire au composé (j) ;

- soit deux équivalents de (j) réagissant avec un composé disulfure aminé (d), pour conduire au colorant disulfure **(Va)** ;
- soit un équivalent molaire de (j) réagissant avec un composé thiol ou thiol protégé (d') pour conduire au colorant thiol ou thiol protégé **(V')** tel que défini dans la revendication 8 ;

selon le schéma suivant :

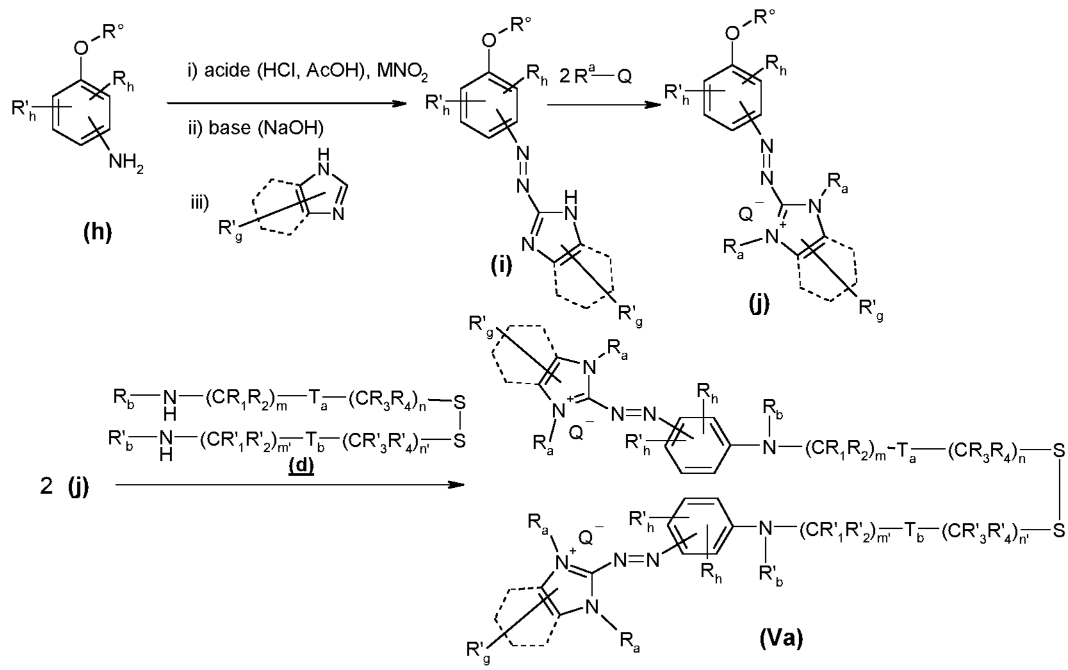


(h) (i) (j)

2 (j)

(Va)

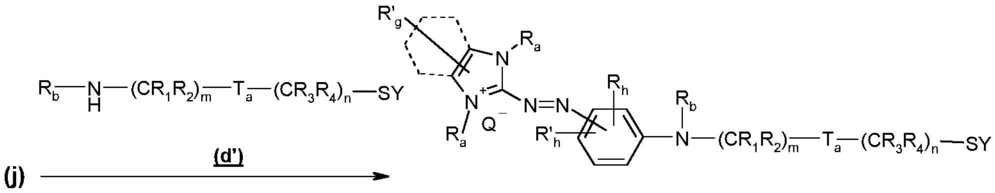


(j)

(V')

avec $R_a$, $R_b$, $R'_b$, $R'_g$, $R_h$, $R'_h$, $R_1$, $R_2$, $R_3$, $R_4$, $R'_1$, $R'_2$, $R'_3$, $R'_4$, Y, m, m', n, n', $T_a$, $T_b$, et

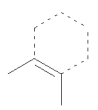

étant tels que définis dans la revendication 8 ou 9, Q étant tel que défini dans la revendication précédente, et R° représentant un groupe ($C_1$-$C_6$)alkyle, (hétéro)aryle ou benzyle.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 1647580 A
- WO 2005097051 A
- EP 2004759 A
- EP 2075289 A
- WO 2007110541 A
- WO 2007110540 A
- WO 2007110539 A
- WO 2007110538 A
- WO 2007110537 A
- WO 2007110536 A
- WO 2007110535 A
- WO 2007110534 A
- WO 2007110533 A
- WO 2007110532 A
- WO 2007110531 A
- EP 2070988 A
- WO 2009040354 A
- WO 2009034059 A
- WO 9515144 A
- WO 9501772 A
- EP 714954 A
- US 5888252 A
- EP 1133975 A
- WO 03029359 A
- EP 860636 A
- EP 850636 A
- FR 2788433
- EP 920856 A
- WO 9948465 A
- FR 2757385
- EP 850637 A
- EP 918053 A
- WO 9744004 A
- FR 2570946
- FR 2285851
- DE 2538363
- FR 2189006
- FR 1560664
- FR 1540423
- FR 1567219
- FR 1516943
- FR 1221122
- DE 4220388
- DE 4137005
- WO 0166646 A
- US 5708151 A
- WO 515144 A
- GB 1195386 A
- US 3524842 A
- US 5879413 A
- EP 1062940 A
- EP 1133976 A
- GB 738585 A
- DE 2527638
- FR 2275462
- GB 197427645 A
- FR 290780
- WO 04039771 A
- EP 1386916 A
- US 4579949 A
- US 4103145 A
- US 4308878 A
- US 5983903 A
- US 5957140 A
- US 5494058 A
- US 5046516 A

### Non-patent literature cited in the description


- **T.W. GREENE.** Protective Groups in Organic Synthesis. John Wiley & Sons, 1981, 193-217
- **P. KOCIENSKI.** Protecting Groups. Thieme, 2005
- Peptide Synthesis. Ullmann's Encyclopedia. Wiley-VCH Verlag GmbH & Co. KGaA, 2005, 4-5
- **T. W. GREENE.** Protective Groups in Organic Synthesis. John Wiley & Sons, 1981, 193-217
- **DYES ; AZO.** Kirk Othmer Encyclopedia of Chemical Technology. J. Wiley & Sons, 19 April 2010
- **K. VENKATARAMAN.** The Chemistry of Synthetic Dyes. Academic Press, 1952, vol. 1 to 7
- Dyes and Dye Intermediates. Kirk-Othmer Encyclopedia of Chemical Technology. Wiley and Sons, 1993
- Ullmann's Encyclopedia of Industrial Chemistry. Wiley and Sons
- *Acta Histochem,* 1978, vol. 61 (1), 48-52
- *Tsitologiya,* 1968, vol. 10 (3), 403-5
- *Zh. Obshch. Khim.,* 1970, vol. 40 (1), 195-202
- *Ann. Chim. (Rome),* 1975, vol. 65 (5-6), 305-14

- *Journal of the Chinese Chemical Society (Taipei),* 1998, vol. 45 (1), 209-211
- *Rev. Roum. Chim.,* 1988, vol. 33 (4), 377-83
- *Text. Res. J.,* 1984, vol. 54 (2), 105-7
- *Chim. Ind. (Milan),* 1974, vol. 56 (9), 600-3
- *Khim. Tekhnol.,* 1979, vol. 22 (5), 548-53
- *Ger. Monatsh. Chem.,* 1975, vol. 106 (3), 643-8
- *MRL Bull. Res. Dev.,* 1992, vol. 6 (2), 21-7
- *Dyes Pigm.,* 1992, vol. 19 (1), 69-79
- *Dyes Pigm.,* 1989, vol. 11 (3), 163-72
- Reactions, Mechanisms and Structures. **J. MARCH.** the books Advanced Organic Chemistry. John Wiley & Sons, 1992
- Protective Groups in Organic Synthesis. **T. W. GREENE.** Color Chemistry. Wiley VCH
- **H ZOLLINGER.** Color Chemistry. Wiley VCH, 166-169
- *J. Org. Chem.,* 1985, vol. 50 (26), 5716-5719
- *J. Am. Chem. Soc.,* 1970, vol. 92 (24), 7224-7225
- *Tetrahedron,* 2004, vol. 60 (51), 11911-11922
- *J. Org. (Rome),* 1990, vol. 55 (59), 2580-2586
- *J. Chinese Chem. Chem.,* 1985, vol. 50 (26), 5716-5719
- *J. Chem. Soc., Chem. Comm.,* 1981, vol. 15, 741-742
- *Tet. Lett.,* 2005, vol. 46 (36), 6097-6099
- *Bioorganic § Medicinal Chemistry Letters,* 2004, vol. 14 (21), 5347-5350
- *Chem. Berichte,* 1983, vol. 116 (1), 323-347
- *Tetrahedron,* 1985, vol. 41 (15), 3063-3069
- *J. Am. Chem. Soc.,* 1987, vol. 109 (25), 7648-7653
- *J. Org. Chem.,* 1995, vol. 60 (8), 2638-2639
- *Sulfur Lett.,* 2000, vol. 24 (3), 137-145
- *Synth. Comm.,* 2005, vol. 35 (23), 3021-3026
- *Synthesis,* 1986, vol. 5, 382-383
- *Liebigs Annalen der Chemie,* 1987, vol. 1, 77-79
- *Bull. Chem. Soc. Jap.,* 1977, vol. 50 (6), 1510-1512
- *J. Org. Chem.,* 1979, vol. 44 (4), 638-639
- *J. Hetero. Chem,* 1984, vol. 21 (2), 501-3
- Thiols and organic Sulfides'', ''Thiocyanates and Isothiocyanates, organic''. Ullmann's Encyclopedia. Wiley-VCH, 2005
- Reactions, Mechanisms and Structures. **J. MARCH.** book Advanced Organic Chemistry. John Wiley & Sons, 1992
- **T. W. GREENE.** *Protective Groups in Organic Synthesis*
- **J. MARCH.** Advanced Organic Chemistry, Reactions, Mechanisms and Structures. John Wiley & Sons, 1992
- **T. W. GREENE.** Protective Groups in Organic Synthesis. John Wiley & Sons, 1981